(19) Europäisches Patentamt
European Patent Office
Office européen des brevets

(11) EP 2 706 070 A1

(12) EUROPEAN PATENT APPLICATION

(43) Date of publication:
**12.03.2014 Bulletin 2014/11**

(51) Int Cl.:
***C07K 16/40*** (2006.01) ***A61K 39/395*** (2006.01)

(21) Application number: **12306068.3**

(22) Date of filing: **06.09.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventor: **The designation of the inventor has not yet been filed**

(74) Representative: **Schneider, Rudolf et al**
**Sanofi-Aventis Deutschland GmbH**
**Global Intellectual Property Department**
**Industriepark Höchst**
**Gebäude K 703**
**65926 Frankfurt am Main (DE)**

(54) **Combination treatments involving antibodies to human PCSK9**

(57) The present invention relates to combination methods for treating diseases or conditions in which proprotein convertase subtilisin/kexin type 9 (PCSK9) expression or activity causes an impact by administration of PCSK9-specific antibodies or antigen-binding fragments thereof. The present invention further relates to PCSK9-specific antibodies or antigen-binding fragments thereof for use in the combination treatment of diseases or conditions in which PCSK9 expression or activity causes an impact.

The present invention also relates to articles of manufacture comprising packaging material, PCSK9-specific antibodies or antigen-binding fragments thereof, a fibrate and/or a lipid lowering drug acting by inhibiting cholesterol uptake, together with a label or packaging insert.

EP 2 706 070 A1

SEQUENCE LISTING

<110> SANOFI

<120> Combination treatments involving antibodies to human PCSK9

<130> DE2012-135

<160> 758

<170> FastSEQ for Windows Version 4.0

<210> 1
<211> 351
<212> DNA
<213> Artificial Sequence

<220>
<223> Synthetic

<400> 1
caggtccagc tggtgcagtc tgggggaggc ttggtacagc ctggggggtc cctgagactc 60
tcctgtgcag cctctggatt tactctaagt agttacgaca tgcactgggt ccgccaatct 120
acaggaaaag gtctggagtg ggtctcagct attggttcta ccggtgacac atactatcca 180
ggctccgtga agggccgatt caccatcacc agagaaaaag ccaagaactc cgtgtatctt 240
caaatgaaca gcctgagagc cggggacacg gctgtgtatt actgtgtaag agaggggtgg 300
gaggtaccct ttgactactg gggccaggga accctggtca ctgtctcctc a 351

<210> 2
<211> 117
<212> PRT
<213> Artificial Sequence

<220>
<223> Synthetic

<400> 2
Gln Val Gln Leu Val Gln Ser Gly Gly Gly Leu Val Gln Pro Gly Gly
1 5 10 15
Ser Leu Arg Leu Ser Cys Ala Ala Ser Gly Phe Thr Leu Ser Ser Tyr
20 25 30
Asp Met His Trp Val Arg Gln Ser Thr Gly Lys Gly Leu Glu Trp Val
35 40 45
Ser Ala Ile Gly Ser Thr Gly Asp Thr Tyr Tyr Pro Gly Ser Val Lys
50 55 60
Gly Arg Phe Thr Ile Thr Arg Glu Lys Ala Lys Asn Ser Val Tyr Leu
65 70 75 80
Gln Met Asn Ser Leu Arg Ala Gly Asp Thr Ala Val Tyr Tyr Cys Val
85 90 95
Arg Glu Gly Trp Glu Val Pro Phe Asp Tyr Trp Gly Gln Gly Thr Leu
100 105 110
Val Thr Val Ser Ser
115

<210> 3
<211> 24
<212> DNA
<213> Artificial Sequence

<220>
<223> Synthetic

<400> 3
ggatttactc taagtagtta cgac 24

<210> 4
<211> 8
<212> PRT
<213> Artificial Sequence

<220>
<223> Synthetic

<400> 4
Gly Phe Thr Leu Ser Ser Tyr Asp
1 5

<210> 5
<211> 21
<212> DNA
<213> Artificial Sequence

<220>
<223> Synthetic

<400> 5
attggttcta ccggtgacac a 21

<210> 6
<211> 7
<212> PRT
<213> Artificial Sequence

<220>
<223> Synthetic

<400> 6
Ile Gly Ser Thr Gly Asp Thr
1 5

<210> 7
<211> 33
<212> DNA
<213> Artificial Sequence

<220>
<223> Synthetic

<400> 7
gtaagagagg ggtgggaggt accctttgac tac 33

<210> 8
<211> 11
<212> PRT
<213> Artificial Sequence

<220>
<223> Synthetic

<400> 8
Val Arg Glu Gly Trp Glu Val Pro Phe Asp Tyr
1 5 10

<210> 9
<211> 327
<212> DNA
<213> Artificial Sequence

<220>
<223> Synthetic

<400> 9
gacatccaga tgacccagtc tccagccacc ctgtctgtgt ctccagggga aagagccgcc 60
ctctcctgca gggccagtca gagtgttagc agcaacttag cctggtacca ccagaaacct 120
ggccaggctc ccaggctcct catctatggt gcatccacca gggccactgg tatcccagcc 180
aggttcagtg gcattgggtc tgggacagag ttcactctca ttatcagcag cctgcagtct 240
gaagattttg catttattt ctgtcagcag tataataact ggcctccatt cactttcggc 300
cctgggacca aggtggagat caaacga 327

<210> 10
<211> 109
<212> PRT
<213> Artificial Sequence

<220>
<223> Synthetic

<400> 10
Asp Ile Gln Met Thr Gln Ser Pro Ala Thr Leu Ser Val Ser Pro Gly
1 5 10 15
Glu Arg Ala Ala Leu Ser Cys Arg Ala Ser Gln Ser Val Ser Ser Asn
20 25 30
Leu Ala Trp Tyr His Gln Lys Pro Gly Gln Ala Pro Arg Leu Leu Ile
35 40 45
Tyr Gly Ala Ser Thr Arg Ala Thr Gly Ile Pro Ala Arg Phe Ser Gly
50 55 60
Ile Gly Ser Gly Thr Glu Phe Thr Leu Ile Ile Ser Ser Leu Gln Ser
65 70 75 80
Glu Asp Phe Ala Phe Tyr Phe Cys Gln Gln Tyr Asn Asn Trp Pro Pro
85 90 95
Phe Thr Phe Gly Pro Gly Thr Lys Val Glu Ile Lys Arg
100 105

<210> 11
<211> 18
<212> DNA
<213> Artificial Sequence

<220>
<223> Synthetic

<400> 11
cagagtgtta gcagcaac 18

<210> 12
<211> 6
<212> PRT
<213> Artificial Sequence

<220>
<223> Synthetic

<400> 12
Gln Ser Val Ser Ser Asn
1 5

<210> 13
<211> 9
<212> DNA
<213> Artificial Sequence

<220>
<223> Synthetic

<400> 13
ggtgcatcc 9

<210> 14
<211> 3
<212> PRT
<213> Artificial Sequence

<220>
<223> Synthetic

<400> 14
Gly Ala Ser
1

<210> 15
<211> 30
<212> DNA
<213> Artificial Sequence

<220>
<223> Synthetic

<400> 15
cagcagtata ataactggcc tccattcact 30

<210> 16
<211> 10
<212> PRT
<213> Artificial Sequence

<220>
<223> Synthetic

<400> 16
Gln Gln Tyr Asn Asn Trp Pro Pro Phe Thr
1 5 10

<210> 17
<211> 351
<212> DNA
<213> Artificial Sequence

<220>
<223> Synthetic

<400> 17
gaggtgcagc tggtggagtc tgggggaggc ttggtacagc ctggggggtc cctgagactc 60
tcctgtgcag cctctggatt tactctaagt agttacgaca tgcactgggt ccgccaatct 120
acaggaaaag gtctggagtg ggtctcagct attggttcta ccggtgacac atactatcca 180
ggctccgtga agggccgatt caccatcacc agagaaaaag ccaagaactc cgtgtatctt 240
caaatgaaca gcctgagagc cggggacacg gctgtgtatt actgtgtaag agaggggtgg 300
gaggtaccct ttgactactg gggccaggga accctggtca ccgtctcctc a 351

<210> 18

<211> 117
<212> PRT
<213> Artificial Sequence

<220>
<223> Synthetic

<400> 18
```
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Leu Ser Ser Tyr
            20                  25                  30
Asp Met His Trp Val Arg Gln Ser Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Thr Arg Glu Lys Ala Lys Asn Ser Val Tyr Leu
65                  70                  75                  80
Gln Met Asn Ser Leu Arg Ala Gly Asp Thr Ala Val Tyr Tyr Cys Val
                85                  90                  95
Arg Glu Gly Trp Glu Val Pro Phe Asp Tyr Trp Gly Gln Gly Thr Leu
            100                 105                 110
Val Thr Val Ser Ser
        115
```

<210> 19
<211> 324
<212> DNA
<213> Artificial Sequence

<220>
<223> Synthetic

<400> 19
```
gaaatagtga tgacgcagtc tccagccacc ctgtctgtgt ctccagggga aagagccgcc 60
ctctcctgca gggccagtca gagtgttagc agcaacttag cctggtacca ccagaaacct 120
ggccaggctc ccaggctcct catctatggt gcatccacca gggccactgg tatcccagcc 180
aggttcagtg gcattgggtc tgggacagag ttcactctca ttatcagcag cctgcagtct 240
gaagattttg cattttattt ctgtcagcag tataataact ggcctccatt cactttcggc 300
cctgggacca aagtggatat caaa                                         324
```

<210> 20
<211> 108
<212> PRT
<213> Artificial Sequence

<220>
<223> Synthetic

<400> 20
```
Glu Ile Val Met Thr Gln Ser Pro Ala Thr Leu Ser Val Ser Pro Gly
 1               5                  10                  15
Glu Arg Ala Ala Leu Ser Cys Arg Ala Ser Gln Ser Val Ser Ser Asn
            20                  25                  30
Leu Ala Trp Tyr His Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ile Gly Ser Gly Thr Glu Phe Thr Leu Ile Ile Ser Ser Leu Gln Ser
65                  70                  75                  80
Glu Asp Phe Ala Phe Tyr Phe Cys Gln Gln Tyr Asn Asn Trp Pro Pro
                85                  90                  95
Phe Thr Phe Gly Pro Gly Thr Lys Val Asp Ile Lys
            100                 105
```

```
<210> 21
<211> 351
<212> DNA
<213> Artificial Sequence

<220>
<223> Synthetic

<400> 21
gaggtgcagc tggtggagtc tgggggaggc ttggtacagc ctggggggtc cctgagactc 60
tcctgtgcag cctctggatt tactctaagt agttacgaca tgcactgggt ccgccaagct 120
acaggaaaag gtctggagtg ggtctcagct attggttcta ccggtgacac atactatcca 180
ggctccgtga agggccgatt caccatctcc agagaaaatg ccaagaactc cttgtatctt 240
caaatgaaca gcctgagagc cggggacacg gctgtgtatt actgtgtaag agaggggtgg 300
gaggtaccct ttgactactg gggccaggga accctggtca ccgtctcctc a          351

<210> 22
<211> 117
<212> PRT
<213> Artificial Sequence

<220>
<223> Synthetic

<400> 22
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Val
                85                  90                  95
Arg Glu Gly Trp Glu Val Pro Phe Asp Tyr Trp Gly Gln Gly Thr Leu
            100                 105                 110
Val Thr Val Ser Ser
        115


<210> 23
<211> 324
<212> DNA
<213> Artificial Sequence

<220>
<223> Synthetic

<400> 23
gaaatagtga tgacgcagtc tccagccacc ctgtctgtgt ctccagggga aagagccacc 60
ctctcctgca gggccagtca gagtgttagc agcaacttag cctggtacca gcagaaacct 120
ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataact ggcctccatt cactttcggc 300
cctgggacca aagtggatat caaa                                        324

<210> 24
<211> 108
<212> PRT
<213> Artificial Sequence
```

<220>
<223> Synthetic

<400> 24
```
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys
            100                 105
```

<210> 25
<211> 342
<212> DNA
<213> Artificial Sequence

<220>
<223> Synthetic

<400> 25
```
caggtgcagc tggtgcagtc tggggggaggc gtggtccagc ctgggaggtc cctgagactc 60
tcctgtgcag cgtctggatt caccttcagt agctatggca tgcactgggt ccgccaggct 120
ccaggcaagg ggctggagtg ggtggcgttt ataggatttg atggaagtaa tatacattat 180
ggagactccg tgagggggccg aatcatcata tccagagaca attccgagaa cacgttgtat 240
ctggaaatga acagcctgag agccgaggac acggcaatgt actattgtgc gagagagaag 300
ggtttagact ggggccaggg aaccacggtc accgtctcct ca                    342
```

<210> 26
<211> 114
<212> PRT
<213> Artificial Sequence

<220>
<223> Synthetic

<400> 26
```
Gln Val Gln Leu Val Gln Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Phe Ile Gly Phe Asp Gly Ser Asn Ile His Tyr Gly Asp Ser Val
    50                  55                  60
Arg Gly Arg Ile Ile Ile Ser Arg Asp Asn Ser Glu Asn Thr Leu Tyr
65                  70                  75                  80
Leu Glu Met Asn Ser Leu Arg Ala Glu Asp Thr Ala Met Tyr Tyr Cys
                85                  90                  95
Ala Arg Glu Lys Gly Leu Asp Trp Gly Gln Gly Thr Thr Val Thr Val
            100                 105                 110
Ser Ser
```

<210> 27

<211> 24
<212> DNA
<213> Artificial Sequence

<220>
<223> Synthetic

<400> 27
ggattcacct tcagtagcta tggc 24

<210> 28
<211> 8
<212> PRT
<213> Artificial Sequence

<220>
<223> Synthetic

<400> 28
Gly Phe Thr Phe Ser Ser Tyr Gly
1 5

<210> 29
<211> 24
<212> DNA
<213> Artificial Sequence

<220>
<223> Synthetic

<400> 29
ataggatttg atggaagtaa tata 24

<210> 30
<211> 8
<212> PRT
<213> Artificial Sequence

<220>
<223> Synthetic

<400> 30
Ile Gly Phe Asp Gly Ser Asn Ile
1 5

<210> 31
<211> 21
<212> DNA
<213> Artificial Sequence

<220>
<223> Synthetic

<400> 31
gcgagagaga agggtttaga c 21

<210> 32
<211> 7
<212> PRT
<213> Artificial Sequence

<220>
<223> Synthetic

<400> 32
Ala Arg Glu Lys Gly Leu Asp
1 5

<210> 33
<211> 321
<212> DNA
<213> Artificial Sequence

<220>
<223> Synthetic

<400> 33
gccatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attacacttt tggccagggg 300
accaaggtgg aaatcaaacg a 321

<210> 34
<211> 107
<212> PRT
<213> Artificial Sequence

<220>
<223> Synthetic

<400> 34
Ala Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Lys Pro Gly Lys Ala Pro Lys Leu Leu Ile
35 40 45
Tyr Lys Ala Ser Ser Leu Glu Ser Gly Val Pro Ser Arg Phe Ser Gly
50 55 60
Ser Gly Ser Gly Thr Glu Phe Thr Leu Thr Ile Ser Ser Leu Gln Pro
65 70 75 80
Asp Asp Phe Ala Thr Tyr Tyr Cys Gln Gln Tyr Asn Ser Tyr Tyr Thr
85 90 95
Phe Gly Gln Gly Thr Lys Val Glu Ile Lys Arg
100 105

<210> 35
<211> 18
<212> DNA
<213> Artificial Sequence

<220>
<223> Synthetic

<400> 35
cagagtatta gtagctgg 18

<210> 36
<211> 6
<212> PRT
<213> Artificial Sequence

<220>
<223> Synthetic

<400> 36
Gln Ser Ile Ser Ser Trp
1               5

<210> 37
<211> 9
<212> DNA
<213> Artificial Sequence

<220>
<223> Synthetic

<400> 37
aaggcgtct                                                                9

<210> 38
<211> 3
<212> PRT
<213> Artificial Sequence

<220>
<223> Synthetic

<400> 38
Lys Ala Ser
1

<210> 39
<211> 24
<212> DNA
<213> Artificial Sequence

<220>
<223> Synthetic

<400> 39
caacagtata atagttatta cact                                               24

<210> 40
<211> 8
<212> PRT
<213> Artificial Sequence

<220>
<223> Synthetic

<400> 40
Gln Gln Tyr Asn Ser Tyr Tyr Thr
1               5

<210> 41
<211> 342
<212> DNA
<213> Artificial Sequence

<220>
<223> Synthetic

<400> 41
caggtgcagc tggtggagtc tgggggaggc gtggtccagc ctgggaggtc cctgagactc 60
tcctgtgcag cgtctggatt caccttcagt agctatggca tgcactgggt ccgccaggct 120

```
ccaggcaagg ggctggagtg ggtggcgttt ataggatttg atggaagtaa tatacattat 180
ggagactccg tgaggggccg aatcatcata tccagagaca attccgagaa cacgttgtat 240
ctggaaatga acagcctgag agccgaggac acggcaatgt actattgtgc gagagagaag 300
ggtttagact ggggccaggg aaccctggtc accgtctcct ca                    342

<210> 42
<211> 114
<212> PRT
<213> Artificial Sequence

<220>
<223> Synthetic

<400> 42
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Phe Ile Gly Phe Asp Gly Ser Asn Ile His Tyr Gly Asp Ser Val
    50                  55                  60
Arg Gly Arg Ile Ile Ile Ser Arg Asp Asn Ser Glu Asn Thr Leu Tyr
65                  70                  75                  80
Leu Glu Met Asn Ser Leu Arg Ala Glu Asp Thr Ala Met Tyr Tyr Cys
                85                  90                  95
Ala Arg Glu Lys Gly Leu Asp Trp Gly Gln Gly Thr Leu Val Thr Val
            100                 105                 110
Ser Ser



<210> 43
<211> 318
<212> DNA
<213> Artificial Sequence

<220>
<223> Synthetic

<400> 43
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attacacttt tggccagggg 300
accaagctgg agatcaaa                                               318

<210> 44
<211> 106
<212> PRT
<213> Artificial Sequence

<220>
<223> Synthetic

<400> 44
Asp Ile Gln Met Thr Gln Ser Pro Ser Thr Leu Ser Ala Ser Val Gly
 1               5                  10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
```

Ser Gly Ser Gly Thr Glu Phe Thr Leu Thr Ile Ser Ser Leu Gln Pro
65 70 75 80
Asp Asp Phe Ala Thr Tyr Tyr Cys Gln Gln Tyr Asn Ser Tyr Tyr Thr
85 90 95
Phe Gly Gln Gly Thr Lys Leu Glu Ile Lys
100 105

<210> 45
<211> 342
<212> DNA
<213> Artificial Sequence

<220>
<223> Synthetic

<400> 45
caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt ataggatttg atggaagtaa tatatactat 180
gcagactccg tgaagggccg attcaccatc tccagagaca attccaagaa cacgctgtat 240
ctgcaaatga acagcctgag agctgaggac acggctgtgt attactgtgc gagagagaag 300
ggtttagact ggggccaggg aaccctggtc accgtctcct ca 342

<210> 46
<211> 114
<212> PRT
<213> Artificial Sequence

<220>
<223> Synthetic

<400> 46
Gln Val Gln Leu Val Glu Ser Gly Gly Gly Val Val Gln Pro Gly Arg
1 5 10 15
Ser Leu Arg Leu Ser Cys Ala Ala Ser Gly Phe Thr Phe Ser Ser Tyr
20 25 30
Gly Met His Trp Val Arg Gln Ala Pro Gly Lys Gly Leu Glu Trp Val
35 40 45
Ala Val Ile Gly Phe Asp Gly Ser Asn Ile Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Arg Glu Lys Gly Leu Asp Trp Gly Gln Gly Thr Leu Val Thr Val
100 105 110
Ser Ser

<210> 47
<211> 319
<212> DNA
<213> Artificial Sequence

<220>
<223> Synthetic

<400> 47
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attacacttt tggccagggg 300

accaagctgg agatcaaac 319

<210> 48
<211> 106
<212> PRT
<213> Artificial Sequence

<220>
<223> Synthetic

<400> 48
Asp Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Lys Pro Gly Lys Ala Pro Lys Leu Leu Ile
35 40 45
Tyr Lys Ala Ser Ser Leu Glu Ser Gly Val Pro Ser Arg Phe Ser Gly
50 55 60
Ser Gly Ser Gly Thr Glu Phe Thr Leu Thr Ile Ser Ser Leu Gln Pro
65 70 75 80
Asp Asp Phe Ala Thr Tyr Tyr Cys Gln Gln Tyr Asn Ser Tyr Tyr Thr
85 90 95
Phe Gly Gln Gly Thr Lys Leu Glu Ile Lys
100 105

<210> 49
<211> 342
<212> DNA
<213> Artificial Sequence

<220>
<223> Synthetic

<400> 49
caggtgcagc tgcaggagtc tgggggaggc gtggtccagc ctgggaggtc cctgagactc 60
tcctgtgcag cgtctggatt caccttcagt agctatggca tgcactgggt ccgccaggct 120
ccaggcaagg ggctggagtg ggtggcgttt ataggatttg atggaagtaa tatatattat 180
ggagactccg tgaggggccg aatcatcata tccagagaca attccgagaa cacgttgtat 240
ctggaaatga acagcctgag agccgaggac acggcagtgt attattgtgc gagagagaag 300
ggtttagact ggggccaggg aaccctggtc actgtctcct ca 342

<210> 50
<211> 114
<212> PRT
<213> Artificial Sequence

<220>
<223> Synthetic

<400> 50
Gln Val Gln Leu Gln Glu Ser Gly Gly Gly Val Val Gln Pro Gly Arg
1 5 10 15
Ser Leu Arg Leu Ser Cys Ala Ala Ser Gly Phe Thr Phe Ser Ser Tyr
20 25 30
Gly Met His Trp Val Arg Gln Ala Pro Gly Lys Gly Leu Glu Trp Val
35 40 45
Ala Phe Ile Gly Phe Asp Gly Ser Asn Ile Tyr Tyr Gly Asp Ser Val
50 55 60
Arg Gly Arg Ile Ile Ile Ser Arg Asp Asn Ser Glu Asn Thr Leu Tyr
65 70 75 80
Leu Glu Met Asn Ser Leu Arg Ala Glu Asp Thr Ala Val Tyr Tyr Cys
85 90 95
Ala Arg Glu Lys Gly Leu Asp Trp Gly Gln Gly Thr Leu Val Thr Val

100 105 110
Ser Ser

<210> 51
<211> 24
<212> DNA
<213> Artificial Sequence

<220>
<223> Synthetic

<400> 51
ggattcacct tcagtagcta tggc 24

<210> 52
<211> 8
<212> PRT
<213> Artificial Sequence

<220>
<223> Synthetic

<400> 52
Gly Phe Thr Phe Ser Ser Tyr Gly
1 5

<210> 53
<211> 24
<212> DNA
<213> Artificial Sequence

<220>
<223> Synthetic

<400> 53
ataggatttg atggaagtaa tata 24

<210> 54
<211> 8
<212> PRT
<213> Artificial Sequence

<220>
<223> Synthetic

<400> 54
Ile Gly Phe Asp Gly Ser Asn Ile
1 5

<210> 55
<211> 21
<212> DNA
<213> Artificial Sequence

<220>
<223> Synthetic

<400> 55
gcgagagaga agggtttaga c 21

<210> 56

<211> 7
<212> PRT
<213> Artificial Sequence

<220>
<223> Synthetic

<400> 56
Ala Arg Glu Lys Gly Leu Asp
1 5

<210> 57
<211> 342
<212> DNA
<213> Artificial Sequence

<220>
<223> Synthetic

<400> 57
gccatccaga tgacccagtc tccagactcc ctggctgtgt ctctgggcga gagggccacc 60
atcaactgca agtccagcca gagtgttttt cacacctcca acaataagaa ctacttagtt 120
tggtatcagc agaaaccagg acagcctcct aagttgctcc tttactgggc ctctacccgg 180
gaatccgggg tccctgaccg attcagtggc agcgggtctg ggacagattt cactctcacc 240
atcagcagcc tgcaggctga agatgtggca aattattact gtcaccaata ttacagtatt 300
ccgtggacgt tcggccaagg gaccaaggtg gagatcaaac ga 342

<210> 58
<211> 114
<212> PRT
<213> Artificial Sequence

<220>
<223> Synthetic

<400> 58
Ala Ile Gln Met Thr Gln Ser Pro Asp Ser Leu Ala Val Ser Leu Gly
1 5 10 15
Glu Arg Ala Thr Ile Asn Cys Lys Ser Ser Gln Ser Val Phe His Thr
20 25 30
Ser Asn Asn Lys Asn Tyr Leu Val Trp Tyr Gln Gln Lys Pro Gly Gln
35 40 45
Pro Pro Lys Leu Leu Leu Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Asn Tyr Tyr Cys His Gln
85 90 95
Tyr Tyr Ser Ile Pro Trp Thr Phe Gly Gln Gly Thr Lys Val Glu Ile
100 105 110
Lys Arg

<210> 59
<211> 36
<212> DNA
<213> Artificial Sequence

<220>
<223> Synthetic

<400> 59
cagagtgttt ttcacacctc caacaataag aactac 36

<210> 60
<211> 12
<212> PRT
<213> Artificial Sequence

<220>
<223> Synthetic

<400> 60
Gln Ser Val Phe His Thr Ser Asn Asn Lys Asn Tyr
1 5 10

<210> 61
<211> 9
<212> DNA
<213> Artificial Sequence

<220>
<223> Synthetic

<400> 61
tgggcctct 9

<210> 62
<211> 3
<212> PRT
<213> Artificial Sequence

<220>
<223> Synthetic

<400> 62
Trp Ala Ser
1

<210> 63
<211> 27
<212> DNA
<213> Artificial Sequence

<220>
<223> Synthetic

<400> 63
caccaatatt acagtattcc gtggacg 27

<210> 64
<211> 9
<212> PRT
<213> Artificial Sequence

<220>
<223> Synthetic

<400> 64
His Gln Tyr Tyr Ser Ile Pro Trp Thr
1 5

<210> 65
<211> 342
<212> DNA

<213> Artificial Sequence

<220>
<223> Synthetic

<400> 65
caggtgcagc tggtggagtc tgggggaggc gtggtccagc ctgggaggtc cctgagactc 60
tcctgtgcag cgtctggatt caccttcagt agctatggca tgcactgggt ccgccaggct 120
ccaggcaagg ggctggagtg ggtggcgttt ataggatttg atggaagtaa tatatattat 180
ggagactccg tgagggggccg aatcatcata tccagagaca attccgagaa cacgttgtat 240
ctggaaatga acagcctgag agccgaggac acggcagtgt attattgtgc gagagagaag 300
ggtttagact ggggccaggg aaccctggtc accgtctcct ca 342

<210> 66
<211> 114
<212> PRT
<213> Artificial Sequence

<220>
<223> Synthetic

<400> 66
Gln Val Gln Leu Val Glu Ser Gly Gly Gly Val Val Gln Pro Gly Arg
1 5 10 15
Ser Leu Arg Leu Ser Cys Ala Ala Ser Gly Phe Thr Phe Ser Ser Tyr
20 25 30
Gly Met His Trp Val Arg Gln Ala Pro Gly Lys Gly Leu Glu Trp Val
35 40 45
Ala Phe Ile Gly Phe Asp Gly Ser Asn Ile Tyr Tyr Gly Asp Ser Val
50 55 60
Arg Gly Arg Ile Ile Ile Ser Arg Asp Asn Ser Glu Asn Thr Leu Tyr
65 70 75 80
Leu Glu Met Asn Ser Leu Arg Ala Glu Asp Thr Ala Val Tyr Tyr Cys
85 90 95
Ala Arg Glu Lys Gly Leu Asp Trp Gly Gln Gly Thr Leu Val Thr Val
100 105 110
Ser Ser

<210> 67
<211> 339
<212> DNA
<213> Artificial Sequence

<220>
<223> Synthetic

<400> 67
gacatcgtga tgacccagtc tccagactcc ctggctgtgt ctctgggcga gagggccacc 60
atcaactgca agtccagcca gagtgttttt cacacctcca acaataagaa ctacttagtt 120
tggtatcagc agaaaccagg acagcctcct aagttgctcc tttactgggc ctctacccgg 180
gaatccgggg tccctgaccg attcagtggc agcgggtctg ggacagattt cactctcacc 240
atcagcagcc tgcaggctga agatgtggca aattattact gtcaccaata ttacagtatt 300
ccgtggacgt tcggccaagg gaccaaggtg gaaatcaaa 339

<210> 68
<211> 113
<212> PRT
<213> Artificial Sequence

<220>
<223> Synthetic

<400> 68

```
Asp Ile Val Met Thr Gln Ser Pro Asp Ser Leu Ala Val Ser Leu Gly
 1               5                  10                  15
Glu Arg Ala Thr Ile Asn Cys Lys Ser Ser Gln Ser Val Phe His Thr
            20                  25                  30
Ser Asn Asn Lys Asn Tyr Leu Val Trp Tyr Gln Gln Lys Pro Gly Gln
        35                  40                  45
Pro Pro Lys Leu Leu Leu Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Asn Tyr Tyr Cys His Gln
                85                  90                  95
Tyr Tyr Ser Ile Pro Trp Thr Phe Gly Gln Gly Thr Lys Val Glu Ile
            100                 105                 110
Lys
```

<210> 69
<211> 342
<212> DNA
<213> Artificial Sequence

<220>
<223> Synthetic

<400> 69

```
caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt ataggatttg atggaagtaa tatatactat 180
gcagactccg tgaagggccg attcaccatc tccagagaca attccaagaa cacgctgtat 240
ctgcaaatga acagcctgag agctgaggac acggctgtgt attactgtgc gagagagaag 300
ggtttagact ggggccaggg aaccctggtc accgtctcct ca                    342
```

<210> 70
<211> 114
<212> PRT
<213> Artificial Sequence

<220>
<223> Synthetic

<400> 70

```
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Gly Phe Asp Gly Ser Asn Ile Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Glu Lys Gly Leu Asp Trp Gly Gln Gly Thr Leu Val Thr Val
            100                 105                 110
Ser Ser
```

<210> 71
<211> 339
<212> DNA
<213> Artificial Sequence

<220>
<223> Synthetic

<400> 71
gacatcgtga tgacccagtc tccagactcc ctggctgtgt ctctgggcga gagggccacc 60
atcaactgca agtccagcca gagtgttttt cacacctcca acaataagaa ctacttagct 120
tggtaccagc agaaaccagg acagcctcct aagctgctca tttactgggc ctctacccgg 180
gaatccgggg tccctgaccg attcagtggc agcgggtctg ggacagattt cactctcacc 240
atcagcagcc tgcaggctga agatgtggca gtttattact gtcaccaata ttacagtatt 300
ccgtggacgt tcggccaagg gaccaaggtg gaaatcaaa 339

<210> 72
<211> 113
<212> PRT
<213> Artificial Sequence

<220>
<223> Synthetic

<400> 72
Asp Ile Val Met Thr Gln Ser Pro Asp Ser Leu Ala Val Ser Leu Gly
1 5 10 15
Glu Arg Ala Thr Ile Asn Cys Lys Ser Ser Gln Ser Val Phe His Thr
20 25 30
Ser Asn Asn Lys Asn Tyr Leu Ala Trp Tyr Gln Gln Lys Pro Gly Gln
35 40 45
Pro Pro Lys Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys His Gln
85 90 95
Tyr Tyr Ser Ile Pro Trp Thr Phe Gly Gln Gly Thr Lys Val Glu Ile
100 105 110
Lys

<210> 73
<211> 354
<212> DNA
<213> Artificial Sequence

<220>
<223> Synthetic

<400> 73
gaagtgcagc tggtgcagtc tgggggaggc ttggtacagc ctggggggtc cctgagactc 60
tcctgtgcag cctctggatt cacctttaac aactatgcca tgaactgggt ccgccaggct 120
ccaggaaagg gactggactg ggtctcaact attagtggta gcggtggtac tacaaactac 180
gcagactccg tgaagggccg tttcattatt tcccgagaca gttccaaaca cacgctgtat 240
ctgcaaatga acagcctgag agccgaggac acggccgtat attactgtgc gaaagattct 300
aactggggaa atttcgatct ctggggccgt ggcaccacgg tcactgtctc ctca 354

<210> 74
<211> 118
<212> PRT
<213> Artificial Sequence

<220>
<223> Synthetic

<400> 74
Glu Val Gln Leu Val Gln Ser Gly Gly Gly Leu Val Gln Pro Gly Gly

```
 1               5                   10                  15
Ser Leu Arg Leu Ser Cys Ala Ala Ser Gly Phe Thr Phe Asn Asn Tyr
            20                  25                  30
Ala Met Asn Trp Val Arg Gln Ala Pro Gly Lys Gly Leu Asp Trp Val
        35                  40                  45
Ser Thr Ile Ser Gly Ser Gly Gly Thr Thr Asn Tyr Ala Asp Ser Val
    50                  55                  60
Lys Gly Arg Phe Ile Ile Ser Arg Asp Ser Ser Lys His Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Ser Asn Trp Gly Asn Phe Asp Leu Trp Gly Arg Gly Thr
            100                 105                 110
Thr Val Thr Val Ser Ser
        115
```

```
<210> 75
<211> 24
<212> DNA
<213> Artificial Sequence

<220>
<223> Synthetic

<400> 75
ggattcacct ttaacaacta tgcc                                          24

<210> 76
<211> 8
<212> PRT
<213> Artificial Sequence

<220>
<223> Synthetic

<400> 76
Gly Phe Thr Phe Asn Asn Tyr Ala
 1               5


<210> 77
<211> 24
<212> DNA
<213> Artificial Sequence

<220>
<223> Synthetic

<400> 77
attagtggta gcggtggtac taca                                          24

<210> 78
<211> 8
<212> PRT
<213> Artificial Sequence

<220>
<223> Synthetic

<400> 78
Ile Ser Gly Ser Gly Gly Thr Thr
 1               5
```

<210> 79
<211> 33
<212> DNA
<213> Artificial Sequence

<220>
<223> Synthetic

<400> 79
gcgaaagatt ctaactgggg aaatttcgat ctc 33

<210> 80
<211> 11
<212> PRT
<213> Artificial Sequence

<220>
<223> Synthetic

<400> 80
Ala Lys Asp Ser Asn Trp Gly Asn Phe Asp Leu
1 5 10

<210> 81
<211> 342
<212> DNA
<213> Artificial Sequence

<220>
<223> Synthetic

<400> 81
gacatccaga tgacccagtc tccagactcc ctggctgtgt ctctgggcga gagggccacc 60
atcaactgca agtccagcca gagtgtttta tacaggtcca acaataggaa cttcttaggt 120
tggtaccagc agaaaccagg gcagcctcct aatctactca tttactgggc atctacccgg 180
gaatccgggg tccctgaccg attcagtggc agcgggtctg ggacagattt cactctcacc 240
atcagcagcc tgcaggctga agatgtggca gtttattact gtcaacaata ttatactact 300
ccgtacactt ttggccaggg gaccaaggtg gaaatcaaac ga 342

<210> 82
<211> 114
<212> PRT
<213> Artificial Sequence

<220>
<223> Synthetic

<400> 82
Asp Ile Gln Met Thr Gln Ser Pro Asp Ser Leu Ala Val Ser Leu Gly
1 5 10 15
Glu Arg Ala Thr Ile Asn Cys Lys Ser Ser Gln Ser Val Leu Tyr Arg
20 25 30
Ser Asn Asn Arg Asn Phe Leu Gly Trp Tyr Gln Gln Lys Pro Gly Gln
35 40 45
Pro Pro Asn Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Gln
85 90 95
Tyr Tyr Thr Thr Pro Tyr Thr Phe Gly Gln Gly Thr Lys Val Glu Ile
100 105 110
Lys Arg

<210> 83
<211> 36
<212> DNA
<213> Artificial Sequence

<220>
<223> Synthetic

<400> 83
cagagtgttt tatacaggtc caacaatagg aacttc 36

<210> 84
<211> 12
<212> PRT
<213> Artificial Sequence

<220>
<223> Synthetic

<400> 84
Gln Ser Val Leu Tyr Arg Ser Asn Asn Arg Asn Phe
1 5 10

<210> 85
<211> 9
<212> DNA
<213> Artificial Sequence

<220>
<223> Synthetic

<400> 85
tgggcatct 9

<210> 86
<211> 3
<212> PRT
<213> Artificial Sequence

<220>
<223> Synthetic

<400> 86
Trp Ala Ser
1

<210> 87
<211> 27
<212> DNA
<213> Artificial Sequence

<220>
<223> Synthetic

<400> 87
caacaatatt atactactcc gtacact 27

<210> 88
<211> 9
<212> PRT
<213> Artificial Sequence

<220>
<223> Synthetic

<400> 88
Gln Gln Tyr Tyr Thr Thr Pro Tyr Thr
1 5

<210> 89
<211> 354
<212> DNA
<213> Artificial Sequence

<220>
<223> Synthetic

<400> 89
gaggtgcagc tggtggagtc tgggggaggc ttggtacagc ctggggggtc cctgagactc 60
tcctgtgcag cctctggatt cacctttaac aactatgcca tgaactgggt ccgccaggct 120
ccaggaaagg gactggactg ggtctcaact attagtggta gcggtggtac tacaaactac 180
gcagactccg tgaagggccg tttcattatt tcccgagaca gttccaaaca cacgctgtat 240
ctgcaaatga acagcctgag agccgaggac acggccgtat attactgtgc gaaagattct 300
aactggggaa atttcgatct ctggggccgt ggcaccctgg tcactgtctc ctca 354

<210> 90
<211> 118
<212> PRT
<213> Artificial Sequence

<220>
<223> Synthetic

<400> 90
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Asn Asn Tyr
20 25 30
Ala Met Asn Trp Val Arg Gln Ala Pro Gly Lys Gly Leu Asp Trp Val
35 40 45
Ser Thr Ile Ser Gly Ser Gly Gly Thr Thr Asn Tyr Ala Asp Ser Val
50 55 60
Lys Gly Arg Phe Ile Ile Ser Arg Asp Ser Ser Lys His Thr Leu Tyr
65 70 75 80
Leu Gln Met Asn Ser Leu Arg Ala Glu Asp Thr Ala Val Tyr Tyr Cys
85 90 95
Ala Lys Asp Ser Asn Trp Gly Asn Phe Asp Leu Trp Gly Arg Gly Thr
100 105 110
Leu Val Thr Val Ser Ser
115

<210> 91
<211> 339
<212> DNA
<213> Artificial Sequence

<220>
<223> Synthetic

<400> 91
gacatcgtga tgacccagtc tccagactcc ctggctgtgt ctctgggcga gagggccacc 60
atcaactgca agtccagcca gagtgtttta tacaggtcca acaataggaa cttcttaggt 120
tggtaccagc agaaaccagg gcagcctcct aatctactca tttactgggc atctacccgg 180
gaatccgggg tccctgaccg attcagtggc agcgggtctg ggacagattt cactctcacc 240

atcagcagcc tgcaggctga agatgtggca gtttattact gtcaacaata ttatactact 300
ccgtacactt ttggccaggg gaccaagctg gagatcaaa 339

<210> 92
<211> 113
<212> PRT
<213> Artificial Sequence

<220>
<223> Synthetic

<400> 92
Asp Ile Val Met Thr Gln Ser Pro Asp Ser Leu Ala Val Ser Leu Gly
1 5 10 15
Glu Arg Ala Thr Ile Asn Cys Lys Ser Ser Gln Ser Val Leu Tyr Arg
20 25 30
Ser Asn Asn Arg Asn Phe Leu Gly Trp Tyr Gln Gln Lys Pro Gly Gln
35 40 45
Pro Pro Asn Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Gln
85 90 95
Tyr Tyr Thr Thr Pro Tyr Thr Phe Gly Gln Gly Thr Lys Leu Glu Ile
100 105 110
Lys

<210> 93
<211> 354
<212> DNA
<213> Artificial Sequence

<220>
<223> Synthetic

<400> 93
gaggtgcagc tggtggagtc tgggggaggc ttggtacagc ctggggggtc cctgagactc 60
tcctgtgcag cctctggatt cacctttaac aactatgcca tgagctgggt ccgccaggct 120
ccagggaagg ggctggagtg ggtctcagct attagtggta gcggtggtac tacatactac 180
gcagactccg tgaagggccg gttcaccatc tccagagaca attccaagaa cacgctgtat 240
ctgcaaatga acagcctgag agccgaggac acggccgtat attactgtgc gaaagattct 300
aactggggaa atttcgatct ctggggccgt ggcaccctgg tcactgtctc ctca 354

<210> 94
<211> 118
<212> PRT
<213> Artificial Sequence

<220>
<223> Synthetic

<400> 94
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Asn Asn Tyr
20 25 30
Ala Met Ser Trp Val Arg Gln Ala Pro Gly Lys Gly Leu Glu Trp Val
35 40 45
Ser Ala Ile Ser Gly Ser Gly Gly Thr Thr Tyr Tyr Ala Asp Ser Val
50 55 60
Lys Gly Arg Phe Thr Ile Ser Arg Asp Asn Ser Lys Asn Thr Leu Tyr
65 70 75 80

```
Leu Gln Met Asn Ser Leu Arg Ala Glu Asp Thr Ala Val Tyr Tyr Cys
                85                  90                  95
Ala Lys Asp Ser Asn Trp Gly Asn Phe Asp Leu Trp Gly Arg Gly Thr
            100                 105                 110
Leu Val Thr Val Ser Ser
        115


<210> 95
<211> 339
<212> DNA
<213> Artificial Sequence

<220>
<223> Synthetic

<400> 95
gacatcgtga tgacccagtc tccagactcc ctggctgtgt ctctgggcga gagggccacc 60
atcaactgca agtccagcca gagtgtttta tacaggtcca acaataggaa cttcttagct 120
tggtaccagc agaaaccagg acagcctcct aagctgctca tttactgggc atctacccgg 180
gaatccgggg tccctgaccg attcagtggc agcgggtctg ggacagattt cactctcacc 240
atcagcagcc tgcaggctga agatgtggca gtttattact gtcaacaata ttatactact 300
ccgtacactt ttggccaggg gaccaagctg gagatcaaa                        339

<210> 96
<211> 113
<212> PRT
<213> Artificial Sequence

<220>
<223> Synthetic

<400> 96
Asp Ile Val Met Thr Gln Ser Pro Asp Ser Leu Ala Val Ser Leu Gly
 1               5                  10                  15
Glu Arg Ala Thr Ile Asn Cys Lys Ser Ser Gln Ser Val Leu Tyr Arg
            20                  25                  30
Ser Asn Asn Arg Asn Phe Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Thr Thr Pro Tyr Thr Phe Gly Gln Gly Thr Lys Leu Glu Ile
            100                 105                 110
Lys



<210> 97
<211> 351
<212> DNA
<213> Artificial Sequence

<220>
<223> Synthetic

<400> 97
caggtgcagc tggtgcagtc tgggggaggc ttggtacagc ctggggggtc cctgagactc 60
tcctgtgcag tctctggatt caccctcagt agctacgata tgcactgggt ccgccaacct 120
acaggaaaag gtctggagtg ggtctcagct attggttcta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggatgg 300
```

gacgtaccct ttgacttctg gggccaggga accctggtca ccgtctcctc a 351

<210> 98
<211> 117
<212> PRT
<213> Artificial Sequence

<220>
<223> Synthetic

<400> 98
Gln Val Gln Leu Val Gln Ser Gly Gly Gly Leu Val Gln Pro Gly Gly
1 5 10 15
Ser Leu Arg Leu Ser Cys Ala Val Ser Gly Phe Thr Leu Ser Ser Tyr
20 25 30
Asp Met His Trp Val Arg Gln Pro Thr Gly Lys Gly Leu Glu Trp Val
35 40 45
Ser Ala Ile Gly Ser Thr Gly Asp Thr Tyr Tyr Pro Gly Ser Val Lys
50 55 60
Gly Arg Phe Thr Ile Ser Arg Glu Asn Ala Lys Asn Ser Leu Tyr Leu
65 70 75 80
Gln Met Asn Ser Leu Arg Ala Gly Asp Thr Ala Val Tyr Tyr Cys Ala
85 90 95
Arg Glu Gly Trp Asp Val Pro Phe Asp Phe Trp Gly Gln Gly Thr Leu
100 105 110
Val Thr Val Ser Ser
115

<210> 99
<211> 24
<212> DNA
<213> Artificial Sequence

<220>
<223> Synthetic

<400> 99
ggattcaccc tcagtagcta cgat 24

<210> 100
<211> 8
<212> PRT
<213> Artificial Sequence

<220>
<223> Synthetic

<400> 100
Gly Phe Thr Leu Ser Ser Tyr Asp
1 5

<210> 101
<211> 21
<212> DNA
<213> Artificial Sequence

<220>
<223> Synthetic

<400> 101
attggttcta ctggtgacac a 21

<210> 102

<211> 7
<212> PRT
<213> Artificial Sequence

<220>
<223> Synthetic

<400> 102
Ile Gly Ser Thr Gly Asp Thr
1 5

<210> 103
<211> 33
<212> DNA
<213> Artificial Sequence

<220>
<223> Synthetic

<400> 103
gcaagagagg gatgggacgt accctttgac ttc 33

<210> 104
<211> 11
<212> PRT
<213> Artificial Sequence

<220>
<223> Synthetic

<400> 104
Ala Arg Glu Gly Trp Asp Val Pro Phe Asp Phe
1 5 10

<210> 105
<211> 324
<212> DNA
<213> Artificial Sequence

<220>
<223> Synthetic

<400> 105
gccatccagt tgacccagtc tccatcctcc ctgtctgcat ctgtaggaga cagagtcacc 60
atcacttgcc gggcaagtca ggacattaga aatgatttag gctggtatca gcagaaacca 120
gggaaagccc ctaagctcct gatctatgct gcatccagtt tacaaagtgg ggtcccatca 180
cggttcagcg gcagtggatc tggcacagat ttcactctca ccatcagcag cctgcagcct 240
gaagattttg caacttatta ctgtctacaa gattacaatt acccgtggac gttcggccaa 300
gggaccaagg tggagatcaa acga 324

<210> 106
<211> 108
<212> PRT
<213> Artificial Sequence

<220>
<223> Synthetic

<400> 106
Ala Ile Gln Leu Thr Gln Ser Pro Ser Ser Leu Ser Ala Ser Val Gly
1 5 10 15
Asp Arg Val Thr Ile Thr Cys Arg Ala Ser Gln Asp Ile Arg Asn Asp
20 25 30

```
Leu Gly Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Asp Tyr Asn Tyr Pro Trp
                85                  90                  95
Thr Phe Gly Gln Gly Thr Lys Val Glu Ile Lys Arg
            100                 105
```

<210> 107
<211> 18
<212> DNA
<213> Artificial Sequence

<220>
<223> Synthetic

<400> 107
caggacatta gaaatgat 18

<210> 108
<211> 6
<212> PRT
<213> Artificial Sequence

<220>
<223> Synthetic

<400> 108

```
Gln Asp Ile Arg Asn Asp
 1               5
```

<210> 109
<211> 9
<212> DNA
<213> Artificial Sequence

<220>
<223> Synthetic

<400> 109
gctgcatcc 9

<210> 110
<211> 3
<212> PRT
<213> Artificial Sequence

<220>
<223> Synthetic

<400> 110

```
Ala Ala Ser
 1
```

<210> 111
<211> 27
<212> DNA
<213> Artificial Sequence

<220>
<223> Synthetic

<400> 111
ctacaagatt acaattaccc gtggacg 27

<210> 112
<211> 9
<212> PRT
<213> Artificial Sequence

<220>
<223> Synthetic

<400> 112
Leu Gln Asp Tyr Asn Tyr Pro Trp Thr
1 5

<210> 113
<211> 351
<212> DNA
<213> Artificial Sequence

<220>
<223> Synthetic

<400> 113
gaggtgcagc tggtggagtc tgggggaggc ttggtacagc ctggggggtc cctgagactc 60
tcctgtgcag tctctggatt caccctcagt agctacgata tgcactgggt ccgccaacct 120
acaggaaaag gtctggagtg ggtctcagct attggttcta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggatgg 300
gacgtaccct ttgacttctg gggccaggga accctggtca ccgtctcctc a 351

<210> 114
<211> 117
<212> PRT
<213> Artificial Sequence

<220>
<223> Synthetic

<400> 114
Glu Val Gln Leu Val Glu Ser Gly Gly Gly Leu Val Gln Pro Gly Gly
1 5 10 15
Ser Leu Arg Leu Ser Cys Ala Val Ser Gly Phe Thr Leu Ser Ser Tyr
20 25 30
Asp Met His Trp Val Arg Gln Pro Thr Gly Lys Gly Leu Glu Trp Val
35 40 45
Ser Ala Ile Gly Ser Thr Gly Asp Thr Tyr Tyr Pro Gly Ser Val Lys
50 55 60
Gly Arg Phe Thr Ile Ser Arg Glu Asn Ala Lys Asn Ser Leu Tyr Leu
65 70 75 80
Gln Met Asn Ser Leu Arg Ala Gly Asp Thr Ala Val Tyr Tyr Cys Ala
85 90 95
Arg Glu Gly Trp Asp Val Pro Phe Asp Phe Trp Gly Gln Gly Thr Leu
100 105 110
Val Thr Val Ser Ser
115

<210> 115
<211> 321
<212> DNA

<213> Artificial Sequence

<220>
<223> Synthetic

<400> 115
gccatccaga tgacccagtc tccatcctcc ctgtctgcat ctgtaggaga cagagtcacc 60
atcacttgcc gggcaagtca ggacattaga aatgatttag gctggtatca gcagaaacca 120
gggaaagccc ctaagctcct gatctatgct gcatccagtt tacaaagtgg ggtcccatca 180
cggttcagcg gcagtggatc tggcacagat ttcactctca ccatcagcag cctgcagcct 240
gaagattttg caacttatta ctgtctacaa gattacaatt acccgtggac gttcggccaa 300
gggaccaagg tggaaatcaa a 321

<210> 116
<211> 107
<212> PRT
<213> Artificial Sequence

<220>
<223> Synthetic

<400> 116
Ala Ile Gln Met Thr Gln Ser Pro Ser Ser Leu Ser Ala Ser Val Gly
1 5 10 15
Asp Arg Val Thr Ile Thr Cys Arg Ala Ser Gln Asp Ile Arg Asn Asp
20 25 30
Leu Gly Trp Tyr Gln Gln Lys Pro Gly Lys Ala Pro Lys Leu Leu Ile
35 40 45
Tyr Ala Ala Ser Ser Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Leu Gln Asp Tyr Asn Tyr Pro Trp
85 90 95
Thr Phe Gly Gln Gly Thr Lys Val Glu Ile Lys
100 105

<210> 117
<211> 351
<212> DNA
<213> Artificial Sequence

<220>
<223> Synthetic

<400> 117
gaggtgcagc tggtggagtc tgggggaggc ttggtacagc ctggggggtc cctgagactc 60
tcctgtgcag cctctggatt caccctcagt agctacgata tgcactgggt ccgccaagct 120
acaggaaaag gtctggagtg ggtctcagct attggttcta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggatgg 300
gacgtaccct ttgacttctg gggccaggga accctggtca ccgtctcctc a 351

<210> 118
<211> 117
<212> PRT
<213> Artificial Sequence

<220>
<223> Synthetic

<400> 118
Glu Val Gln Leu Val Glu Ser Gly Gly Gly Leu Val Gln Pro Gly Gly
1 5 10 15

```
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Gly Trp Asp Val Pro Phe Asp Phe Trp Gly Gln Gly Thr Leu
            100                 105                 110
Val Thr Val Ser Ser
        115
```

<210> 119
<211> 321
<212> DNA
<213> Artificial Sequence

<220>
<223> Synthetic

<400> 119

```
gccatccaga tgacccagtc tccatcctcc ctgtctgcat ctgtaggaga cagagtcacc 60
atcacttgcc gggcaagtca ggacattaga aatgatttag gctggtatca gcagaaacca 120
gggaaagccc ctaagctcct gatctatgct gcatccagtt tacaaagtgg ggtcccatca 180
aggttcagcg gcagtggatc tggcacagat ttcactctca ccatcagcag cctgcagcct 240
gaagattttg caacttatta ctgtctacaa gattacaatt acccgtggac gttcggccaa 300
gggaccaagg tggaaatcaa a                                           321
```

<210> 120
<211> 107
<212> PRT
<213> Artificial Sequence

<220>
<223> Synthetic

<400> 120

```
Ala Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Arg Asn Asp
            20                  25                  30
Leu Gly Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Asp Tyr Asn Tyr Pro Trp
                85                  90                  95
Thr Phe Gly Gln Gly Thr Lys Val Glu Ile Lys
            100                 105
```

<210> 121
<211> 384
<212> DNA
<213> Artificial Sequence

<220>
<223> Synthetic

<400> 121
caggtgcagc tgcaggagtc ggggccagga ctggtgaagc cttcggagac cctgtccctc 60
acctgcactg tctctgggga ctccatcaat acttactact ggagctggtt ccggcagccc 120
ccagggaagg gactggagtg gattgggtat atctattata gtggaaccac caactacaac 180
ccctccctca agagtcgagt caccatatca atagacacgc ccaggaacca gttctccctg 240
aagctgatct ctgtgaccgc agcggacacg gccgtgtatt actgtgcgag agagaggatt 300
actatgattc ggggagttac cctctactat tactcctacg gtatggacgt ctggggccaa 360
gggaccacgg tcaccgtctc ctca 384

<210> 122
<211> 128
<212> PRT
<213> Artificial Sequence

<220>
<223> Synthetic

<400> 122
Gln Val Gln Leu Gln Glu Ser Gly Pro Gly Leu Val Lys Pro Ser Glu
1 5 10 15
Thr Leu Ser Leu Thr Cys Thr Val Ser Gly Asp Ser Ile Asn Thr Tyr
20 25 30
Tyr Trp Ser Trp Phe Arg Gln Pro Pro Gly Lys Gly Leu Glu Trp Ile
35 40 45
Gly Tyr Ile Tyr Tyr Ser Gly Thr Thr Asn Tyr Asn Pro Ser Leu Lys
50 55 60
Ser Arg Val Thr Ile Ser Ile Asp Thr Pro Arg Asn Gln Phe Ser Leu
65 70 75 80
Lys Leu Ile Ser Val Thr Ala Ala Asp Thr Ala Val Tyr Tyr Cys Ala
85 90 95
Arg Glu Arg Ile Thr Met Ile Arg Gly Val Thr Leu Tyr Tyr Tyr Ser
100 105 110
Tyr Gly Met Asp Val Trp Gly Gln Gly Thr Thr Val Thr Val Ser Ser
115 120 125

<210> 123
<211> 24
<212> DNA
<213> Artificial Sequence

<220>
<223> Synthetic

<400> 123
ggggactcca tcaatactta ctac 24

<210> 124
<211> 8
<212> PRT
<213> Artificial Sequence

<220>
<223> Synthetic

<400> 124
Gly Asp Ser Ile Asn Thr Tyr Tyr
1 5

<210> 125
<211> 21
<212> DNA
<213> Artificial Sequence

<220>
<223> Synthetic

<400> 125
atctattata gtggaaccac c 21

<210> 126
<211> 7
<212> PRT
<213> Artificial Sequence

<220>
<223> Synthetic

<400> 126
Ile Tyr Tyr Ser Gly Thr Thr
1 5

<210> 127
<211> 66
<212> DNA
<213> Artificial Sequence

<220>
<223> Synthetic

<400> 127
gcgagagaga ggattactat gattcgggga gttaccctct actattactc ctacggtatg 60
gacgtc 66

<210> 128
<211> 22
<212> PRT
<213> Artificial Sequence

<220>
<223> Synthetic

<400> 128
Ala Arg Glu Arg Ile Thr Met Ile Arg Gly Val Thr Leu Tyr Tyr Tyr
1 5 10 15
Ser Tyr Gly Met Asp Val
20

<210> 129
<211> 324
<212> DNA
<213> Artificial Sequence

<220>
<223> Synthetic

<400> 129
gacatccaga tgacccagtc tccatccttc ctgtctgcat ctgtaggaga cagagtcacc 60
atcacttgct gggccagtca ggacattagc agttatttag cctggtatca gcaaaaacca 120
gggatagccc ctaagctcct gatctatgct gcatccactt tgcaaagtgg ggtcccatca 180
aggttcggcg gcagtggatc tgggacagaa ttcactctca caatcagcag cctgcagcct 240
gaagattttg caacttatta ctgtcaacag cttaatagtt accctcggac gttcggccaa 300
gggaccaagg tggaaatcaa acga 324

<210> 130
<211> 108
<212> PRT

<213> Artificial Sequence

<220>
<223> Synthetic

<400> 130
Asp Ile Gln Met Thr Gln Ser Pro Ser Phe Leu Ser Ala Ser Val Gly
1 5 10 15
Asp Arg Val Thr Ile Thr Cys Trp Ala Ser Gln Asp Ile Ser Ser Tyr
20 25 30
Leu Ala Trp Tyr Gln Gln Lys Pro Gly Ile Ala Pro Lys Leu Leu Ile
35 40 45
Tyr Ala Ala Ser Thr Leu Gln Ser Gly Val Pro Ser Arg Phe Gly Gly
50 55 60
Ser Gly Ser Gly Thr Glu Phe Thr Leu Thr Ile Ser Ser Leu Gln Pro
65 70 75 80
Glu Asp Phe Ala Thr Tyr Tyr Cys Gln Gln Leu Asn Ser Tyr Pro Arg
85 90 95
Thr Phe Gly Gln Gly Thr Lys Val Glu Ile Lys Arg
100 105

<210> 131
<211> 18
<212> DNA
<213> Artificial Sequence

<220>
<223> Synthetic

<400> 131
caggacatta gcagttat 18

<210> 132
<211> 6
<212> PRT
<213> Artificial Sequence

<220>
<223> Synthetic

<400> 132
Gln Asp Ile Ser Ser Tyr
1 5

<210> 133
<211> 9
<212> DNA
<213> Artificial Sequence

<220>
<223> Synthetic

<400> 133
gctgcatcc 9

<210> 134
<211> 3
<212> PRT
<213> Artificial Sequence

<220>
<223> Synthetic

<400> 134
Ala Ala Ser
1

<210> 135
<211> 27
<212> DNA
<213> Artificial Sequence

<220>
<223> Synthetic

<400> 135
caacagctta atagttaccc tcggacg 27

<210> 136
<211> 9
<212> PRT
<213> Artificial Sequence

<220>
<223> Synthetic

<400> 136
Gln Gln Leu Asn Ser Tyr Pro Arg Thr
1 5

<210> 137
<211> 384
<212> DNA
<213> Artificial Sequence

<220>
<223> Synthetic

<400> 137
caggtgcagc tgcaggagtc ggggccagga ctggtgaagc cttcggagac cctgtccctc 60
acctgcactg tctctgggga ctccatcaat acttactact ggagctggtt ccggcagccc 120
ccagggaagg gactggagtg gattgggtat atctattata gtggaaccac caactacaac 180
ccctccctca agagtcgagt caccatatca atagacacgc ccaggaacca gttctccctg 240
aagctgatct ctgtgaccgc agcggacacg gccgtgtatt actgtgcgag agagaggatt 300
actatgattc ggggagttac cctctactat tactcctacg gtatggacgt ctggggccaa 360
gggaccacgg tcaccgtctc ctca 384

<210> 138
<211> 128
<212> PRT
<213> Artificial Sequence

<220>
<223> Synthetic

<400> 138
Gln Val Gln Leu Gln Glu Ser Gly Pro Gly Leu Val Lys Pro Ser Glu
1 5 10 15
Thr Leu Ser Leu Thr Cys Thr Val Ser Gly Asp Ser Ile Asn Thr Tyr
20 25 30
Tyr Trp Ser Trp Phe Arg Gln Pro Pro Gly Lys Gly Leu Glu Trp Ile
35 40 45
Gly Tyr Ile Tyr Tyr Ser Gly Thr Thr Asn Tyr Asn Pro Ser Leu Lys
50 55 60
Ser Arg Val Thr Ile Ser Ile Asp Thr Pro Arg Asn Gln Phe Ser Leu
65 70 75 80

```
Lys Leu Ile Ser Val Thr Ala Ala Asp Thr Ala Val Tyr Tyr Cys Ala
                85                  90                  95
Arg Glu Arg Ile Thr Met Ile Arg Gly Val Thr Leu Tyr Tyr Tyr Ser
            100                 105                 110
Tyr Gly Met Asp Val Trp Gly Gln Gly Thr Thr Val Thr Val Ser Ser
        115                 120                 125


<210> 139
<211> 321
<212> DNA
<213> Artificial Sequence

<220>
<223> Synthetic

<400> 139
gacatccaga tgacccagtc tccatccttc ctgtctgcat ctgtaggaga cagagtcacc 60
atcacttgct gggccagtca ggacattagc agttatttag cctggtatca gcaaaaacca 120
gggatagccc ctaagctcct gatctatgct gcatccactt tgcaaagtgg ggtcccatca 180
aggttcggcg gcagtggatc tgggacagaa ttcactctca caatcagcag cctgcagcct 240
gaagattttg caacttatta ctgtcaacag cttaatagtt accctcggac gttcggccaa 300
gggaccaagg tggaaatcaa a                                           321

<210> 140
<211> 107
<212> PRT
<213> Artificial Sequence

<220>
<223> Synthetic

<400> 140
Asp Ile Gln Met Thr Gln Ser Pro Ser Phe Leu Ser Ala Ser Val Gly
1               5                   10                  15
Asp Arg Val Thr Ile Thr Cys Trp Ala Ser Gln Asp Ile Ser Ser Tyr
            20                  25                  30
Leu Ala Trp Tyr Gln Gln Lys Pro Gly Ile Ala Pro Lys Leu Leu Ile
        35                  40                  45
Tyr Ala Ala Ser Thr Leu Gln Ser Gly Val Pro Ser Arg Phe Gly Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Leu Asn Ser Tyr Pro Arg
                85                  90                  95
Thr Phe Gly Gln Gly Thr Lys Val Glu Ile Lys
            100                 105


<210> 141
<211> 384
<212> DNA
<213> Artificial Sequence

<220>
<223> Synthetic

<400> 141
caggtgcagc tgcaggagtc gggcccagga ctggtgaagc cttcggagac cctgtccctc 60
acctgcactg tctctgggga ctccatcaat acttactact ggagctggat ccggcagccc 120
ccagggaagg gactggagtg gattgggtat atctattata gtggaaccac caactacaac 180
ccctccctca agagtcgagt caccatatca gtagacacgt ccaagaacca gttctccctg 240
aagctgagct ctgtgaccgc tgcggacacg gccgtgtatt actgtgcgag agagaggatt 300
actatgattc ggggagttac cctctactat tactcctacg gtatggacgt ctggggccaa 360
gggaccacgg tcaccgtctc ctca                                        384
```

<210> 142
<211> 128
<212> PRT
<213> Artificial Sequence

<220>
<223> Synthetic

<400> 142
Gln Val Gln Leu Gln Glu Ser Gly Pro Gly Leu Val Lys Pro Ser Glu
1 5 10 15
Thr Leu Ser Leu Thr Cys Thr Val Ser Gly Asp Ser Ile Asn Thr Tyr
20 25 30
Tyr Trp Ser Trp Ile Arg Gln Pro Pro Gly Lys Gly Leu Glu Trp Ile
35 40 45
Gly Tyr Ile Tyr Tyr Ser Gly Thr Thr Asn Tyr Asn Pro Ser Leu Lys
50 55 60
Ser Arg Val Thr Ile Ser Val Asp Thr Ser Lys Asn Gln Phe Ser Leu
65 70 75 80
Lys Leu Ser Ser Val Thr Ala Ala Asp Thr Ala Val Tyr Tyr Cys Ala
85 90 95
Arg Glu Arg Ile Thr Met Ile Arg Gly Val Thr Leu Tyr Tyr Tyr Ser
100 105 110
Tyr Gly Met Asp Val Trp Gly Gln Gly Thr Thr Val Thr Val Ser Ser
115 120 125

<210> 143
<211> 321
<212> DNA
<213> Artificial Sequence

<220>
<223> Synthetic

<400> 143
gacatccaga tgacccagtc tccatcctcc ctgtctgcat ctgtaggaga cagagtcacc 60
atcacttgcc gggcaagtca ggacattagc agttatttag gctggtatca gcagaaacca 120
gggaaagccc ctaagcgcct gatctatgct gcatccagtt tgcaaagtgg ggtcccatca 180
aggttcagcg gcagtggatc tgggacagaa ttcactctca caatcagcag cctgcagcct 240
gaagattttg caacttatta ctgtcaacag cttaatagtt accctcggac gttcggccaa 300
gggaccaagg tggaaatcaa a 321

<210> 144
<211> 107
<212> PRT
<213> Artificial Sequence

<220>
<223> Synthetic

<400> 144
Asp Ile Gln Met Thr Gln Ser Pro Ser Ser Leu Ser Ala Ser Val Gly
1 5 10 15
Asp Arg Val Thr Ile Thr Cys Arg Ala Ser Gln Asp Ile Ser Ser Tyr
20 25 30
Leu Gly Trp Tyr Gln Gln Lys Pro Gly Lys Ala Pro Lys Arg Leu Ile
35 40 45
Tyr Ala Ala Ser Ser Leu Gln Ser Gly Val Pro Ser Arg Phe Ser Gly
50 55 60
Ser Gly Ser Gly Thr Glu Phe Thr Leu Thr Ile Ser Ser Leu Gln Pro
65 70 75 80
Glu Asp Phe Ala Thr Tyr Tyr Cys Gln Gln Leu Asn Ser Tyr Pro Arg
85 90 95

```
Thr Phe Gly Gln Gly Thr Lys Val Glu Ile Lys
            100                 105
```

<210> 145
<211> 378
<212> DNA
<213> Artificial Sequence

<220>
<223> Synthetic

<400> 145
```
caggtgcagc tggtgcagtc tggagctgag gtgaagaagc ctggggcctc agtgaaggtc 60
tcctgcaagg cttctggtta cacctttacc aactatggta tcagctgggt gcgacaggcc 120
cctggacaag gacttgagtt aatgggatgg attagtggtt acaatggtaa cacaaactat 180
gcacaagaac tccaggccag agtcaccatg accacagaca catccacgag cacagcctac 240
atggagctga ggaacctgag atctgacgac acggccgtat attactgtgc gagagataga 300
gtcgttgtag cagctgctaa ttactacttt tattctatgg acgtctgggg ccaagggacc 360
acggtcaccg tctcctca                                                378
```

<210> 146
<211> 126
<212> PRT
<213> Artificial Sequence

<220>
<223> Synthetic

<400> 146
```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Gly Ile Ser Trp Val Arg Gln Ala Pro Gly Gln Gly Leu Glu Leu Met
        35                  40                  45
Gly Trp Ile Ser Gly Tyr Asn Gly Asn Thr Asn Tyr Ala Gln Glu Leu
    50                  55                  60
Gln Ala Arg Val Thr Met Thr Thr Asp Thr Ser Thr Ser Thr Ala Tyr
65                  70                  75                  80
Met Glu Leu Arg Asn Leu Arg Ser Asp Asp Thr Ala Val Tyr Tyr Cys
                85                  90                  95
Ala Arg Asp Arg Val Val Val Ala Ala Ala Asn Tyr Tyr Phe Tyr Ser
            100                 105                 110
Met Asp Val Trp Gly Gln Gly Thr Thr Val Thr Val Ser Ser
        115                 120                 125
```

<210> 147
<211> 24
<212> DNA
<213> Artificial Sequence

<220>
<223> Synthetic

<400> 147
```
ggttacacct ttaccaacta tggt                                         24
```

<210> 148
<211> 8
<212> PRT
<213> Artificial Sequence

<220>

<223> Synthetic

<400> 148
Gly Tyr Thr Phe Thr Asn Tyr Gly
1 5

<210> 149
<211> 24
<212> DNA
<213> Artificial Sequence

<220>
<223> Synthetic

<400> 149
attagtggtt acaatggtaa caca 24

<210> 150
<211> 8
<212> PRT
<213> Artificial Sequence

<220>
<223> Synthetic

<400> 150
Ile Ser Gly Tyr Asn Gly Asn Thr
1 5

<210> 151
<211> 57
<212> DNA
<213> Artificial Sequence

<220>
<223> Synthetic

<400> 151
gcgagagata gagtcgttgt agcagctgct aattactact tttattctat ggacgtc 57

<210> 152
<211> 19
<212> PRT
<213> Artificial Sequence

<220>
<223> Synthetic

<400> 152
Ala Arg Asp Arg Val Val Val Ala Ala Ala Asn Tyr Tyr Phe Tyr Ser
1 5 10 15
Met Asp Val

<210> 153
<211> 339
<212> DNA
<213> Artificial Sequence

<220>
<223> Synthetic

<400> 153
gccatccaga tgacccagtc tccactctcc ctgtccgtca cccttggaca gccggcctcc 60
atctcctgca ggtctagtca aagcctcgta tacagtgatg gagacaccta cttgaattgg 120
tttcagcaga ggccaggcca atctccaagg cgcctaattt ataaggtttc taaccgggac 180
tctggggtcc cagacagatt cagcggcagt gggtcaggca ctgctttcac actgaaaatc 240
agcggggtgg aggccgagga tgttggggtt tactactgca tgcaagctac acactggcct 300
cggacgttcg gccaagggac caaggtggaa atcaaacga 339

<210> 154
<211> 113
<212> PRT
<213> Artificial Sequence

<220>
<223> Synthetic

<400> 154
Ala Ile Gln Met Thr Gln Ser Pro Leu Ser Leu Ser Val Thr Leu Gly
1 5 10 15
Gln Pro Ala Ser Ile Ser Cys Arg Ser Ser Gln Ser Leu Val Tyr Ser
20 25 30
Asp Gly Asp Thr Tyr Leu Asn Trp Phe Gln Gln Arg Pro Gly Gln Ser
35 40 45
Pro Arg Arg Leu Ile Tyr Lys Val Ser Asn Arg Asp Ser Gly Val Pro
50 55 60
Asp Arg Phe Ser Gly Ser Gly Ser Gly Thr Ala Phe Thr Leu Lys Ile
65 70 75 80
Ser Gly Val Glu Ala Glu Asp Val Gly Val Tyr Tyr Cys Met Gln Ala
85 90 95
Thr His Trp Pro Arg Thr Phe Gly Gln Gly Thr Lys Val Glu Ile Lys
100 105 110
Arg

<210> 155
<211> 33
<212> DNA
<213> Artificial Sequence

<220>
<223> Synthetic

<400> 155
caaagcctcg tatacagtga tggagacacc tac 33

<210> 156
<211> 11
<212> PRT
<213> Artificial Sequence

<220>
<223> Synthetic

<400> 156
Gln Ser Leu Val Tyr Ser Asp Gly Asp Thr Tyr
1 5 10

<210> 157
<211> 9
<212> DNA
<213> Artificial Sequence

<220>

<223> Synthetic

<400> 157
aaggtttct 9

<210> 158
<211> 3
<212> PRT
<213> Artificial Sequence

<220>
<223> Synthetic

<400> 158
Lys Val Ser
1

<210> 159
<211> 27
<212> DNA
<213> Artificial Sequence

<220>
<223> Synthetic

<400> 159
atgcaagcta cacactggcc tcggacg 27

<210> 160
<211> 9
<212> PRT
<213> Artificial Sequence

<220>
<223> Synthetic

<400> 160
Met Gln Ala Thr His Trp Pro Arg Thr
1 5

<210> 161
<211> 378
<212> DNA
<213> Artificial Sequence

<220>
<223> Synthetic

<400> 161
caggttcagc tggtgcagtc tggagctgag gtgaagaagc ctggggcctc agtgaaggtc 60
tcctgcaagg cttctggtta cacctttacc aactatggta tcagctgggt gcgacaggcc 120
cctggacaag gacttgagtt aatgggatgg attagtggtt acaatggtaa cacaaactat 180
gcacaagaac tccaggccag agtcaccatg accacagaca catccacgag cacagcctac 240
atggagctga ggaacctgag atctgacgac acggccgtat attactgtgc gagagataga 300
gtcgttgtag cagctgctaa ttactacttt tattctatgg acgtctgggg ccaagggacc 360
acggtcaccg tctcctca 378

<210> 162
<211> 126
<212> PRT
<213> Artificial Sequence

<220>

<223> Synthetic

<400> 162
```
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Asn Tyr
            20                  25                  30
Gly Ile Ser Trp Val Arg Gln Ala Pro Gly Gln Gly Leu Glu Leu Met
        35                  40                  45
Gly Trp Ile Ser Gly Tyr Asn Gly Asn Thr Asn Tyr Ala Gln Glu Leu
    50                  55                  60
Gln Ala Arg Val Thr Met Thr Thr Asp Thr Ser Thr Ser Thr Ala Tyr
65                  70                  75                  80
Met Glu Leu Arg Asn Leu Arg Ser Asp Asp Thr Ala Val Tyr Tyr Cys
                85                  90                  95
Ala Arg Asp Arg Val Val Val Ala Ala Ala Asn Tyr Tyr Phe Tyr Ser
            100                 105                 110
Met Asp Val Trp Gly Gln Gly Thr Thr Val Thr Val Ser Ser
        115                 120                 125
```

<210> 163
<211> 336
<212> DNA
<213> Artificial Sequence

<220>
<223> Synthetic

<400> 163
```
gatgttgtga tgactcagtc tccactctcc ctgtccgtca cccttggaca gccggcctcc 60
atctcctgca ggtctagtca aagcctcgta tacagtgatg gagacaccta cttgaattgg 120
tttcagcaga ggccaggcca atctccaagg cgcctaattt ataaggtttc taaccgggac 180
tctggggtcc cagacagatt cagcggcagt gggtcaggca ctgctttcac actgaaaatc 240
agcggggtgg aggccgagga tgttggggtt tactactgca tgcaagctac acactggcct 300
cggacgttcg gccaagggac caaggtggaa atcaaa                           336
```

<210> 164
<211> 112
<212> PRT
<213> Artificial Sequence

<220>
<223> Synthetic

<400> 164
```
Asp Val Val Met Thr Gln Ser Pro Leu Ser Leu Ser Val Thr Leu Gly
 1               5                  10                  15
Gln Pro Ala Ser Ile Ser Cys Arg Ser Ser Gln Ser Leu Val Tyr Ser
            20                  25                  30
Asp Gly Asp Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Ala Phe Thr Leu Lys Ile
65                  70                  75                  80
Ser Gly Val Glu Ala Glu Asp Val Gly Val Tyr Tyr Cys Met Gln Ala
                85                  90                  95
Thr His Trp Pro Arg Thr Phe Gly Gln Gly Thr Lys Val Glu Ile Lys
            100                 105                 110
```

<210> 165
<211> 378
<212> DNA

<213> Artificial Sequence

<220>
<223> Synthetic

<400> 165
caggttcagc tggtgcagtc tggagctgag gtgaagaagc ctggggcctc agtgaaggtc 60
tcctgcaagg cttctggtta cacctttacc aactatggta tcagctgggt gcgacaggcc 120
cctggacaag ggcttgagtg gatgggatgg attagtggtt acaatggtaa cacaaactat 180
gcacagaagc tccagggcag agtcaccatg accacagaca catccacgag cacagcctac 240
atggagctga ggagcctgag atctgacgac acggccgtgt attactgtgc gagagataga 300
gtcgttgtag cagctgctaa ttactacttt tattctatgg acgtctgggg ccaagggacc 360
acggtcaccg tctcctca 378

<210> 166
<211> 126
<212> PRT
<213> Artificial Sequence

<220>
<223> Synthetic

<400> 166
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Asn Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Asn Thr Asn Tyr Ala Gln Lys Leu
50 55 60
Gln Gly Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ala Arg Asp Arg Val Val Val Ala Ala Ala Asn Tyr Tyr Phe Tyr Ser
100 105 110
Met Asp Val Trp Gly Gln Gly Thr Thr Val Thr Val Ser Ser
115 120 125

<210> 167
<211> 336
<212> DNA
<213> Artificial Sequence

<220>
<223> Synthetic

<400> 167
gatgttgtga tgactcagtc tccactctcc ctgcccgtca cccttggaca gccggcctcc 60
atctcctgca ggtctagtca aagcctcgta tacagtgatg gagacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaagctac acactggcct 300
cggacgttcg gccaagggac caaggtggaa atcaaa 336

<210> 168
<211> 112
<212> PRT
<213> Artificial Sequence

<220>
<223> Synthetic

<400> 168
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asp Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Thr His Trp Pro Arg Thr Phe Gly Gln Gly Thr Lys Val Glu Ile Lys
            100                 105                 110

<210> 169
<211> 375
<212> DNA
<213> Artificial Sequence

<220>
<223> Synthetic

<400> 169
caggtccact tgaaggagtc tggtcctacg ctggtgaaac ccacacagac cctcacgctg 60
acctgcacct tctctggatt ctcactcatc actagtggag tgggtgtggg ctggattcgt 120
cagccccccg gaaaggccct ggagtggctt gcactcattt attggaatgg tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacagg 300
ataactgaaa ctagttacta cttctactac ggtatggacg tctggggcca agggaccacg 360
gtcaccgtct cctca                                                  375

<210> 170
<211> 125
<212> PRT
<213> Artificial Sequence

<220>
<223> Synthetic

<400> 170
Gln Val His Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ile Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Gly Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile Thr Glu Thr Ser Tyr Tyr Phe Tyr Tyr Gly Met
            100                 105                 110
Asp Val Trp Gly Gln Gly Thr Thr Val Thr Val Ser Ser
        115                 120                 125

<210> 171
<211> 30
<212> DNA
<213> Artificial Sequence

<220>
<223> Synthetic

<400> 171
ggattctcac tcatcactag tggagtgggt 30

<210> 172
<211> 10
<212> PRT
<213> Artificial Sequence

<220>
<223> Synthetic

<400> 172
Gly Phe Ser Leu Ile Thr Ser Gly Val Gly
1 5 10

<210> 173
<211> 21
<212> DNA
<213> Artificial Sequence

<220>
<223> Synthetic

<400> 173
atttattgga atggtgataa g 21

<210> 174
<211> 7
<212> PRT
<213> Artificial Sequence

<220>
<223> Synthetic

<400> 174
Ile Tyr Trp Asn Gly Asp Lys
1 5

<210> 175
<211> 51
<212> DNA
<213> Artificial Sequence

<220>
<223> Synthetic

<400> 175
gcacacagga taactgaaac tagttactac ttctactacg gtatggacgt c 51

<210> 176
<211> 17
<212> PRT
<213> Artificial Sequence

<220>
<223> Synthetic

<400> 176
Ala His Arg Ile Thr Glu Thr Ser Tyr Tyr Phe Tyr Tyr Gly Met Asp

```
 1               5                   10                  15
Val

<210> 177
<211> 339
<212> DNA
<213> Artificial Sequence

<220>
<223> Synthetic

<400> 177
gacatccaga tgacccagtc tccactctcc ctgcccgtca cccctggaga gccggcctcc 60
atctcctgca ggtctagtca gagcctcctg catagtcatg gatacgacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactccg 300
ctcactttcg gcggagggac caaggtggaa atcaaacga                        339

<210> 178
<211> 113
<212> PRT
<213> Artificial Sequence

<220>
<223> Synthetic

<400> 178
Asp Ile Gln Met Thr Gln Ser Pro Leu Ser Leu Pro Val Thr Pro Gly
 1               5                   10                  15
Glu Pro Ala Ser Ile Ser Cys Arg Ser Ser Gln Ser Leu Leu His Ser
            20                  25                  30
His Gly Tyr Asp Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110
Arg

<210> 179
<211> 33
<212> DNA
<213> Artificial Sequence

<220>
<223> Synthetic

<400> 179
cagagcctcc tgcatagtca tggatacgac tat                              33

<210> 180
<211> 11
<212> PRT
<213> Artificial Sequence

<220>
```

<223> Synthetic

<400> 180
Gln Ser Leu Leu His Ser His Gly Tyr Asp Tyr
1 5 10

<210> 181
<211> 9
<212> DNA
<213> Artificial Sequence

<220>
<223> Synthetic

<400> 181
ttgggttct 9

<210> 182
<211> 3
<212> PRT
<213> Artificial Sequence

<220>
<223> Synthetic

<400> 182
Leu Gly Ser
1

<210> 183
<211> 27
<212> DNA
<213> Artificial Sequence

<220>
<223> Synthetic

<400> 183
atgcaagctc tacaaactcc gctcact 27

<210> 184
<211> 9
<212> PRT
<213> Artificial Sequence

<220>
<223> Synthetic

<400> 184
Met Gln Ala Leu Gln Thr Pro Leu Thr
1 5

<210> 185
<211> 375
<212> DNA
<213> Artificial Sequence

<220>
<223> Synthetic

<400> 185
cagatcacct tgaaggagtc tggtcctacg ctggtgaaac ccacacagac cctcacgctg 60

acctgcacct tctctggatt ctcactcatc actagtggag tgggtgtggg ctggattcgt 120
cagcccccg gaaaggccct ggagtggctt gcactcattt attggaatgg tgataagcgc 180
tacagcccat ctctgaagag caggctcacc atcaccaagg acacctccaa aaccaggtg 240
gtccttacaa tgaccaacat ggaccctgtg gacacagcca catattactg tgcacacagg 300
ataactgaaa ctagttacta cttctactac ggtatggacg tctggggcca agggaccacg 360
gtcaccgtct cctca 375

<210> 186
<211> 125
<212> PRT
<213> Artificial Sequence

<220>
<223> Synthetic

<400> 186
Gln Ile Thr Leu Lys Glu Ser Gly Pro Thr Leu Val Lys Pro Thr Gln
1 5 10 15
Thr Leu Thr Leu Thr Cys Thr Phe Ser Gly Phe Ser Leu Ile Thr Ser
20 25 30
Gly Val Gly Val Gly Trp Ile Arg Gln Pro Pro Gly Lys Ala Leu Glu
35 40 45
Trp Leu Ala Leu Ile Tyr Trp Asn Gly Asp Lys Arg Tyr Ser Pro Ser
50 55 60
Leu Lys Ser Arg Leu Thr Ile Thr Lys Asp Thr Ser Lys Asn Gln Val
65 70 75 80
Val Leu Thr Met Thr Asn Met Asp Pro Val Asp Thr Ala Thr Tyr Tyr
85 90 95
Cys Ala His Arg Ile Thr Glu Thr Ser Tyr Tyr Phe Tyr Tyr Gly Met
100 105 110
Asp Val Trp Gly Gln Gly Thr Thr Val Thr Val Ser Ser
115 120 125

<210> 187
<211> 336
<212> DNA
<213> Artificial Sequence

<220>
<223> Synthetic

<400> 187
gatattgtga tgactcagtc tccactctcc ctgcccgtca cccctggaga gccggcctcc 60
atctcctgca ggtctagtca gagcctcctg catagtcatg gatacgacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactccg 300
ctcactttcg gcggagggac caaggtggag atcaaa 336

<210> 188
<211> 112
<212> PRT
<213> Artificial Sequence

<220>
<223> Synthetic

<400> 188
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
His Gly Tyr Asp Tyr Leu Asp Trp Tyr Leu Gln Lys Pro Gly Gln Ser
35 40 45

```
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110
```

<210> 189
<211> 375
<212> DNA
<213> Artificial Sequence

<220>
<223> Synthetic

<400> 189

```
cagatcacct tgaaggagtc tggtcctacg ctggtgaaac ccacacagac cctcacgctg 60
acctgcacct tctctggatt ctcactcatc actagtggag tgggtgtggg ctggatccgt 120
cagcccccag gaaaggccct ggagtggctt gcactcattt attggaatgg tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacagg 300
ataactgaaa ctagttacta cttctactac ggtatggacg tctggggcca agggaccacg 360
gtcaccgtct cctca                                                  375
```

<210> 190
<211> 125
<212> PRT
<213> Artificial Sequence

<220>
<223> Synthetic

<400> 190

```
Gln Ile Thr Leu Lys Glu Ser Gly Pro Thr Leu Val Lys Pro Thr Gln
 1               5                  10                  15
Thr Leu Thr Leu Thr Cys Thr Phe Ser Gly Phe Ser Leu Ile Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Gly Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile Thr Glu Thr Ser Tyr Tyr Phe Tyr Tyr Gly Met
            100                 105                 110
Asp Val Trp Gly Gln Gly Thr Thr Val Thr Val Ser Ser
        115                 120                 125
```

<210> 191
<211> 336
<212> DNA
<213> Artificial Sequence

<220>
<223> Synthetic

<400> 191

```
gatattgtga tgactcagtc tccactctcc ctgcccgtca cccctggaga gccggcctcc 60
atctcctgca ggtctagtca gagcctcctg catagtcatg gatacgacta tttggattgg 120
```

tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactccg 300
ctcactttcg gcggagggac caaggtggag atcaaa 336

<210> 192
<211> 112
<212> PRT
<213> Artificial Sequence

<220>
<223> Synthetic

<400> 192
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
His Gly Tyr Asp Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Leu Thr Phe Gly Gly Gly Thr Lys Val Glu Ile Lys
100 105 110

<210> 193
<211> 375
<212> DNA
<213> Artificial Sequence

<220>
<223> Synthetic

<400> 193
cagatcacct tgaaggagtc tggtcctact ctggtgaaac cctcacagac cctcacgctg 60
acctgcacct tctctgggtt ctcactcagc actagtggag tgggtgtggg ctggatccgt 120
cagcccccag gaaaggccct ggagtggctt gcactcattt attggaattc tgataagcgc 180
tacagcccat ctctgaagag caggctcacc atcaccaagg acacctccaa aaaccaggta 240
gtccttacaa tgaccaacat ggaccctgtg gacacagcca catattactg tgcacacaga 300
catgacagct cgtcctacta cttctactac ggtatggacg tctggggcca agggatcacg 360
gtcaccgtct cctca 375

<210> 194
<211> 125
<212> PRT
<213> Artificial Sequence

<220>
<223> Synthetic

<400> 194
Gln Ile Thr Leu Lys Glu Ser Gly Pro Thr Leu Val Lys Pro Ser Gln
1 5 10 15
Thr Leu Thr Leu Thr Cys Thr Phe Ser Gly Phe Ser Leu Ser Thr Ser
20 25 30
Gly Val Gly Val Gly Trp Ile Arg Gln Pro Pro Gly Lys Ala Leu Glu
35 40 45
Trp Leu Ala Leu Ile Tyr Trp Asn Ser Asp Lys Arg Tyr Ser Pro Ser
50 55 60
Leu Lys Ser Arg Leu Thr Ile Thr Lys Asp Thr Ser Lys Asn Gln Val

```
65                  70                  75                  80
Val Leu Thr Met Thr Asn Met Asp Pro Val Asp Thr Ala Thr Tyr Tyr
                85                  90                  95
Cys Ala His Arg His Asp Ser Ser Ser Tyr Tyr Phe Tyr Tyr Gly Met
            100                 105                 110
Asp Val Trp Gly Gln Gly Ile Thr Val Thr Val Ser Ser
        115                 120                 125
```

<210> 195
<211> 30
<212> DNA
<213> Artificial Sequence

<220>
<223> Synthetic

<400> 195
gggttctcac tcagcactag tggagtgggt 30

<210> 196
<211> 10
<212> PRT
<213> Artificial Sequence

<220>
<223> Synthetic

<400> 196

```
Gly Phe Ser Leu Ser Thr Ser Gly Val Gly
 1               5                  10
```

<210> 197
<211> 21
<212> DNA
<213> Artificial Sequence

<220>
<223> Synthetic

<400> 197
atttattgga attctgataa g 21

<210> 198
<211> 7
<212> PRT
<213> Artificial Sequence

<220>
<223> Synthetic

<400> 198

```
Ile Tyr Trp Asn Ser Asp Lys
 1               5
```

<210> 199
<211> 51
<212> DNA
<213> Artificial Sequence

<220>
<223> Synthetic

<400> 199
gcacacagac atgacagctc gtcctactac ttctactacg gtatggacgt c 51

<210> 200
<211> 17
<212> PRT
<213> Artificial Sequence

<220>
<223> Synthetic

<400> 200
Ala His Arg His Asp Ser Ser Ser Tyr Tyr Phe Tyr Tyr Gly Met Asp
1 5 10 15
Val

<210> 201
<211> 339
<212> DNA
<213> Artificial Sequence

<220>
<223> Synthetic

<400> 201
gacatccaga tgacccagtc tccgctctcc ctgcccgtca cccctggaga gccggcctcc 60
atctcctgca ggtctagtca gagcctcctc catagtcatg gatacaacta tttggattgg 120
tacctgcaga agccagggca gtctccacaa ctcctgatct atttgggttc taatcgggcc 180
tccggggtcc ctgacaggtt cagtggcggt ggatcaggca cagattttac actgaaaatc 240
agcagagtgg aggctgagga tgttgggatt tattactgca tgcaagctct acagactcct 300
ctcactttcg gcggagggac caaggtggag atcaaacga 339

<210> 202
<211> 113
<212> PRT
<213> Artificial Sequence

<220>
<223> Synthetic

<400> 202
Asp Ile Gln Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
His Gly Tyr Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Gly Gly Ser Gly Thr Asp Phe Thr Leu Lys Ile
65 70 75 80
Ser Arg Val Glu Ala Glu Asp Val Gly Ile Tyr Tyr Cys Met Gln Ala
85 90 95
Leu Gln Thr Pro Leu Thr Phe Gly Gly Gly Thr Lys Val Glu Ile Lys
100 105 110
Arg

<210> 203
<211> 33
<212> DNA
<213> Artificial Sequence

<220>
<223> Synthetic

<400> 203
cagagcctcc tccatagtca tggatacaac tat 33

<210> 204
<211> 11
<212> PRT
<213> Artificial Sequence

<220>
<223> Synthetic

<400> 204
Gln Ser Leu Leu His Ser His Gly Tyr Asn Tyr
1 5 10

<210> 205
<211> 9
<212> DNA
<213> Artificial Sequence

<220>
<223> Synthetic

<400> 205
ttgggttct 9

<210> 206
<211> 3
<212> PRT
<213> Artificial Sequence

<220>
<223> Synthetic

<400> 206
Leu Gly Ser
1

<210> 207
<211> 27
<212> DNA
<213> Artificial Sequence

<220>
<223> Synthetic

<400> 207
atgcaagctc tacagactcc tctcact 27

<210> 208
<211> 9
<212> PRT
<213> Artificial Sequence

<220>
<223> Synthetic

<400> 208
Met Gln Ala Leu Gln Thr Pro Leu Thr

1               5

<210> 209
<211> 375
<212> DNA
<213> Artificial Sequence

<220>
<223> Synthetic

<400> 209
cagatcacct tgaaggagtc tggtcctact ctggtgaaac cctcacagac cctcacgctg 60
acctgcacct tctctgggtt ctcactcagc actagtggag tgggtgtggg ctggatccgt 120
cagcccccag gaaaggccct ggagtggctt gcactcattt attggaattc tgataagcgc 180
tacagcccat ctctgaagag caggctcacc atcaccaagg acacctccaa aaaccaggta 240
gtccttacaa tgaccaacat ggaccctgtg gacacagcca catattactg tgcacacaga 300
catgacagct cgtcctacta cttctactac ggtatggacg tctggggcca agggaccacg 360
gtcaccgtct cctca 375

<210> 210
<211> 125
<212> PRT
<213> Artificial Sequence

<220>
<223> Synthetic

<400> 210
Gln Ile Thr Leu Lys Glu Ser Gly Pro Thr Leu Val Lys Pro Ser Gln
1               5                   10                  15
Thr Leu Thr Leu Thr Cys Thr Phe Ser Gly Phe Ser Leu Ser Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Ser Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg His Asp Ser Ser Ser Tyr Tyr Phe Tyr Tyr Gly Met
            100                 105                 110
Asp Val Trp Gly Gln Gly Thr Thr Val Thr Val Ser Ser
        115                 120                 125

<210> 211
<211> 336
<212> DNA
<213> Artificial Sequence

<220>
<223> Synthetic

<400> 211
gatattgtga tgactcagtc tccgctctcc ctgcccgtca cccctggaga gccggcctcc 60
atctcctgca ggtctagtca gagcctcctc catagtcatg gatacaacta tttggattgg 120
tacctgcaga agccagggca gtctccacaa ctcctgatct atttgggttc taatcgggcc 180
tccggggtcc ctgacaggtt cagtggcggt ggatcaggca cagattttac actgaaaatc 240
agcagagtgg aggctgagga tgttgggatt tattactgca tgcaagctct acagactcct 300
ctcactttcg gcggagggac caaggtggag atcaaa 336

<210> 212
<211> 112

<212> PRT
<213> Artificial Sequence

<220>
<223> Synthetic

<400> 212
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
His Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Gly Gly Ser Gly Thr Asp Phe Thr Leu Lys Ile
65                  70                  75                  80
Ser Arg Val Glu Ala Glu Asp Val Gly Ile Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 213
<211> 375
<212> DNA
<213> Artificial Sequence

<220>
<223> Synthetic

<400> 213
cagatcacct tgaaggagtc tggtcctacg ctggtgaaac ccacacagac cctcacgctg 60
acctgcacct tctctgggtt ctcactcagc actagtggag tgggtgtggg ctggatccgt 120
cagcccccag gaaaggccct ggagtggctt gcactcattt attggaattc tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacaga 300
catgacagct cgtcctacta cttctactac ggtatggacg tctggggcca agggaccacg 360
gtcaccgtct cctca 375

<210> 214
<211> 125
<212> PRT
<213> Artificial Sequence

<220>
<223> Synthetic

<400> 214
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Ser Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg His Asp Ser Ser Ser Tyr Tyr Phe Tyr Tyr Gly Met
            100                 105                 110
Asp Val Trp Gly Gln Gly Thr Thr Val Thr Val Ser Ser
        115                 120                 125

<210> 215
<211> 336
<212> DNA
<213> Artificial Sequence

<220>
<223> Synthetic

<400> 215
gatattgtga tgactcagtc tccactctcc ctgcccgtca cccctggaga gccggcctcc 60
atctcctgca ggtctagtca gagcctcctc catagtcatg gatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acagactcct 300
ctcactttcg gcggagggac caaggtggag atcaaa 336

<210> 216
<211> 112
<212> PRT
<213> Artificial Sequence

<220>
<223> Synthetic

<400> 216
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
His Gly Tyr Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Leu Thr Phe Gly Gly Gly Thr Lys Val Glu Ile Lys
100 105 110

<210> 217
<211> 381
<212> DNA
<213> Artificial Sequence

<220>
<223> Synthetic

<400> 217
gagatgcaac tggtggagtc tgggggaggc ttggtccagc ctggggggtc cctgagactc 60
tcctgtgcag cctctggatt cacctttagt agtcactgga tgaagtgggt ccgccaggct 120
ccagggaagg ggctggagtg ggtggccaac ataaaccaag atggaagtga gaaatactat 180
gtggactctg tgaagggccg attcaccatc tccagagaca acgccaagaa ctcactgttt 240
ctgcaaatga acagcctgag agccgaggac acggctgtgt attactgtgc gagagatatt 300
gtactaatgg tctatgatat ggactactac tactacggta tggacgtctg gggccaaggg 360
accacggtca ccgtctcctc a 381

<210> 218
<211> 127
<212> PRT
<213> Artificial Sequence

<220>
<223> Synthetic

<400> 218
Glu Met Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser His
20 25 30
Trp Met Lys Trp Val Arg Gln Ala Pro Gly Lys Gly Leu Glu Trp Val
35 40 45
Ala Asn Ile Asn Gln Asp Gly Ser Glu Lys Tyr Tyr Val Asp Ser Val
50 55 60
Lys Gly Arg Phe Thr Ile Ser Arg Asp Asn Ala Lys Asn Ser Leu Phe
65 70 75 80
Leu Gln Met Asn Ser Leu Arg Ala Glu Asp Thr Ala Val Tyr Tyr Cys
85 90 95
Ala Arg Asp Ile Val Leu Met Val Tyr Asp Met Asp Tyr Tyr Tyr Tyr
100 105 110
Gly Met Asp Val Trp Gly Gln Gly Thr Thr Val Thr Val Ser Ser
115 120 125

<210> 219
<211> 24
<212> DNA
<213> Artificial Sequence

<220>
<223> Synthetic

<400> 219
ggattcacct ttagtagtca ctgg 24

<210> 220
<211> 8
<212> PRT
<213> Artificial Sequence

<220>
<223> Synthetic

<400> 220
Gly Phe Thr Phe Ser Ser His Trp
1 5

<210> 221
<211> 24
<212> DNA
<213> Artificial Sequence

<220>
<223> Synthetic

<400> 221
ataaaccaag atggaagtga gaaa 24

<210> 222
<211> 8
<212> PRT
<213> Artificial Sequence

<220>
<223> Synthetic

<400> 222
Ile Asn Gln Asp Gly Ser Glu Lys
1 5

<210> 223
<211> 60
<212> DNA
<213> Artificial Sequence

<220>
<223> Synthetic

<400> 223
gcgagagata ttgtactaat ggtctatgat atggactact actactacgg tatggacgtc 60

<210> 224
<211> 20
<212> PRT
<213> Artificial Sequence

<220>
<223> Synthetic

<400> 224
Ala Arg Asp Ile Val Leu Met Val Tyr Asp Met Asp Tyr Tyr Tyr Tyr
1 5 10 15
Gly Met Asp Val
20

<210> 225
<211> 336
<212> DNA
<213> Artificial Sequence

<220>
<223> Synthetic

<400> 225
gatattgtga tgactcagtc tccactctcc ctgcccgtca cccctggaga gccggcctcc 60
atctcctgca ggtctagtca gagcctcctg catagtaatg gaaacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaaactct acaaactccg 300
ctcactttcg gcggagggac caaggtggag atcaaa 336

<210> 226
<211> 112
<212> PRT
<213> Artificial Sequence

<220>
<223> Synthetic

<400> 226
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
Asn Gly Asn Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60

Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Thr
85 90 95
Leu Gln Thr Pro Leu Thr Phe Gly Gly Gly Thr Lys Val Glu Ile Lys
100 105 110

<210> 227
<211> 33
<212> DNA
<213> Artificial Sequence

<220>
<223> Synthetic

<400> 227
cagagcctcc tgcatagtaa tggaaacaac tat 33

<210> 228
<211> 11
<212> PRT
<213> Artificial Sequence

<220>
<223> Synthetic

<400> 228
Ser Ser Gln Ser Leu Leu His Ser Asn Gly Asn
1 5 10

<210> 229
<211> 9
<212> DNA
<213> Artificial Sequence

<220>
<223> Synthetic

<400> 229
ttgggttct 9

<210> 230
<211> 3
<212> PRT
<213> Artificial Sequence

<220>
<223> Synthetic

<400> 230
Leu Gly Ser
1

<210> 231
<211> 27
<212> DNA
<213> Artificial Sequence

<220>
<223> Synthetic

<400> 231

atgcaaactc tacaaactcc gctcact 27

<210> 232
<211> 9
<212> PRT
<213> Artificial Sequence

<220>
<223> Synthetic

<400> 232
Met Gln Thr Leu Gln Thr Pro Leu Thr
1 5

<210> 233
<211> 381
<212> DNA
<213> Artificial Sequence

<220>
<223> Synthetic

<400> 233
gaggtgcagc tggtggagtc tgggggaggc ttggtccagc ctggggggtc cctgagactc 60
tcctgtgcag cctctggatt cacctttagt agtcactgga tgaagtgggt ccgccaggct 120
ccagggaagg ggctggagtg ggtggccaac ataaaccaag atggaagtga gaaatactat 180
gtggactctg tgaagggccg attcaccatc tccagagaca acgccaagaa ctcactgttt 240
ctgcaaatga acagcctgag agccgaggac acggctgtgt attactgtgc gagagatatt 300
gtactaatgg tctatgatat ggactactac tactacggta tggacgtctg gggccaaggg 360
accacggtca ccgtctcctc a 381

<210> 234
<211> 127
<212> PRT
<213> Artificial Sequence

<220>
<223> Synthetic

<400> 234
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser His
20 25 30
Trp Met Lys Trp Val Arg Gln Ala Pro Gly Lys Gly Leu Glu Trp Val
35 40 45
Ala Asn Ile Asn Gln Asp Gly Ser Glu Lys Tyr Tyr Val Asp Ser Val
50 55 60
Lys Gly Arg Phe Thr Ile Ser Arg Asp Asn Ala Lys Asn Ser Leu Phe
65 70 75 80
Leu Gln Met Asn Ser Leu Arg Ala Glu Asp Thr Ala Val Tyr Tyr Cys
85 90 95
Ala Arg Asp Ile Val Leu Met Val Tyr Asp Met Asp Tyr Tyr Tyr Tyr
100 105 110
Gly Met Asp Val Trp Gly Gln Gly Thr Thr Val Thr Val Ser Ser
115 120 125

<210> 235
<211> 336
<212> DNA
<213> Artificial Sequence

<220>

<223> Synthetic

<400> 235
gatattgtga tgactcagtc tccactctcc ctgcccgtca cccctggaga gccggcctcc 60
atctcctgca ggtctagtca gagcctcctg catagtaatg gaaacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaaactct acaaactccg 300
ctcactttcg gcggagggac caaggtggag atcaaa 336

<210> 236
<211> 112
<212> PRT
<213> Artificial Sequence

<220>
<223> Synthetic

<400> 236
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
Asn Gly Asn Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Thr
85 90 95
Leu Gln Thr Pro Leu Thr Phe Gly Gly Gly Thr Lys Val Glu Ile Lys
100 105 110

<210> 237
<211> 381
<212> DNA
<213> Artificial Sequence

<220>
<223> Synthetic

<400> 237
gaggtgcagc tggtggagtc tgggggaggc ttggtccagc ctggggggtc cctgagactc 60
tcctgtgcag cctctggatt cacctttagt agtcactgga tgagctgggt ccgccaggct 120
ccagggaagg ggctggagtg ggtggccaac ataaaccaag atggaagtga gaaatactat 180
gtggactctg tgaagggccg attcaccatc tccagagaca acgccaagaa ctcactgtat 240
ctgcaaatga acagcctgag agccgaggac acggctgtgt attactgtgc gagagatatt 300
gtactaatgg tctatgatat ggactactac tactacggta tggacgtctg gggcaaggg 360
accacggtca ccgtctcctc a 381

<210> 238
<211> 127
<212> PRT
<213> Artificial Sequence

<220>
<223> Synthetic

<400> 238
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser His
20 25 30

```
Trp Met Ser Trp Val Arg Gln Ala Pro Gly Lys Gly Leu Glu Trp Val
        35                  40                  45
Ala Asn Ile Asn Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Ile Val Leu Met Val Tyr Asp Met Asp Tyr Tyr Tyr Tyr
            100                 105                 110
Gly Met Asp Val Trp Gly Gln Gly Thr Thr Val Thr Val Ser Ser
        115                 120                 125


<210> 239
<211> 336
<212> DNA
<213> Artificial Sequence

<220>
<223> Synthetic

<400> 239
gatattgtga tgactcagtc tccactctcc ctgcccgtca cccctggaga gccggcctcc 60
atctcctgca ggtctagtca gagcctcctg catagtaatg gaaacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaaactct acaaactccg 300
ctcactttcg gcggagggac caaggtggag atcaaa                           336

<210> 240
<211> 112
<212> PRT
<213> Artificial Sequence

<220>
<223> Synthetic

<400> 240
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Asn Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Thr
                85                  90                  95
Leu Gln Thr Pro Leu Thr Phe Gly Gly Gly Thr Lys Val Glu Ile Lys
            100                 105                 110


<210> 241
<211> 381
<212> DNA
<213> Artificial Sequence

<220>
<223> Synthetic

<400> 241
caggtgcagc tggtggagtc tgggggaggc gtggtccagc ctgggaggtc cctgagactc 60
```

tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg ggccaaggg 360
accacggtca ccgtctcctc a 381

<210> 242
<211> 127
<212> PRT
<213> Artificial Sequence

<220>
<223> Synthetic

<400> 242
Gln Val Gln Leu Val Glu Ser Gly Gly Gly Val Val Gln Pro Gly Arg
1 5 10 15
Ser Leu Arg Leu Ser Cys Ala Val Ser Gly Phe Thr Phe Ser Ser Tyr
20 25 30
Gly Met His Trp Val Arg Gln Ala Pro Gly Lys Gly Leu Glu Trp Val
35 40 45
Ala Ala Ile Ser Tyr Asp Gly Ser Asn Lys Tyr Tyr Val Asp Ser Val
50 55 60
Lys Gly Arg Phe Thr Ile Ser Arg Asp Asn Ser Lys Lys Thr Leu Tyr
65 70 75 80
Leu Gln Met Asn Ser Leu Arg Ala Glu Asp Thr Ala Val Tyr Asn Cys
85 90 95
Ala Lys Asn Ile Val Leu Val Met Tyr Asp Ile Asp Tyr His Tyr Tyr
100 105 110
Gly Met Asp Val Trp Gly Gln Gly Thr Thr Val Thr Val Ser Ser
115 120 125

<210> 243
<211> 24
<212> DNA
<213> Artificial Sequence

<220>
<223> Synthetic

<400> 243
ggattcacct tcagtagcta tggc 24

<210> 244
<211> 8
<212> PRT
<213> Artificial Sequence

<220>
<223> Synthetic

<400> 244
Gly Phe Thr Phe Ser Ser Tyr Gly
1 5

<210> 245
<211> 24
<212> DNA
<213> Artificial Sequence

<220>
<223> Synthetic

<400> 245
atatcatatg atggaagtaa taaa 24

<210> 246
<211> 8
<212> PRT
<213> Artificial Sequence

<220>
<223> Synthetic

<400> 246
Ile Ser Tyr Asp Gly Ser Asn Lys
1 5

<210> 247
<211> 60
<212> DNA
<213> Artificial Sequence

<220>
<223> Synthetic

<400> 247
gcgaaaaata ttgtactagt gatgtatgat atagactatc actactatgg gatggacgtc 60

<210> 248
<211> 20
<212> PRT
<213> Artificial Sequence

<220>
<223> Synthetic

<400> 248
Ala Lys Asn Ile Val Leu Val Met Tyr Asp Ile Asp Tyr His Tyr Tyr
1 5 10 15
Gly Met Asp Val
20

<210> 249
<211> 336
<212> DNA
<213> Artificial Sequence

<220>
<223> Synthetic

<400> 249
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaga 336

<210> 250
<211> 112
<212> PRT
<213> Artificial Sequence

<220>
<223> Synthetic

<400> 250
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Arg
            100                 105                 110

<210> 251
<211> 33
<212> DNA
<213> Artificial Sequence

<220>
<223> Synthetic

<400> 251
cagagcctcc tgcatagtaa tggatacaac tat 33

<210> 252
<211> 11
<212> PRT
<213> Artificial Sequence

<220>
<223> Synthetic

<400> 252
Gln Ser Leu Leu His Ser Asn Gly Tyr Asn Tyr
1               5                   10

<210> 253
<211> 9
<212> DNA
<213> Artificial Sequence

<220>
<223> Synthetic

<400> 253
ttgggtttt 9

<210> 254
<211> 3
<212> PRT
<213> Artificial Sequence

<220>
<223> Synthetic

<400> 254
Leu Gly Phe

1

<210> 255
<211> 27
<212> DNA
<213> Artificial Sequence

<220>
<223> Synthetic

<400> 255
atgcaagctc tacaaactcc tctcact 27

<210> 256
<211> 9
<212> PRT
<213> Artificial Sequence

<220>
<223> Synthetic

<400> 256
Met Gln Ala Leu Gln Thr Pro Leu Thr
1 5

<210> 257
<211> 381
<212> DNA
<213> Artificial Sequence

<220>
<223> Synthetic

<400> 257
caggtgcagc tggtggagtc tgggggaggc gtggtccagc ctgggaggtc cctgagactc 60
tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg gggccaaggg 360
accacggtca ccgtctcctc a 381

<210> 258
<211> 127
<212> PRT
<213> Artificial Sequence

<220>
<223> Synthetic

<400> 258
Gln Val Gln Leu Val Glu Ser Gly Gly Gly Val Val Gln Pro Gly Arg
1 5 10 15
Ser Leu Arg Leu Ser Cys Ala Val Ser Gly Phe Thr Phe Ser Ser Tyr
20 25 30
Gly Met His Trp Val Arg Gln Ala Pro Gly Lys Gly Leu Glu Trp Val
35 40 45
Ala Ala Ile Ser Tyr Asp Gly Ser Asn Lys Tyr Tyr Val Asp Ser Val
50 55 60
Lys Gly Arg Phe Thr Ile Ser Arg Asp Asn Ser Lys Lys Thr Leu Tyr
65 70 75 80
Leu Gln Met Asn Ser Leu Arg Ala Glu Asp Thr Ala Val Tyr Asn Cys
85 90 95

Ala Lys Asn Ile Val Leu Val Met Tyr Asp Ile Asp Tyr His Tyr Tyr
100 105 110
Gly Met Asp Val Trp Gly Gln Gly Thr Thr Val Thr Val Ser Ser
115 120 125

<210> 259
<211> 336
<212> DNA
<213> Artificial Sequence

<220>
<223> Synthetic

<400> 259
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa 336

<210> 260
<211> 112
<212> PRT
<213> Artificial Sequence

<220>
<223> Synthetic

<400> 260
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
Asn Gly Tyr Asn Tyr Leu Asp Trp Tyr Leu Gln Lys Pro Gly Gln Ser
35 40 45
Pro Gln Leu Leu Ile Tyr Leu Gly Phe Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Leu Thr Phe Gly Gly Gly Thr Lys Val Glu Ile Lys
100 105 110

<210> 261
<211> 381
<212> DNA
<213> Artificial Sequence

<220>
<223> Synthetic

<400> 261
caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt atatcatatg atggaagtaa taaatactat 180
gcagactccg tgaagggccg attcaccatc tccagagaca attccaagaa cacgctgtat 240
ctgcaaatga acagcctgag agctgaggac acggctgtgt attactgtgc gaaaaatatt 300
gtactagtga tgtatgatat agactatcac tactatggga tggacgtctg gggcaaggg 360
accacggtca ccgtctcctc a 381

<210> 262

<211> 127
<212> PRT
<213> Artificial Sequence

<220>
<223> Synthetic

<400> 262
Gln Val Gln Leu Val Glu Ser Gly Gly Gly Val Val Gln Pro Gly Arg
1 5 10 15
Ser Leu Arg Leu Ser Cys Ala Ala Ser Gly Phe Thr Phe Ser Ser Tyr
20 25 30
Gly Met His Trp Val Arg Gln Ala Pro Gly Lys Gly Leu Glu Trp Val
35 40 45
Ala Val Ile Ser Tyr Asp Gly Ser Asn Lys Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Lys Asn Ile Val Leu Val Met Tyr Asp Ile Asp Tyr His Tyr Tyr
100 105 110
Gly Met Asp Val Trp Gly Gln Gly Thr Thr Val Thr Val Ser Ser
115 120 125

<210> 263
<211> 336
<212> DNA
<213> Artificial Sequence

<220>
<223> Synthetic

<400> 263
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa 336

<210> 264
<211> 112
<212> PRT
<213> Artificial Sequence

<220>
<223> Synthetic

<400> 264
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
Asn Gly Tyr Asn Tyr Leu Asp Trp Tyr Leu Gln Lys Pro Gly Gln Ser
35 40 45
Pro Gln Leu Leu Ile Tyr Leu Gly Phe Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Leu Thr Phe Gly Gly Gly Thr Lys Val Glu Ile Lys
100 105 110

<210> 265
<211> 381
<212> DNA
<213> Artificial Sequence

<220>
<223> Synthetic

<400> 265
caggtgcagc tggtggagtc tgggggaggc gtggtccagc ctgggaggtc cctgagactc 60
tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg gggccaaggg 360
accacggtca ccgtctcctc a 381

<210> 266
<211> 127
<212> PRT
<213> Artificial Sequence

<220>
<223> Synthetic

<400> 266
Gln Val Gln Leu Val Glu Ser Gly Gly Gly Val Val Gln Pro Gly Arg
1 5 10 15
Ser Leu Arg Leu Ser Cys Ala Val Ser Gly Phe Thr Phe Ser Ser Tyr
20 25 30
Gly Met His Trp Val Arg Gln Ala Pro Gly Lys Gly Leu Glu Trp Val
35 40 45
Ala Ala Ile Ser Tyr Asp Gly Ser Asn Lys Tyr Tyr Val Asp Ser Val
50 55 60
Lys Gly Arg Phe Thr Ile Ser Arg Asp Asn Ser Lys Lys Thr Leu Tyr
65 70 75 80
Leu Gln Met Asn Ser Leu Arg Ala Glu Asp Thr Ala Val Tyr Asn Cys
85 90 95
Ala Lys Asn Ile Val Leu Val Met Tyr Asp Ile Asp Tyr His Tyr Tyr
100 105 110
Gly Met Asp Val Trp Gly Gln Gly Thr Thr Val Thr Val Ser Ser
115 120 125

<210> 267
<211> 24
<212> DNA
<213> Artificial Sequence

<220>
<223> Synthetic

<400> 267
ggattcacct tcagtagcta tggc 24

<210> 268
<211> 8
<212> PRT
<213> Artificial Sequence

<220>
<223> Synthetic

<400> 268
Gly Phe Thr Phe Ser Ser Tyr Gly
 1               5

<210> 269
<211> 24
<212> DNA
<213> Artificial Sequence

<220>
<223> Synthetic

<400> 269
atatcatatg atggaagtaa taaa                                        24

<210> 270
<211> 8
<212> PRT
<213> Artificial Sequence

<220>
<223> Synthetic

<400> 270
Ile Ser Tyr Asp Gly Ser Asn Lys
 1               5

<210> 271
<211> 60
<212> DNA
<213> Artificial Sequence

<220>
<223> Synthetic

<400> 271
gcgaaaaata ttgtactagt gatgtatgat atagactatc actactatgg gatggacgtc 60

<210> 272
<211> 20
<212> PRT
<213> Artificial Sequence

<220>
<223> Synthetic

<400> 272
Ala Lys Asn Ile Val Leu Val Met Tyr Asp Ile Asp Tyr His Tyr Tyr
 1               5                  10                  15
Gly Met Asp Val
            20

<210> 273
<211> 336
<212> DNA
<213> Artificial Sequence

<220>
<223> Synthetic

<400> 273

```
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaga                           336

<210> 274
<211> 112
<212> PRT
<213> Artificial Sequence

<220>
<223> Synthetic

<400> 274
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Arg
            100                 105                 110


<210> 275
<211> 33
<212> DNA
<213> Artificial Sequence

<220>
<223> Synthetic

<400> 275
cagagcctcc tgcatagtaa tggatacaac tat                              33

<210> 276
<211> 11
<212> PRT
<213> Artificial Sequence

<220>
<223> Synthetic

<400> 276
Gln Ser Leu Leu His Ser Asn Gly Tyr Asn Tyr
1               5                   10


<210> 277
<211> 9
<212> DNA
<213> Artificial Sequence

<220>
<223> Synthetic

<400> 277
```

ttgggtttt 9

<210> 278
<211> 3
<212> PRT
<213> Artificial Sequence

<220>
<223> Synthetic

<400> 278
Leu Gly Phe
1

<210> 279
<211> 27
<212> DNA
<213> Artificial Sequence

<220>
<223> Synthetic

<400> 279
atgcaagctc tacaaactcc tctcact 27

<210> 280
<211> 9
<212> PRT
<213> Artificial Sequence

<220>
<223> Synthetic

<400> 280
Met Gln Ala Leu Gln Thr Pro Leu Thr
1 5

<210> 281
<211> 381
<212> DNA
<213> Artificial Sequence

<220>
<223> Synthetic

<400> 281
caggtgcagc tggtggagtc tgggggaggc gtggtccagc ctgggaggtc cctgagactc 60
tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg gggccaaggg 360
accacggtca ccgtctcctc a 381

<210> 282
<211> 127
<212> PRT
<213> Artificial Sequence

<220>
<223> Synthetic

<400> 282

```
Gln Val Gln Leu Val Glu Ser Gly Gly Gly Val Val Gln Pro Gly Arg
1               5                   10                  15
Ser Leu Arg Leu Ser Cys Ala Val Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Ala Ile Ser Tyr Asp Gly Ser Asn Lys Tyr Tyr Val Asp Ser Val
    50                  55                  60
Lys Gly Arg Phe Thr Ile Ser Arg Asp Asn Ser Lys Lys Thr Leu Tyr
65                  70                  75                  80
Leu Gln Met Asn Ser Leu Arg Ala Glu Asp Thr Ala Val Tyr Asn Cys
                85                  90                  95
Ala Lys Asn Ile Val Leu Val Met Tyr Asp Ile Asp Tyr His Tyr Tyr
            100                 105                 110
Gly Met Asp Val Trp Gly Gln Gly Thr Thr Val Thr Val Ser Ser
        115                 120                 125
```

<210> 283
<211> 336
<212> DNA
<213> Artificial Sequence

<220>
<223> Synthetic

<400> 283

```
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa                           336
```

<210> 284
<211> 112
<212> PRT
<213> Artificial Sequence

<220>
<223> Synthetic

<400> 284

```
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110
```

<210> 285
<211> 381
<212> DNA
<213> Artificial Sequence

<220>

<223> Synthetic

<400> 285
caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt atatcatatg atggaagtaa taaatactat 180
gcagactccg tgaagggccg attcaccatc tccagagaca attccaagaa cacgctgtat 240
ctgcaaatga acagcctgag agctgaggac acggctgtgt attactgtgc gaaaaatatt 300
gtactagtga tgtatgatat agactatcac tactatggga tggacgtctg gggcaaggg 360
accacggtca ccgtctcctc a 381

<210> 286
<211> 127
<212> PRT
<213> Artificial Sequence

<220>
<223> Synthetic

<400> 286
Gln Val Gln Leu Val Glu Ser Gly Gly Gly Val Val Gln Pro Gly Arg
1 5 10 15
Ser Leu Arg Leu Ser Cys Ala Ala Ser Gly Phe Thr Phe Ser Ser Tyr
20 25 30
Gly Met His Trp Val Arg Gln Ala Pro Gly Lys Gly Leu Glu Trp Val
35 40 45
Ala Val Ile Ser Tyr Asp Gly Ser Asn Lys Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Lys Asn Ile Val Leu Val Met Tyr Asp Ile Asp Tyr His Tyr Tyr
100 105 110
Gly Met Asp Val Trp Gly Gln Gly Thr Thr Val Thr Val Ser Ser
115 120 125

<210> 287
<211> 336
<212> DNA
<213> Artificial Sequence

<220>
<223> Synthetic

<400> 287
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa 336

<210> 288
<211> 112
<212> PRT
<213> Artificial Sequence

<220>
<223> Synthetic

<400> 288
Asp Ile Val Met Thr Gln Ser Pro Leu Ser Leu Pro Val Thr Pro Gly
1 5 10 15

```
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110
```

<210> 289
<211> 372
<212> DNA
<213> Artificial Sequence

<220>
<223> Synthetic

<400> 289

```
cagatcacct tgaaggagtc tggtcctacg ctggtaaaac ccacacagac cctcacgctg 60
acctgcacct tctctgggtt ctcactcagc gctagtggag tgggtgtggg ctggttccgt 120
cagcccccag gaaaggccct ggagtggctt gcactcattt attggaatga tgataagcgt 180
tacagcccat ctctaaagaa cagcctcacc atcaccaagg acacctccaa aaaccaggtg 240
gtccttacaa tgaccaacat ggaccctgtg gacacagcca catattactg tgcacacaga 300
atacatctat ggtcctactt ctactacggt atggacgtct ggggccaagg gaccacggtc 360
accgtctcct ca                                                     372
```

<210> 290
<211> 124
<212> PRT
<213> Artificial Sequence

<220>
<223> Synthetic

<400> 290

```
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Ala Ser
            20                  25                  30
Gly Val Gly Val Gly Trp Phe Arg Gln Pro Pro Gly Lys Ala Leu Glu
        35                  40                  45
Trp Leu Ala Leu Ile Tyr Trp Asn Asp Asp Lys Arg Tyr Ser Pro Ser
    50                  55                  60
Leu Lys Asn Ser Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile His Leu Trp Ser Tyr Phe Tyr Tyr Gly Met Asp
            100                 105                 110
Val Trp Gly Gln Gly Thr Thr Val Thr Val Ser Ser
        115                 120
```

<210> 291
<211> 30
<212> DNA
<213> Artificial Sequence

<220>
<223> Synthetic

<400> 291
gggttctcac tcagcgctag tggagtgggt 30

<210> 292
<211> 10
<212> PRT
<213> Artificial Sequence

<220>
<223> Synthetic

<400> 292
Gly Phe Ser Leu Ser Ala Ser Gly Val Gly
1 5 10

<210> 293
<211> 21
<212> DNA
<213> Artificial Sequence

<220>
<223> Synthetic

<400> 293
atttattgga atgatgataa g 21

<210> 294
<211> 7
<212> PRT
<213> Artificial Sequence

<220>
<223> Synthetic

<400> 294
Ile Tyr Trp Asn Asp Asp Lys
1 5

<210> 295
<211> 48
<212> DNA
<213> Artificial Sequence

<220>
<223> Synthetic

<400> 295
gcacacagaa tacatctatg gtcctacttc tactacggta tggacgtc 48

<210> 296
<211> 16
<212> PRT
<213> Artificial Sequence

<220>
<223> Synthetic

<400> 296
Ala His Arg Ile His Leu Trp Ser Tyr Phe Tyr Tyr Gly Met Asp Val
1 5 10 15

<210> 297
<211> 336
<212> DNA
<213> Artificial Sequence

<220>
<223> Synthetic

<400> 297
gatattgtga tgactcagtc tccactctcc ctgcccgtca cccctggaga gccggcctcc 60
atctcctgca ggtctagtca gactctcctg catagtaatg gatacaacta tttcgattgg 120
tacctgcaga agccagggca gtctccacag ctcctgatct atttgggttc taatcgggcc 180
tccggggtcc ctgacagatt cagtggcagt ggatcaggca cagattttac actgaaaatc 240
agcagagtgg aggctgagga tgttggaatt tattactgca tgcaagctct acaaactcct 300
ctcactttcg gcggagggac caaggtggag atcaga 336

<210> 298
<211> 112
<212> PRT
<213> Artificial Sequence

<220>
<223> Synthetic

<400> 298
Asp Ile Val Met Thr Gln Ser Pro Leu Ser Leu Pro Val Thr Pro Gly
1 5 10 15
Glu Pro Ala Ser Ile Ser Cys Arg Ser Ser Gln Thr Leu Leu His Ser
20 25 30
Asn Gly Tyr Asn Tyr Phe Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Ile Tyr Tyr Cys Met Gln Ala
85 90 95
Leu Gln Thr Pro Leu Thr Phe Gly Gly Gly Thr Lys Val Glu Ile Arg
100 105 110

<210> 299
<211> 33
<212> DNA
<213> Artificial Sequence

<220>
<223> Synthetic

<400> 299
cagactctcc tgcatagtaa tggatacaac tat 33

<210> 300
<211> 11
<212> PRT
<213> Artificial Sequence

<220>
<223> Synthetic

<400> 300
Gln Thr Leu Leu His Ser Asn Gly Tyr Asn Tyr
1 5 10

<210> 301
<211> 9
<212> DNA
<213> Artificial Sequence

<220>
<223> Synthetic

<400> 301
ttgggttct 9

<210> 302
<211> 3
<212> PRT
<213> Artificial Sequence

<220>
<223> Synthetic

<400> 302
Leu Gly Ser
1

<210> 303
<211> 27
<212> DNA
<213> Artificial Sequence

<220>
<223> Synthetic

<400> 303
atgcaagctc tacaaactcc tctcact 27

<210> 304
<211> 9
<212> PRT
<213> Artificial Sequence

<220>
<223> Synthetic

<400> 304
Met Gln Ala Leu Gln Thr Pro Leu Thr
1 5

<210> 305
<211> 372
<212> DNA
<213> Artificial Sequence

<220>
<223> Synthetic

<400> 305
cagatcacct tgaaggagtc tggtcctacg ctggtaaaac ccacacagac cctcacgctg 60
acctgcacct tctctgggtt ctcactcagc gctagtggag tgggtgtggg ctggttccgt 120
cagcccccag gaaaggccct ggagtggctt gcactcattt attggaatga tgataagcgt 180
tacagcccat ctctaaagaa cagcctcacc atcaccaagg acacctccaa aaaccaggtg 240
gtccttacaa tgaccaacat ggaccctgtg gacacagcca catattactg tgcacacaga 300
atacatctat ggtcctactt ctactacggt atggacgtct ggggccaagg gaccacggtc 360
accgtctcct ca 372

<210> 306
<211> 124
<212> PRT
<213> Artificial Sequence

<220>
<223> Synthetic

<400> 306
```
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Ala Ser
            20                  25                  30
Gly Val Gly Val Gly Trp Phe Arg Gln Pro Pro Gly Lys Ala Leu Glu
        35                  40                  45
Trp Leu Ala Leu Ile Tyr Trp Asn Asp Asp Lys Arg Tyr Ser Pro Ser
    50                  55                  60
Leu Lys Asn Ser Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile His Leu Trp Ser Tyr Phe Tyr Tyr Gly Met Asp
            100                 105                 110
Val Trp Gly Gln Gly Thr Thr Val Thr Val Ser Ser
        115                 120
```

<210> 307
<211> 336
<212> DNA
<213> Artificial Sequence

<220>
<223> Synthetic

<400> 307
```
gatattgtga tgactcagtc tccactctcc ctgcccgtca cccctggaga gccggcctcc 60
atctcctgca ggtctagtca gactctcctg catagtaatg gatacaacta tttcgattgg 120
tacctgcaga agccagggca gtctccacag ctcctgatct atttgggttc taatcgggcc 180
tccggggtcc ctgacagatt cagtggcagt ggatcaggca cagattttac actgaaaatc 240
agcagagtgg aggctgagga tgttggaatt tattactgca tgcaagctct acaaactcct 300
ctcactttcg gcggagggac caaggtggag atcaaa                           336
```

<210> 308
<211> 112
<212> PRT
<213> Artificial Sequence

<220>
<223> Synthetic

<400> 308
```
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Thr Leu Leu His Ser
            20                  25                  30
Asn Gly Tyr Asn Tyr Phe Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Ile Tyr Tyr Cys Met Gln Ala
                85                  90                  95
Leu Gln Thr Pro Leu Thr Phe Gly Gly Gly Thr Lys Val Glu Ile Lys
```

100 105 110

<210> 309
<211> 372
<212> DNA
<213> Artificial Sequence

<220>
<223> Synthetic

<400> 309
cagatcacct tgaaggagtc tggtcctacg ctggtgaaac ccacacagac cctcacgctg 60
acctgcacct tctctgggtt ctcactcagc gctagtggag tgggtgtggg ctggatccgt 120
cagcccccag gaaaggccct ggagtggctt gcactcattt attggaatga tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacaga 300
atacatctat ggtcctactt ctactacggt atggacgtct gggggcaagg gaccacggtc 360
accgtctcct ca 372

<210> 310
<211> 124
<212> PRT
<213> Artificial Sequence

<220>
<223> Synthetic

<400> 310
Gln Ile Thr Leu Lys Glu Ser Gly Pro Thr Leu Val Lys Pro Thr Gln
1 5 10 15
Thr Leu Thr Leu Thr Cys Thr Phe Ser Gly Phe Ser Leu Ser Ala Ser
20 25 30
Gly Val Gly Val Gly Trp Ile Arg Gln Pro Pro Gly Lys Ala Leu Glu
35 40 45
Trp Leu Ala Leu Ile Tyr Trp Asn Asp Asp Lys Arg Tyr Ser Pro Ser
50 55 60
Leu Lys Ser Arg Leu Thr Ile Thr Lys Asp Thr Ser Lys Asn Gln Val
65 70 75 80
Val Leu Thr Met Thr Asn Met Asp Pro Val Asp Thr Ala Thr Tyr Tyr
85 90 95
Cys Ala His Arg Ile His Leu Trp Ser Tyr Phe Tyr Tyr Gly Met Asp
100 105 110
Val Trp Gly Gln Gly Thr Thr Val Thr Val Ser Ser
115 120

<210> 311
<211> 336
<212> DNA
<213> Artificial Sequence

<220>
<223> Synthetic

<400> 311
gatattgtga tgactcagtc tccactctcc ctgcccgtca cccctggaga gccggcctcc 60
atctcctgca ggtctagtca gactctcctg catagtaatg gatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa 336

<210> 312
<211> 112

<212> PRT
<213> Artificial Sequence

<220>
<223> Synthetic

<400> 312
Asp Ile Val Met Thr Gln Ser Pro Leu Ser Leu Pro Val Thr Pro Gly
1 5 10 15
Glu Pro Ala Ser Ile Ser Cys Arg Ser Ser Gln Thr Leu Leu His Ser
20 25 30
Asn Gly Tyr Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Leu Thr Phe Gly Gly Gly Thr Lys Val Glu Ile Lys
100 105 110

<210> 313
<211> 381
<212> DNA
<213> Artificial Sequence

<220>
<223> Synthetic

<400> 313
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattcc tactacgtta tggacgtctg gggccaaggg 360
accacggtca ccgtctcctc a 381

<210> 314
<211> 127
<212> PRT
<213> Artificial Sequence

<220>
<223> Synthetic

<400> 314
Gln Val Gln Leu Val Gln Ser Gly Pro Glu Val Lys Asn Pro Gly Ala
1 5 10 15
Ser Val Lys Val Ser Cys Lys Ala Ser Gly Tyr Thr Phe Thr Thr Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Lys Thr Asn Asp Ala Gln Lys Phe
50 55 60
Gln Asp Arg Val Ala Met Thr Thr Asp Thr Ser Thr Ser Thr Ala Tyr
65 70 75 80
Met Glu Leu Arg Ser Leu Arg Ser Asp Asp Thr Ala Ile Tyr Tyr Cys
85 90 95
Ser Arg Asp Arg Leu Val Val Pro Pro Ala Leu Asn Tyr Ser Tyr Tyr
100 105 110
Val Met Asp Val Trp Gly Gln Gly Thr Thr Val Thr Val Ser Ser
115 120 125

<210> 315
<211> 24
<212> DNA
<213> Artificial Sequence

<220>
<223> Synthetic

<400> 315
ggttacacct ttaccaccta tggt 24

<210> 316
<211> 8
<212> PRT
<213> Artificial Sequence

<220>
<223> Synthetic

<400> 316
Gly Tyr Thr Phe Thr Thr Tyr Gly
1 5

<210> 317
<211> 24
<212> DNA
<213> Artificial Sequence

<220>
<223> Synthetic

<400> 317
atcagcggtt acaatggtaa aaca 24

<210> 318
<211> 8
<212> PRT
<213> Artificial Sequence

<220>
<223> Synthetic

<400> 318
Ile Ser Gly Tyr Asn Gly Lys Thr
1 5

<210> 319
<211> 60
<212> DNA
<213> Artificial Sequence

<220>
<223> Synthetic

<400> 319
tcgagagatc gtttagtagt accacctgcc cttaattatt cctactacgt tatggacgtc 60

<210> 320
<211> 20
<212> PRT

<213> Artificial Sequence

<220>
<223> Synthetic

<400> 320
Ser Arg Asp Arg Leu Val Val Pro Pro Ala Leu Asn Tyr Ser Tyr Tyr
1               5                   10                  15
Val Met Asp Val
            20

<210> 321
<211> 336
<212> DNA
<213> Artificial Sequence

<220>
<223> Synthetic

<400> 321
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tctcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 322
<211> 112
<212> PRT
<213> Artificial Sequence

<220>
<223> Synthetic

<400> 322
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Ser Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 323
<211> 33
<212> DNA
<213> Artificial Sequence

<220>
<223> Synthetic

<400> 323
caaagcctcg tatacagtga tggaaacacc tac 33

<210> 324

<211> 11
<212> PRT
<213> Artificial Sequence

<220>
<223> Synthetic

<400> 324
Gln Ser Leu Val Tyr Ser Asp Gly Asn Thr Tyr
1 5 10

<210> 325
<211> 9
<212> DNA
<213> Artificial Sequence

<220>
<223> Synthetic

<400> 325
aaggtttct 9

<210> 326
<211> 3
<212> PRT
<213> Artificial Sequence

<220>
<223> Synthetic

<400> 326
Lys Val Ser
1

<210> 327
<211> 27
<212> DNA
<213> Artificial Sequence

<220>
<223> Synthetic

<400> 327
atgcaaggta cacactggcc gtacact 27

<210> 328
<211> 9
<212> PRT
<213> Artificial Sequence

<220>
<223> Synthetic

<400> 328
Met Gln Gly Thr His Trp Pro Tyr Thr
1 5

<210> 329
<211> 381
<212> DNA
<213> Artificial Sequence

<220>
<223> Synthetic

<400> 329
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattcc tactacgtta tggacgtctg gggccaaggg 360
accacggtca ccgtctcctc a 381

<210> 330
<211> 127
<212> PRT
<213> Artificial Sequence

<220>
<223> Synthetic

<400> 330
Gln Val Gln Leu Val Gln Ser Gly Pro Glu Val Lys Asn Pro Gly Ala
1 5 10 15
Ser Val Lys Val Ser Cys Lys Ala Ser Gly Tyr Thr Phe Thr Thr Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Lys Thr Asn Asp Ala Gln Lys Phe
50 55 60
Gln Asp Arg Val Ala Met Thr Thr Asp Thr Ser Thr Ser Thr Ala Tyr
65 70 75 80
Met Glu Leu Arg Ser Leu Arg Ser Asp Asp Thr Ala Ile Tyr Tyr Cys
85 90 95
Ser Arg Asp Arg Leu Val Val Pro Pro Ala Leu Asn Tyr Ser Tyr Tyr
100 105 110
Val Met Asp Val Trp Gly Gln Gly Thr Thr Val Thr Val Ser Ser
115 120 125

<210> 331
<211> 336
<212> DNA
<213> Artificial Sequence

<220>
<223> Synthetic

<400> 331
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tctcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 332
<211> 112
<212> PRT
<213> Artificial Sequence

<220>
<223> Synthetic

<400> 332
Asp Val Val Met Thr Gln Ser Pro Leu Ser Leu Pro Val Thr Leu Gly

```
 1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Ser Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110


<210> 333
<211> 381
<212> DNA
<213> Artificial Sequence

<220>
<223> Synthetic

<400> 333
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct taattattcc tactacgtta tggacgtctg gggcaaggg 360
accacggtca ccgtctcctc a                                           381

<210> 334
<211> 127
<212> PRT
<213> Artificial Sequence

<220>
<223> Synthetic

<400> 334
Gln Val Gln Leu Val Gln Ser Gly Ala Glu Val Lys Lys Pro Gly Ala
 1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ser Arg Asp Arg Leu Val Val Pro Pro Ala Leu Asn Tyr Ser Tyr Tyr
            100                 105                 110
Val Met Asp Val Trp Gly Gln Gly Thr Thr Val Thr Val Ser Ser
        115                 120                 125


<210> 335
<211> 336
<212> DNA
<213> Artificial Sequence

<220>
```

<223> Synthetic

<400> 335
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 336
<211> 112
<212> PRT
<213> Artificial Sequence

<220>
<223> Synthetic

<400> 336
Asp Val Val Met Thr Gln Ser Pro Leu Ser Leu Pro Val Thr Leu Gly
1 5 10 15
Gln Pro Ala Ser Ile Ser Cys Arg Ser Ser Gln Ser Leu Val Tyr Ser
20 25 30
Asp Gly Asn Thr Tyr Leu Asn Trp Phe Gln Gln Arg Pro Gly Gln Ser
35 40 45
Pro Arg Arg Leu Ile Tyr Lys Val Ser Asn Arg Asp Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Gly
85 90 95
Thr His Trp Pro Tyr Thr Phe Gly Gln Gly Thr Lys Leu Glu Ile Lys
100 105 110

<210> 337
<211> 354
<212> DNA
<213> Artificial Sequence

<220>
<223> Synthetic

<400> 337
gaggtgcagc tggtggagtc tgggggaggc ctggtcaagc ctggggggtc cctgagactc 60
tcctgtgcag cctctggatt caccttcagt agctatagca tggactgggt ccgccaggct 120
ccagggaagg ggctggagtg ggtctcatcc attagtagta gtagtagtta catatactac 180
gcagactctg tgaagggccg attcaccatc tccagagaca ccgccaagaa ctcactgtat 240
ctgcaaatga acagcctgag agacgaggac acggctgttt attactgtgc gagagagggc 300
agtagcagac tttttgacta ctggggccag ggaaccctgg tcaccgtctc ctca 354

<210> 338
<211> 118
<212> PRT
<213> Artificial Sequence

<220>
<223> Synthetic

<400> 338
Glu Val Gln Leu Val Glu Ser Gly Gly Gly Leu Val Lys Pro Gly Gly
1 5 10 15
Ser Leu Arg Leu Ser Cys Ala Ala Ser Gly Phe Thr Phe Ser Ser Tyr
20 25 30
Ser Met Asp Trp Val Arg Gln Ala Pro Gly Lys Gly Leu Glu Trp Val

```
        35                  40                  45
Ser Ser Ile Ser Ser Ser Ser Ser Tyr Ile Tyr Tyr Ala Asp Ser Val
    50                  55                  60
Lys Gly Arg Phe Thr Ile Ser Arg Asp Thr Ala Lys Asn Ser Leu Tyr
65                  70                  75                  80
Leu Gln Met Asn Ser Leu Arg Asp Glu Asp Thr Ala Val Tyr Tyr Cys
                85                  90                  95
Ala Arg Glu Gly Ser Ser Arg Leu Phe Asp Tyr Trp Gly Gln Gly Thr
            100                 105                 110
Leu Val Thr Val Ser Ser
        115
```

<210> 339
<211> 24
<212> DNA
<213> Artificial Sequence

<220>
<223> Synthetic

<400> 339
ggattcacct tcagtagcta tagc 24

<210> 340
<211> 8
<212> PRT
<213> Artificial Sequence

<220>
<223> Synthetic

<400> 340

```
Gly Phe Thr Phe Ser Ser Tyr Ser
 1               5
```

<210> 341
<211> 24
<212> DNA
<213> Artificial Sequence

<220>
<223> Synthetic

<400> 341
attagtagta gtagtagtta cata 24

<210> 342
<211> 8
<212> PRT
<213> Artificial Sequence

<220>
<223> Synthetic

<400> 342

```
Ile Ser Ser Ser Ser Ser Tyr Ile
 1               5
```

<210> 343
<211> 33
<212> DNA
<213> Artificial Sequence

<220>
<223> Synthetic

<400> 343
gcgagagagg gcagtagcag actttttgac tac 33

<210> 344
<211> 11
<212> PRT
<213> Artificial Sequence

<220>
<223> Synthetic

<400> 344
Ala Arg Glu Gly Ser Ser Arg Leu Phe Asp Tyr
1 5 10

<210> 345
<211> 321
<212> DNA
<213> Artificial Sequence

<220>
<223> Synthetic

<400> 345
gacatccaga tgacccagtc tccttccacc ctgtctgcat ctgtaggaga cagagtcacc 60
atcacttgcc gggccagtca gagtattagt agctggttgg cctggtatca gcagagacca 120
gggaaagccc ctaagctcct gatctataag gcgtctagtt tagaaggtgg agtcccatca 180
aggttcagcg gcagtggatc tgggacagaa ttcactctca ccatcagcag cctgcagcct 240
gaggattttg caacttatta ctgccaacag tataatagtt attggtacac ttttggccag 300
gggaccaagc tggagatcaa a 321

<210> 346
<211> 107
<212> PRT
<213> Artificial Sequence

<220>
<223> Synthetic

<400> 346
Asp Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Arg Pro Gly Lys Ala Pro Lys Leu Leu Ile
35 40 45
Tyr Lys Ala Ser Ser Leu Glu Gly Gly Val Pro Ser Arg Phe Ser Gly
50 55 60
Ser Gly Ser Gly Thr Glu Phe Thr Leu Thr Ile Ser Ser Leu Gln Pro
65 70 75 80
Glu Asp Phe Ala Thr Tyr Tyr Cys Gln Gln Tyr Asn Ser Tyr Trp Tyr
85 90 95
Thr Phe Gly Gln Gly Thr Lys Leu Glu Ile Lys
100 105

<210> 347
<211> 18
<212> DNA
<213> Artificial Sequence

<220>
<223> Synthetic

<400> 347
cagagtatta gtagctgg 18

<210> 348
<211> 6
<212> PRT
<213> Artificial Sequence

<220>
<223> Synthetic

<400> 348
Gln Ser Ile Ser Ser Trp
1 5

<210> 349
<211> 9
<212> DNA
<213> Artificial Sequence

<220>
<223> Synthetic

<400> 349
aaggcgtct 9

<210> 350
<211> 3
<212> PRT
<213> Artificial Sequence

<220>
<223> Synthetic

<400> 350
Lys Ala Ser
1

<210> 351
<211> 27
<212> DNA
<213> Artificial Sequence

<220>
<223> Synthetic

<400> 351
caacagtata atagttattg gtacact 27

<210> 352
<211> 9
<212> PRT
<213> Artificial Sequence

<220>
<223> Synthetic

<400> 352
Gln Gln Tyr Asn Ser Tyr Trp Tyr Thr

1               5

<210> 353
<211> 354
<212> DNA
<213> Artificial Sequence

<220>
<223> Synthetic

<400> 353
gaggtgcagc tggtggagtc tgggggaggc ctggtcaagc ctggggggtc cctgagactc 60
tcctgtgcag cctctggatt caccttcagt agctatagca tggactgggt ccgccaggct 120
ccagggaagg ggctggagtg ggtctcatcc attagtagta gtagtagtta catatactac 180
gcagactctg tgaagggccg attcaccatc tccagagaca ccgccaagaa ctcactgtat 240
ctgcaaatga acagcctgag agacgaggac acggctgttt attactgtgc gagagagggc 300
agtagcagac tttttgacta ctggggccag ggaaccctgg tcaccgtctc ctca 354

<210> 354
<211> 118
<212> PRT
<213> Artificial Sequence

<220>
<223> Synthetic

<400> 354
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Ser Tyr
            20                  25                  30
Ser Met Asp Trp Val Arg Gln Ala Pro Gly Lys Gly Leu Glu Trp Val
        35                  40                  45
Ser Ser Ile Ser Ser Ser Ser Ser Tyr Ile Tyr Tyr Ala Asp Ser Val
    50                  55                  60
Lys Gly Arg Phe Thr Ile Ser Arg Asp Thr Ala Lys Asn Ser Leu Tyr
65                  70                  75                  80
Leu Gln Met Asn Ser Leu Arg Asp Glu Asp Thr Ala Val Tyr Tyr Cys
                85                  90                  95
Ala Arg Glu Gly Ser Ser Arg Leu Phe Asp Tyr Trp Gly Gln Gly Thr
            100                 105                 110
Leu Val Thr Val Ser Ser
        115

<210> 355
<211> 321
<212> DNA
<213> Artificial Sequence

<220>
<223> Synthetic

<400> 355
gacatccaga tgacccagtc tccttccacc ctgtctgcat ctgtaggaga cagagtcacc 60
atcacttgcc gggccagtca gagtattagt agctggttgg cctggtatca gcagagacca 120
gggaaagccc ctaagctcct gatctataag gcgtctagtt tagaaggtgg agtcccatca 180
aggttcagcg gcagtggatc tgggacagaa ttcactctca ccatcagcag cctgcagcct 240
gaggattttg caacttatta ctgccaacag tataatagtt attggtacac ttttggccag 300
gggaccaagc tggagatcaa a 321

<210> 356
<211> 107
<212> PRT

<213> Artificial Sequence

<220>
<223> Synthetic

<400> 356
Asp Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Arg Pro Gly Lys Ala Pro Lys Leu Leu Ile
35 40 45
Tyr Lys Ala Ser Ser Leu Glu Gly Gly Val Pro Ser Arg Phe Ser Gly
50 55 60
Ser Gly Ser Gly Thr Glu Phe Thr Leu Thr Ile Ser Ser Leu Gln Pro
65 70 75 80
Glu Asp Phe Ala Thr Tyr Tyr Cys Gln Gln Tyr Asn Ser Tyr Trp Tyr
85 90 95
Thr Phe Gly Gln Gly Thr Lys Leu Glu Ile Lys
100 105

<210> 357
<211> 354
<212> DNA
<213> Artificial Sequence

<220>
<223> Synthetic

<400> 357
gaggtgcagc tggtggagtc tgggggaggc ctggtcaagc ctggggggtc cctgagactc 60
tcctgtgcag cctctggatt caccttcagt agctatagca tgaactgggt ccgccaggct 120
ccagggaagg ggctggagtg ggtctcatcc attagtagta gtagtagtta catatactac 180
gcagactcag tgaagggccg attcaccatc tccagagaca acgccaagaa ctcactgtat 240
ctgcaaatga acagcctgag agccgaggac acggctgtgt attactgtgc gagagagggc 300
agtagcagac tttttgacta ctggggccaa ggaaccctgg tcaccgtctc ctca 354

<210> 358
<211> 118
<212> PRT
<213> Artificial Sequence

<220>
<223> Synthetic

<400> 358
Glu Val Gln Leu Val Glu Ser Gly Gly Gly Leu Val Lys Pro Gly Gly
1 5 10 15
Ser Leu Arg Leu Ser Cys Ala Ala Ser Gly Phe Thr Phe Ser Ser Tyr
20 25 30
Ser Met Asn Trp Val Arg Gln Ala Pro Gly Lys Gly Leu Glu Trp Val
35 40 45
Ser Ser Ile Ser Ser Ser Ser Ser Tyr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Glu Gly Ser Ser Arg Leu Phe Asp Tyr Trp Gly Gln Gly Thr
100 105 110
Leu Val Thr Val Ser Ser
115

<210> 359
<211> 321
<212> DNA
<213> Artificial Sequence

<220>
<223> Synthetic

<400> 359
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attggtacac ttttggccag 300
gggaccaagc tggagatcaa a 321

<210> 360
<211> 107
<212> PRT
<213> Artificial Sequence

<220>
<223> Synthetic

<400> 360
Asp Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Lys Pro Gly Lys Ala Pro Lys Leu Leu Ile
35 40 45
Tyr Lys Ala Ser Ser Leu Glu Ser Gly Val Pro Ser Arg Phe Ser Gly
50 55 60
Ser Gly Ser Gly Thr Glu Phe Thr Leu Thr Ile Ser Ser Leu Gln Pro
65 70 75 80
Asp Asp Phe Ala Thr Tyr Tyr Cys Gln Gln Tyr Asn Ser Tyr Trp Tyr
85 90 95
Thr Phe Gly Gln Gly Thr Lys Leu Glu Ile Lys
100 105

<210> 361
<211> 384
<212> DNA
<213> Artificial Sequence

<220>
<223> Synthetic

<400> 361
caggtgcacc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtcgc ctca 384

<210> 362
<211> 128
<212> PRT
<213> Artificial Sequence

<220>
<223> Synthetic

<400> 362
Gln Val His Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ala Ser
        115                 120                 125

<210> 363
<211> 24
<212> DNA
<213> Artificial Sequence

<220>
<223> Synthetic

<400> 363
ggattcacct tcagtgacca ctac 24

<210> 364
<211> 8
<212> PRT
<213> Artificial Sequence

<220>
<223> Synthetic

<400> 364
Gly Phe Thr Phe Ser Asp His Tyr
1               5

<210> 365
<211> 24
<212> DNA
<213> Artificial Sequence

<220>
<223> Synthetic

<400> 365
attagtaatg atggtggtac caaa 24

<210> 366
<211> 8
<212> PRT
<213> Artificial Sequence

<220>
<223> Synthetic

<400> 366
Ile Ser Asn Asp Gly Gly Thr Lys

1 5

<210> 367
<211> 63
<212> DNA
<213> Artificial Sequence

<220>
<223> Synthetic

<400> 367
gcgagagatc agggatatat tggctacgac tcgtattatt actattccta cggtatggac 60
gtc 63

<210> 368
<211> 21
<212> PRT
<213> Artificial Sequence

<220>
<223> Synthetic

<400> 368
Ala Arg Asp Gln Gly Tyr Ile Gly Tyr Asp Ser Tyr Tyr Tyr Tyr Ser
1 5 10 15
Tyr Gly Met Asp Val
20

<210> 369
<211> 321
<212> DNA
<213> Artificial Sequence

<220>
<223> Synthetic

<400> 369
aaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tctcggcgga 300
gggaccaagg tggagatcaa g 321

<210> 370
<211> 107
<212> PRT
<213> Artificial Sequence

<220>
<223> Synthetic

<400> 370
Lys Ile Val Leu Thr Gln Ser Pro Gly Thr Leu Pro Leu Phe Pro Gly
1 5 10 15
Glu Arg Ala Thr Leu Ser Cys Arg Ala Ser Gln Ser Val Asn Asn Lys
20 25 30
Phe Leu Ala Trp Tyr Gln Gln Lys Ser Gly Gln Ala Pro Arg Leu Leu
35 40 45
Ile Tyr Gly Ala Ser Ser Arg Ala Thr Gly Ile Pro Asp Arg Phe Ser
50 55 60
Gly Ser Gly Ser Gly Thr Asp Phe Thr Leu Thr Ile Ser Gly Leu Glu
65 70 75 80

Pro Glu Asp Phe Glu Val Tyr Tyr Cys Gln Val Tyr Gly Asn Ser Leu
85 90 95
Thr Leu Gly Gly Gly Thr Lys Val Glu Ile Lys
100 105

<210> 371
<211> 21
<212> DNA
<213> Artificial Sequence

<220>
<223> Synthetic

<400> 371
cagagtgtta acaacaaatt c 21

<210> 372
<211> 7
<212> PRT
<213> Artificial Sequence

<220>
<223> Synthetic

<400> 372
Gln Ser Val Asn Asn Lys Phe
1 5

<210> 373
<211> 9
<212> DNA
<213> Artificial Sequence

<220>
<223> Synthetic

<400> 373
ggtgcatcc 9

<210> 374
<211> 3
<212> PRT
<213> Artificial Sequence

<220>
<223> Synthetic

<400> 374
Gly Ala Ser
1

<210> 375
<211> 24
<212> DNA
<213> Artificial Sequence

<220>
<223> Synthetic

<400> 375
caagtatatg gtaactcact cact 24

<210> 376
<211> 8
<212> PRT
<213> Artificial Sequence

<220>
<223> Synthetic

<400> 376
Gln Val Tyr Gly Asn Ser Leu Thr
1               5

<210> 377
<211> 384
<212> DNA
<213> Artificial Sequence

<220>
<223> Synthetic

<400> 377
caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtctc ctca 384

<210> 378
<211> 128
<212> PRT
<213> Artificial Sequence

<220>
<223> Synthetic

<400> 378
Gln Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125

<210> 379
<211> 321
<212> DNA
<213> Artificial Sequence

<220>
<223> Synthetic

<400> 379
gaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tctcggcgga 300
gggaccaagg tggagatcaa a 321

<210> 380
<211> 107
<212> PRT
<213> Artificial Sequence

<220>
<223> Synthetic

<400> 380
Glu Ile Val Leu Thr Gln Ser Pro Gly Thr Leu Pro Leu Phe Pro Gly
1 5 10 15
Glu Arg Ala Thr Leu Ser Cys Arg Ala Ser Gln Ser Val Asn Asn Lys
20 25 30
Phe Leu Ala Trp Tyr Gln Gln Lys Ser Gly Gln Ala Pro Arg Leu Leu
35 40 45
Ile Tyr Gly Ala Ser Ser Arg Ala Thr Gly Ile Pro Asp Arg Phe Ser
50 55 60
Gly Ser Gly Ser Gly Thr Asp Phe Thr Leu Thr Ile Ser Gly Leu Glu
65 70 75 80
Pro Glu Asp Phe Glu Val Tyr Tyr Cys Gln Val Tyr Gly Asn Ser Leu
85 90 95
Thr Leu Gly Gly Gly Thr Lys Val Glu Ile Lys
100 105

<210> 381
<211> 384
<212> DNA
<213> Artificial Sequence

<220>
<223> Synthetic

<400> 381
caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg ggtttcatac attagtaatg atggtggtac caaatactac 180
gcagactctg tgaagggccg attcaccatc tccagggaca acgccaagaa ctcactgtat 240
ctgcaaatga acagcctgag agccgaggac acggccgtgt attactgtgc gagagatcag 300
ggatatattg gctacgactc gtattattac tattcctacg gtatggacgt ctgggggcaa 360
gggaccacgg tcaccgtctc ctca 384

<210> 382
<211> 128
<212> PRT
<213> Artificial Sequence

<220>
<223> Synthetic

<400> 382
Gln Val Gln Leu Val Glu Ser Gly Gly Gly Leu Val Lys Pro Gly Gly
1 5 10 15
Ser Leu Arg Leu Ser Cys Ala Ala Ser Gly Phe Thr Phe Ser Asp His
20 25 30
Tyr Met Ser Trp Ile Arg Gln Ala Pro Gly Lys Gly Leu Glu Trp Val
35 40 45

```
Ser Tyr Ile Ser Asn Asp Gly Gly Thr Lys Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125
```

<210> 383
<211> 321
<212> DNA
<213> Artificial Sequence

<220>
<223> Synthetic

<400> 383
```
gaaattgtgt tgacgcagtc tccaggcacc ctgtctttgt ctccagggga aagagccacc 60
ctctcctgca gggccagtca gagtgttaac aacaaattct tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240
cctgaagatt ttgcagtgta ttactgtcaa gtatatggta actcactcac tttcggcgga 300
gggaccaagg tggagatcaa a                                           321
```

<210> 384
<211> 107
<212> PRT
<213> Artificial Sequence

<220>
<223> Synthetic

<400> 384
```
Glu Ile Val Leu Thr Gln Ser Pro Gly Thr Leu Ser Leu Ser Pro Gly
 1               5                  10                  15
Glu Arg Ala Thr Leu Ser Cys Arg Ala Ser Gln Ser Val Asn Asn Lys
            20                  25                  30
Phe Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Val Tyr Gly Asn Ser Leu
                85                  90                  95
Thr Phe Gly Gly Gly Thr Lys Val Glu Ile Lys
            100                 105
```

<210> 385
<211> 360
<212> DNA
<213> Artificial Sequence

<220>
<223> Synthetic

<400> 385
```
gaggtgcaga aggtggagtc tgggggaggc ctggtcaagc cggggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
```

gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 386
<211> 120
<212> PRT
<213> Artificial Sequence

<220>
<223> Synthetic

<400> 386
Glu Val Gln Lys Val Glu Ser Gly Gly Gly Leu Val Lys Pro Gly Gly
1 5 10 15
Ser Leu Arg Leu Ser Cys Thr Ala Ser Gly Phe Thr Phe Ser Thr Tyr
20 25 30
Asn Met Asn Trp Val Arg Gln Ala Pro Gly Lys Gly Leu Glu Trp Val
35 40 45
Ser Ser Ile Arg Ser Ser Ser Asn Tyr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Asp Asp Thr Ala Val Tyr Tyr Cys
85 90 95
Ala Arg Asp Gly Ser Ser Trp Tyr Asp Tyr Ser Asp Tyr Trp Gly Gln
100 105 110
Gly Thr Leu Val Thr Val Ser Ser
115 120

<210> 387
<211> 24
<212> DNA
<213> Artificial Sequence

<220>
<223> Synthetic

<400> 387
ggattcacct tcagtactta taac 24

<210> 388
<211> 8
<212> PRT
<213> Artificial Sequence

<220>
<223> Synthetic

<400> 388
Gly Phe Thr Phe Ser Thr Tyr Asn
1 5

<210> 389
<211> 24
<212> DNA
<213> Artificial Sequence

<220>
<223> Synthetic

<400> 389

attaggagta gtagtaatta cata 24

<210> 390
<211> 8
<212> PRT
<213> Artificial Sequence

<220>
<223> Synthetic

<400> 390
Ile Arg Ser Ser Ser Asn Tyr Ile
1 5

<210> 391
<211> 39
<212> DNA
<213> Artificial Sequence

<220>
<223> Synthetic

<400> 391
gcgagagatg gcagcagttg gtacgactac tctgactac 39

<210> 392
<211> 13
<212> PRT
<213> Artificial Sequence

<220>
<223> Synthetic

<400> 392
Ala Arg Asp Gly Ser Ser Trp Tyr Asp Tyr Ser Asp Tyr
1 5 10

<210> 393
<211> 321
<212> DNA
<213> Artificial Sequence

<220>
<223> Synthetic

<400> 393
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 394
<211> 107
<212> PRT
<213> Artificial Sequence

<220>
<223> Synthetic

<400> 394
Asp Ile Gln Met Thr Gln Ser Pro Ser Thr Leu Ser Ala Ser Val Gly

```
 1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105
```

<210> 395
<211> 18
<212> DNA
<213> Artificial Sequence

<220>
<223> Synthetic

<400> 395
cagagtatta gtagctgg 18

<210> 396
<211> 6
<212> PRT
<213> Artificial Sequence

<220>
<223> Synthetic

<400> 396

```
Gln Ser Ile Ser Ser Trp
 1               5
```

<210> 397
<211> 9
<212> DNA
<213> Artificial Sequence

<220>
<223> Synthetic

<400> 397
aaggcgtct 9

<210> 398
<211> 3
<212> PRT
<213> Artificial Sequence

<220>
<223> Synthetic

<400> 398

```
Lys Ala Ser
 1
```

<210> 399
<211> 27

<212> DNA
<213> Artificial Sequence

<220>
<223> Synthetic

<400> 399
caacagtata ttagttattc tcggacg 27

<210> 400
<211> 9
<212> PRT
<213> Artificial Sequence

<220>
<223> Synthetic

<400> 400
Gln Gln Tyr Ile Ser Tyr Ser Arg Thr
1 5

<210> 401
<211> 360
<212> DNA
<213> Artificial Sequence

<220>
<223> Synthetic

<400> 401
gaggtgcagc tggtggagtc tgggggaggc ctggtcaagc cggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 402
<211> 120
<212> PRT
<213> Artificial Sequence

<220>
<223> Synthetic

<400> 402
Glu Val Gln Leu Val Glu Ser Gly Gly Gly Leu Val Lys Pro Gly Gly
1 5 10 15
Ser Leu Arg Leu Ser Cys Thr Ala Ser Gly Phe Thr Phe Ser Thr Tyr
20 25 30
Asn Met Asn Trp Val Arg Gln Ala Pro Gly Lys Gly Leu Glu Trp Val
35 40 45
Ser Ser Ile Arg Ser Ser Ser Asn Tyr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Asp Asp Thr Ala Val Tyr Tyr Cys
85 90 95
Ala Arg Asp Gly Ser Ser Trp Tyr Asp Tyr Ser Asp Tyr Trp Gly Gln
100 105 110
Gly Thr Leu Val Thr Val Ser Ser
115 120

<210> 403
<211> 321
<212> DNA
<213> Artificial Sequence

<220>
<223> Synthetic

<400> 403
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 404
<211> 107
<212> PRT
<213> Artificial Sequence

<220>
<223> Synthetic

<400> 404
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 405
<211> 360
<212> DNA
<213> Artificial Sequence

<220>
<223> Synthetic

<400> 405
gaggtgcagc tggtggagtc tgggggaggc ctggtcaagc ctgggggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
agcagttggt acgactactc tgactactgg ggccaaggaa ccctggtcac cgtctcctca 360

<210> 406
<211> 120
<212> PRT
<213> Artificial Sequence

<220>

<223> Synthetic

<400> 406
Glu Val Gln Leu Val Glu Ser Gly Gly Gly Leu Val Lys Pro Gly Gly
1 5 10 15
Ser Leu Arg Leu Ser Cys Ala Ala Ser Gly Phe Thr Phe Ser Thr Tyr
20 25 30
Asn Met Asn Trp Val Arg Gln Ala Pro Gly Lys Gly Leu Glu Trp Val
35 40 45
Ser Ser Ile Arg Ser Ser Ser Asn Tyr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Asp Gly Ser Ser Trp Tyr Asp Tyr Ser Asp Tyr Trp Gly Gln
100 105 110
Gly Thr Leu Val Thr Val Ser Ser
115 120

<210> 407
<211> 321
<212> DNA
<213> Artificial Sequence

<220>
<223> Synthetic

<400> 407
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a 321

<210> 408
<211> 107
<212> PRT
<213> Artificial Sequence

<220>
<223> Synthetic

<400> 408
Asp Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Lys Pro Gly Lys Ala Pro Lys Leu Leu Ile
35 40 45
Tyr Lys Ala Ser Ser Leu Glu Ser Gly Val Pro Ser Arg Phe Ser Gly
50 55 60
Ser Gly Ser Gly Thr Glu Phe Thr Leu Thr Ile Ser Ser Leu Gln Pro
65 70 75 80
Asp Asp Phe Ala Thr Tyr Tyr Cys Gln Gln Tyr Ile Ser Tyr Ser Arg
85 90 95
Thr Phe Gly Gln Gly Thr Lys Val Glu Ile Lys
100 105

<210> 409
<211> 360
<212> DNA

<213> Artificial Sequence

<220>
<223> Synthetic

<400> 409
gaggtgcagc tggtggagtc tgggggaggc ctggtcaagc cggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 410
<211> 120
<212> PRT
<213> Artificial Sequence

<220>
<223> Synthetic

<400> 410
Glu Val Gln Leu Val Glu Ser Gly Gly Gly Leu Val Lys Pro Gly Gly
1 5 10 15
Ser Leu Arg Leu Ser Cys Thr Ala Ser Gly Phe Thr Phe Ser Thr Tyr
20 25 30
Asn Met Asn Trp Val Arg Gln Ala Pro Gly Lys Gly Leu Glu Trp Val
35 40 45
Ser Ser Ile Arg Ser Ser Ser Asn Tyr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Asp Asp Thr Ala Val Tyr Tyr Cys
85 90 95
Ala Arg Asp Gly Ser Ser Trp Tyr Asp Tyr Ser Asp Tyr Trp Gly Gln
100 105 110
Gly Thr Leu Val Thr Val Ser Ser
115 120

<210> 411
<211> 24
<212> DNA
<213> Artificial Sequence

<220>
<223> Synthetic

<400> 411
ggattcacct tcagtactta taac 24

<210> 412
<211> 8
<212> PRT
<213> Artificial Sequence

<220>
<223> Synthetic

<400> 412
Gly Phe Thr Phe Ser Thr Tyr Asn
1 5

<210> 413
<211> 24
<212> DNA
<213> Artificial Sequence

<220>
<223> Synthetic

<400> 413
attaggagta gtagtaatta cata 24

<210> 414
<211> 8
<212> PRT
<213> Artificial Sequence

<220>
<223> Synthetic

<400> 414
Ile Arg Ser Ser Ser Asn Tyr Ile
1 5

<210> 415
<211> 39
<212> DNA
<213> Artificial Sequence

<220>
<223> Synthetic

<400> 415
gcgagagatg gcagcagttg gtacgactac tctgactac 39

<210> 416
<211> 13
<212> PRT
<213> Artificial Sequence

<220>
<223> Synthetic

<400> 416
Ala Arg Asp Gly Ser Ser Trp Tyr Asp Tyr Ser Asp Tyr
1 5 10

<210> 417
<211> 321
<212> DNA
<213> Artificial Sequence

<220>
<223> Synthetic

<400> 417
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 418

<211> 107
<212> PRT
<213> Artificial Sequence

<220>
<223> Synthetic

<400> 418
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 419
<211> 18
<212> DNA
<213> Artificial Sequence

<220>
<223> Synthetic

<400> 419
cagagtatta gtagctgg 18

<210> 420
<211> 6
<212> PRT
<213> Artificial Sequence

<220>
<223> Synthetic

<400> 420
Gln Ser Ile Ser Ser Trp
1               5

<210> 421
<211> 9
<212> DNA
<213> Artificial Sequence

<220>
<223> Synthetic

<400> 421
aaggcgtct 9

<210> 422
<211> 3
<212> PRT
<213> Artificial Sequence

<220>

<223> Synthetic

<400> 422
Lys Ala Ser
 1

<210> 423
<211> 27
<212> DNA
<213> Artificial Sequence

<220>
<223> Synthetic

<400> 423
caacagtata ttagttattc tcggacg 27

<210> 424
<211> 9
<212> PRT
<213> Artificial Sequence

<220>
<223> Synthetic

<400> 424
Gln Gln Tyr Ile Ser Tyr Ser Arg Thr
 1               5

<210> 425
<211> 360
<212> DNA
<213> Artificial Sequence

<220>
<223> Synthetic

<400> 425
gaggtgcagc tggtggagtc tgggggaggc ctggtcaagc cggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 426
<211> 120
<212> PRT
<213> Artificial Sequence

<220>
<223> Synthetic

<400> 426
Glu Val Gln Leu Val Glu Ser Gly Gly Gly Leu Val Lys Pro Gly Gly
 1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60

```
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120


<210> 427
<211> 321
<212> DNA
<213> Artificial Sequence

<220>
<223> Synthetic

<400> 427
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                           321

<210> 428
<211> 107
<212> PRT
<213> Artificial Sequence

<220>
<223> Synthetic

<400> 428
Asp Ile Gln Met Thr Gln Ser Pro Ser Thr Leu Ser Ala Ser Val Gly
 1               5                  10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105


<210> 429
<211> 360
<212> DNA
<213> Artificial Sequence

<220>
<223> Synthetic

<400> 429
gaggtgcagc tggtggagtc tgggggaggc ctggtcaagc ctggggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
```

agcagttggt acgactactc tgactactgg ggccaaggaa ccctggtcac cgtctcctca 360

<210> 430
<211> 120
<212> PRT
<213> Artificial Sequence

<220>
<223> Synthetic

<400> 430
```
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120
```

<210> 431
<211> 321
<212> DNA
<213> Artificial Sequence

<220>
<223> Synthetic

<400> 431
```
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a                                           321
```

<210> 432
<211> 107
<212> PRT
<213> Artificial Sequence

<220>
<223> Synthetic

<400> 432
```
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
```

```
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105


<210> 433
<211> 360
<212> DNA
<213> Artificial Sequence

<220>
<223> Synthetic

<400> 433
gaggtgcagc tggtggagtc tgggggaggc ctggtcaagc cggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagag ttcactgtat 240
ctgcaaatga acagcctgag agccgaggac acggctgtgt attactgtgc gagagatggc 300
agcagttggt acgactactc tgactactgg ggccagggaa ccctggtcac cgtctcctca 360


<210> 434
<211> 120
<212> PRT
<213> Artificial Sequence

<220>
<223> Synthetic

<400> 434
Glu Val Gln Leu Val Glu Ser Gly Gly Gly Leu Val Lys Pro Gly Gly
 1               5                  10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Ser Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120


<210> 435
<211> 24
<212> DNA
<213> Artificial Sequence

<220>
<223> Synthetic

<400> 435
ggattcacct tcagtactta taac                                          24

<210> 436
<211> 8
<212> PRT
<213> Artificial Sequence
```

<220>
<223> Synthetic

<400> 436
Gly Phe Thr Phe Ser Thr Tyr Asn
1 5

<210> 437
<211> 24
<212> DNA
<213> Artificial Sequence

<220>
<223> Synthetic

<400> 437
attaggagta gtagtaatta cata 24

<210> 438
<211> 8
<212> PRT
<213> Artificial Sequence

<220>
<223> Synthetic

<400> 438
Ile Arg Ser Ser Ser Asn Tyr Ile
1 5

<210> 439
<211> 39
<212> DNA
<213> Artificial Sequence

<220>
<223> Synthetic

<400> 439
gcgagagatg gcagcagttg gtacgactac tctgactac 39

<210> 440
<211> 13
<212> PRT
<213> Artificial Sequence

<220>
<223> Synthetic

<400> 440
Ala Arg Asp Gly Ser Ser Trp Tyr Asp Tyr Ser Asp Tyr
1 5 10

<210> 441
<211> 321
<212> DNA
<213> Artificial Sequence

<220>
<223> Synthetic

<400> 441
gacatccaga tgacccagtc tccttccacc ctgtctgcat ctgtaggaga cagagtcacc 60
atcacttgcc gggccagtca gagtattagt agctggttgg cctggtatca acaggtacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 442
<211> 107
<212> PRT
<213> Artificial Sequence

<220>
<223> Synthetic

<400> 442
Asp Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Val Pro Gly Lys Ala Pro Lys Leu Leu Ile
35 40 45
Tyr Lys Ala Ser Ser Leu Glu Asn Gly Val Pro Ser Arg Phe Ser Gly
50 55 60
Ser Gly Ser Gly Thr Glu Phe Thr Leu Ile Ile Ser Ser Leu Gln Pro
65 70 75 80
Asp Asp Phe Ala Thr Tyr Tyr Cys Gln Gln Tyr Ile Ser Tyr Ser Arg
85 90 95
Thr Phe Gly Gln Gly Thr Lys Val Glu Ile Lys
100 105

<210> 443
<211> 18
<212> DNA
<213> Artificial Sequence

<220>
<223> Synthetic

<400> 443
cagagtatta gtagctgg 18

<210> 444
<211> 6
<212> PRT
<213> Artificial Sequence

<220>
<223> Synthetic

<400> 444
Gln Ser Ile Ser Ser Trp
1 5

<210> 445
<211> 9
<212> DNA
<213> Artificial Sequence

<220>
<223> Synthetic

<400> 445
aaggcgtct 9

<210> 446
<211> 3
<212> PRT
<213> Artificial Sequence

<220>
<223> Synthetic

<400> 446
Lys Ala Ser
1

<210> 447
<211> 27
<212> DNA
<213> Artificial Sequence

<220>
<223> Synthetic

<400> 447
caacagtata ttagttattc tcggacg 27

<210> 448
<211> 9
<212> PRT
<213> Artificial Sequence

<220>
<223> Synthetic

<400> 448
Gln Gln Tyr Ile Ser Tyr Ser Arg Thr
1 5

<210> 449
<211> 360
<212> DNA
<213> Artificial Sequence

<220>
<223> Synthetic

<400> 449
gaggtgcagc tggtggagtc tgggggaggc ctggtcaagc cggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagag ttcactgtat 240
ctgcaaatga acagcctgag agccgaggac acggctgtgt attactgtgc gagagatggc 300
agcagttggt acgactactc tgactactgg ggccagggaa ccctggtcac cgtctcctca 360

<210> 450
<211> 120
<212> PRT
<213> Artificial Sequence

<220>
<223> Synthetic

<400> 450
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Ser Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 451
<211> 321
<212> DNA
<213> Artificial Sequence

<220>
<223> Synthetic

<400> 451
gacatccaga tgacccagtc tccttccacc ctgtctgcat ctgtaggaga cagagtcacc 60
atcacttgcc gggccagtca gagtattagt agctggttgg cctggtatca acaggtacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 452
<211> 107
<212> PRT
<213> Artificial Sequence

<220>
<223> Synthetic

<400> 452
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Val Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 453
<211> 360
<212> DNA
<213> Artificial Sequence

<220>
<223> Synthetic

<400> 453
gaggtgcagc tggtggagtc tgggggaggc ctggtcaagc ctgggggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
agcagttggt acgactactc tgactactgg ggccagggaa ccctggtcac cgtctcctca 360

<210> 454
<211> 120
<212> PRT
<213> Artificial Sequence

<220>
<223> Synthetic

<400> 454
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 455
<211> 321
<212> DNA
<213> Artificial Sequence

<220>
<223> Synthetic

<400> 455
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a 321

<210> 456
<211> 107
<212> PRT
<213> Artificial Sequence

<220>
<223> Synthetic

<400> 456
Asp Ile Gln Met Thr Gln Ser Pro Ser Thr Leu Ser Ala Ser Val Gly

```
 1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105
```

<210> 457
<211> 360
<212> DNA
<213> Artificial Sequence

<220>
<223> Synthetic

<400> 457

```
gaggtgcagc tggtggagtc tgggggaggc ctggtcaagc cggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360
```

<210> 458
<211> 120
<212> PRT
<213> Artificial Sequence

<220>
<223> Synthetic

<400> 458

```
Glu Val Gln Leu Val Glu Ser Gly Gly Gly Leu Val Lys Pro Gly Gly
 1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120
```

<210> 459
<211> 24
<212> DNA
<213> Artificial Sequence

<220>

<223> Synthetic

<400> 459
ggattcacct tcagtactta taac 24

<210> 460
<211> 8
<212> PRT
<213> Artificial Sequence

<220>
<223> Synthetic

<400> 460
Gly Phe Thr Phe Ser Thr Tyr Asn
1 5

<210> 461
<211> 24
<212> DNA
<213> Artificial Sequence

<220>
<223> Synthetic

<400> 461
attaggagta gtagtaatta cata 24

<210> 462
<211> 8
<212> PRT
<213> Artificial Sequence

<220>
<223> Synthetic

<400> 462
Ile Arg Ser Ser Ser Asn Tyr Ile
1 5

<210> 463
<211> 39
<212> DNA
<213> Artificial Sequence

<220>
<223> Synthetic

<400> 463
gcgagagatg gcagcagttg gtacgactac tctgactac 39

<210> 464
<211> 13
<212> PRT
<213> Artificial Sequence

<220>
<223> Synthetic

<400> 464
Ala Arg Asp Gly Ser Ser Trp Tyr Asp Tyr Ser Asp Tyr
1 5 10

<210> 465
<211> 321
<212> DNA
<213> Artificial Sequence

<220>
<223> Synthetic

<400> 465
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 466
<211> 107
<212> PRT
<213> Artificial Sequence

<220>
<223> Synthetic

<400> 466
Asp Ile Gln Met Thr Gln Ser Pro Ser Thr Leu Ser Ala Ser Val Gly
1 5 10 15
Asp Arg Val Thr Ile Thr Cys Arg Ala Ser Gln Ser Ile Ser Ser Trp
20 25 30
Leu Ala Trp Tyr Gln Gln Ile Pro Gly Lys Ala Pro Lys Leu Leu Ile
35 40 45
Tyr Lys Ala Ser Ser Leu Glu Asn Gly Val Pro Ser Arg Phe Ser Gly
50 55 60
Ser Gly Ser Gly Thr Glu Phe Thr Leu Ile Ile Ser Ser Leu Gln Pro
65 70 75 80
Asp Asp Phe Ala Thr Tyr Tyr Cys Gln Gln Tyr Ile Ser Tyr Ser Arg
85 90 95
Thr Phe Gly Gln Gly Thr Lys Val Glu Ile Lys
100 105

<210> 467
<211> 18
<212> DNA
<213> Artificial Sequence

<220>
<223> Synthetic

<400> 467
cagagtatta gtagctgg 18

<210> 468
<211> 6
<212> PRT
<213> Artificial Sequence

<220>
<223> Synthetic

<400> 468
Gln Ser Ile Ser Ser Trp
1 5

<210> 469
<211> 9
<212> DNA
<213> Artificial Sequence

<220>
<223> Synthetic

<400> 469
aaggcgtct 9

<210> 470
<211> 3
<212> PRT
<213> Artificial Sequence

<220>
<223> Synthetic

<400> 470
Lys Ala Ser
1

<210> 471
<211> 27
<212> DNA
<213> Artificial Sequence

<220>
<223> Synthetic

<400> 471
caacagtata ttagttattc tcggacg 27

<210> 472
<211> 9
<212> PRT
<213> Artificial Sequence

<220>
<223> Synthetic

<400> 472
Gln Gln Tyr Ile Ser Tyr Ser Arg Thr
1 5

<210> 473
<211> 360
<212> DNA
<213> Artificial Sequence

<220>
<223> Synthetic

<400> 473
gaggtgcagc tggtggagtc tgggggaggc ctggtcaagc cggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 474
<211> 120
<212> PRT
<213> Artificial Sequence

<220>
<223> Synthetic

<400> 474
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 475
<211> 321
<212> DNA
<213> Artificial Sequence

<220>
<223> Synthetic

<400> 475
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 476
<211> 107
<212> PRT
<213> Artificial Sequence

<220>
<223> Synthetic

<400> 476
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95

Thr Phe Gly Gln Gly Thr Lys Val Glu Ile Lys
100 105

<210> 477
<211> 360
<212> DNA
<213> Artificial Sequence

<220>
<223> Synthetic

<400> 477
gaggtgcagc tggtggagtc tgggggaggc ctggtcaagc ctgggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
agcagttggt acgactactc tgactactgg ggccaaggaa ccctggtcac cgtctcctca 360

<210> 478
<211> 120
<212> PRT
<213> Artificial Sequence

<220>
<223> Synthetic

<400> 478
Glu Val Gln Leu Val Glu Ser Gly Gly Gly Leu Val Lys Pro Gly Gly
1 5 10 15
Ser Leu Arg Leu Ser Cys Ala Ala Ser Gly Phe Thr Phe Ser Thr Tyr
20 25 30
Asn Met Asn Trp Val Arg Gln Ala Pro Gly Lys Gly Leu Glu Trp Val
35 40 45
Ser Ser Ile Arg Ser Ser Ser Asn Tyr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Asp Gly Ser Ser Trp Tyr Asp Tyr Ser Asp Tyr Trp Gly Gln
100 105 110
Gly Thr Leu Val Thr Val Ser Ser
115 120

<210> 479
<211> 321
<212> DNA
<213> Artificial Sequence

<220>
<223> Synthetic

<400> 479
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a 321

<210> 480

<211> 107
<212> PRT
<213> Artificial Sequence

<220>
<223> Synthetic

<400> 480
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 481
<211> 354
<212> DNA
<213> Artificial Sequence

<220>
<223> Synthetic

<400> 481
gaggtgcaac tagtggagtc tgggggaggc ttggtacagc ctggggggtc cctgagactc 60
tcctgtgtag tctctggatt caccttcggt gactacgaca tgcactgggt ccgtcaagct 120
acaggaagag gtctggagtg ggtctcaggt attgctcctg ctggtgacac atcctataca 180
ggctccgtga agggccgatt caccatctcc agagagaatg ccaagaactc cttgcatctt 240
caaatgaaca gcctgacaac cggggacacg gctatatatt attgtgctag agaggatata 300
gcagtgcctg gttttgatta ctggggccag ggaaccctgg tcaccgtctc ctca 354

<210> 482
<211> 118
<212> PRT
<213> Artificial Sequence

<220>
<223> Synthetic

<400> 482
Glu Val Gln Leu Val Glu Ser Gly Gly Gly Leu Val Gln Pro Gly Gly
1               5                   10                  15
Ser Leu Arg Leu Ser Cys Val Val Ser Gly Phe Thr Phe Gly Asp Tyr
            20                  25                  30
Asp Met His Trp Val Arg Gln Ala Thr Gly Arg Gly Leu Glu Trp Val
        35                  40                  45
Ser Gly Ile Ala Pro Ala Gly Asp Thr Ser Tyr Thr Gly Ser Val Lys
    50                  55                  60
Gly Arg Phe Thr Ile Ser Arg Glu Asn Ala Lys Asn Ser Leu His Leu
65                  70                  75                  80
Gln Met Asn Ser Leu Thr Thr Gly Asp Thr Ala Ile Tyr Tyr Cys Ala
                85                  90                  95
Arg Glu Asp Ile Ala Val Pro Gly Phe Asp Tyr Trp Gly Gln Gly Thr
            100                 105                 110
Leu Val Thr Val Ser Ser
        115

<210> 483
<211> 24
<212> DNA
<213> Artificial Sequence

<220>
<223> Synthetic

<400> 483
ggattcacct tcggtgacta cgac 24

<210> 484
<211> 8
<212> PRT
<213> Artificial Sequence

<220>
<223> Synthetic

<400> 484
Gly Phe Thr Phe Gly Asp Tyr Asp
1 5

<210> 485
<211> 21
<212> DNA
<213> Artificial Sequence

<220>
<223> Synthetic

<400> 485
attgctcctg ctggtgacac a 21

<210> 486
<211> 7
<212> PRT
<213> Artificial Sequence

<220>
<223> Synthetic

<400> 486
Ile Ala Pro Ala Gly Asp Thr
1 5

<210> 487
<211> 36
<212> DNA
<213> Artificial Sequence

<220>
<223> Synthetic

<400> 487
gctagagagg atatagcagt gcctggtttt gattac 36

<210> 488
<211> 12
<212> PRT
<213> Artificial Sequence

<220>
<223> Synthetic

<400> 488
Ala Arg Glu Asp Ile Ala Val Pro Gly Phe Asp Tyr
1               5                   10

<210> 489
<211> 324
<212> DNA
<213> Artificial Sequence

<220>
<223> Synthetic

<400> 489
gaaatagtga tgacgcagtc tccagccacc ctgtctgtgt ctccagggga acgaggcacc 60
ctctcctgca gggccagtca gagtgttagc agcaacttag cctggtacca gcagaaacct 120
ggccaggctc ccagactcct catctatggt gcatccacga gggccactgg cttcccagcc 180
aggttcagtg gcagtgggtc tgggacagag ttcactctca ccatcagcag cctgcagtct 240
gaagattttg cagtttatta ctgtcagcag tataataagt ggcctccgtt cactttcggc 300
cctgggacca aagtggattt caaa 324

<210> 490
<211> 108
<212> PRT
<213> Artificial Sequence

<220>
<223> Synthetic

<400> 490
Glu Ile Val Met Thr Gln Ser Pro Ala Thr Leu Ser Val Ser Pro Gly
1               5                   10                  15
Glu Arg Gly Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Phe Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Lys Trp Pro Pro
                85                  90                  95
Phe Thr Phe Gly Pro Gly Thr Lys Val Asp Phe Lys
            100                 105

<210> 491
<211> 18
<212> DNA
<213> Artificial Sequence

<220>
<223> Synthetic

<400> 491
cagagtgtta gcagcaac 18

<210> 492
<211> 6
<212> PRT
<213> Artificial Sequence

<220>
<223> Synthetic

<400> 492
Gln Ser Val Ser Ser Asn
1 5

<210> 493
<211> 9
<212> DNA
<213> Artificial Sequence

<220>
<223> Synthetic

<400> 493
ggtgcatcc 9

<210> 494
<211> 3
<212> PRT
<213> Artificial Sequence

<220>
<223> Synthetic

<400> 494
Gly Ala Ser
1

<210> 495
<211> 30
<212> DNA
<213> Artificial Sequence

<220>
<223> Synthetic

<400> 495
cagcagtata ataagtggcc tccgttcact 30

<210> 496
<211> 10
<212> PRT
<213> Artificial Sequence

<220>
<223> Synthetic

<400> 496
Gln Gln Tyr Asn Lys Trp Pro Pro Phe Thr
1 5 10

<210> 497
<211> 354
<212> DNA
<213> Artificial Sequence

<220>
<223> Synthetic

<400> 497
gaggtgcaac tagtggagtc tgggggaggc ttggtacagc ctggggggtc cctgagactc 60
tcctgtgtag tctctggatt caccttcggt gactacgaca tgcactgggt ccgtcaagct 120
acaggaagag gtctggagtg ggtctcaggt attgctcctg ctggtgacac atcctataca 180
ggctccgtga agggccgatt caccatctcc agagagaatg ccaagaactc cttgcatctt 240
caaatgaaca gcctgacaac cggggacacg gctatatatt attgtgctag agaggatata 300
gcagtgcctg gttttgatta ctggggccag ggaaccctgg tcaccgtctc ctca 354

<210> 498
<211> 118
<212> PRT
<213> Artificial Sequence

<220>
<223> Synthetic

<400> 498
Glu Val Gln Leu Val Glu Ser Gly Gly Gly Leu Val Gln Pro Gly Gly
1 5 10 15
Ser Leu Arg Leu Ser Cys Val Val Ser Gly Phe Thr Phe Gly Asp Tyr
20 25 30
Asp Met His Trp Val Arg Gln Ala Thr Gly Arg Gly Leu Glu Trp Val
35 40 45
Ser Gly Ile Ala Pro Ala Gly Asp Thr Ser Tyr Thr Gly Ser Val Lys
50 55 60
Gly Arg Phe Thr Ile Ser Arg Glu Asn Ala Lys Asn Ser Leu His Leu
65 70 75 80
Gln Met Asn Ser Leu Thr Thr Gly Asp Thr Ala Ile Tyr Tyr Cys Ala
85 90 95
Arg Glu Asp Ile Ala Val Pro Gly Phe Asp Tyr Trp Gly Gln Gly Thr
100 105 110
Leu Val Thr Val Ser Ser
115

<210> 499
<211> 324
<212> DNA
<213> Artificial Sequence

<220>
<223> Synthetic

<400> 499
gaaatagtga tgacgcagtc tccagccacc ctgtctgtgt ctccagggga acgaggcacc 60
ctctcctgca gggccagtca gagtgttagc agcaacttag cctggtacca gcagaaacct 120
ggccaggctc ccagactcct catctatggt gcatccacga gggccactgg cttcccagcc 180
aggttcagtg gcagtgggtc tgggacagag ttcactctca ccatcagcag cctgcagtct 240
gaagattttg cagtttatta ctgtcagcag tataataagt ggcctccgtt cactttcggc 300
cctgggacca aagtggatat caaa 324

<210> 500
<211> 108
<212> PRT
<213> Artificial Sequence

<220>
<223> Synthetic

<400> 500
Glu Ile Val Met Thr Gln Ser Pro Ala Thr Leu Ser Val Ser Pro Gly
1 5 10 15
Glu Arg Gly Thr Leu Ser Cys Arg Ala Ser Gln Ser Val Ser Ser Asn
20 25 30
Leu Ala Trp Tyr Gln Gln Lys Pro Gly Gln Ala Pro Arg Leu Leu Ile

```
        35                  40                  45
Tyr Gly Ala Ser Thr Arg Ala Thr Gly Phe Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Lys Trp Pro Pro
                85                  90                  95
Phe Thr Phe Gly Pro Gly Thr Lys Val Asp Ile Lys
            100                 105
```

<210> 501
<211> 354
<212> DNA
<213> Artificial Sequence

<220>
<223> Synthetic

<400> 501

```
gaggtgcagc tggtggagtc tgggggaggc ttggtacagc ctggggggtc cctgagactc 60
tcctgtgcag cctctggatt caccttcggt gactacgaca tgcactgggt ccgccaagct 120
acaggaaaag gtctggagtg ggtctcagct attgctcctg ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgctag agaggatata 300
gcagtgcctg gttttgatta ctggggccaa ggaaccctgg tcaccgtctc ctca       354
```

<210> 502
<211> 118
<212> PRT
<213> Artificial Sequence

<220>
<223> Synthetic

<400> 502

```
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Phe Gly Asp Tyr
            20                  25                  30
Asp Met His Trp Val Arg Gln Ala Thr Gly Lys Gly Leu Glu Trp Val
        35                  40                  45
Ser Ala Ile Ala Pro Ala Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Asp Ile Ala Val Pro Gly Phe Asp Tyr Trp Gly Gln Gly Thr
            100                 105                 110
Leu Val Thr Val Ser Ser
        115
```

<210> 503
<211> 324
<212> DNA
<213> Artificial Sequence

<220>
<223> Synthetic

<400> 503

```
gaaatagtga tgacgcagtc tccagccacc ctgtctgtgt ctccagggga aagagccacc 60
ctctcctgca gggccagtca gagtgttagc agcaacttag cctggtacca gcagaaacct 120
```

ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataagt ggcctccgtt cactttcggc 300
cctgggacca aagtggatat caaa 324

<210> 504
<211> 108
<212> PRT
<213> Artificial Sequence

<220>
<223> Synthetic

<400> 504
Glu Ile Val Met Thr Gln Ser Pro Ala Thr Leu Ser Val Ser Pro Gly
1 5 10 15
Glu Arg Ala Thr Leu Ser Cys Arg Ala Ser Gln Ser Val Ser Ser Asn
20 25 30
Leu Ala Trp Tyr Gln Gln Lys Pro Gly Gln Ala Pro Arg Leu Leu Ile
35 40 45
Tyr Gly Ala Ser Thr Arg Ala Thr Gly Ile Pro Ala Arg Phe Ser Gly
50 55 60
Ser Gly Ser Gly Thr Glu Phe Thr Leu Thr Ile Ser Ser Leu Gln Ser
65 70 75 80
Glu Asp Phe Ala Val Tyr Tyr Cys Gln Gln Tyr Asn Lys Trp Pro Pro
85 90 95
Phe Thr Phe Gly Pro Gly Thr Lys Val Asp Ile Lys
100 105

<210> 505
<211> 378
<212> DNA
<213> Artificial Sequence

<220>
<223> Synthetic

<400> 505
caaattctgc tggtgcaatc tggacctgag gtgaaggagc ctggggcctc agtgaaggtc 60
tcctgcaagg cttctggtta cacctttacc aactacgcta tcagctgggt gcgacaggtc 120
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca 378

<210> 506
<211> 126
<212> PRT
<213> Artificial Sequence

<220>
<223> Synthetic

<400> 506
Gln Ile Leu Leu Val Gln Ser Gly Pro Glu Val Lys Glu Pro Gly Ala
1 5 10 15
Ser Val Lys Val Ser Cys Lys Ala Ser Gly Tyr Thr Phe Thr Asn Tyr
20 25 30
Ala Ile Ser Trp Val Arg Gln Val Pro Gly Gln Gly Leu Glu Trp Met
35 40 45
Gly Trp Val Ser Ala Tyr Asn Gly His Thr Asn Tyr Ala His Glu Val
50 55 60
Gln Gly Arg Val Thr Met Thr Thr Asp Thr Ser Thr Thr Thr Ala Tyr

```
65                  70                  75                  80
Met Glu Leu Arg Ser Leu Arg Ser Asp Asp Thr Ala Met Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125
```

<210> 507
<211> 24
<212> DNA
<213> Artificial Sequence

<220>
<223> Synthetic

<400> 507
ggttacacct ttaccaacta cgct 24

<210> 508
<211> 8
<212> PRT
<213> Artificial Sequence

<220>
<223> Synthetic

<400> 508

```
Gly Tyr Thr Phe Thr Asn Tyr Ala
 1               5
```

<210> 509
<211> 24
<212> DNA
<213> Artificial Sequence

<220>
<223> Synthetic

<400> 509
gtcagcgctt acaatggtca caca 24

<210> 510
<211> 8
<212> PRT
<213> Artificial Sequence

<220>
<223> Synthetic

<400> 510

```
Val Ser Ala Tyr Asn Gly His Thr
 1               5
```

<210> 511
<211> 57
<212> DNA
<213> Artificial Sequence

<220>
<223> Synthetic

<400> 511
gcgagagggg gtgtagtcgt gccagttgct ccccacttct acaacggtat ggacgtc 57

<210> 512
<211> 19
<212> PRT
<213> Artificial Sequence

<220>
<223> Synthetic

<400> 512
Ala Arg Gly Gly Val Val Val Pro Val Ala Pro His Phe Tyr Asn Gly
1 5 10 15
Met Asp Val

<210> 513
<211> 336
<212> DNA
<213> Artificial Sequence

<220>
<223> Synthetic

<400> 513
gatattgtga tgactcagtt tccactctcc ctgcccgtca cccctggaga gccggcctcc 60
atctcctgca ggtctagtca gagcctcctg catattaatg aatacaacta tttggattgg 120
tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttag gccaagggac caaggtggaa atcaaa 336

<210> 514
<211> 112
<212> PRT
<213> Artificial Sequence

<220>
<223> Synthetic

<400> 514
Asp Ile Val Met Thr Gln Phe Pro Leu Ser Leu Pro Val Thr Pro Gly
1 5 10 15
Glu Pro Ala Ser Ile Ser Cys Arg Ser Ser Gln Ser Leu Leu His Ile
20 25 30
Asn Glu Tyr Asn Tyr Leu Asp Trp Tyr Leu Lys Lys Pro Gly Gln Ser
35 40 45
Pro Gln Leu Leu Ile Tyr Leu Gly Phe Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Trp Thr Leu Gly Gln Gly Thr Lys Val Glu Ile Lys
100 105 110

<210> 515
<211> 33
<212> DNA
<213> Artificial Sequence

<220>

<223> Synthetic

<400> 515
cagagcctcc tgcatattaa tgaatacaac tat 33

<210> 516
<211> 11
<212> PRT
<213> Artificial Sequence

<220>
<223> Synthetic

<400> 516
Gln Ser Leu Leu His Ile Asn Glu Tyr Asn Tyr
1 5 10

<210> 517
<211> 9
<212> DNA
<213> Artificial Sequence

<220>
<223> Synthetic

<400> 517
ttgggtttt 9

<210> 518
<211> 3
<212> PRT
<213> Artificial Sequence

<220>
<223> Synthetic

<400> 518
Leu Gly Phe
1

<210> 519
<211> 27
<212> DNA
<213> Artificial Sequence

<220>
<223> Synthetic

<400> 519
atgcaagctc ttcaaactcc gtggacg 27

<210> 520
<211> 9
<212> PRT
<213> Artificial Sequence

<220>
<223> Synthetic

<400> 520
Met Gln Ala Leu Gln Thr Pro Trp Thr
1 5

<210> 521
<211> 378
<212> DNA
<213> Artificial Sequence

<220>
<223> Synthetic

<400> 521
caggttcagc tggtgcagtc tggacctgag gtgaaggagc ctggggcctc agtgaaggtc 60
tcctgcaagg cttctggtta cacctttacc aactacgcta tcagctgggt gcgacaggtc 120
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca 378

<210> 522
<211> 126
<212> PRT
<213> Artificial Sequence

<220>
<223> Synthetic

<400> 522
Gln Val Gln Leu Val Gln Ser Gly Pro Glu Val Lys Glu Pro Gly Ala
1 5 10 15
Ser Val Lys Val Ser Cys Lys Ala Ser Gly Tyr Thr Phe Thr Asn Tyr
20 25 30
Ala Ile Ser Trp Val Arg Gln Val Pro Gly Gln Gly Leu Glu Trp Met
35 40 45
Gly Trp Val Ser Ala Tyr Asn Gly His Thr Asn Tyr Ala His Glu Val
50 55 60
Gln Gly Arg Val Thr Met Thr Thr Asp Thr Ser Thr Thr Thr Ala Tyr
65 70 75 80
Met Glu Leu Arg Ser Leu Arg Ser Asp Asp Thr Ala Met Tyr Tyr Cys
85 90 95
Ala Arg Gly Gly Val Val Val Pro Val Ala Pro His Phe Tyr Asn Gly
100 105 110
Met Asp Val Trp Gly Gln Gly Thr Thr Val Thr Val Ser Ser
115 120 125

<210> 523
<211> 336
<212> DNA
<213> Artificial Sequence

<220>
<223> Synthetic

<400> 523
gatattgtga tgactcagtc tccactctcc ctgcccgtca cccctggaga gccggcctcc 60
atctcctgca ggtctagtca gagcctcctg catattaatg aatacaacta tttggattgg 120
tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttag gccaagggac caaggtggaa atcaaa 336

<210> 524
<211> 112
<212> PRT
<213> Artificial Sequence

<220>
<223> Synthetic

<400> 524

```
Asp Ile Val Met Thr Gln Ser Pro Leu Ser Leu Pro Val Thr Pro Gly
 1               5                  10                  15
Glu Pro Ala Ser Ile Ser Cys Arg Ser Ser Gln Ser Leu Leu His Ile
            20                  25                  30
Asn Glu Tyr Asn Tyr Leu Asp Trp Tyr Leu Lys Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Trp Thr Leu Gly Gln Gly Thr Lys Val Glu Ile Lys
            100                 105                 110
```

<210> 525
<211> 378
<212> DNA
<213> Artificial Sequence

<220>
<223> Synthetic

<400> 525

```
caggttcagc tggtgcagtc tggagctgag gtgaagaagc ctggggcctc agtgaaggtc 60
tcctgcaagg cttctggtta cacctttacc aactacgcta tcagctgggt gcgacaggcc 120
cctggacaag ggcttgagtg gatgggatgg gtcagcgctt acaatggtca cacaaactat 180
gcacagaagc tccagggcag agtcaccatg accacagaca catccacgag cacagcctac 240
atggagctga ggagcctgag atctgacgac acggccgtgt attactgtgc gagagggggt 300
gtagtcgtgc cagttgctcc ccacttctac aacggtatgg acgtctgggg gcaagggacc 360
acggtcaccg tctcctca                                                378
```

<210> 526
<211> 126
<212> PRT
<213> Artificial Sequence

<220>
<223> Synthetic

<400> 526

```
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Asn Tyr
            20                  25                  30
Ala Ile Ser Trp Val Arg Gln Ala Pro Gly Gln Gly Leu Glu Trp Met
        35                  40                  45
Gly Trp Val Ser Ala Tyr Asn Gly His Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125
```

<210> 527
<211> 336
<212> DNA
<213> Artificial Sequence

<220>
<223> Synthetic

<400> 527
gatattgtga tgactcagtc tccactctcc ctgcccgtca cccctggaga gccggcctcc 60
atctcctgca ggtctagtca gagcctcctg catattaatg aatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct tcaaactccg 300
tggacgttcg gccaagggac caaggtggaa atcaaa 336

<210> 528
<211> 112
<212> PRT
<213> Artificial Sequence

<220>
<223> Synthetic

<400> 528
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ile
20 25 30
Asn Glu Tyr Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Trp Thr Phe Gly Gln Gly Thr Lys Val Glu Ile Lys
100 105 110

<210> 529
<211> 351
<212> DNA
<213> Artificial Sequence

<220>
<223> Synthetic

<400> 529
gaggtgcagc tggtggagtc tgggggaggc ttggtacagc ctggggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180
ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga gcccgggtca ccgtctcctc a 351

<210> 530
<211> 117
<212> PRT
<213> Artificial Sequence

<220>
<223> Synthetic

<400> 530
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Thr Gly Ser Val Met
    50                  55                  60
Gly Arg Phe Thr Ile Ser Arg Asp Ala Ala Lys Asn Ser Phe Tyr Leu
65                  70                  75                  80
Glu Met Asn Ser Leu Arg Val Gly Asp Thr Ala Val Tyr Tyr Cys Ala
                85                  90                  95
Arg Glu Gly Ile Arg Thr Pro Tyr Asp Tyr Trp Gly Gln Gly Ala Arg
            100                 105                 110
Val Thr Val Ser Ser
        115

<210> 531
<211> 24
<212> DNA
<213> Artificial Sequence

<220>
<223> Synthetic

<400> 531
ggattcaccc taagtagcta cgac 24

<210> 532
<211> 8
<212> PRT
<213> Artificial Sequence

<220>
<223> Synthetic

<400> 532
Gly Phe Thr Leu Ser Ser Tyr Asp
1               5

<210> 533
<211> 21
<212> DNA
<213> Artificial Sequence

<220>
<223> Synthetic

<400> 533
attggcagta ctggtgacac a 21

<210> 534
<211> 7
<212> PRT
<213> Artificial Sequence

<220>
<223> Synthetic

<400> 534
Ile Gly Ser Thr Gly Asp Thr
1               5

<210> 535
<211> 33
<212> DNA
<213> Artificial Sequence

<220>
<223> Synthetic

<400> 535
gcaagagagg gaataagaac accctatgat tat 33

<210> 536
<211> 11
<212> PRT
<213> Artificial Sequence

<220>
<223> Synthetic

<400> 536
Ala Arg Glu Gly Ile Arg Thr Pro Tyr Asp Tyr
1 5 10

<210> 537
<211> 324
<212> DNA
<213> Artificial Sequence

<220>
<223> Synthetic

<400> 537
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240
gaagattttg cagtttatta ctgtcagcag tataataatt ggcctccatt cactttcggc 300
cctgggacca aagtggatat caaa 324

<210> 538
<211> 108
<212> PRT
<213> Artificial Sequence

<220>
<223> Synthetic

<400> 538
Glu Ile Val Met Thr Gln Ser Pro Ala Thr Leu Ser Val Ser Pro Gly
1 5 10 15
Glu Arg Ala Thr Leu Ser Cys Arg Ala Ser Gln Ser Val Ser Ser Asn
20 25 30
Val Ala Trp Tyr Gln Gln Lys Pro Gly Gln Ala Pro Arg Leu Leu Ile
35 40 45
Tyr Gly Ala Ser Thr Arg Ala Thr Gly Ile Pro Ala Arg Phe Ser Gly
50 55 60
Ser Gly Ser Gly Thr Glu Phe Thr Leu Thr Ile Ser Ser Leu Gln Ser
65 70 75 80
Glu Asp Phe Ala Val Tyr Tyr Cys Gln Gln Tyr Asn Asn Trp Pro Pro
85 90 95
Phe Thr Phe Gly Pro Gly Thr Lys Val Asp Ile Lys
100 105

<210> 539
<211> 18
<212> DNA
<213> Artificial Sequence

<220>
<223> Synthetic

<400> 539
cagagtgtta gcagcaat 18

<210> 540
<211> 6
<212> PRT
<213> Artificial Sequence

<220>
<223> Synthetic

<400> 540
Gln Ser Val Ser Ser Asn
1 5

<210> 541
<211> 9
<212> DNA
<213> Artificial Sequence

<220>
<223> Synthetic

<400> 541
ggtgcatcc 9

<210> 542
<211> 3
<212> PRT
<213> Artificial Sequence

<220>
<223> Synthetic

<400> 542
Gly Ala Ser
1

<210> 543
<211> 30
<212> DNA
<213> Artificial Sequence

<220>
<223> Synthetic

<400> 543
cagcagtata ataattggcc tccattcact 30

<210> 544
<211> 10
<212> PRT
<213> Artificial Sequence

<220>
<223> Synthetic

<400> 544
Gln Gln Tyr Asn Asn Trp Pro Pro Phe Thr
1               5                   10

<210> 545
<211> 351
<212> DNA
<213> Artificial Sequence

<220>
<223> Synthetic

<400> 545
gaggtgcagc tggtggagtc tgggggaggc ttggtacagc ctggggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180
ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga accctggtca ccgtctcctc a          351

<210> 546
<211> 117
<212> PRT
<213> Artificial Sequence

<220>
<223> Synthetic

<400> 546
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Thr Gly Ser Val Met
    50                  55                  60
Gly Arg Phe Thr Ile Ser Arg Asp Ala Ala Lys Asn Ser Phe Tyr Leu
65                  70                  75                  80
Glu Met Asn Ser Leu Arg Val Gly Asp Thr Ala Val Tyr Tyr Cys Ala
                85                  90                  95
Arg Glu Gly Ile Arg Thr Pro Tyr Asp Tyr Trp Gly Gln Gly Thr Leu
            100                 105                 110
Val Thr Val Ser Ser
        115

<210> 547
<211> 324
<212> DNA
<213> Artificial Sequence

<220>
<223> Synthetic

<400> 547
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240

gaagattttg cagtttatta ctgtcagcag tataataatt ggcctccatt cactttcggc 300
cctgggacca aagtggatat caaa 324

<210> 548
<211> 108
<212> PRT
<213> Artificial Sequence

<220>
<223> Synthetic

<400> 548
Glu Ile Val Met Thr Gln Ser Pro Ala Thr Leu Ser Val Ser Pro Gly
1 5 10 15
Glu Arg Ala Thr Leu Ser Cys Arg Ala Ser Gln Ser Val Ser Ser Asn
20 25 30
Val Ala Trp Tyr Gln Gln Lys Pro Gly Gln Ala Pro Arg Leu Leu Ile
35 40 45
Tyr Gly Ala Ser Thr Arg Ala Thr Gly Ile Pro Ala Arg Phe Ser Gly
50 55 60
Ser Gly Ser Gly Thr Glu Phe Thr Leu Thr Ile Ser Ser Leu Gln Ser
65 70 75 80
Glu Asp Phe Ala Val Tyr Tyr Cys Gln Gln Tyr Asn Asn Trp Pro Pro
85 90 95
Phe Thr Phe Gly Pro Gly Thr Lys Val Asp Ile Lys
100 105

<210> 549
<211> 351
<212> DNA
<213> Artificial Sequence

<220>
<223> Synthetic

<400> 549
gaggtgcagc tggtggagtc tgggggaggc ttggtacagc ctggggggtc cctgagactc 60
tcctgtgcag cctctggatt caccctaagt agctacgaca tgcactgggt ccgccaagct 120
acaggaaaag gtctggagtg ggtctcagct attggcagta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggaata 300
agaacaccct atgattattg gggccaagga accctggtca ccgtctcctc a 351

<210> 550
<211> 117
<212> PRT
<213> Artificial Sequence

<220>
<223> Synthetic

<400> 550
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Leu Ser Ser Tyr
20 25 30
Asp Met His Trp Val Arg Gln Ala Thr Gly Lys Gly Leu Glu Trp Val
35 40 45
Ser Ala Ile Gly Ser Thr Gly Asp Thr Tyr Tyr Pro Gly Ser Val Lys
50 55 60
Gly Arg Phe Thr Ile Ser Arg Glu Asn Ala Lys Asn Ser Leu Tyr Leu
65 70 75 80
Gln Met Asn Ser Leu Arg Ala Gly Asp Thr Ala Val Tyr Tyr Cys Ala
85 90 95

Arg Glu Gly Ile Arg Thr Pro Tyr Asp Tyr Trp Gly Gln Gly Thr Leu
100 105 110
Val Thr Val Ser Ser
115

<210> 551
<211> 324
<212> DNA
<213> Artificial Sequence

<220>
<223> Synthetic

<400> 551
gaaatagtga tgacgcagtc tccagccacc ctgtctgtgt ctccagggga aagagccacc 60
ctctcctgca gggccagtca gagtgttagc agcaatttag cctggtacca gcagaaacct 120
ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataatt ggcctccatt cactttcggc 300
cctgggacca aagtggatat caaa 324

<210> 552
<211> 108
<212> PRT
<213> Artificial Sequence

<220>
<223> Synthetic

<400> 552
Glu Ile Val Met Thr Gln Ser Pro Ala Thr Leu Ser Val Ser Pro Gly
1 5 10 15
Glu Arg Ala Thr Leu Ser Cys Arg Ala Ser Gln Ser Val Ser Ser Asn
20 25 30
Leu Ala Trp Tyr Gln Gln Lys Pro Gly Gln Ala Pro Arg Leu Leu Ile
35 40 45
Tyr Gly Ala Ser Thr Arg Ala Thr Gly Ile Pro Ala Arg Phe Ser Gly
50 55 60
Ser Gly Ser Gly Thr Glu Phe Thr Leu Thr Ile Ser Ser Leu Gln Ser
65 70 75 80
Glu Asp Phe Ala Val Tyr Tyr Cys Gln Gln Tyr Asn Asn Trp Pro Pro
85 90 95
Phe Thr Phe Gly Pro Gly Thr Lys Val Asp Ile Lys
100 105

<210> 553
<211> 351
<212> DNA
<213> Artificial Sequence

<220>
<223> Synthetic

<400> 553
gaggtgcagc tggtggagtc tgggggaggc ttggtacagc ctggggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180
ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga gcccgggtca ccgtctcctc a 351

<210> 554
<211> 117

<212> PRT
<213> Artificial Sequence

<220>
<223> Synthetic

<400> 554
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Thr Gly Ser Val Met
    50                  55                  60
Gly Arg Phe Thr Ile Ser Arg Asp Ala Ala Lys Asn Ser Phe Tyr Leu
65                  70                  75                  80
Glu Met Asn Ser Leu Arg Val Gly Asp Thr Ala Val Tyr Tyr Cys Ala
                85                  90                  95
Arg Glu Gly Ile Arg Thr Pro Tyr Asp Tyr Trp Gly Gln Gly Ala Arg
            100                 105                 110
Val Thr Val Ser Ser
        115

<210> 555
<211> 24
<212> DNA
<213> Artificial Sequence

<220>
<223> Synthetic

<400> 555
ggattcaccc taagtagcta cgac 24

<210> 556
<211> 8
<212> PRT
<213> Artificial Sequence

<220>
<223> Synthetic

<400> 556
Gly Phe Thr Leu Ser Ser Tyr Asp
1               5

<210> 557
<211> 21
<212> DNA
<213> Artificial Sequence

<220>
<223> Synthetic

<400> 557
attggcagta ctggtgacac a 21

<210> 558
<211> 7
<212> PRT
<213> Artificial Sequence

<220>
<223> Synthetic

<400> 558
Ile Gly Ser Thr Gly Asp Thr
1 5

<210> 559
<211> 33
<212> DNA
<213> Artificial Sequence

<220>
<223> Synthetic

<400> 559
gcaagagagg gaataagaac accctatgat tat 33

<210> 560
<211> 11
<212> PRT
<213> Artificial Sequence

<220>
<223> Synthetic

<400> 560
Ala Arg Glu Gly Ile Arg Thr Pro Tyr Asp Tyr
1 5 10

<210> 561
<211> 324
<212> DNA
<213> Artificial Sequence

<220>
<223> Synthetic

<400> 561
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240
gaagattttg cagtttatta ctgtcagcag tataataatt ggcctccatt cactttcggc 300
cctgggacca aagtggatat caaa 324

<210> 562
<211> 108
<212> PRT
<213> Artificial Sequence

<220>
<223> Synthetic

<400> 562
Glu Ile Val Met Thr Gln Ser Pro Ala Thr Leu Ser Val Ser Pro Gly
1 5 10 15
Glu Arg Ala Thr Leu Ser Cys Arg Ala Ser Gln Ser Val Ser Ser Asn
20 25 30
Val Ala Trp Tyr Gln Gln Lys Pro Gly Gln Ala Pro Arg Leu Leu Ile
35 40 45
Tyr Gly Ala Ser Thr Arg Ala Thr Gly Ile Pro Ala Arg Phe Ser Gly
50 55 60

Ser Gly Ser Gly Thr Glu Phe Thr Leu Thr Ile Ser Ser Leu Gln Ser
65 70 75 80
Glu Asp Phe Ala Val Tyr Tyr Cys Gln Gln Tyr Asn Asn Trp Pro Pro
85 90 95
Phe Thr Phe Gly Pro Gly Thr Lys Val Asp Ile Lys
100 105

<210> 563
<211> 18
<212> DNA
<213> Artificial Sequence

<220>
<223> Synthetic

<400> 563
cagagtgtta gcagcaat 18

<210> 564
<211> 6
<212> PRT
<213> Artificial Sequence

<220>
<223> Synthetic

<400> 564
Gln Ser Val Ser Ser Asn
1 5

<210> 565
<211> 9
<212> DNA
<213> Artificial Sequence

<220>
<223> Synthetic

<400> 565
ggtgcatcc 9

<210> 566
<211> 3
<212> PRT
<213> Artificial Sequence

<220>
<223> Synthetic

<400> 566
Gly Ala Ser
1

<210> 567
<211> 30
<212> DNA
<213> Artificial Sequence

<220>
<223> Synthetic

<400> 567

cagcagtata ataattggcc tccattcact 30

<210> 568
<211> 10
<212> PRT
<213> Artificial Sequence

<220>
<223> Synthetic

<400> 568
Gln Gln Tyr Asn Asn Trp Pro Pro Phe Thr
1 5 10

<210> 569
<211> 351
<212> DNA
<213> Artificial Sequence

<220>
<223> Synthetic

<400> 569
gaggtgcagc tggtggagtc tgggggaggc ttggtacagc ctgggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180
ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga accctggtca ccgtctcctc a 351

<210> 570
<211> 117
<212> PRT
<213> Artificial Sequence

<220>
<223> Synthetic

<400> 570
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Leu Ser Ser Tyr
20 25 30
Asp Met His Trp Val Arg Gln Ala Thr Gly Lys Gly Leu Glu Trp Val
35 40 45
Ser Ala Ile Gly Ser Thr Gly Asp Thr Tyr Tyr Thr Gly Ser Val Met
50 55 60
Gly Arg Phe Thr Ile Ser Arg Asp Ala Ala Lys Asn Ser Phe Tyr Leu
65 70 75 80
Glu Met Asn Ser Leu Arg Val Gly Asp Thr Ala Val Tyr Tyr Cys Ala
85 90 95
Arg Glu Gly Ile Arg Thr Pro Tyr Asp Tyr Trp Gly Gln Gly Thr Leu
100 105 110
Val Thr Val Ser Ser
115

<210> 571
<211> 324
<212> DNA
<213> Artificial Sequence

<220>
<223> Synthetic

<400> 571
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240
gaagattttg cagtttatta ctgtcagcag tataataatt ggcctccatt cactttcggc 300
cctgggacca aagtggatat caaa 324

<210> 572
<211> 108
<212> PRT
<213> Artificial Sequence

<220>
<223> Synthetic

<400> 572
Glu Ile Val Met Thr Gln Ser Pro Ala Thr Leu Ser Val Ser Pro Gly
1 5 10 15
Glu Arg Ala Thr Leu Ser Cys Arg Ala Ser Gln Ser Val Ser Ser Asn
20 25 30
Val Ala Trp Tyr Gln Gln Lys Pro Gly Gln Ala Pro Arg Leu Leu Ile
35 40 45
Tyr Gly Ala Ser Thr Arg Ala Thr Gly Ile Pro Ala Arg Phe Ser Gly
50 55 60
Ser Gly Ser Gly Thr Glu Phe Thr Leu Thr Ile Ser Ser Leu Gln Ser
65 70 75 80
Glu Asp Phe Ala Val Tyr Tyr Cys Gln Gln Tyr Asn Asn Trp Pro Pro
85 90 95
Phe Thr Phe Gly Pro Gly Thr Lys Val Asp Ile Lys
100 105

<210> 573
<211> 351
<212> DNA
<213> Artificial Sequence

<220>
<223> Synthetic

<400> 573
gaggtgcagc tggtggagtc tgggggaggc ttggtacagc ctggggggtc cctgagactc 60
tcctgtgcag cctctggatt caccctaagt agctacgaca tgcactgggt ccgccaagct 120
acaggaaaag gtctggagtg ggtctcagct attggcagta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggaata 300
agaacaccct atgattattg gggccaagga accctggtca ccgtctcctc a 351

<210> 574
<211> 117
<212> PRT
<213> Artificial Sequence

<220>
<223> Synthetic

<400> 574
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Leu Ser Ser Tyr
20 25 30
Asp Met His Trp Val Arg Gln Ala Thr Gly Lys Gly Leu Glu Trp Val
35 40 45

```
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Gly Ile Arg Thr Pro Tyr Asp Tyr Trp Gly Gln Gly Thr Leu
            100                 105                 110
Val Thr Val Ser Ser
        115
```

<210> 575
<211> 324
<212> DNA
<213> Artificial Sequence

<220>
<223> Synthetic

<400> 575
```
gaaatagtga tgacgcagtc tccagccacc ctgtctgtgt ctccagggga aagagccacc 60
ctctcctgca gggccagtca gagtgttagc agcaatttag cctggtacca gcagaaacct 120
ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataatt ggcctccatt cactttcggc 300
cctgggacca aagtggatat caaa                                        324
```

<210> 576
<211> 108
<212> PRT
<213> Artificial Sequence

<220>
<223> Synthetic

<400> 576
```
Glu Ile Val Met Thr Gln Ser Pro Ala Thr Leu Ser Val Ser Pro Gly
 1               5                  10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys
            100                 105
```

<210> 577
<211> 363
<212> DNA
<213> Artificial Sequence

<220>
<223> Synthetic

<400> 577
```
gaagtgcagc tggtggagtc tgggggaggc ttggtacagc ctggcaggtc cctgagactc 60
tcctgtgcag cctctggatt cacctttgat gattatgcca tgcactgggt ccggcaagct 120
ccagggaagg gcctggagtg ggtctcaggt attaattgga acagtggtag cataggctat 180
```

gcggactctg tgaagggccg attcaccatc tccagagaca acgccaagca ctccctgtat 240
ctgcaaatga acagtctgag acctgaggac acggccttgt attactgtgt aaaagaggtg 300
actacgggat actactacgg tatggacgtc tggggccaag ggaccacggt caccgtctcc 360
tca 363

<210> 578
<211> 121
<212> PRT
<213> Artificial Sequence

<220>
<223> Synthetic

<400> 578
Glu Val Gln Leu Val Glu Ser Gly Gly Gly Leu Val Gln Pro Gly Arg
1 5 10 15
Ser Leu Arg Leu Ser Cys Ala Ala Ser Gly Phe Thr Phe Asp Asp Tyr
20 25 30
Ala Met His Trp Val Arg Gln Ala Pro Gly Lys Gly Leu Glu Trp Val
35 40 45
Ser Gly Ile Asn Trp Asn Ser Gly Ser Ile Gly Tyr Ala Asp Ser Val
50 55 60
Lys Gly Arg Phe Thr Ile Ser Arg Asp Asn Ala Lys His Ser Leu Tyr
65 70 75 80
Leu Gln Met Asn Ser Leu Arg Pro Glu Asp Thr Ala Leu Tyr Tyr Cys
85 90 95
Val Lys Glu Val Thr Thr Gly Tyr Tyr Tyr Gly Met Asp Val Trp Gly
100 105 110
Gln Gly Thr Thr Val Thr Val Ser Ser
115 120

<210> 579
<211> 24
<212> DNA
<213> Artificial Sequence

<220>
<223> Synthetic

<400> 579
ggattcacct ttgatgatta tgcc 24

<210> 580
<211> 8
<212> PRT
<213> Artificial Sequence

<220>
<223> Synthetic

<400> 580
Gly Phe Thr Phe Asp Asp Tyr Ala
1 5

<210> 581
<211> 24
<212> DNA
<213> Artificial Sequence

<220>
<223> Synthetic

<400> 581

attaattgga acagtggtag cata 24

<210> 582
<211> 8
<212> PRT
<213> Artificial Sequence

<220>
<223> Synthetic

<400> 582
Ile Asn Trp Asn Ser Gly Ser Ile
1 5

<210> 583
<211> 42
<212> DNA
<213> Artificial Sequence

<220>
<223> Synthetic

<400> 583
gtaaaagagg tgactacggg atactactac ggtatggacg tc 42

<210> 584
<211> 14
<212> PRT
<213> Artificial Sequence

<220>
<223> Synthetic

<400> 584
Val Lys Glu Val Thr Thr Gly Tyr Tyr Tyr Gly Met Asp Val
1 5 10

<210> 585
<211> 321
<212> DNA
<213> Artificial Sequence

<220>
<223> Synthetic

<400> 585
gacatccagt tgacccagtc tccatccttc ctgtctgcat ctgtaggaga cagagtcacc 60
atcacttgct gggccagtca gggcattagc agttatttag cctggtatca gaaaaaacca 120
gggaaagccc ctaacctcct gatctatgat gcatccactt tgcaaagtgg ggtcccatca 180
aggttcagcg gcagtggatc tgggacagaa ttcactctca cactcagcag cctgcagcct 240
gaagattttg caacttatta ctgtcaacag cttaatattt acccattcac tttcggccct 300
gggaccaaag tggatatcaa a 321

<210> 586
<211> 107
<212> PRT
<213> Artificial Sequence

<220>
<223> Synthetic

<400> 586
Asp Ile Gln Leu Thr Gln Ser Pro Ser Phe Leu Ser Ala Ser Val Gly

```
1               5                   10                  15
Asp Arg Val Thr Ile Thr Cys Trp Ala Ser Gln Gly Ile Ser Ser Tyr
            20                  25                  30
Leu Ala Trp Tyr Gln Lys Lys Pro Gly Lys Ala Pro Asn Leu Leu Ile
        35                  40                  45
Tyr Asp Ala Ser Thr Leu Gln Ser Gly Val Pro Ser Arg Phe Ser Gly
    50                  55                  60
Ser Gly Ser Gly Thr Glu Phe Thr Leu Thr Leu Ser Ser Leu Gln Pro
65                  70                  75                  80
Glu Asp Phe Ala Thr Tyr Tyr Cys Gln Gln Leu Asn Ile Tyr Pro Phe
                85                  90                  95
Thr Phe Gly Pro Gly Thr Lys Val Asp Ile Lys
            100                 105
```

<210> 587
<211> 18
<212> DNA
<213> Artificial Sequence

<220>
<223> Synthetic

<400> 587
caggcatta gcagttat 18

<210> 588
<211> 6
<212> PRT
<213> Artificial Sequence

<220>
<223> Synthetic

<400> 588
Gln Gly Ile Ser Ser Tyr
1               5

<210> 589
<211> 9
<212> DNA
<213> Artificial Sequence

<220>
<223> Synthetic

<400> 589
gatgcatcc 9

<210> 590
<211> 3
<212> PRT
<213> Artificial Sequence

<220>
<223> Synthetic

<400> 590
Asp Ala Ser
1

<210> 591
<211> 27

<212> DNA
<213> Artificial Sequence

<220>
<223> Synthetic

<400> 591
caacagctta atatttaccc attcact 27

<210> 592
<211> 9
<212> PRT
<213> Artificial Sequence

<220>
<223> Synthetic

<400> 592
Gln Gln Leu Asn Ile Tyr Pro Phe Thr
1 5

<210> 593
<211> 363
<212> DNA
<213> Artificial Sequence

<220>
<223> Synthetic

<400> 593
gaagtgcagc tggtggagtc tgggggaggc ttggtacagc ctggcaggtc cctgagactc 60
tcctgtgcag cctctggatt cacctttgat gattatgcca tgcactgggt ccggcaagct 120
ccagggaagg gcctggagtg ggtctcaggt attaattgga acagtggtag cataggctat 180
gcggactctg tgaagggccg attcaccatc tccagagaca acgccaagca ctccctgtat 240
ctgcaaatga acagtctgag acctgaggac acggccttgt attactgtgt aaaagaggtg 300
actacgggat actactacgg tatggacgtc tggggccaag ggaccacggt caccgtctcc 360
tca 363

<210> 594
<211> 121
<212> PRT
<213> Artificial Sequence

<220>
<223> Synthetic

<400> 594
Glu Val Gln Leu Val Glu Ser Gly Gly Gly Leu Val Gln Pro Gly Arg
1 5 10 15
Ser Leu Arg Leu Ser Cys Ala Ala Ser Gly Phe Thr Phe Asp Asp Tyr
20 25 30
Ala Met His Trp Val Arg Gln Ala Pro Gly Lys Gly Leu Glu Trp Val
35 40 45
Ser Gly Ile Asn Trp Asn Ser Gly Ser Ile Gly Tyr Ala Asp Ser Val
50 55 60
Lys Gly Arg Phe Thr Ile Ser Arg Asp Asn Ala Lys His Ser Leu Tyr
65 70 75 80
Leu Gln Met Asn Ser Leu Arg Pro Glu Asp Thr Ala Leu Tyr Tyr Cys
85 90 95
Val Lys Glu Val Thr Thr Gly Tyr Tyr Tyr Gly Met Asp Val Trp Gly
100 105 110
Gln Gly Thr Thr Val Thr Val Ser Ser
115 120

<210> 595
<211> 321
<212> DNA
<213> Artificial Sequence

<220>
<223> Synthetic

<400> 595
gacatccagt tgacccagtc tccatccttc ctgtctgcat ctgtaggaga cagagtcacc 60
atcacttgct gggccagtca gggcattagc agttatttag cctggtatca gaaaaaacca 120
gggaaagccc ctaacctcct gatctatgat gcatccactt tgcaaagtgg ggtcccatca 180
aggttcagcg gcagtggatc tgggacagaa ttcactctca cactcagcag cctgcagcct 240
gaagattttg caacttatta ctgtcaacag cttaatattt acccattcac tttcggccct 300
gggaccaaag tggatatcaa a 321

<210> 596
<211> 107
<212> PRT
<213> Artificial Sequence

<220>
<223> Synthetic

<400> 596
Asp Ile Gln Leu Thr Gln Ser Pro Ser Phe Leu Ser Ala Ser Val Gly
1 5 10 15
Asp Arg Val Thr Ile Thr Cys Trp Ala Ser Gln Gly Ile Ser Ser Tyr
20 25 30
Leu Ala Trp Tyr Gln Lys Lys Pro Gly Lys Ala Pro Asn Leu Leu Ile
35 40 45
Tyr Asp Ala Ser Thr Leu Gln Ser Gly Val Pro Ser Arg Phe Ser Gly
50 55 60
Ser Gly Ser Gly Thr Glu Phe Thr Leu Thr Leu Ser Ser Leu Gln Pro
65 70 75 80
Glu Asp Phe Ala Thr Tyr Tyr Cys Gln Gln Leu Asn Ile Tyr Pro Phe
85 90 95
Thr Phe Gly Pro Gly Thr Lys Val Asp Ile Lys
100 105

<210> 597
<211> 363
<212> DNA
<213> Artificial Sequence

<220>
<223> Synthetic

<400> 597
gaagtgcagc tggtggagtc tgggggaggc ttggtacagc ctggcaggtc cctgagactc 60
tcctgtgcag cctctggatt cacctttgat gattatgcca tgcactgggt ccggcaagct 120
ccagggaagg gcctggagtg ggtctcaggt attaattgga acagtggtag cataggctat 180
gcggactctg tgaagggccg attcaccatc tccagagaca acgccaagaa ctccctgtat 240
ctgcaaatga acagtctgag agctgaggac acggccttgt attactgtgt aaaagaggtg 300
actacgggat actactacgg tatggacgtc tgggggcaag ggaccacggt caccgtctcc 360
tca 363

<210> 598
<211> 121
<212> PRT
<213> Artificial Sequence

<220>

<223> Synthetic

<400> 598

```
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Gly Ile Asn Trp Asn Ser Gly Ser Ile Gly Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Leu Tyr Tyr Cys
                85                  90                  95
Val Lys Glu Val Thr Thr Gly Tyr Tyr Tyr Gly Met Asp Val Trp Gly
            100                 105                 110
Gln Gly Thr Thr Val Thr Val Ser Ser
        115                 120
```

<210> 599
<211> 321
<212> DNA
<213> Artificial Sequence

<220>
<223> Synthetic

<400> 599

```
gacatccagt tgacccagtc tccatccttc ctgtctgcat ctgtaggaga cagagtcacc 60
atcacttgcc gggccagtca gggcattagc agttatttag cctggtatca gcaaaaacca 120
gggaaagccc ctaagctcct gatctatgat gcatccactt tgcaaagtgg ggtcccatca 180
aggttcagcg gcagtggatc tgggacagaa ttcactctca caatcagcag cctgcagcct 240
gaagattttg caacttatta ctgtcaacag cttaatattt acccattcac tttcggccct 300
gggaccaaag tggatatcaa a                                           321
```

<210> 600
<211> 107
<212> PRT
<213> Artificial Sequence

<220>
<223> Synthetic

<400> 600

```
Asp Ile Gln Leu Thr Gln Ser Pro Ser Phe Leu Ser Ala Ser Val Gly
1               5                   10                  15
Asp Arg Val Thr Ile Thr Cys Arg Ala Ser Gln Gly Ile Ser Ser Tyr
            20                  25                  30
Leu Ala Trp Tyr Gln Gln Lys Pro Gly Lys Ala Pro Lys Leu Leu Ile
        35                  40                  45
Tyr Asp Ala Ser Thr Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Leu Asn Ile Tyr Pro Phe
                85                  90                  95
Thr Phe Gly Pro Gly Thr Lys Val Asp Ile Lys
            100                 105
```

<210> 601
<211> 366
<212> DNA

<213> Artificial Sequence

<220>
<223> Synthetic

<400> 601
gaggtgcagt tgttggagtc tgggggaggc ttggtacagc ctggggggtc cctgagactc 60
tcctgtgcag cctctggatt cacgtttagt agctatgcca tgaactgggt ccgccaggct 120
ccagggaagg ggctggattg ggtctcaggt atcagtggta atggtggtag cacctactac 180
gcagactccg tgaagggccg gttcaccatc tccagagaca tttccaagaa cacgctgtat 240
gtgcaaatgc acagcctgag agtcgaggac acggccgttt actactgtgc gaaagcccgt 300
tattacgatt tttggggggg gaatttcgat ctctggggcc gtggcaccca ggtcactgtc 360
tcctca 366

<210> 602
<211> 122
<212> PRT
<213> Artificial Sequence

<220>
<223> Synthetic

<400> 602
Glu Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Ala Met Asn Trp Val Arg Gln Ala Pro Gly Lys Gly Leu Asp Trp Val
35 40 45
Ser Gly Ile Ser Gly Asn Gly Gly Ser Thr Tyr Tyr Ala Asp Ser Val
50 55 60
Lys Gly Arg Phe Thr Ile Ser Arg Asp Ile Ser Lys Asn Thr Leu Tyr
65 70 75 80
Val Gln Met His Ser Leu Arg Val Glu Asp Thr Ala Val Tyr Tyr Cys
85 90 95
Ala Lys Ala Arg Tyr Tyr Asp Phe Trp Gly Gly Asn Phe Asp Leu Trp
100 105 110
Gly Arg Gly Thr Gln Val Thr Val Ser Ser
115 120

<210> 603
<211> 24
<212> DNA
<213> Artificial Sequence

<220>
<223> Synthetic

<400> 603
ggattcacgt ttagtagcta tgcc 24

<210> 604
<211> 8
<212> PRT
<213> Artificial Sequence

<220>
<223> Synthetic

<400> 604
Gly Phe Thr Phe Ser Ser Tyr Ala
1 5

<210> 605
<211> 24
<212> DNA
<213> Artificial Sequence

<220>
<223> Synthetic

<400> 605
atcagtggta atggtggtag cacc 24

<210> 606
<211> 8
<212> PRT
<213> Artificial Sequence

<220>
<223> Synthetic

<400> 606
Ile Ser Gly Asn Gly Gly Ser Thr
1 5

<210> 607
<211> 45
<212> DNA
<213> Artificial Sequence

<220>
<223> Synthetic

<400> 607
gcgaaagccc gttattacga tttttggggg gggaatttcg atctc 45

<210> 608
<211> 15
<212> PRT
<213> Artificial Sequence

<220>
<223> Synthetic

<400> 608
Ala Lys Ala Arg Tyr Tyr Asp Phe Trp Gly Gly Asn Phe Asp Leu
1 5 10 15

<210> 609
<211> 324
<212> DNA
<213> Artificial Sequence

<220>
<223> Synthetic

<400> 609
gaaattgtgt tgacgcagtc tccaggcacc ctgtctttgt ctccagggga aagagccacc 60
ctctcctgca gggccagtca gagtgttagc atcaggtact tagcctggta tcagcagaaa 120
cctggccagg ctcccaggct cctcatctat ggtgcatcca gcagggccac tggcatccca 180
gacaggttca gtgtcagtgt gtctgggaca gacttcactc tcaccatcac tagactggag 240
cctgaagatt ttgcagtcta ttactgtcag caatatggta gttcaccgct cactttcggc 300
ggagggacca aggtggagat caaa 324

<210> 610

<211> 108
<212> PRT
<213> Artificial Sequence

<220>
<223> Synthetic

<400> 610
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ile Arg
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Val Ser Val Ser Gly Thr Asp Phe Thr Leu Thr Ile Thr Arg Leu Glu
65                  70                  75                  80
Pro Glu Asp Phe Ala Val Tyr Tyr Cys Gln Gln Tyr Gly Ser Ser Pro
                85                  90                  95
Leu Thr Phe Gly Gly Gly Thr Lys Val Glu Ile Lys
            100                 105

<210> 611
<211> 21
<212> DNA
<213> Artificial Sequence

<220>
<223> Synthetic

<400> 611
cagagtgtta gcatcaggta c 21

<210> 612
<211> 7
<212> PRT
<213> Artificial Sequence

<220>
<223> Synthetic

<400> 612
Gln Ser Val Ser Ile Arg Tyr
1               5

<210> 613
<211> 9
<212> DNA
<213> Artificial Sequence

<220>
<223> Synthetic

<400> 613
ggtgcatcc 9

<210> 614
<211> 3
<212> PRT
<213> Artificial Sequence

<220>

<223> Synthetic

<400> 614
Gly Ala Ser
1

<210> 615
<211> 27
<212> DNA
<213> Artificial Sequence

<220>
<223> Synthetic

<400> 615
cagcaatatg gtagttcacc gctcact 27

<210> 616
<211> 9
<212> PRT
<213> Artificial Sequence

<220>
<223> Synthetic

<400> 616
Gln Gln Tyr Gly Ser Ser Pro Leu Thr
1 5

<210> 617
<211> 366
<212> DNA
<213> Artificial Sequence

<220>
<223> Synthetic

<400> 617
gaggtgcagt tgttggagtc tgggggaggc ttggtacagc ctggggggtc cctgagactc 60
tcctgtgcag cctctggatt cacgtttagt agctatgcca tgaactgggt ccgccaggct 120
ccagggaagg ggctggattg ggtctcaggt atcagtggta atggtggtag cacctactac 180
gcagactccg tgaagggccg gttcaccatc tccagagaca tttccaagaa cacgctgtat 240
gtgcaaatgc acagcctgag agtcgaggac acggccgttt actactgtgc gaaagcccgt 300
tattacgatt tttggggggg gaatttcgat ctctggggcc gtggcaccct ggtcactgtc 360
tcctca 366

<210> 618
<211> 122
<212> PRT
<213> Artificial Sequence

<220>
<223> Synthetic

<400> 618
Glu Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Ala Met Asn Trp Val Arg Gln Ala Pro Gly Lys Gly Leu Asp Trp Val
35 40 45
Ser Gly Ile Ser Gly Asn Gly Gly Ser Thr Tyr Tyr Ala Asp Ser Val
50 55 60

```
Lys Gly Arg Phe Thr Ile Ser Arg Asp Ile Ser Lys Asn Thr Leu Tyr
65                  70                  75                  80
Val Gln Met His Ser Leu Arg Val Glu Asp Thr Ala Val Tyr Tyr Cys
                85                  90                  95
Ala Lys Ala Arg Tyr Tyr Asp Phe Trp Gly Gly Asn Phe Asp Leu Trp
            100                 105                 110
Gly Arg Gly Thr Leu Val Thr Val Ser Ser
        115                 120


<210> 619
<211> 324
<212> DNA
<213> Artificial Sequence

<220>
<223> Synthetic

<400> 619
gaaattgtgt tgacgcagtc tccaggcacc ctgtctttgt ctccagggga aagagccacc 60
ctctcctgca gggccagtca gagtgttagc atcaggtact tagcctggta tcagcagaaa 120
cctggccagg ctcccaggct cctcatctat ggtgcatcca gcagggccac tggcatccca 180
gacaggttca gtgtcagtgt gtctgggaca gacttcactc tcaccatcac tagactggag 240
cctgaagatt ttgcagtcta ttactgtcag caatatggta gttcaccgct cactttcggc 300
ggagggacca aggtggagat caaa                                        324

<210> 620
<211> 108
<212> PRT
<213> Artificial Sequence

<220>
<223> Synthetic

<400> 620
Glu Ile Val Leu Thr Gln Ser Pro Gly Thr Leu Ser Leu Ser Pro Gly
 1              5                   10                  15
Glu Arg Ala Thr Leu Ser Cys Arg Ala Ser Gln Ser Val Ser Ile Arg
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Val Ser Val Ser Gly Thr Asp Phe Thr Leu Thr Ile Thr Arg Leu Glu
65                  70                  75                  80
Pro Glu Asp Phe Ala Val Tyr Tyr Cys Gln Gln Tyr Gly Ser Ser Pro
                85                  90                  95
Leu Thr Phe Gly Gly Gly Thr Lys Val Glu Ile Lys
            100                 105


<210> 621
<211> 366
<212> DNA
<213> Artificial Sequence

<220>
<223> Synthetic

<400> 621
gaggtgcagc tgttggagtc tgggggaggc ttggtacagc ctggggggtc cctgagactc 60
tcctgtgcag cctctggatt cacgtttagt agctatgcca tgagctgggt ccgccaggct 120
ccagggaagg ggctggagtg ggtctcagct atcagtggta atggtggtag cacctactac 180
gcagactccg tgaagggccg gttcaccatc tccagagaca attccaagaa cacgctgtat 240
ctgcaaatga acagcctgag agccgaggac acggccgtat attactgtgc gaaagcccgt 300
```

```
tattacgatt tttggggggg gaatttcgat ctctggggcc gtggcaccct ggtcactgtc 360
tcctca                                                            366

<210> 622
<211> 122
<212> PRT
<213> Artificial Sequence

<220>
<223> Synthetic

<400> 622
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Asn Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ala Arg Tyr Tyr Asp Phe Trp Gly Gly Asn Phe Asp Leu Trp
            100                 105                 110
Gly Arg Gly Thr Leu Val Thr Val Ser Ser
        115                 120


<210> 623
<211> 324
<212> DNA
<213> Artificial Sequence

<220>
<223> Synthetic

<400> 623
gaaattgtgt tgacgcagtc tccaggcacc ctgtctttgt ctccagggga aagagccacc 60
ctctcctgca gggccagtca gagtgttagc atcaggtact tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240
cctgaagatt ttgcagtgta ttactgtcag caatatggta gttcaccgct cactttcggc 300
ggagggacca aggtggagat caaa                                        324

<210> 624
<211> 108
<212> PRT
<213> Artificial Sequence

<220>
<223> Synthetic

<400> 624
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ile Arg
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
```

```
Pro Glu Asp Phe Ala Val Tyr Tyr Cys Gln Gln Tyr Gly Ser Ser Pro
                85                  90                  95
Leu Thr Phe Gly Gly Gly Thr Lys Val Glu Ile Lys
            100                 105


<210> 625
<211> 381
<212> DNA
<213> Artificial Sequence

<220>
<223> Synthetic

<400> 625
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattcc tactacgtta tggacgtctg gggccaaggg 360
accacggtca ccgtctcctc a                                           381

<210> 626
<211> 127
<212> PRT
<213> Artificial Sequence

<220>
<223> Synthetic

<400> 626
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Tyr Tyr Ser Tyr Tyr
            100                 105                 110
Val Met Asp Val Trp Gly Gln Gly Thr Thr Val Thr Val Ser Ser
        115                 120                 125


<210> 627
<211> 24
<212> DNA
<213> Artificial Sequence

<220>
<223> Synthetic

<400> 627
ggttacacct ttaccaccta tggt                                        24

<210> 628
<211> 8
<212> PRT
<213> Artificial Sequence
```

<220>
<223> Synthetic

<400> 628
Gly Tyr Thr Phe Thr Thr Tyr Gly
1 5

<210> 629
<211> 24
<212> DNA
<213> Artificial Sequence

<220>
<223> Synthetic

<400> 629
atcagcggtt acaatggtaa aaca 24

<210> 630
<211> 8
<212> PRT
<213> Artificial Sequence

<220>
<223> Synthetic

<400> 630
Ile Ser Gly Tyr Asn Gly Lys Thr
1 5

<210> 631
<211> 60
<212> DNA
<213> Artificial Sequence

<220>
<223> Synthetic

<400> 631
tcgagagatc gtttagtagt accacctgcc ctttattatt cctactacgt tatggacgtc 60

<210> 632
<211> 20
<212> PRT
<213> Artificial Sequence

<220>
<223> Synthetic

<400> 632
Ser Arg Asp Arg Leu Val Val Pro Pro Ala Leu Tyr Tyr Ser Tyr Tyr
1 5 10 15
Val Met Asp Val
20

<210> 633
<211> 336
<212> DNA
<213> Artificial Sequence

<220>
<223> Synthetic

<400> 633
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 634
<211> 112
<212> PRT
<213> Artificial Sequence

<220>
<223> Synthetic

<400> 634
Asp Val Val Met Thr Gln Ser Pro Leu Ser Leu Pro Val Thr Leu Gly
1 5 10 15
Gln Pro Ala Ser Ile Ser Cys Arg Ser Ser Gln Ser Leu Val Tyr Ser
20 25 30
Asp Gly Asn Thr Tyr Leu Asn Trp Phe Gln Gln Arg Pro Gly Gln Ser
35 40 45
Pro Arg Arg Leu Ile Tyr Lys Val Ser Asn Arg Asp Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Gly
85 90 95
Thr His Trp Pro Tyr Thr Phe Gly Gln Gly Thr Lys Leu Glu Ile Lys
100 105 110

<210> 635
<211> 33
<212> DNA
<213> Artificial Sequence

<220>
<223> Synthetic

<400> 635
caaagcctcg tatacagtga tggaaacacc tac 33

<210> 636
<211> 11
<212> PRT
<213> Artificial Sequence

<220>
<223> Synthetic

<400> 636
Gln Ser Leu Val Tyr Ser Asp Gly Asn Thr Tyr
1 5 10

<210> 637
<211> 9
<212> DNA
<213> Artificial Sequence

<220>
<223> Synthetic

<400> 637
aaggtttct 9

<210> 638
<211> 3
<212> PRT
<213> Artificial Sequence

<220>
<223> Synthetic

<400> 638
Lys Val Ser
1

<210> 639
<211> 27
<212> DNA
<213> Artificial Sequence

<220>
<223> Synthetic

<400> 639
atgcaaggta cacactggcc gtacact 27

<210> 640
<211> 9
<212> PRT
<213> Artificial Sequence

<220>
<223> Synthetic

<400> 640
Met Gln Gly Thr His Trp Pro Tyr Thr
1 5

<210> 641
<211> 381
<212> DNA
<213> Artificial Sequence

<220>
<223> Synthetic

<400> 641
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattcc tactacgtta tggacgtctg gggccaaggg 360
accacggtca ccgtctcctc a 381

<210> 642
<211> 127
<212> PRT
<213> Artificial Sequence

<220>
<223> Synthetic

<400> 642
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Tyr Tyr Ser Tyr Tyr
            100                 105                 110
Val Met Asp Val Trp Gly Gln Gly Thr Thr Val Thr Val Ser Ser
        115                 120                 125

<210> 643
<211> 336
<212> DNA
<213> Artificial Sequence

<220>
<223> Synthetic

<400> 643
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 644
<211> 112
<212> PRT
<213> Artificial Sequence

<220>
<223> Synthetic

<400> 644
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 645
<211> 381

<212> DNA
<213> Artificial Sequence

<220>
<223> Synthetic

<400> 645
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct ttattattcc tactacgtta tggacgtctg gggcaaggg 360
accacggtca ccgtctcctc a 381

<210> 646
<211> 127
<212> PRT
<213> Artificial Sequence

<220>
<223> Synthetic

<400> 646
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Thr Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Lys Thr Asn Tyr Ala Gln Lys Leu
50 55 60
Gln Gly Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ser Arg Asp Arg Leu Val Val Pro Pro Ala Leu Tyr Tyr Ser Tyr Tyr
100 105 110
Val Met Asp Val Trp Gly Gln Gly Thr Thr Val Thr Val Ser Ser
115 120 125

<210> 647
<211> 336
<212> DNA
<213> Artificial Sequence

<220>
<223> Synthetic

<400> 647
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 648
<211> 112
<212> PRT
<213> Artificial Sequence

<220>
<223> Synthetic

<400> 648
Asp Val Val Met Thr Gln Ser Pro Leu Ser Leu Pro Val Thr Leu Gly
1 5 10 15
Gln Pro Ala Ser Ile Ser Cys Arg Ser Ser Gln Ser Leu Val Tyr Ser
20 25 30
Asp Gly Asn Thr Tyr Leu Asn Trp Phe Gln Gln Arg Pro Gly Gln Ser
35 40 45
Pro Arg Arg Leu Ile Tyr Lys Val Ser Asn Arg Asp Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Gly
85 90 95
Thr His Trp Pro Tyr Thr Phe Gly Gln Gly Thr Lys Leu Glu Ile Lys
100 105 110

<210> 649
<211> 381
<212> DNA
<213> Artificial Sequence

<220>
<223> Synthetic

<400> 649
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattac tactacgtta tggacgtctg gggccaaggg 360
accacggtca ccgtctcctc a 381

<210> 650
<211> 127
<212> PRT
<213> Artificial Sequence

<220>
<223> Synthetic

<400> 650
Gln Val Gln Leu Val Gln Ser Gly Pro Glu Val Lys Asn Pro Gly Ala
1 5 10 15
Ser Val Lys Val Ser Cys Lys Ala Ser Gly Tyr Thr Phe Thr Thr Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Lys Thr Asn Asp Ala Gln Lys Phe
50 55 60
Gln Asp Arg Val Ala Met Thr Thr Asp Thr Ser Thr Ser Thr Ala Tyr
65 70 75 80
Met Glu Leu Arg Ser Leu Arg Ser Asp Asp Thr Ala Ile Tyr Tyr Cys
85 90 95
Ser Arg Asp Arg Leu Val Val Pro Pro Ala Leu Asn Tyr Tyr Tyr Tyr
100 105 110
Val Met Asp Val Trp Gly Gln Gly Thr Thr Val Thr Val Ser Ser
115 120 125

<210> 651
<211> 24
<212> DNA

<213> Artificial Sequence

<220>
<223> Synthetic

<400> 651
ggttacacct ttaccaccta tggt 24

<210> 652
<211> 8
<212> PRT
<213> Artificial Sequence

<220>
<223> Synthetic

<400> 652
Gly Tyr Thr Phe Thr Thr Tyr Gly
1 5

<210> 653
<211> 24
<212> DNA
<213> Artificial Sequence

<220>
<223> Synthetic

<400> 653
atcagcggtt acaatggtaa aaca 24

<210> 654
<211> 8
<212> PRT
<213> Artificial Sequence

<220>
<223> Synthetic

<400> 654
Ile Ser Gly Tyr Asn Gly Lys Thr
1 5

<210> 655
<211> 60
<212> DNA
<213> Artificial Sequence

<220>
<223> Synthetic

<400> 655
tcgagagatc gtttagtagt accacctgcc cttaattatt actactacgt tatggacgtc 60

<210> 656
<211> 20
<212> PRT
<213> Artificial Sequence

<220>
<223> Synthetic

<400> 656
Ser Arg Asp Arg Leu Val Val Pro Pro Ala Leu Asn Tyr Tyr Tyr Tyr
1               5                   10                  15
Val Met Asp Val
            20

<210> 657
<211> 336
<212> DNA
<213> Artificial Sequence

<220>
<223> Synthetic

<400> 657
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 658
<211> 112
<212> PRT
<213> Artificial Sequence

<220>
<223> Synthetic

<400> 658
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 659
<211> 33
<212> DNA
<213> Artificial Sequence

<220>
<223> Synthetic

<400> 659
caaagcctcg tatacagtga tggaaacacc tac 33

<210> 660
<211> 11
<212> PRT
<213> Artificial Sequence

<220>

<223> Synthetic

<400> 660
Gln Ser Leu Val Tyr Ser Asp Gly Asn Thr Tyr
1 5 10

<210> 661
<211> 9
<212> DNA
<213> Artificial Sequence

<220>
<223> Synthetic

<400> 661
aaggtttct 9

<210> 662
<211> 3
<212> PRT
<213> Artificial Sequence

<220>
<223> Synthetic

<400> 662
Lys Val Ser
1

<210> 663
<211> 27
<212> DNA
<213> Artificial Sequence

<220>
<223> Synthetic

<400> 663
atgcaaggta cacactggcc gtacact 27

<210> 664
<211> 9
<212> PRT
<213> Artificial Sequence

<220>
<223> Synthetic

<400> 664
Met Gln Gly Thr His Trp Pro Tyr Thr
1 5

<210> 665
<211> 381
<212> DNA
<213> Artificial Sequence

<220>
<223> Synthetic

<400> 665
caggttcagc tggtgcagtc tggacctgag gtgaagaacc ctggggcctc agtgaaggtc 60

```
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattac tactacgtta tggacgtctg gggccaaggg 360
accacggtca ccgtctcctc a                                           381

<210> 666
<211> 127
<212> PRT
<213> Artificial Sequence

<220>
<223> Synthetic

<400> 666
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Asn Tyr Tyr Tyr Tyr
            100                 105                 110
Val Met Asp Val Trp Gly Gln Gly Thr Thr Val Thr Val Ser Ser
        115                 120                 125


<210> 667
<211> 336
<212> DNA
<213> Artificial Sequence

<220>
<223> Synthetic

<400> 667
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                           336

<210> 668
<211> 112
<212> PRT
<213> Artificial Sequence

<220>
<223> Synthetic

<400> 668
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
```

```
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110


<210> 669
<211> 381
<212> DNA
<213> Artificial Sequence

<220>
<223> Synthetic

<400> 669
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct taattattac tactacgtta tggacgtctg gggcaaggg 360
accacggtca ccgtctcctc a                                           381

<210> 670
<211> 127
<212> PRT
<213> Artificial Sequence

<220>
<223> Synthetic

<400> 670
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ser Arg Asp Arg Leu Val Val Pro Pro Ala Leu Asn Tyr Tyr Tyr Tyr
            100                 105                 110
Val Met Asp Val Trp Gly Gln Gly Thr Thr Val Thr Val Ser Ser
        115                 120                 125


<210> 671
<211> 336
<212> DNA
<213> Artificial Sequence

<220>
<223> Synthetic

<400> 671
gatgttgtga tgactcagtc tccactctcc ctgcccgtca cccttggaca gccggcctcc 60
atctcctgca ggtctagtca aagcctcgta tacagtgatg gaaacaccta cttgaattgg 120
```

tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 672
<211> 112
<212> PRT
<213> Artificial Sequence

<220>
<223> Synthetic

<400> 672
Asp Val Val Met Thr Gln Ser Pro Leu Ser Leu Pro Val Thr Leu Gly
1 5 10 15
Gln Pro Ala Ser Ile Ser Cys Arg Ser Ser Gln Ser Leu Val Tyr Ser
20 25 30
Asp Gly Asn Thr Tyr Leu Asn Trp Phe Gln Gln Arg Pro Gly Gln Ser
35 40 45
Pro Arg Arg Leu Ile Tyr Lys Val Ser Asn Arg Asp Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Gly
85 90 95
Thr His Trp Pro Tyr Thr Phe Gly Gln Gly Thr Lys Leu Glu Ile Lys
100 105 110

<210> 673
<211> 381
<212> DNA
<213> Artificial Sequence

<220>
<223> Synthetic

<400> 673
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattac tactacgtta tggacgtctg ggggcaaggg 360
accacggtca ccgtctcctc a 381

<210> 674
<211> 127
<212> PRT
<213> Artificial Sequence

<220>
<223> Synthetic

<400> 674
Gln Val Gln Leu Val Gln Ser Gly Pro Glu Val Lys Asn Pro Gly Ala
1 5 10 15
Ser Val Lys Val Ser Cys Lys Ala Ser Gly Tyr Thr Phe Thr Thr Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Lys Thr Asn Asp Ala Gln Lys Phe
50 55 60
Gln Asp Arg Val Ala Met Thr Thr Asp Thr Ser Thr Ser Thr Ala Tyr

```
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Tyr Tyr Tyr Tyr Tyr
            100                 105                 110
Val Met Asp Val Trp Gly Gln Gly Thr Thr Val Thr Val Ser Ser
        115                 120                 125
```

<210> 675
<211> 24
<212> DNA
<213> Artificial Sequence

<220>
<223> Synthetic

<400> 675
ggttacacct ttaccaccta tggt 24

<210> 676
<211> 8
<212> PRT
<213> Artificial Sequence

<220>
<223> Synthetic

<400> 676

```
Gly Tyr Thr Phe Thr Thr Tyr Gly
 1               5
```

<210> 677
<211> 24
<212> DNA
<213> Artificial Sequence

<220>
<223> Synthetic

<400> 677
atcagcggtt acaatggtaa aaca 24

<210> 678
<211> 8
<212> PRT
<213> Artificial Sequence

<220>
<223> Synthetic

<400> 678

```
Ile Ser Gly Tyr Asn Gly Lys Thr
 1               5
```

<210> 679
<211> 60
<212> DNA
<213> Artificial Sequence

<220>
<223> Synthetic

<400> 679
tcgagagatc gtttagtagt accacctgcc ctttattatt actactacgt tatggacgtc 60

<210> 680
<211> 20
<212> PRT
<213> Artificial Sequence

<220>
<223> Synthetic

<400> 680
Ser Arg Asp Arg Leu Val Val Pro Pro Ala Leu Tyr Tyr Tyr Tyr Tyr
1               5                   10                  15
Val Met Asp Val
            20

<210> 681
<211> 336
<212> DNA
<213> Artificial Sequence

<220>
<223> Synthetic

<400> 681
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 682
<211> 112
<212> PRT
<213> Artificial Sequence

<220>
<223> Synthetic

<400> 682
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 683
<211> 33
<212> DNA
<213> Artificial Sequence

<220>
<223> Synthetic

<400> 683
caaagcctcg tatacagtga tggaaacacc tac 33

<210> 684
<211> 11
<212> PRT
<213> Artificial Sequence

<220>
<223> Synthetic

<400> 684
Gln Ser Leu Val Tyr Ser Asp Gly Asn Thr Tyr
1 5 10

<210> 685
<211> 9
<212> DNA
<213> Artificial Sequence

<220>
<223> Synthetic

<400> 685
aaggtttct 9

<210> 686
<211> 3
<212> PRT
<213> Artificial Sequence

<220>
<223> Synthetic

<400> 686
Lys Val Ser
1

<210> 687
<211> 27
<212> DNA
<213> Artificial Sequence

<220>
<223> Synthetic

<400> 687
atgcaaggta cacactggcc gtacact 27

<210> 688
<211> 9
<212> PRT
<213> Artificial Sequence

<220>
<223> Synthetic

<400> 688
Met Gln Gly Thr His Trp Pro Tyr Thr
1 5

<210> 689
<211> 381
<212> DNA
<213> Artificial Sequence

<220>
<223> Synthetic

<400> 689
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattac tactacgtta tggacgtctg gggccaaggg 360
accacggtca ccgtctcctc a 381

<210> 690
<211> 127
<212> PRT
<213> Artificial Sequence

<220>
<223> Synthetic

<400> 690
Gln Val Gln Leu Val Gln Ser Gly Pro Glu Val Lys Asn Pro Gly Ala
1 5 10 15
Ser Val Lys Val Ser Cys Lys Ala Ser Gly Tyr Thr Phe Thr Thr Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Lys Thr Asn Asp Ala Gln Lys Phe
50 55 60
Gln Asp Arg Val Ala Met Thr Thr Asp Thr Ser Thr Ser Thr Ala Tyr
65 70 75 80
Met Glu Leu Arg Ser Leu Arg Ser Asp Asp Thr Ala Ile Tyr Tyr Cys
85 90 95
Ser Arg Asp Arg Leu Val Val Pro Pro Ala Leu Tyr Tyr Tyr Tyr Tyr
100 105 110
Val Met Asp Val Trp Gly Gln Gly Thr Thr Val Thr Val Ser Ser
115 120 125

<210> 691
<211> 336
<212> DNA
<213> Artificial Sequence

<220>
<223> Synthetic

<400> 691
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 692
<211> 112
<212> PRT

<213> Artificial Sequence

<220>
<223> Synthetic

<400> 692
Asp Val Val Met Thr Gln Ser Pro Leu Ser Leu Pro Val Thr Leu Gly
1 5 10 15
Gln Pro Ala Ser Ile Ser Cys Arg Ser Ser Gln Ser Leu Val Tyr Ser
20 25 30
Asp Gly Asn Thr Tyr Leu Asn Trp Phe Gln Gln Arg Pro Gly Gln Ser
35 40 45
Pro Arg Arg Leu Ile Tyr Lys Val Ser Asn Arg Asp Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Gly
85 90 95
Thr His Trp Pro Tyr Thr Phe Gly Gln Gly Thr Lys Leu Glu Ile Lys
100 105 110

<210> 693
<211> 381
<212> DNA
<213> Artificial Sequence

<220>
<223> Synthetic

<400> 693
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct ttattattac tactacgtta tggacgtctg gggcaaggg 360
accacggtca ccgtctcctc a 381

<210> 694
<211> 127
<212> PRT
<213> Artificial Sequence

<220>
<223> Synthetic

<400> 694
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Thr Tyr
20 25 30
Gly Ile Ser Trp Val Arg Gln Ala Pro Gly Gln Gly Leu Glu Trp Met
35 40 45
Gly Trp Ile Ser Gly Tyr Asn Gly Lys Thr Asn Tyr Ala Gln Lys Leu
50 55 60
Gln Gly Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ser Arg Asp Arg Leu Val Val Pro Pro Ala Leu Tyr Tyr Tyr Tyr Tyr
100 105 110
Val Met Asp Val Trp Gly Gln Gly Thr Thr Val Thr Val Ser Ser
115 120 125

<210> 695
<211> 336
<212> DNA
<213> Artificial Sequence

<220>
<223> Synthetic

<400> 695
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 696
<211> 112
<212> PRT
<213> Artificial Sequence

<220>
<223> Synthetic

<400> 696
Asp Val Val Met Thr Gln Ser Pro Leu Ser Leu Pro Val Thr Leu Gly
1 5 10 15
Gln Pro Ala Ser Ile Ser Cys Arg Ser Ser Gln Ser Leu Val Tyr Ser
20 25 30
Asp Gly Asn Thr Tyr Leu Asn Trp Phe Gln Gln Arg Pro Gly Gln Ser
35 40 45
Pro Arg Arg Leu Ile Tyr Lys Val Ser Asn Arg Asp Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Gly
85 90 95
Thr His Trp Pro Tyr Thr Phe Gly Gln Gly Thr Lys Leu Glu Ile Lys
100 105 110

<210> 697
<211> 384
<212> DNA
<213> Artificial Sequence

<220>
<223> Synthetic

<400> 697
caggtgcacc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtcgc ctca 384

<210> 698
<211> 128
<212> PRT
<213> Artificial Sequence

<220>

<223> Synthetic

<400> 698
Gln Val His Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ala Ser
        115                 120                 125

<210> 699
<211> 24
<212> DNA
<213> Artificial Sequence

<220>
<223> Synthetic

<400> 699
ggattcacct tcagtgacca ctac 24

<210> 700
<211> 8
<212> PRT
<213> Artificial Sequence

<220>
<223> Synthetic

<400> 700
Gly Phe Thr Phe Ser Asp His Tyr
1               5

<210> 701
<211> 24
<212> DNA
<213> Artificial Sequence

<220>
<223> Synthetic

<400> 701
attagtaatg atggtggtac caaa 24

<210> 702
<211> 8
<212> PRT
<213> Artificial Sequence

<220>
<223> Synthetic

<400> 702

```
Ile Ser Asn Asp Gly Gly Thr Lys
 1               5


<210> 703
<211> 63
<212> DNA
<213> Artificial Sequence

<220>
<223> Synthetic

<400> 703
gcgagagatc agggatatat tggctacgac tcgtattatt actattccta cggtatggac 60
gtc                                                               63

<210> 704
<211> 21
<212> PRT
<213> Artificial Sequence

<220>
<223> Synthetic

<400> 704
Ala Arg Asp Gln Gly Tyr Ile Gly Tyr Asp Ser Tyr Tyr Tyr Tyr Ser
 1               5                  10                  15
Tyr Gly Met Asp Val
            20


<210> 705
<211> 321
<212> DNA
<213> Artificial Sequence

<220>
<223> Synthetic

<400> 705
aaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tttcggcgga 300
gggaccaagg tggagatcaa g                                           321

<210> 706
<211> 107
<212> PRT
<213> Artificial Sequence

<220>
<223> Synthetic

<400> 706
Lys Ile Val Leu Thr Gln Ser Pro Gly Thr Leu Pro Leu Phe Pro Gly
 1               5                  10                  15
Glu Arg Ala Thr Leu Ser Cys Arg Ala Ser Gln Ser Val Asn Asn Lys
            20                  25                  30
Phe Leu Ala Trp Tyr Gln Gln Lys Ser Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Gly Leu Glu
```

```
65                  70                  75                  80
Pro Glu Asp Phe Glu Val Tyr Tyr Cys Gln Val Tyr Gly Asn Ser Leu
                85                  90                  95
Thr Phe Gly Gly Gly Thr Lys Val Glu Ile Lys
            100                 105


<210> 707
<211> 21
<212> DNA
<213> Artificial Sequence

<220>
<223> Synthetic

<400> 707
cagagtgtta acaacaaatt c                                             21

<210> 708
<211> 7
<212> PRT
<213> Artificial Sequence

<220>
<223> Synthetic

<400> 708
Gln Ser Val Asn Asn Lys Phe
1               5


<210> 709
<211> 9
<212> DNA
<213> Artificial Sequence

<220>
<223> Synthetic

<400> 709
ggtgcatcc                                                           9

<210> 710
<211> 3
<212> PRT
<213> Artificial Sequence

<220>
<223> Synthetic

<400> 710
Gly Ala Ser
1


<210> 711
<211> 24
<212> DNA
<213> Artificial Sequence

<220>
<223> Synthetic

<400> 711
caagtatatg gtaactcact cact                                          24
```

<210> 712
<211> 8
<212> PRT
<213> Artificial Sequence

<220>
<223> Synthetic

<400> 712
Gln Val Tyr Gly Asn Ser Leu Thr
1               5

<210> 713
<211> 384
<212> DNA
<213> Artificial Sequence

<220>
<223> Synthetic

<400> 713
caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtctc ctca 384

<210> 714
<211> 128
<212> PRT
<213> Artificial Sequence

<220>
<223> Synthetic

<400> 714
Gln Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125

<210> 715
<211> 321
<212> DNA
<213> Artificial Sequence

<220>
<223> Synthetic

<400> 715
gaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tttcggcgga 300
gggaccaagg tggagatcaa a 321

<210> 716
<211> 107
<212> PRT
<213> Artificial Sequence

<220>
<223> Synthetic

<400> 716
Glu Ile Val Leu Thr Gln Ser Pro Gly Thr Leu Pro Leu Phe Pro Gly
1 5 10 15
Glu Arg Ala Thr Leu Ser Cys Arg Ala Ser Gln Ser Val Asn Asn Lys
20 25 30
Phe Leu Ala Trp Tyr Gln Gln Lys Ser Gly Gln Ala Pro Arg Leu Leu
35 40 45
Ile Tyr Gly Ala Ser Ser Arg Ala Thr Gly Ile Pro Asp Arg Phe Ser
50 55 60
Gly Ser Gly Ser Gly Thr Asp Phe Thr Leu Thr Ile Ser Gly Leu Glu
65 70 75 80
Pro Glu Asp Phe Glu Val Tyr Tyr Cys Gln Val Tyr Gly Asn Ser Leu
85 90 95
Thr Phe Gly Gly Gly Thr Lys Val Glu Ile Lys
100 105

<210> 717
<211> 384
<212> DNA
<213> Artificial Sequence

<220>
<223> Synthetic

<400> 717
caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg ggtttcatac attagtaatg atggtggtac caaatactac 180
gcagactctg tgaagggccg attcaccatc tccagggaca acgccaagaa ctcactgtat 240
ctgcaaatga acagcctgag agccgaggac acggccgtgt attactgtgc gagagatcag 300
ggatatattg gctacgactc gtattattac tattcctacg gtatggacgt ctgggggcaa 360
gggaccacgg tcaccgtctc ctca 384

<210> 718
<211> 128
<212> PRT
<213> Artificial Sequence

<220>
<223> Synthetic

<400> 718
Gln Val Gln Leu Val Glu Ser Gly Gly Gly Leu Val Lys Pro Gly Gly
1 5 10 15
Ser Leu Arg Leu Ser Cys Ala Ala Ser Gly Phe Thr Phe Ser Asp His
20 25 30
Tyr Met Ser Trp Ile Arg Gln Ala Pro Gly Lys Gly Leu Glu Trp Val

```
        35                  40                  45
Ser Tyr Ile Ser Asn Asp Gly Gly Thr Lys Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125
```

<210> 719
<211> 321
<212> DNA
<213> Artificial Sequence

<220>
<223> Synthetic

<400> 719

```
gaaattgtgt tgacgcagtc tccaggcacc ctgtctttgt ctccagggga aagagccacc 60
ctctcctgca gggccagtca gagtgttaac aacaaattct tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240
cctgaagatt ttgcagtgta ttactgtcaa gtatatggta actcactcac tttcggcgga 300
gggaccaagg tggagatcaa a                                           321
```

<210> 720
<211> 107
<212> PRT
<213> Artificial Sequence

<220>
<223> Synthetic

<400> 720

```
Glu Ile Val Leu Thr Gln Ser Pro Gly Thr Leu Ser Leu Ser Pro Gly
 1               5                  10                  15
Glu Arg Ala Thr Leu Ser Cys Arg Ala Ser Gln Ser Val Asn Asn Lys
            20                  25                  30
Phe Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Val Tyr Gly Asn Ser Leu
                85                  90                  95
Thr Phe Gly Gly Gly Thr Lys Val Glu Ile Lys
            100                 105
```

<210> 721
<211> 378
<212> DNA
<213> Artificial Sequence

<220>
<223> Synthetic

<400> 721

```
caaattctgc tggtgcaatc tggacctgag gtgaaggagc ctggggcctc agtgaaggtc 60
tcctgcaagg cttctggtta cacctttacc aactacgcta tcagctgggt gcgacaggtc 120
```

```
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca                                                378
```

<210> 722
<211> 126
<212> PRT
<213> Artificial Sequence

<220>
<223> Synthetic

<400> 722

```
Gln Ile Leu Leu Val Gln Ser Gly Pro Glu Val Lys Glu Pro Gly Ala
1               5                   10                  15
Ser Val Lys Val Ser Cys Lys Ala Ser Gly Tyr Thr Phe Thr Asn Tyr
            20                  25                  30
Ala Ile Ser Trp Val Arg Gln Val Pro Gly Gln Gly Leu Glu Trp Met
        35                  40                  45
Gly Trp Val Ser Ala Tyr Asn Gly His Thr Asn Tyr Ala His Glu Val
    50                  55                  60
Gln Gly Arg Val Thr Met Thr Thr Asp Thr Ser Thr Thr Thr Ala Tyr
65                  70                  75                  80
Met Glu Leu Arg Ser Leu Arg Ser Asp Asp Thr Ala Met Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125
```

<210> 723
<211> 24
<212> DNA
<213> Artificial Sequence

<220>
<223> Synthetic

<400> 723

```
ggttacacct ttaccaacta cgct                                         24
```

<210> 724
<211> 8
<212> PRT
<213> Artificial Sequence

<220>
<223> Synthetic

<400> 724

```
Gly Tyr Thr Phe Thr Asn Tyr Ala
1               5
```

<210> 725
<211> 24
<212> DNA
<213> Artificial Sequence

<220>
<223> Synthetic

<400> 725
gtcagcgctt acaatggtca caca 24

<210> 726
<211> 8
<212> PRT
<213> Artificial Sequence

<220>
<223> Synthetic

<400> 726
Val Ser Ala Tyr Asn Gly His Thr
1 5

<210> 727
<211> 57
<212> DNA
<213> Artificial Sequence

<220>
<223> Synthetic

<400> 727
gcgagagggg gtgtagtcgt gccagttgct ccccacttct acaacggtat ggacgtc 57

<210> 728
<211> 19
<212> PRT
<213> Artificial Sequence

<220>
<223> Synthetic

<400> 728
Ala Arg Gly Gly Val Val Val Pro Val Ala Pro His Phe Tyr Asn Gly
1 5 10 15
Met Asp Val

<210> 729
<211> 336
<212> DNA
<213> Artificial Sequence

<220>
<223> Synthetic

<400> 729
gatattgtga tgactcagtt tccactctcc ctgcccgtca cccctggaga gccggcctcc 60
atctcctgca ggtctagtca gagcctcctg catattaatg aatacaacta tttggattgg 120
tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttcg gccaagggac caaggtggaa atcaaa 336

<210> 730
<211> 112
<212> PRT
<213> Artificial Sequence

<220>
<223> Synthetic

<400> 730
Asp Ile Val Met Thr Gln Phe Pro Leu Ser Leu Pro Val Thr Pro Gly
1 5 10 15
Glu Pro Ala Ser Ile Ser Cys Arg Ser Ser Gln Ser Leu Leu His Ile
20 25 30
Asn Glu Tyr Asn Tyr Leu Asp Trp Tyr Leu Lys Lys Pro Gly Gln Ser
35 40 45
Pro Gln Leu Leu Ile Tyr Leu Gly Phe Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Trp Thr Phe Gly Gln Gly Thr Lys Val Glu Ile Lys
100 105 110

<210> 731
<211> 33
<212> DNA
<213> Artificial Sequence

<220>
<223> Synthetic

<400> 731
cagagcctcc tgcatattaa tgaatacaac tat 33

<210> 732
<211> 11
<212> PRT
<213> Artificial Sequence

<220>
<223> Synthetic

<400> 732
Gln Ser Leu Leu His Ile Asn Glu Tyr Asn Tyr
1 5 10

<210> 733
<211> 9
<212> DNA
<213> Artificial Sequence

<220>
<223> Synthetic

<400> 733
ttgggtttt 9

<210> 734
<211> 3
<212> PRT
<213> Artificial Sequence

<220>
<223> Synthetic

<400> 734
Leu Gly Phe
1

<210> 735
<211> 27
<212> DNA
<213> Artificial Sequence

<220>
<223> Synthetic

<400> 735
atgcaagctc ttcaaactcc gtggacg 27

<210> 736
<211> 9
<212> PRT
<213> Artificial Sequence

<220>
<223> Synthetic

<400> 736
Met Gln Ala Leu Gln Thr Pro Trp Thr
1 5

<210> 737
<211> 378
<212> DNA
<213> Artificial Sequence

<220>
<223> Synthetic

<400> 737
caggttcagc tggtgcagtc tggacctgag gtgaaggagc ctggggcctc agtgaaggtc 60
tcctgcaagg cttctggtta cacctttacc aactacgcta tcagctgggt gcgacaggtc 120
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca 378

<210> 738
<211> 126
<212> PRT
<213> Artificial Sequence

<220>
<223> Synthetic

<400> 738
Gln Val Gln Leu Val Gln Ser Gly Pro Glu Val Lys Glu Pro Gly Ala
1 5 10 15
Ser Val Lys Val Ser Cys Lys Ala Ser Gly Tyr Thr Phe Thr Asn Tyr
20 25 30
Ala Ile Ser Trp Val Arg Gln Val Pro Gly Gln Gly Leu Glu Trp Met
35 40 45
Gly Trp Val Ser Ala Tyr Asn Gly His Thr Asn Tyr Ala His Glu Val
50 55 60
Gln Gly Arg Val Thr Met Thr Thr Asp Thr Ser Thr Thr Thr Ala Tyr
65 70 75 80
Met Glu Leu Arg Ser Leu Arg Ser Asp Asp Thr Ala Met Tyr Tyr Cys
85 90 95
Ala Arg Gly Gly Val Val Val Pro Val Ala Pro His Phe Tyr Asn Gly
100 105 110

Met Asp Val Trp Gly Gln Gly Thr Thr Val Thr Val Ser Ser
115 120 125

<210> 739
<211> 336
<212> DNA
<213> Artificial Sequence

<220>
<223> Synthetic

<400> 739
gatattgtga tgactcagtc tccactctcc ctgcccgtca cccctggaga gccggcctcc 60
atctcctgca ggtctagtca gagcctcctg catattaatg aatacaacta tttggattgg 120
tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttcg gccaagggac caaggtggaa atcaaa 336

<210> 740
<211> 112
<212> PRT
<213> Artificial Sequence

<220>
<223> Synthetic

<400> 740
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ile
20 25 30
Asn Glu Tyr Asn Tyr Leu Asp Trp Tyr Leu Lys Lys Pro Gly Gln Ser
35 40 45
Pro Gln Leu Leu Ile Tyr Leu Gly Phe Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Trp Thr Phe Gly Gln Gly Thr Lys Val Glu Ile Lys
100 105 110

<210> 741
<211> 378
<212> DNA
<213> Artificial Sequence

<220>
<223> Synthetic

<400> 741
caggttcagc tggtgcagtc tggagctgag gtgaagaagc ctggggcctc agtgaaggtc 60
tcctgcaagg cttctggtta cacctttacc aactacgcta tcagctgggt gcgacaggcc 120
cctggacaag ggcttgagtg gatgggatgg gtcagcgctt acaatggtca cacaaactat 180
gcacagaagc tccagggcag agtcaccatg accacagaca catccacgag cacagcctac 240
atggagctga ggagcctgag atctgacgac acggccgtgt attactgtgc gagagggggt 300
gtagtcgtgc cagttgctcc ccacttctac aacggtatgg acgtctgggg gcaagggacc 360
acggtcaccg tctcctca 378

<210> 742
<211> 126
<212> PRT

<213> Artificial Sequence

<220>
<223> Synthetic

<400> 742
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Asn Tyr
20 25 30
Ala Ile Ser Trp Val Arg Gln Ala Pro Gly Gln Gly Leu Glu Trp Met
35 40 45
Gly Trp Val Ser Ala Tyr Asn Gly His Thr Asn Tyr Ala Gln Lys Leu
50 55 60
Gln Gly Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ala Arg Gly Gly Val Val Val Pro Val Ala Pro His Phe Tyr Asn Gly
100 105 110
Met Asp Val Trp Gly Gln Gly Thr Thr Val Thr Val Ser Ser
115 120 125

<210> 743
<211> 336
<212> DNA
<213> Artificial Sequence

<220>
<223> Synthetic

<400> 743
gatattgtga tgactcagtc tccactctcc ctgcccgtca cccctggaga gccggcctcc 60
atctcctgca ggtctagtca gagcctcctg catattaatg aatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct tcaaactccg 300
tggacgttcg gccaagggac caaggtggaa atcaaa 336

<210> 744
<211> 112
<212> PRT
<213> Artificial Sequence

<220>
<223> Synthetic

<400> 744
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ile
20 25 30
Asn Glu Tyr Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Trp Thr Phe Gly Gln Gly Thr Lys Val Glu Ile Lys
100 105 110

<210> 745
<211> 8
<212> PRT
<213> Artificial Sequence

<220>
<223> Synthetic

<221> VARIANT
<222> (1)...(8)
<223> Xaa = Any amino acid

<400> 745
Xaa Xaa Xaa Xaa Xaa Xaa Xaa Xaa
1 5

<210> 746
<211> 8
<212> PRT
<213> Artificial Sequence

<220>
<223> Synthetic

<221> VARIANT
<222> (1)...(8)
<223> Xaa - Any amino acid

<400> 746
Xaa Xaa Xaa Xaa Xaa Xaa Xaa Xaa
1 5

<210> 747
<211> 16
<212> PRT
<213> Artificial Sequence

<220>
<223> Synthetic

<221> VARIANT
<222> (1)...(16)
<223> Xaa = Any amino acid

<400> 747
Xaa Xaa Xaa Xaa Xaa Xaa Xaa Xaa Xaa Xaa Xaa Xaa Xaa Xaa Xaa Xaa
1 5 10 15

<210> 748
<211> 12
<212> PRT
<213> Artificial Sequence

<220>
<223> Synthetic

<221> VARIANT
<222> (1)...(12)
<223> Xaa = Any amino acid

<400> 748
Xaa Xaa Xaa Xaa Xaa Xaa Xaa Xaa Xaa Xaa Xaa Xaa

1 5 10

<210> 749
<211> 3
<212> PRT
<213> Artificial Sequence

<220>
<223> Synthetic

<221> VARIANT
<222> (1)...(3)
<223> Xaa = Any amino acid

<400> 749
Xaa Xaa Xaa
1

<210> 750
<211> 9
<212> PRT
<213> Artificial Sequence

<220>
<223> Synthetic

<221> VARIANT
<222> (1)...(9)
<223> Xaa = Any amino acid

<400> 750
Xaa Xaa Xaa Xaa Xaa Xaa Xaa Xaa Xaa
1 5

<210> 751
<211> 330
<212> PRT
<213> Artificial Sequence

<220>
<223> Synthetic

<400> 751
Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Ser Leu Gly Thr Gln Thr
65 70 75 80
Tyr Ile Cys Asn Val Asn His Lys Pro Ser Asn Thr Lys Val Asp Lys
85 90 95
Lys Val Glu Pro Lys Ser Cys Asp Lys Thr His Thr Cys Pro Pro Cys
100 105 110
Pro Ala Pro Glu Leu Leu Gly Gly Pro Ser Val Phe Leu Phe Pro Pro
115 120 125
Lys Pro Lys Asp Thr Leu Met Ile Ser Arg Thr Pro Glu Val Thr Cys
130 135 140
Val Val Val Asp Val Ser His Glu Asp Pro Glu Val Lys Phe Asn Trp

```
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330
```

<210> 752
<211> 327
<212> PRT
<213> Artificial Sequence

<220>
<223> Synthetic

<400> 752

```
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Ser Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
```

```
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325


<210> 753
<211> 327
<212> PRT
<213> Artificial Sequence

<220>
<223> Synthetic

<400> 753
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325
```

<210> 754
<211> 2076
<212> DNA
<213> Homo sapiens

<400> 754
atgggcaccg tcagctccag gcggtcctgg tggccgctgc cactgctgct gctgctgctg 60
ctgctcctgg gtcccgcggg cgcccgtgcg caggaggacg aggacggcga ctacgaggag 120
ctggtgctag ccttgcgttc cgaggaggac ggcctggccg aagcacccga gcacggaacc 180
acagccacct tccaccgctg cgccaaggat ccgtggaggt tgcctggcac ctacgtggtg 240
gtgctgaagg aggagaccca cctctcgcag tcagagcgca ctgcccgccg cctgcaggcc 300
caggctgccc gccgggggata cctcaccaag atcctgcatg tcttccatgg ccttcttcct 360
ggcttcctgg tgaagatgag tggcgacctg ctggagctgg ccttgaagtt gccccatgtc 420
gactacatcg aggaggactc ctctgtcttt gcccagagca tcccgtggaa cctggagcgg 480
attacccctc cacggtaccg ggcggatgaa taccagcccc ccgacggagg cagcctggtg 540
gaggtgtatc tcctagacac cagcatacag agtgaccacc gggaaatcga gggcagggtc 600
atggtcaccg acttcgagaa tgtgcccgag gaggacggga cccgcttcca cagacaggcc 660
agcaagtgtg acagtcatgg cacccacctg gcaggggtgg tcagcggccg ggatgccggc 720
gtggccaagg gtgccagcat gcgcagcctg cgcgtgctca actgccaagg gaagggcacg 780
gttagcggca ccctcatagg cctggagttt attcggaaaa gccagctggt ccagcctgtg 840
gggccactgg tggtgctgct gcccctggcg ggtgggtaca gccgcgtcct caacgccgcc 900
tgccagcgcc tggcgagggc tggggtcgtg ctggtcaccg ctgccggcaa cttccgggac 960
gatgcctgcc tctactcccc agcctcagct cccgaggtca tcacagttgg ggccaccaat 1020
gcccaagacc agccggtgac cctggggact ttggggacca actttggccg ctgtgtggac 1080
ctctttgccc caggggagga catcattggt gcctccagcg actgcagcac ctgctttgtg 1140
tcacagagtg ggacatcaca ggctgctgcc cacgtggctg gcattgcagc catgatgctg 1200
tctgccgagc cggagctcac cctggccgag ttgaggcaga gactgatcca cttctctgcc 1260
aaagatgtca tcaatgaggc ctggttccct gaggaccagc gggtactgac ccccaacctg 1320
gtggccgccc tgccccccag cacccatggg gcaggttggc agctgttttg caggactgta 1380
tggtcagcac actcggggcc tacacggatg gccacagccg tcgcccgctg cgccccagat 1440
gaggagctgc tgagctgctc cagtttctcc aggagtggga agcggcgggg cgagcgcatg 1500
gaggcccaag gggcaagct ggtctgccgg gcccacaacg cttttggggg tgagggtgtc 1560
tacgccattg ccaggtgctg cctgctaccc caggccaact gcagcgtcca cacagctcca 1620
ccagctgagg ccagcatggg gacccgtgtc cactgccacc aacagggcca cgtcctcaca 1680
ggctgcagct cccactggga ggtggaggac cttggcaccc acaagccgcc tgtgctgagg 1740
ccacgaggtc agcccaacca gtgcgtgggc cacagggagg ccagcatcca cgcttcctgc 1800
tgccatgccc caggtctgga atgcaaagtc aaggagcatg gaatcccggc ccctcaggag 1860
caggtgaccg tggcctgcga ggagggctgg accctgactg gctgcagtgc cctccctggg 1920
acctcccacg tcctgggggc ctacgccgta gacaacacgt gtgtagtcag gagccgggac 1980
gtcagcacta caggcagcac cagcgaaggg gccgtgacag ccgttgccat ctgctgccgg 2040
agccggcacc tggcgcaggc ctcccaggag ctccag 2076

<210> 755
<211> 692
<212> PRT
<213> Homo sapiens

<400> 755
Met Gly Thr Val Ser Ser Arg Arg Ser Trp Trp Pro Leu Pro Leu Leu
1 5 10 15
Leu Leu Leu Leu Leu Leu Leu Gly Pro Ala Gly Ala Arg Ala Gln Glu
20 25 30
Asp Glu Asp Gly Asp Tyr Glu Glu Leu Val Leu Ala Leu Arg Ser Glu
35 40 45
Glu Asp Gly Leu Ala Glu Ala Pro Glu His Gly Thr Thr Ala Thr Phe
50 55 60
His Arg Cys Ala Lys Asp Pro Trp Arg Leu Pro Gly Thr Tyr Val Val
65 70 75 80
Val Leu Lys Glu Glu Thr His Leu Ser Gln Ser Glu Arg Thr Ala Arg
85 90 95
Arg Leu Gln Ala Gln Ala Ala Arg Arg Gly Tyr Leu Thr Lys Ile Leu
100 105 110
His Val Phe His Gly Leu Leu Pro Gly Phe Leu Val Lys Met Ser Gly
115 120 125

```
Asp Leu Leu Glu Leu Ala Leu Lys Leu Pro His Val Asp Tyr Ile Glu
    130                 135                 140
Glu Asp Ser Ser Val Phe Ala Gln Ser Ile Pro Trp Asn Leu Glu Arg
145                 150                 155                 160
Ile Thr Pro Pro Arg Tyr Arg Ala Asp Glu Tyr Gln Pro Pro Asp Gly
                165                 170                 175
Gly Ser Leu Val Glu Val Tyr Leu Leu Asp Thr Ser Ile Gln Ser Asp
            180                 185                 190
His Arg Glu Ile Glu Gly Arg Val Met Val Thr Asp Phe Glu Asn Val
        195                 200                 205
Pro Glu Glu Asp Gly Thr Arg Phe His Arg Gln Ala Ser Lys Cys Asp
    210                 215                 220
Ser His Gly Thr His Leu Ala Gly Val Val Ser Gly Arg Asp Ala Gly
225                 230                 235                 240
Val Ala Lys Gly Ala Ser Met Arg Ser Leu Arg Val Leu Asn Cys Gln
                245                 250                 255
Gly Lys Gly Thr Val Ser Gly Thr Leu Ile Gly Leu Glu Phe Ile Arg
            260                 265                 270
Lys Ser Gln Leu Val Gln Pro Val Gly Pro Leu Val Val Leu Leu Pro
        275                 280                 285
Leu Ala Gly Gly Tyr Ser Arg Val Leu Asn Ala Ala Cys Gln Arg Leu
    290                 295                 300
Ala Arg Ala Gly Val Val Leu Val Thr Ala Ala Gly Asn Phe Arg Asp
305                 310                 315                 320
Asp Ala Cys Leu Tyr Ser Pro Ala Ser Ala Pro Glu Val Ile Thr Val
                325                 330                 335
Gly Ala Thr Asn Ala Gln Asp Gln Pro Val Thr Leu Gly Thr Leu Gly
            340                 345                 350
Thr Asn Phe Gly Arg Cys Val Asp Leu Phe Ala Pro Gly Glu Asp Ile
        355                 360                 365
Ile Gly Ala Ser Ser Asp Cys Ser Thr Cys Phe Val Ser Gln Ser Gly
    370                 375                 380
Thr Ser Gln Ala Ala Ala His Val Ala Gly Ile Ala Ala Met Met Leu
385                 390                 395                 400
Ser Ala Glu Pro Glu Leu Thr Leu Ala Glu Leu Arg Gln Arg Leu Ile
                405                 410                 415
His Phe Ser Ala Lys Asp Val Ile Asn Glu Ala Trp Phe Pro Glu Asp
            420                 425                 430
Gln Arg Val Leu Thr Pro Asn Leu Val Ala Ala Leu Pro Pro Ser Thr
        435                 440                 445
His Gly Ala Gly Trp Gln Leu Phe Cys Arg Thr Val Trp Ser Ala His
    450                 455                 460
Ser Gly Pro Thr Arg Met Ala Thr Ala Val Ala Arg Cys Ala Pro Asp
465                 470                 475                 480
Glu Glu Leu Leu Ser Cys Ser Ser Phe Ser Arg Ser Gly Lys Arg Arg
                485                 490                 495
Gly Glu Arg Met Glu Ala Gln Gly Gly Lys Leu Val Cys Arg Ala His
            500                 505                 510
Asn Ala Phe Gly Gly Glu Gly Val Tyr Ala Ile Ala Arg Cys Cys Leu
        515                 520                 525
Leu Pro Gln Ala Asn Cys Ser Val His Thr Ala Pro Pro Ala Glu Ala
    530                 535                 540
Ser Met Gly Thr Arg Val His Cys His Gln Gln Gly His Val Leu Thr
545                 550                 555                 560
Gly Cys Ser Ser His Trp Glu Val Glu Asp Leu Gly Thr His Lys Pro
                565                 570                 575
Pro Val Leu Arg Pro Arg Gly Gln Pro Asn Gln Cys Val Gly His Arg
            580                 585                 590
Glu Ala Ser Ile His Ala Ser Cys Cys His Ala Pro Gly Leu Glu Cys
        595                 600                 605
Lys Val Lys Glu His Gly Ile Pro Ala Pro Gln Glu Gln Val Thr Val
    610                 615                 620
Ala Cys Glu Glu Gly Trp Thr Leu Thr Gly Cys Ser Ala Leu Pro Gly
625                 630                 635                 640
Thr Ser His Val Leu Gly Ala Tyr Ala Val Asp Asn Thr Cys Val Val
```

```
                645                 650                 655
Arg Ser Arg Asp Val Ser Thr Thr Gly Ser Thr Ser Glu Gly Ala Val
            660                 665                 670
Thr Ala Val Ala Ile Cys Cys Arg Ser Arg His Leu Ala Gln Ala Ser
        675                 680                 685
Gln Glu Leu Gln
    690


<210> 756
<211> 692
<212> PRT
<213> Macaca mulata

<400> 756
Met Gly Thr Val Ser Ser Arg Arg Ser Trp Trp Pro Leu Pro Leu Pro
1               5                   10                  15
Leu Leu Leu Leu Leu Leu Leu Gly Pro Ala Gly Ala Arg Ala Gln Glu
            20                  25                  30
Asp Glu Asp Gly Asp Tyr Glu Glu Leu Val Leu Ala Leu Arg Ser Glu
        35                  40                  45
Glu Asp Gly Leu Ala Asp Ala Pro Glu His Gly Ala Thr Ala Thr Phe
    50                  55                  60
His Arg Cys Ala Lys Asp Pro Trp Arg Leu Pro Gly Thr Tyr Val Val
65                  70                  75                  80
Val Leu Lys Glu Glu Thr His Arg Ser Gln Ser Glu Arg Thr Ala Arg
                85                  90                  95
Arg Leu Gln Ala Gln Ala Ala Arg Arg Gly Tyr Leu Thr Lys Ile Leu
            100                 105                 110
His Val Phe His His Leu Leu Pro Gly Phe Leu Val Lys Met Ser Gly
        115                 120                 125
Asp Leu Leu Glu Leu Ala Leu Lys Leu Pro His Val Asp Tyr Ile Glu
    130                 135                 140
Glu Asp Ser Ser Val Phe Ala Gln Ser Ile Pro Trp Asn Leu Glu Arg
145                 150                 155                 160
Ile Thr Pro Ala Arg Tyr Arg Ala Asp Glu Tyr Gln Pro Pro Lys Gly
                165                 170                 175
Gly Ser Leu Val Glu Val Tyr Leu Leu Asp Thr Ser Ile Gln Ser Asp
            180                 185                 190
His Arg Glu Ile Glu Gly Arg Val Met Val Thr Asp Phe Glu Ser Val
        195                 200                 205
Pro Glu Glu Asp Gly Thr Arg Phe His Arg Gln Ala Ser Lys Cys Asp
    210                 215                 220
Ser His Gly Thr His Leu Ala Gly Val Val Ser Gly Arg Asp Ala Gly
225                 230                 235                 240
Val Ala Lys Gly Ala Gly Leu Arg Ser Leu Arg Val Leu Asn Cys Gln
                245                 250                 255
Gly Lys Gly Thr Val Ser Gly Thr Leu Ile Gly Leu Glu Phe Ile Arg
            260                 265                 270
Lys Ser Gln Leu Val Gln Pro Val Gly Pro Leu Val Val Leu Leu Pro
        275                 280                 285
Leu Ala Gly Gly Tyr Ser Arg Val Phe Asn Ala Ala Cys Gln Arg Leu
    290                 295                 300
Ala Arg Ala Gly Val Val Leu Val Thr Ala Ala Gly Asn Phe Arg Asp
305                 310                 315                 320
Asp Ala Cys Leu Tyr Ser Pro Ala Ser Ala Pro Glu Val Ile Thr Val
                325                 330                 335
Gly Ala Thr Asn Ala Gln Asp Gln Pro Val Thr Leu Gly Thr Leu Gly
            340                 345                 350
Thr Asn Phe Gly Arg Cys Val Asp Leu Phe Ala Pro Gly Glu Asp Ile
        355                 360                 365
Ile Gly Ala Ser Ser Asp Cys Ser Thr Cys Phe Val Ser Arg Ser Gly
    370                 375                 380
Thr Ser Gln Ala Ala Ala His Val Ala Gly Ile Ala Ala Met Met Leu
385                 390                 395                 400
```

```
Ser Ala Glu Pro Glu Leu Thr Leu Ala Glu Leu Arg Gln Arg Leu Ile
                405                 410                 415
His Phe Ser Ala Lys Asp Val Ile Asn Glu Ala Trp Phe Pro Glu Asp
            420                 425                 430
Gln Arg Val Leu Thr Pro Asn Leu Val Ala Ala Leu Pro Pro Ser Thr
        435                 440                 445
His Arg Ala Gly Trp Gln Leu Phe Cys Arg Thr Val Trp Ser Ala His
    450                 455                 460
Ser Gly Pro Thr Arg Met Ala Thr Ala Val Ala Arg Cys Ala Gln Asp
465                 470                 475                 480
Glu Glu Leu Leu Ser Cys Ser Ser Phe Ser Arg Ser Gly Lys Arg Arg
                485                 490                 495
Gly Glu Arg Ile Glu Ala Gln Gly Gly Lys Arg Val Cys Arg Ala His
            500                 505                 510
Asn Ala Phe Gly Gly Glu Gly Val Tyr Ala Ile Ala Arg Cys Cys Leu
        515                 520                 525
Leu Pro Gln Val Asn Cys Ser Val His Thr Ala Pro Pro Ala Gly Ala
    530                 535                 540
Ser Met Gly Thr Arg Val His Cys His Gln Gln Gly His Val Leu Thr
545                 550                 555                 560
Gly Cys Ser Ser His Trp Glu Val Glu Asp Leu Gly Thr His Lys Pro
                565                 570                 575
Pro Val Leu Arg Pro Arg Gly Gln Pro Asn Gln Cys Val Gly His Arg
            580                 585                 590
Glu Ala Ser Ile His Ala Ser Cys Cys His Ala Pro Gly Leu Glu Cys
        595                 600                 605
Lys Val Lys Glu His Gly Ile Pro Ala Pro Gln Glu Gln Val Ile Val
    610                 615                 620
Ala Cys Glu Asp Gly Trp Thr Leu Thr Gly Cys Ser Pro Leu Pro Gly
625                 630                 635                 640
Thr Ser His Val Leu Gly Ala Tyr Ala Val Asp Asn Thr Cys Val Val
                645                 650                 655
Arg Ser Arg Asp Val Ser Thr Thr Gly Ser Thr Ser Lys Glu Ala Val
            660                 665                 670
Ala Ala Val Ala Ile Cys Cys Arg Ser Arg His Leu Val Gln Ala Ser
        675                 680                 685
Gln Glu Leu Gln
    690


<210> 757
<211> 694
<212> PRT
<213> Mus muscular

<400> 757
Met Gly Thr His Cys Ser Ala Trp Leu Arg Trp Pro Leu Leu Pro Leu
 1               5                  10                  15
Leu Pro Pro Leu Leu Leu Leu Leu Leu Leu Leu Cys Pro Thr Gly Ala
            20                  25                  30
Gly Ala Gln Asp Glu Asp Gly Asp Tyr Glu Glu Leu Met Leu Ala Leu
        35                  40                  45
Pro Ser Gln Glu Asp Gly Leu Ala Asp Glu Ala Ala His Val Ala Thr
    50                  55                  60
Ala Thr Phe Arg Arg Cys Ser Lys Glu Ala Trp Arg Leu Pro Gly Thr
65                  70                  75                  80
Tyr Ile Val Val Leu Met Glu Glu Thr Gln Arg Leu Gln Ile Glu Gln
                85                  90                  95
Thr Ala His Arg Leu Gln Thr Arg Ala Ala Arg Arg Gly Tyr Val Ile
            100                 105                 110
Lys Val Leu His Ile Phe Tyr Asp Leu Phe Pro Gly Phe Leu Val Lys
        115                 120                 125
Met Ser Ser Asp Leu Leu Gly Leu Ala Leu Lys Leu Pro His Val Glu
    130                 135                 140
Tyr Ile Glu Glu Asp Ser Phe Val Phe Ala Gln Ser Ile Pro Trp Asn
```

```
145                 150                 155                 160
Leu Glu Arg Ile Ile Pro Ala Trp His Gln Thr Glu Glu Asp Arg Ser
                165                 170                 175
Pro Asp Gly Ser Ser Gln Val Glu Val Tyr Leu Leu Asp Thr Ser Ile
            180                 185                 190
Gln Gly Ala His Arg Glu Ile Glu Gly Arg Val Thr Ile Thr Asp Phe
        195                 200                 205
Asn Ser Val Pro Glu Glu Asp Gly Thr Arg Phe His Arg Gln Ala Ser
    210                 215                 220
Lys Cys Asp Ser His Gly Thr His Leu Ala Gly Val Val Ser Gly Arg
225                 230                 235                 240
Asp Ala Gly Val Ala Lys Gly Thr Ser Leu His Ser Leu Arg Val Leu
                245                 250                 255
Asn Cys Gln Gly Lys Gly Thr Val Ser Gly Thr Leu Ile Gly Leu Glu
            260                 265                 270
Phe Ile Arg Lys Ser Gln Leu Ile Gln Pro Ser Gly Pro Leu Val Val
        275                 280                 285
Leu Leu Pro Leu Ala Gly Gly Tyr Ser Arg Ile Leu Asn Ala Ala Cys
    290                 295                 300
Arg His Leu Ala Arg Thr Gly Val Val Leu Val Ala Ala Ala Gly Asn
305                 310                 315                 320
Phe Arg Asp Asp Ala Cys Leu Tyr Ser Pro Ala Ser Ala Pro Glu Val
                325                 330                 335
Ile Thr Val Gly Ala Thr Asn Ala Gln Asp Gln Pro Val Thr Leu Gly
            340                 345                 350
Thr Leu Gly Thr Asn Phe Gly Arg Cys Val Asp Leu Phe Ala Pro Gly
        355                 360                 365
Lys Asp Ile Ile Gly Ala Ser Ser Asp Cys Ser Thr Cys Phe Met Ser
    370                 375                 380
Gln Ser Gly Thr Ser Gln Ala Ala Ala His Val Ala Gly Ile Val Ala
385                 390                 395                 400
Arg Met Leu Ser Arg Glu Pro Thr Leu Thr Leu Ala Glu Leu Arg Gln
                405                 410                 415
Arg Leu Ile His Phe Ser Thr Lys Asp Val Ile Asn Met Ala Trp Phe
            420                 425                 430
Pro Glu Asp Gln Gln Val Leu Thr Pro Asn Leu Val Ala Thr Leu Pro
        435                 440                 445
Pro Ser Thr His Glu Thr Gly Gly Gln Leu Leu Cys Arg Thr Val Trp
    450                 455                 460
Ser Ala His Ser Gly Pro Thr Arg Thr Ala Thr Ala Thr Ala Arg Cys
465                 470                 475                 480
Ala Pro Glu Glu Glu Leu Leu Ser Cys Ser Ser Phe Ser Arg Ser Gly
                485                 490                 495
Arg Arg Arg Gly Asp Trp Ile Glu Ala Ile Gly Gly Gln Gln Val Cys
            500                 505                 510
Lys Ala Leu Asn Ala Phe Gly Gly Glu Gly Val Tyr Ala Val Ala Arg
        515                 520                 525
Cys Cys Leu Val Pro Arg Ala Asn Cys Ser Ile His Asn Thr Pro Ala
    530                 535                 540
Ala Arg Ala Gly Leu Glu Thr His Val His Cys His Gln Lys Asp His
545                 550                 555                 560
Val Leu Thr Gly Cys Ser Phe His Trp Glu Val Glu Asp Leu Ser Val
                565                 570                 575
Arg Arg Gln Pro Ala Leu Arg Ser Arg Arg Gln Pro Gly Gln Cys Val
            580                 585                 590
Gly His Gln Ala Ala Ser Val Tyr Ala Ser Cys Cys His Ala Pro Gly
        595                 600                 605
Leu Glu Cys Lys Ile Lys Glu His Gly Ile Ser Gly Pro Ser Glu Gln
    610                 615                 620
Val Thr Val Ala Cys Glu Ala Gly Trp Thr Leu Thr Gly Cys Asn Val
625                 630                 635                 640
Leu Pro Gly Ala Ser Leu Thr Leu Gly Ala Tyr Ser Val Asp Asn Leu
                645                 650                 655
Cys Val Ala Arg Val His Asp Thr Ala Arg Ala Asp Arg Thr Ser Gly
            660                 665                 670
```

Glu Ala Thr Val Ala Ala Ala Ile Cys Cys Arg Ser Arg Pro Ser Ala
675 680 685
Lys Ala Ser Trp Val Gln
690

<210> 758
<211> 653
<212> PRT
<213> Homo sapiens

<400> 758
Glu Phe Arg Cys His Asp Gly Lys Cys Ile Ser Arg Gln Phe Val Cys
1 5 10 15
Asp Ser Asp Arg Asp Cys Leu Asp Gly Ser Asp Glu Ala Ser Cys Pro
20 25 30
Val Leu Thr Cys Gly Pro Ala Ser Phe Gln Cys Asn Ser Ser Thr Cys
35 40 45
Ile Pro Gln Leu Trp Ala Cys Asp Asn Asp Pro Asp Cys Glu Asp Gly
50 55 60
Ser Asp Glu Trp Pro Gln Arg Cys Arg Gly Leu Tyr Val Phe Gln Gly
65 70 75 80
Asp Ser Ser Pro Cys Ser Ala Phe Glu Phe His Cys Leu Ser Gly Glu
85 90 95
Cys Ile His Ser Ser Trp Arg Cys Asp Gly Gly Pro Asp Cys Lys Asp
100 105 110
Lys Ser Asp Glu Glu Asn Cys Ala Val Ala Thr Cys Arg Pro Asp Glu
115 120 125
Phe Gln Cys Ser Asp Gly Asn Cys Ile His Gly Ser Arg Gln Cys Asp
130 135 140
Arg Glu Tyr Asp Cys Lys Asp Met Ser Asp Glu Val Gly Cys Val Asn
145 150 155 160
Val Thr Leu Cys Glu Gly Pro Asn Lys Phe Lys Cys His Ser Gly Glu
165 170 175
Cys Ile Thr Leu Asp Lys Val Cys Asn Met Ala Arg Asp Cys Arg Asp
180 185 190
Trp Ser Asp Glu Pro Ile Lys Glu Cys Gly Thr Asn Glu Cys Leu Asp
195 200 205
Asn Asn Gly Gly Cys Ser His Val Cys Asn Asp Leu Lys Ile Gly Tyr
210 215 220
Glu Cys Leu Cys Pro Asp Gly Phe Gln Leu Val Ala Gln Arg Arg Cys
225 230 235 240
Glu Asp Ile Asp Glu Cys Gln Asp Pro Asp Thr Cys Ser Gln Leu Cys
245 250 255
Val Asn Leu Glu Gly Gly Tyr Lys Cys Gln Cys Glu Glu Gly Phe Gln
260 265 270
Leu Asp Pro His Thr Lys Ala Cys Lys Ala Val Gly Ser Ile Ala Tyr
275 280 285
Leu Phe Phe Thr Asn Arg His Glu Val Arg Lys Met Thr Leu Asp Arg
290 295 300
Ser Glu Tyr Thr Ser Leu Ile Pro Asn Leu Arg Asn Val Val Ala Leu
305 310 315 320
Asp Thr Glu Val Ala Ser Asn Arg Ile Tyr Trp Ser Asp Leu Ser Gln
325 330 335
Arg Met Ile Cys Ser Thr Gln Leu Asp Arg Ala His Gly Val Ser Ser
340 345 350
Tyr Asp Thr Val Ile Ser Arg Asp Ile Gln Ala Pro Asp Gly Leu Ala
355 360 365
Val Asp Trp Ile His Ser Asn Ile Tyr Trp Thr Asp Ser Val Leu Gly
370 375 380
Thr Val Ser Val Ala Asp Thr Lys Gly Val Lys Arg Lys Thr Leu Phe
385 390 395 400
Arg Glu Asn Gly Ser Lys Pro Arg Ala Ile Val Val Asp Pro Val His
405 410 415
Gly Phe Met Tyr Trp Thr Asp Trp Gly Thr Pro Ala Lys Ile Lys Lys

```
                420                 425                 430
Gly Gly Leu Asn Gly Val Asp Ile Tyr Ser Leu Val Thr Glu Asn Ile
        435                 440                 445
Gln Trp Pro Asn Gly Ile Thr Leu Asp Leu Leu Ser Gly Arg Leu Tyr
    450                 455                 460
Trp Val Asp Ser Lys Leu His Ser Ile Ser Ser Ile Asp Val Asn Gly
465                 470                 475                 480
Gly Asn Arg Lys Thr Ile Leu Glu Asp Glu Lys Arg Leu Ala His Pro
                485                 490                 495
Phe Ser Leu Ala Val Phe Glu Asp Lys Val Phe Trp Thr Asp Ile Ile
            500                 505                 510
Asn Glu Ala Ile Phe Ser Ala Asn Arg Leu Thr Gly Ser Asp Val Asn
        515                 520                 525
Leu Leu Ala Glu Asn Leu Leu Ser Pro Glu Asp Met Val Leu Phe His
    530                 535                 540
Asn Leu Thr Gln Pro Arg Gly Val Asn Trp Cys Glu Arg Thr Thr Leu
545                 550                 555                 560
Ser Asn Gly Gly Cys Gln Tyr Leu Cys Leu Pro Ala Pro Gln Ile Asn
                565                 570                 575
Pro His Ser Pro Lys Phe Thr Cys Ala Cys Pro Asp Gly Met Leu Leu
            580                 585                 590
Ala Arg Asp Met Arg Ser Cys Leu Thr Glu Ala Glu Ala Ala Val Ala
        595                 600                 605
Thr Gln Glu Thr Ser Thr Val Arg Leu Lys Val Ser Ser Thr Ala Val
    610                 615                 620
Arg Thr Gln His Thr Thr Thr Arg Pro Val Pro Asp Thr Ser Arg Leu
625                 630                 635                 640
Pro Gly Ala Thr Pro Gly Leu Thr Thr Val Glu Ile Val
                645                 650
```

1

**Description**

The present invention relates to methods for treating diseases or conditions in which proprotein convertase subtilisin/kexin type 9 (PCSK9) expression or activity causes an impact by administration of PCSK9-specific antibodies or antigen-binding fragments thereof. The present invention further relates to PCSK9-specific antibodies or antigen-binding fragments thereof for use in the treatment of diseases or conditions in which PCSK9 expression or activity causes an impact.

The present invention also relates to articles of manufacture comprising packaging material, PCSK9-specific antibodies or antigen-binding fragments thereof, and a label or packaging insert indicating which groups of patients can be treated with said antibodies or fragments, which groups of patients must not be treated with said antibodies or fragments, and/or which dosage regimen should be used.

BACKGROUND OF THE INVENTION

Proprotein convertase subtilisin/kexin type 9 (PCSK9) is a proprotein convertase belonging to the proteinase K subfamily of the secretory subtilase family. The encoded protein is synthesized as a soluble zymogen that undergoes autocatalytic intramolecular processing in the endoplasmic reticulum. Evidence suggest that PCSK9 increases plasma LDL cholesterol by promoting degradation of the LDL receptor, which mediates LDL endocytosis in the liver, the major route of LDL clearance from circulation. The structure of PCSK9 protein shows that it has a signal sequence, followed by a prodomain, a catalytic domain that contains a conserved triad of residues (D186, H226 and S386), and a C-terminal domain. It is synthesized as a soluble 74-kDa precursor that undergoes autocatalytic cleavage in the ER, generating a 14-kDa prodomain and 60-kDa catalytic fragment. The autocatalytic activity has been shown to be required for secretion. After cleavage the prodomain remains tightly associated with the catalytic domain.

Antibodies to PCSK9 are described in, for example, WO 2008/057457, WO 2008/057458, WO 2008/057459, WO 2008/063382, WO 2008/125623, and US 2008/0008697. Anti-PCSK9 antibodies that are particularly well-suited for practicing the present invention are disclosed in US 2010/0166768 A1, the content of which is hereby incorporated by reference in its entirety.

TECHNICAL PROBLEMS UNDERLYING THE PRESENT INVENTION

Statins and other small molecule lipid lowering drugs are among the most widely used drugs in the world. However, about up to 50% of the patients worldwide (with even higher percentages in patients having a form of familial hypercholesterolemia) fail to achieve their lipid target. This is partly due to the fact that some patient populations are statin intolerant (particularly at the highest approved dose) or cannot reach their lipid target despite treatment with the highest approved dose of statin (statin-resistant populations). It is thus necessary to provide alternative methods of treatment that are also amenable for statin intolerant or statin-resistant patient populations.

Despite the widespread availability of lipid-lowering agents such as statins, approximately 30% of all adult patients treated for hypercholesterolemia in the United States between 1999 and 2006 failed to achieve their recommended LDL-C targets. Reasons for this include poor adherence to therapy, drug-resistance/intolerance and the positive relationship between adverse event rates and increasing dosage. Moreover, since the most effective lipid-lowering agents can only reduce LDL-C levels by up to 55%, target attainment rates in patients that require substantial reductions in LDL-C, such as those with familial hypercholesterolemia, are often significantly lower than might be expected. More effective lipid-lowering agents and treatment regimes are therefore required to improve target attainment rates in these patients.

Quite surprisingly, the inventors of the present invention found that the administration of the anti-PCSK9 antibodies or fragments thereof herein described is very effective in combination with a lipid lowering agent inhibiting cholesterol absorption into the intestine such as ezetimibe and is very effective in combination with a fibrate.

The above overview does not necessarily describe all problems solved by the present invention.

SUMMARY OF THE INVENTION

In a first aspect, present invention concerns a method for treating a disease or condition in which PCSK9 expression or activity causes an impact comprising administering a therapeutic amount of an antibody or antigen-binding fragment thereof which specifically binds hPCSK9 (human proprotein convertase subtilisin/kexin type 9) to a subject in need thereof, wherein the method comprises administering of the antibody or antigen-binding fragment thereof together with

a. a therapeutic amount of a lipid lowering agent acting by decreasing cholesterol adsorption in the intestine, or

b. a therapeutic amount of a fibrate, or

c. a therapeutic amount of the lipid lowering agent of a) and a therapeutic amount of a fibrate.

In a second aspect, present invention concerns a method of lowering cholesterol levels in a subject in need thereof, comprising:

(a) selecting a subject with a blood low density lipoprotein cholesterol (LDL-C) level > 100 mg/dL; and
(b) administering to said subject a composition comprising an antibody or antigen-binding fragment thereof or antigen binding fragment thereof that specifically binds to human proprotein convertase subtilisin/kexin type 9 (hPCSK9); thereby lowering cholesterol levels in the subject in need thereof, wherein the method comprises administering of the antibody or antigen-binding fragment thereof or fragment thereof together with

(i) a therapeutic amount of a lipid lowering agent acting by decreasing cholesterol adsorption in the intestine, or

(ii) a therapeutic amount of a fibrate, or

(iii) a therapeutic amount of the lipid lowering agent of a) and a therapeutic amount of a fibrate.

In a third aspect present invention concerns a method of preventing effects of a (persistently) increased LDL-C level in the blood comprising administering a therapeutic amount of an antibody or antigen-binding fragment thereof which specifically binds hPCSK9 (human proprotein convertase subtilisin/kexin type 9) to a subject having an increased LDL-C level in the blood, and

(a) administering a therapeutic amount of a lipid lowering agent acting by decreasing cholesterol adsorption in the intestine, or

(b) administering a therapeutic amount of a fibrate, or

(c) administering a therapeutic amount of the lipid lowering agent of a) and a therapeutic amount of a fibrate.

In a fourth aspect, present invention concerns an antibody or antigen-binding fragment thereof which specifically binds hPCSK9 (human proprotein convertase subtilisin/kexin type 9) for use in a method according to the first, second or third aspect of present invention.
In a fifth aspect, present invention concerns a pharmaceutical composition comprising an antibody or antigen-binding fragment thereof of present invention for use in a method according to the first, second or third aspect of present invention.
In a sixth aspect, present invention concerns a pharmaceutical composition comprising the antibody or antigen-binding fragment thereof of present invention together with a fibrate and a pharmaceutically acceptable excipient.
In a seventh aspect, present invention concerns an article of manufacture comprising

(a) a packaging material or container;

(b) an antibody or antigen-binding fragment thereof which specifically binds hPCSK9; and

(c) a fibrate and/or a lipid lowering drug acting by decreasing cholesterol adsorption in the intestine; and

(d) a label or packaging insert.

In an eighth aspect, present invention concerns a method for preparing an article of manufacture according to the seventh aspect comprising packaging the antibody or antigen-binding fragment thereof and packaging the lipid lowering agent and/or the fibrate in a container and assembling them with a label or packaging insert.
In a ninth aspect, present invention concerns a unit dosage form comprising an antibody or antigen-binding fragment thereof which specifically binds hPCSK9 (human proprotein convertase subtilisin/kexin type 9) together with a fibrate.
In a tenth aspect, present invention concerns a unit dosage form comprising an antibody or antigen-binding fragment thereof which specifically binds hPCSK9 (human proprotein convertase subtilisin/kexin type 9) for use in a method according to the first, second or third aspect.

This summary of the invention does not necessarily describe all features of the present invention. Other embodiments will become apparent from a review of the ensuing detailed description.

DETAILED DESCRIPTION OF THE INVENTION

Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art to which this invention belongs.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents (for example: patents, patent applications, scientific publications, manufacturer's specifications, instructions, GenBank Accession Number sequence submissions etc.) are cited throughout the text of this specification. Nothing herein is to be construed as an admission that the invention is not entitled to antedate such disclosure by virtue of prior invention. Some of the documents cited herein are characterized as being *"incorporated by reference".* In the event of a conflict between the definitions or teachings of such incorporated references and definitions or teachings recited in the present specification, the text of the present specification takes precedence.

Sequences: All sequences referred to herein are disclosed in the attached sequence listing that, with its whole content and disclosure, is a part of this specification.

The term "about" when used in connection with a numerical value is meant to encompass numerical values within a range having a lower limit that is 5% smaller than the indicated numerical value and having an upper limit that is 5% larger than the indicated numerical value.

The term "human proprotein convertase subtilisin/kexin type 9" or "hPCSK9", as used herein, refers to hPCSK9 having the nucleic acid sequence shown in SEQ ID NO: 754 and the amino acid sequence of SEQ ID NO: 755, or a biologically active fragment thereof.

The terms "specifically binds", "specific binding" or the like, mean that an antibody or antigen-binding fragment thereof forms a complex with an antigen that is relatively stable under physiologic conditions. Specific binding can be characterized by an equilibrium dissociation constant of at least about $1x10^{-6}$ M or less (e.g., a smaller $K_D$ denotes a tighter binding). Methods for determining whether two molecules specifically bind are well known in the art and include, for example, equilibrium dialysis, surface plasmon resonance, and the like. An isolated antibody that specifically binds hPCSK9 may, however, exhibit cross-reactivity to other antigens such as PCSK9 molecules from other species. Moreover, multi-specific antibodies (e.g., bispecifics) that bind to hPCSK9 and one or more additional antigens are nonetheless considered antibodies that "specifically bind" hPCSK9, as used herein.

The term "$K_D$ ", as used herein, is intended to refer to the equilibrium dissociation constant of a particular antibody-antigen interaction. The equilibrium dissociation constant is typically measured in "mol/L" (abbreviated as "M").

By the term "slow off rate", "Koff" or "kd" is meant an antibody that dissociates from hPCSK9 with a rate constant of $1 \times 10^{-3}$ $s^{-1}$ or less, preferably $1 \times 10^{-4}$ $s^{-1}$ or less, as determined by surface plasmon resonance, e.g., BIACORE™.

The term "high affinity" antibody refers to those mAbs having a binding affinity to hPCSK9 of at least $10^{-10}$ M; preferably $10^{-11}$ M; even more preferably $10^{-12}$ M, as measured by surface plasmon resonance, e.g., BIACORE™ or solution-affinity ELISA.

The term "surface plasmon resonance", as used herein, refers to an optical phenomenon that allows for the analysis of real-time biospecific interactions by detection of alterations in protein concentrations within a biosensor matrix, for example using the BIACORE™ system (Pharmacia Biosensor AB, Uppsala, Sweden and Piscataway, N.J.).

An "epitope", also known as antigenic determinant, is the region of an antigen that is recognized by the immune system, specifically by antibodies, B cells, or T cells. As used herein, an "epitope" is the part of an antigen capable of binding to an antibody or antigen-binding fragment thereof as described herein. In this context, the term "binding" preferably relates to a "specific binding", as defined herein. Epitopes usually consist of chemically active surface groupings of molecules such as amino acids, sugar side chains, phosphoryl groups, or sulfonyl groups and may have specific three-dimensional structural characteristics and/or specific charge characteristics. Conformational and non-conformational epitopes can be distinguished in that the binding to the former but not the latter is lost in the presence of denaturing

solvents.

A "paratope" is the part of an antibody that specifically binds to the epitope.

The term "antibody", as used herein, is intended to refer to immunoglobulin molecules comprised of four polypeptide chains, two heavy (H) chains and two light (L) chains inter-connected by disulfide bonds. The term "antibody" also includes all recombinant forms of antibodies, in particular of the antibodies described herein, e.g. antibodies expressed in prokaryotes, unglycosylated antibodies, and any antigen-binding antibody fragments and derivatives as described below. Each heavy chain is comprised of a heavy chain variable region ("HCVR" or "VH") and a heavy chain constant region (comprised of domains CH1, CH2 and CH3). Each light chain is comprised of a light chain variable region ("LCVR or "VL") and a light chain constant region (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1 q) of the classical complement system.

Substitution of one or more CDR residues or omission of one or more CDRs is also possible. Antibodies have been described in the scientific literature in which one or two CDRs can be dispensed with for binding. Padlan et al. (1995 FASEB J. 9:133-139) analyzed the contact regions between antibodies and their antigens, based on published crystal structures, and concluded that only about one fifth to one third of CDR residues actually contact the antigen. Padlan also found many antibodies in which one or two CDRs had no amino acids in contact with an antigen (see also, Vajdos et al. 2002 J Mol Biol 320:415-428).

CDR residues not contacting antigen can be identified based on previous studies (for example residues H60-H65 in CDRH2 are often not required), from regions of Kabat CDRs lying outside Chothia CDRs, by molecular modeling and/or empirically. If a CDR or residue(s) thereof is omitted, it is usually substituted with an amino acid occupying the corresponding position in another human antibody sequence or a consensus of such sequences. Positions for substitution within CDRs and amino acids to substitute can also be selected empirically. Empirical substitutions can be conservative or non-conservative substitutions.

The term "antigen-binding fragment" of an antibody (or simply "binding portion"), as used herein, refers to one or more fragments of an antibody that retain the ability to specifically bind to hPCSK9. It has been shown that the antigen-binding function of an antibody can be performed by fragments of a full-length antibody. Examples of binding fragments encompassed within the term "antigen-binding fragment" of an antibody include (i) Fab fragments, monovalent fragments consisting of the VL, VH, CL and CH domains; (ii) $F(ab')_2$ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is a binding-domain immunoglobulin fusion protein comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies. Further examples of "antigen-binding fragments" are so-called microantibodies, which are derived from single CDRs. For example, Heap et al. describe a 17 amino acid residue microantibody derived from the heavy chain CDR3 of an antibody directed against the gp120 envelope glycoprotein of HIV-1 (Heap CJ et al. (2005) J. Gen. Virol. 86:1791-1800). Other examples include small antibody mimetics comprising two or more CDR regions that are fused to each other, preferably by cognate framework regions. Such a small antibody mimetic comprising VH CDR1 and VL CDR3 linked by the cognate VH FR2 has been described by Qiu et al. (Qiu X-Q, et al. (2007) Nature biotechnology 25(8):921-929).

Thus, the term "antibody or antigen-binding fragment thereof", as used herein, refers to immunoglobulin molecules and immunologically active portions of immunoglobulin molecules, i.e. molecules that contain an antigen-binding site that immunospecifically binds an antigen.

Antibodies and antigen-binding fragments thereof usable in the invention may be from any animal origin including birds and mammals. Preferably, the antibodies or fragments are from human, chimpanzee, rodent (e.g. mouse, rat,

guinea pig, or rabbit), chicken, turkey, pig, sheep, goat, camel, cow, horse, donkey, cat, or dog origin. It is particularly preferred that the antibodies are of human or murine origin. Antibodies of the invention also include chimeric molecules in which an antibody constant region derived from one species, preferably human, is combined with the antigen binding site derived from another species, e.g. mouse. Moreover antibodies of the invention include humanized molecules in which the antigen binding sites of an antibody derived from a non-human species (e.g. from mouse) are combined with constant and framework regions of human origin. Antibody molecules of the invention particularly also include fully human antibody molecules such as fully human monoclonal antibodies that are generated by in vitro systems such as phage display libraries or are generated in vivo, e.g. in transgenic mice carrying human immunoglobulin genes as transgenes in their genome.

As exemplified herein, antibodies of the invention can be obtained directly from hybridomas which express the antibody, or can be cloned and recombinantly expressed in a host cell (e.g., a CHO cell, or a lymphocytic cell). Further examples of host cells are microorganisms, such as *E. coli,* and fungi, such as yeast. Alternatively, they can be produced recombinantly in a transgenic non-human animal or plant.

The term "chimeric antibody" refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another species or class. Typically the variable region of both light and heavy chains mimics the variable regions of antibodies derived from one species of mammals, while the constant portions are homologous to sequences of antibodies derived from another. One clear advantage to such chimeric forms is that the variable region can conveniently be derived from presently known sources using readily available B-cells or hybridomas from non-human host organisms in combination with constant regions derived from, for example, human cell preparations. While the variable region has the advantage of ease of preparation and the specificity is not affected by the source, the constant region being human is less likely to elicit an immune response from a human subject when the antibodies are injected than would the constant region from a non-human source. However, the definition is not limited to this particular example.

The term "humanized antibody" refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen-binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "fully human antibody" refers to an immunoglobulin molecule, in which all variable or constant domains or regions present in the molecule are from one or more human immunoglobulin molecules and comprises a fully human mAb or fragment tereof as well as bispecifics, fusion proteins etc., in which all immunoglobulin-derived domains and regions present in the molecule are derived from human immunoglobulin(s). The terms "fully human antibody" and "human antibody" are used synonymously herin.

Different methods for humanizing antibodies are known to the skilled person, as reviewed by Almagro & Fransson, the content of which is herein incorporated by reference in its entirety (Almagro JC and Fransson J (2008) Frontiers in Bioscience 13:1619-1633). Almagro & Fransson distinguish between rational approaches and empirical approaches. Rational approaches are characterized by generating few variants of the engineered antibody and assessing their binding or any other property of interest. If the designed variants do not produce the expected results, a new cycle of design and binding assessment is initiated. Rational approaches include CDR grafting, Resurfacing, Superhumanization, and Human String Content Optimization. In contrast, empirical approaches are based on the generation of large libraries of humanized variants and selection of the best clones using enrichment technologies or high-throughput screening. Accordingly, empirical approaches are dependent on a reliable selection and/or screening system that is able to search through a vast space of antibody variants. *In vitro* display technologies, such as phage display and ribosome display fulfill these requirements and are well-known to the skilled person. Empirical approaches include FR libraries, Guided selection, Framework-shuffling, and Humaneering.

The term "human antibody", as used herein, is intended to include antibodies having variable and constant regions derived from human germline immunoglobulin sequences. The human mAbs of the invention may include amino acid residues not encoded by human germline immunoglobulin sequences (e.g., mutations introduced by random or site-specific mutagenesis *in vitro* or by somatic mutation *in vivo),* for example in the CDRs and in particular CDR3. However, the term "human antibody", as used herein, is not intended to include mAbs in which CDR sequences derived from the germline of another mammalian species (e.g., mouse), have been grafted onto human FR sequences. Human antibodies of the invention include antibodies isolated from human immunoglobulin libraries or from animals transgenic for one or more human immunoglobulin and that do not express endogenous immunoglobulins, as described for example

in U.S. Patent No. 5,939,598 by Kucherlapati & Jakobovits.

The term "monoclonal antibody" as used herein refers to a preparation of antibody molecules of single molecular composition. A monoclonal antibody displays a single binding specificity and affinity for a particular epitope. In one embodiment, the monoclonal antibodies are produced by a hybridoma which includes a B cell obtained from a non-human animal, e.g. mouse, fused to an immortalized cell.

The term "recombinant antibody", as used herein, includes all antibodies that are prepared, expressed, created or isolated by recombinant means, such as (a) antibodies isolated from an animal (e.g., a mouse) that is transgenic or transchromosomal with respect to the immunoglobulin genes or a hybridoma prepared therefrom, (b) antibodies isolated from a host cell transformed to express the antibody, e.g. from a transfectoma, (c) antibodies isolated from a recombinant, combinatorial antibody library, and (d) antibodies prepared, expressed, created or isolated by any other means that involve splicing of immunoglobulin gene sequences to other DNA sequences.

The term "transfectoma", as used herein, includes recombinant eukaryotic host cells expressing an antibody, such as CHO cells, NS/0 cells, HEK293 cells, HEK293T cells, plant cells, or fungi, including yeast cells.

As used herein, a "heterologous antibody" is defined in relation to a transgenic organism producing such an antibody. This term refers to an antibody having an amino acid sequence or an encoding nucleic acid sequence corresponding to that found in an organism not consisting of the transgenic organism, and being generally derived from a species other than the transgenic organism.

As used herein, a "heterohybrid antibody" refers to an antibody having light and heavy chains of different organismal origins. For example, an antibody having a human heavy chain associated with a murine light chain is a heterohybrid antibody.

Thus, "antibodies and antigen-binding fragments thereof" suitable for use in the present invention include, but are not limited to, polyclonal, monoclonal, monovalent, bispecific, heteroconjugate, multispecific, recombinant, heterologous, heterohybrid, chimeric, humanized (in particular CDR-grafted), deimmunized, or human antibodies, Fab fragments, Fab' fragments, $F(ab')_2$ fragments, fragments produced by a Fab expression library, Fd, Fv, disulfide-linked Fvs (dsFv), single chain antibodies (e.g. scFv), diabodies or tetrabodies (Holliger P. et al. (1993) Proc. Natl. Acad. Sci. U.S.A. 90(14), 6444-6448), nanobodies (also known as single domain antibodies), anti-idiotypic (anti-id) antibodies (including, e.g., anti-id antibodies to antibodies of the invention), and epitope-binding fragments of any of the above.

The antibodies described herein are preferably isolated. An "isolated antibody", as used herein, is intended to refer to an antibody that is substantially free of other mAbs having different antigenic specificities (e.g., an isolated antibody that specifically binds hPCSK9 is substantially free of mAbs that specifically bind antigens other than hPCSK9). An isolated antibody that specifically binds hPCSK9 may, however, have cross-reactivity to other antigens, such as PCSK9 molecules from other species.

As used herein, a "PCSK9 antagonist" denotes a compound that inhibits at least one biological activity of PCSK9, preferably the proteinase activity of PCSK9. Preferred PCSK9 antagonists are characterized in that they bind from 10% to 100% (preferably from 50% to 100%) of the PCSK9 present in the blood when used in stoichiometric amounts. Preferred PCSK9 antagonists of the present invention are neutralizing antibodies.

A "neutralizing antibody", as used herein (or an "antibody that neutralizes PCSK9 activity"), is intended to refer to an antibody whose binding to hPCSK9 results in inhibition of at least one biological activity of PCSK9, preferably inhibition of the proteinase activity of PCSK9. This inhibition of the biological activity of PCSK9 can be assessed by measuring one or more indicators of PCSK9 biological activity by one or more of several standard *in vitro* or *in vivo* assays known in the art. Such assays are described for example in US 2010/0166768 A1, the content of which is hereby incorporated by reference in its entirety.

Since PCSK9 increases plasma LDL cholesterol by promoting degradation of the LDL receptor, the activity of PCSK9 has an effect on several diseases associated with increased plasma LDL cholesterol levels. Accordingly, PCSK9 antagonists, such as neutralizing anti-hPCSK9 antibodies or antigen-binding fragments thereof, are useful to reduce elevated total cholesterol, non-HDL cholesterol, LDL cholesterol, and/or apolipoprotein B100 (ApoB100). Consequently, PCSK9 antagonists are useful for ameliorating, improving, inhibiting or preventing several such diseases, including without limitation hypercholesterolemia, hyperlipidemia, dyslipidemia (such as mixed dyslipidemia), atherosclerosis and cardiovascular disease(s).

In the context of present application, the term "a disease or condition in which PCSK9 expression or activity causes an impact" is understood to comprise any disease or condition in which the application or administration of a PCSK-9 inhibitory, activatory, antagonistic, agonistic, neutralizing or blocking molecule, such as an inhibitory, activatory, antagonistic, agonistic, neutralizing or blocking antibody causes an impact and preferably causes an amelioration, improvement, inhibition or prevention of the disease or condition.

Exemplary diseases or conditions that can be ameliorated, improved, inhibited or prevented by administration of an PCSK9 antibody include without limitation hypercholesterolemia, hyperlipidemia, dyslipidemia (such as mixed dyslipidemia), atherosclerosis and cardiovascular disease(s).

In specific embodiments, the anti-PCSK9 antibodies or antigen-binding fragments thereof described herein

may be conjugated to a therapeutic moiety ("immunoconjugate"), such as a cytotoxin, a chemotherapeutic drug, an immunosuppressant or a radioisotope.

A "conservative amino acid substitution" is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent or degree of similarity may be adjusted upwards to correct for the conservative nature of the substitution. Means for making this adjustment are well known to those of skill in the art. See, e.g., Pearson (1994) Methods Mol. Biol. 24: 307-331. Examples of groups of amino acids that have side chains with similar chemical properties include

1) aliphatic side chains: glycine, alanine, valine, leucine and isoleucine;

2) aliphatic- hydroxyl side chains: serine and threonine;

3) amide-containing side chains: asparagine and glutamine;

4) aromatic side chains: phenylalanine, tyrosine, and tryptophan;

5) basic side chains: lysine, arginine, and histidine;

6) acidic side chains: aspartate and glutamate, and

7) sulfur-containing side chains: cysteine and methionine.

Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256: 1443-45. A "moderately conservative" replacement is any change having a nonnegative value in the PAM250 log-likelihood matrix. Given the known genetic code, and recombinant and synthetic DNA techniques, the skilled scientist can readily construct DNAs encoding conservative amino acid variants.

As used herein, "non-conservative substitutions" or "non-conservative amino acid exchanges" are defined as exchanges of an amino acid by another amino acid listed in a different group of the seven standard amino acid groups 1) to 7) shown above.

The term "substantial identity" or "substantially identical," when referring to a nucleic acid or fragment thereof, indicates that, when optimally aligned with appropriate nucleotide insertions or deletions with another nucleic acid (or its complementary strand), there is nucleotide sequence identity in at least about 90%, and more preferably at least about 95%, 96%, 97%, 98% or 99% of the nucleotide bases, as measured by any well-known algorithm of sequence identity, such as FASTA, BLAST or GAP, as discussed below.

As applied to polypeptides, the term "substantial similarity" or "substantially similar" means that two peptide sequences, when optimally aligned, such as by the programs GAP or BESTFIT using default gap weights, share at least 80% sequence identity, and preferably at least 90%, 95%, 98% or 99% or 99.5% sequence identity. Preferably, residue positions which are not identical differ by conservative amino acid substitutions.

Sequence similarity for polypeptides is typically measured using sequence analysis software. Protein analysis software matches similar sequences using measures of similarity assigned to various substitutions, deletions and other modifications, including conservative amino acid substitutions. For instance, GCG software contains programs such as GAP and BESTFIT which can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA with default or recommended parameters; a program in GCG Version 6.1. FASTA (e.g., FASTA2 and FASTA3) provides alignments and percent sequence identity of the regions of the best overlap between the query and search sequences (Pearson (2000) *supra).* Another preferred algorithm when comparing a sequence of the invention to a database containing a large number of sequences from different organisms is the computer program BLAST, especially BLASTP or TBLASTN, using default parameters. See, e.g., Altschul et al. (1990) J. Mol. Biol. 215: 403 410 and (1997) Nucleic Acids Res. 25:3389 402, each of which is herein incorporated by reference.

When percentages of sequence identity are referred to in the present application, these percentages are calculated in relation to the full length of the longer sequence, if not specifically indicated otherwise. This calculation in relation to the full length of the longer sequence applies both to nucleic acid sequences and to polypeptide sequences.

As used herein, "treat", "treating" or "treatment" of a disease or disorder means accomplishing one or more of

the following: (a) reducing the severity and/or duration of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "prevent", "preventing", "prevention", or "prophylaxis" of a disease or disorder means preventing that a disorder occurs in subject.

The terms, "disease", "disorder" and "condition" are used synonymously.

As used herein, the expressions "is for administration" and "is to be administered" have the same meaning as "is prepared to be administered". In other words, the statement that an active compound "is for administration" has to be understood in that said active compound has been formulated and made up into doses so that said active compound is in a state capable of exerting its therapeutic activity.

The terms "therapeutically effective amount" or "therapeutic amount" are intended to mean that amount of a drug or pharmaceutical agent that will elicit the biological or medical response of a tissue, a system, animal or human that is being sought by a researcher, veterinarian, medical doctor or other clinician. The term "prophylactically effective amount" is intended to mean that amount of a pharmaceutical drug that will prevent or reduce the risk of occurrence of the biological or medical event that is sought to be prevented in a tissue, a system, animal or human by a researcher, veterinarian, medical doctor or other clinician. Particularly, the dosage a patient receives can be selected so as to achieve the amount of LDL (low density lipoprotein) cholesterol lowering desired; the dosage a patient receives may also be titrated over time in order to reach a target LDL level. The dosage regimen utilizing an antibody or an antigen-binding fragment thereof as described herein is selected in accordance with a variety of factors including type, species, age, weight, body mass index, sex and medical condition of the patient; the severity of the condition to be treated; the potency of the compound chosen to be administered; the route of administration; the purpose of the administration; and the renal and hepatic function of the patient.

As used herein, a "patient" means any human or non-human animal, such as mammal, reptile or bird who may benefit from a treatment with the antibodies and antigen-biding fragments thereof described herein. Preferably, a "patient" is selected from the group consisting of laboratory animals (e.g. mouse or rat), domestic animals (including e.g. guinea pig, rabbit, chicken, turkey, pig, sheep, goat, camel, cow, horse, donkey, cat, or dog), rodent or primates including chimpanzee, gorilla, bonobo and human beings. It is particularly preferred that the "patient" is a human being.

The terms "subject" or "individual" are used interchangeably herein. As used herein, a "subject" refers to a human or a non-human animal (e.g. a mammal, avian, reptile, fish, amphibian or invertebrate; preferably an individual that can either benefit from one of the different aspects of present invention (e.g. a method of treatment or a drug identified by present methods) or that can be used as laboratory animal for the identification or characterisation of a drug or a method of treatment. The subject can e.g. be a human, a wild-animal, domestic animal or laboratory animal; examples comprise: mammal, e.g. human, non-human primate (chimpanzee, bonobo, gorilla), dog, cat, rodent (e.g. mouse, guinea pig, rat, hamster or rabbit, horse, donkey, cow, sheep, goat, pig, camel; avian, such as duck, dove, turkey, goose or chick; reptile such as: turtle, tortoise, snake, lizard, amphibian such as frog (e.g. Xenopus laevis); fish such as koy or zebrafish; invertebrate such as a worm (e.g. c.elegans) or an insect (such as a fly, e.g. drosophila melanogaster). The term subject also comprises the different morphological developmental stages of avian, fish, reptile or insects, such as egg, pupa, larva or imago. The term "subject" comprises the term "patient". According to a preferred embodiment, the subject is a "patient".

As used herein, "unit dosage form" refers to physically discrete units suitable as unitary dosages for human and/or animal subjects, each unit containing a predetermined quantity of active material (e.g., about 50 to about 500mg of an anti PCSK5 antibody and/or of e.g. 75 to 180 mg of a fibrate such as fenofibrate and/or about 7 to 15 mg of a lipid lowering drug acting by decreasing the cholesterol uptake in the intestine such as ezetimibe) calculated to produce the desired therapeutic effect in association with the required pharmaceutical diluent, carrier or vehicle. The unit dosage form can comprise one or more active ingredients. Conceivable are for example unit dosage forms comprising only the antibody or the fibrate or the lipid lowering drug acting by decreasing cholesterol uptake in the intestine. Conceivable are also for example unit dosage forms comprising the antibody and the lipid lowering drug acting by decreasing cholesterol uptake in the intestine such as ezetimibe or the antibody and the fibrate such as fenofibrate or comprising all three of these active substances (antibody and fibrate and lipid lowering agent acting by decreasing cholesterol uptake in the intestine). One example concerns a unit dosage form comprising one or half an effective dose of the antibody of fragment thereof of present invention (e.g. comprising about 1 ml injection solution comprising one effective dose of about 50, 75, 100, 150, 200 or 300 mg of the antibody or comprising either about 0.5 ml or about 1ml injection solution comprising half an effective dose of 25, 50, 75 or 100 mg of the antibody). One particular example concerns a unit dosage form comprising an anti-PCSK9 antibody or antigen-binding fragment thereof comprising about 150mg of active substance in 1ml injection solution. Another example of a unit dosage form concerns a unit dosage form for oral administration such as a tablet or capsule or overencapsulatet tablet comprising about 160 mg of fenofibrate. Another example of a unit dosage form concerns a unit dosage form for oral administration such as a tablet or capsule or overencapsulated

tablet comprising about 10 mg ezetimibe. Another example of a unit dosage form concerns a unit dosage form for oral administration such as a tablet or capsule comprising an effective dose of fenofibrate (e.g. comprising about 160mg of fenofibrate) and an effective dose of ezetimibe (e.g. about 10mg of ezetimibe). The specifications for the novel unit dosage forms of this invention are dictated by and are directly dependent on (a) the unique characteristics of the active material and the particular therapeutic effect to be achieved, and (b) the limitation inherent in the art of compounding such an active material for therapeutic use in animals or humans, as disclosed in this specification, these being features of the present invention. Examples of suitable unit dosage forms in accord with this invention are vials, tablets, capsules, troches, suppositories, powder packets, wafers, cachets, ampoules, segregated multiples of any of the foregoing, and other forms as herein described or generally known in the art. One or more such unit dosage forms of the antibody can be comprised in an article of manufacture of present invention, optionally further comprising one or more unit dosage forms of a lipid lowering agent acting by decreasing cholesterol uptake in the intestine such as ezetimibe (e.g. a blister of tablets comprising as active ingredient the lipid lowering agent acting by decreasing cholesterol uptake in the intestine such as ezetimibe) and optionally further comprising one or more unit dosage forms of a fibrate such as fenofibrate (e.g. a blister of tablets comprising as active ingredient a fibrate such as fenofibrate). One or more such unit dosage forms of the antibody can also be comprised in an article of manufacture of present invention further comprising one or more unit dosage forms, wherein the further comprised unit dosage forms comprise two active ingredients such as fenofibrate and ezetimibe.

The term "active material" refers to any material with therapeutic activity, such as one or more active ingredients. The active ingredients to be employed as therapeutic agents can be easily prepared in such unit dosage form with the employment of pharmaceutical materials which themselves are available in the art and can be prepared by established procedures.

The following preparations are illustrative of the preparation of the unit dosage forms of the present invention, and not as a limitation thereof. Several dosage forms may be prepared embodying the present invention. For example, a unit dosage per vial may contain 0,5 ml, 1 ml, 2 ml, 3 ml, 4 ml, 5 ml, 6 ml, 7 ml, 8 ml, 9 ml, 10 ml, 15 ml, or 20 ml of PCSK5 antibody or a fragment thereof ranging from about 40 to about 500 mg of PCSK5 antibody. If necessary, these preparations can be adjusted to a desired concentration by adding a sterile diluent to each vial. In one embodiment, the ingredients of formulation of the invention are supplied either separately or mixed together in unit dosage form, for example, as a dry lyophilized powder or water free concentrate in a hermetically sealed container such as a vial, an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The formulations as herein described include bulk drug compositions useful in the manufacture of pharmaceutical compositions (e.g., compositions that are suitable for administration to a subject or patient) which can be used in the preparation of unit dosage forms. In a preferred embodiment, a composition of the invention is a pharmaceutical composition. Such compositions comprise a prophylactically or therapeutically effective amount of one or more prophylactic or therapeutic agents (e.g., an anti PCSK9 antibody or antigen-binding fragment thereof, a fibrate, a lipid lowering agent acting by decreasing cholesterol uptake in the intestine or another prophylactic or therapeutic agent), and a pharmaceutically acceptable carrier. Preferably, the pharmaceutical compositions are formulated to be suitable for the route of administration to a subject such as a patient.

The active materials, agents or ingredients (e.g. antibodies or antigen-binding fragments thereof, fibrates and lipid lowering agents acting by decreasing cholesterol uptake into the intestine or other lipid lowering agents) can be formulated as various dosage forms including solid dosage forms for oral administration such as capsules, tablets (e.g. over-encapsulated tablets), pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

In a specific embodiment, the term "pharmaceutically acceptable" means approved by a regulatory agency of the U.S. Federal or a state government or by the EMA (European Medicines Agency) or listed in the U.S. Pharmacopeia Pharmacopeia (United States Pharmacopeia-33/National Formulary-28 Reissue, published by the United States Pharmacopeia Convention, Inc., Rockville Md., publication date: April 2010) or other generally recognized pharmacopeia for use in animals, and more particularly in humans. The term "carrier" refers to a diluent, adjuvant {e.g., Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. For the use of (further) excipients and their use see also "Handbook of Pharmaceutical Excipients", fifth edition, R.C. Rowe, P.J. Seskey and S.C. Owen, Pharmaceutical Press, London, Chicago. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a prophylactically or therapeutically effective amount of the antibody, preferably in purified form, together with a suitable amount of carrier so as to provide the form for proper administration to the patient. The formulation should suit the mode of administration.

Generally, the ingredients of compositions of the invention are supplied either separately or mixed together in a unit dosage form, for example, as a dry formulation for dissolution such as a lyophilized powder, freeze-dried powder or water free concentrate in a hermetically sealed container, such as an ampoule or sachette indicating the quantity of active agent. The ingredients of compositions of the invention can also be supplied as admixed liquid formulation (i.e. injection or infusion solution) in a hermetically sealed container such as an ampoule, sachette, a pre-filled syringe or autoinjector, or a cartridge for a reusable syringe or applicator (e.g. pen or autoinjector). Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The invention also provides that the formulation is packaged in a hermetically sealed container such as an ampoule or sachette indicating the quantity of antibody. In one embodiment, the formulation of the invention comprising an antibody is supplied as a dry formulation, such as a sterilized lyophilized powder, freeze-dried powder, spray - dried powder or water free concentrate in a hermetically sealed container and can be reconstituted, e.g., with water or saline to the appropriate concentration for administration to a subject. In another embodiment the antibody or antigen-binding fragment thereof is supplied as a liquid formulation such as an injection or infusion solution. In one embodiment, the formulation of the invention or used in the context of present invention comprising an antibody is supplied as a dry formulation or as a liquid formulation in a hermetically sealed container at a unit dosage of at least 40 mg, at least 50 mg, more preferably at least 75 mg, at least 100 mg, at least 150 mg, at least 200 mg, at least 250 mg, at least 300 mg, at least 350 mg, at least 400 mg, at least 450 mg, or at least 500 mg, of antibody or antigen-binding fragment thereof. The lyophilized formulation of the invention comprising an antibody should be stored at between 2 and 8° C in its original container and the antibody should be administered within 12 hours, preferably within 6 hours, within 5 hours, within 3 hours, within 2 hours or within 1 hour after being reconstituted. The formulation of the invention comprising antibodies can for example be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Adult subjects are characterized as having "hypertension" or a high blood pressure when they have a systolic blood pressure of more than 140 mmHg and/or a diastolic blood pressure of more than 90 mmHg.

Specific populations treatable by the therapeutic methods of the invention include subjects with one or more of the following conditions: subjects indicated for LDL apheresis, subjects with PCSK9-activating mutations (gain of function mutations, "GOF"), subjects with elevated total cholesterol levels, subjects with elevated low-density lipoprotein cholesterol (LDL-C) levels, subjects with primary hypercholesterolemia, such as subjects primary with Familial or Non-Familial Hypercholesterolemia, subjects with heterozygous Familial Hypercholesterolemia (heFH); subjects with hypercholesterolemia, especially primary hypercholesterolemia, who are statin intolerant or statin uncontrolled; and subjects at risk for developing hypercholesterolemia who may be preventably treated.

Other indications include hyperlipidemia and dyslipidemia (such as mixed dyslipidemia), especially if associated with secondary causes such as Type 2 diabetes mellitus, cholestatic liver diseases (primary biliary cirrhosis), nephrotic syndrome, hypothyroidism, obesity; and the prevention and treatment of atherosclerosis and cardiovascular disease(s), such as coronary heart disease (CHD). The conditions or disorders as listed for the above populations or subjects are conditions or disorders, for which treatment with the combination therapy of the invention is especially suitable.

However, depending on the severity of the afore-mentioned diseases and conditions, the treatment of subjects with the antibodies and antigen-binding fragments of the invention may be contraindicated for certain diseases and conditions.

The term "adverse effect" (or side-effect) refers to a harmful and undesired effect resulting from a medication. An adverse effect may be termed a "side effect", when judged to be secondary to a main or therapeutic effect. Some adverse effects occur only when starting, increasing or discontinuing a treatment. Adverse effects may cause medical complications of a disease and negatively affect its prognosis. Examples of side effects are allergic reactions, vomiting,

headache, or dizziness or any other effect herein described.

As used herein, "treat", "treating" or "treatment" of a disease or disorder means accomplishing one or more of the following: (a) reducing the severity and/or duration of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "prevent", "preventing", "prevention", or "prophylaxis" of a disease, condition or disorder means preventing that a disorder, disease or condition occurs in subject.

Elevated total cholesterol levels are understood in the context of present invention to preferably be total cholesterol levels of 200 mg/dL or more, especially 240mg/dL or more. International treatment guidelines recommend lowering LDL-C to <2.0-2.6 mmol/L (<77-100 mg/dL) in patients with established cardiovascular diseases (CVDs) and to <1.8-2.0 mmol/L (<70-77 mg/dL) in high-risk groups such as those with CVDs plus diabetes, smoking, poorly controlled hypertension, metabolic syndrome, or previous myocardial infarction. Elevated LDL-C levels are thus understood in the context of present invention to be LDL-C levels of 77 mg/dL or more (especially for patients with one or more of the following characteristics: established CVDs and one or more of [diabetes, with smoking, poorly controlled hypertension, metabolic syndrome or previous myocardial infarction]) and 100 mg/dL or more (especially for patients with established CVDs) ,130mg/dL or more , or 160 mg/dL or 190mg/dL or more. Low High-density lipoprotein levels (HDL-levels) in the context of present invention are understood to be preferably less than about 40mg/dL.

The terms "uncontrolled by lipid lowering agent(s)" or "not or not adequately controlled by lipid lowering or modifying agents" or "not or not adequately controlled by therapy or treatment with lipid lowering or modifying agents" etc. especially in context of hyperlipidemia, hypercholesterolemia etc., are used synonymously herein and refer to conditions such as hyperlipidemia, wherein treatment with a lipid modifying, e.g. a statin (i.e. regular administration of a statin such as atorvastatin to a patient) does not significantly lower total cholesterol or LDL-C or does not suffice to establish normolipidemic levels for the patient or to establish a lipidemic (e.g. total cholesterol or LDL-C) level that is not a significant risk factor for developing cardiovascular diseases. This means for example that therapy with the lipid modifying or lipid lowering agent does not suffice to establish levels of less than 130 mg/dL in general, or of less than 100 mg/dL (e.g. about >77mg/dL to about 100 mg/dL), especially in patients with established cardiovascular diseases, or to establish levels of about less than 77 mg/dL (e.g. about >70-77 mg/dL), especially in high-risk groups such as those with CVDs plus diabetes, smoking, poorly controlled hypertension, metabolic syndrome, or previous myocardial infarction.

The terms "lipid modifying therapy" or "lipid lowering therapy" as used herein refer to the treatment or therapy with a lipid modifying or a lipid lowering agent, and particularly comprise cholesterol lowering therapies.

The terms "uncontrolled by statins" or "statin-resistant" or "not adequately controlled by statins or by statin therapy" especially in the context of hyperlipidemia, hypercholesterolemia etc., are used synonymously herein. Theyefer to conditions with elevated lipid levels such as hyperlipidemia, wherein treatment with a statin (i.e. regular administration of a statin such as atorvastatin to a patient)...

- does not sufficiently lower total cholesterol or LDL-C levels or
- does not suffice to establish the lipid target
- does not suffice to establish normolipidemic levels for the patient
- does not suffice to establish a lipidemic (e.g. total cholesterol or LDL-C) level that is not a significant risk factor for developing cardiovascular diseases.

This means for example that statin-treatment does not suffice to establish levels of less than 130 mg/dL in general, or of less than 100 mg/dL (e.g. about >77mg/dL to about 100 mg/dL), especially in patients with established cardiovascular diseases, or to establish levels of about less than 77 mg/dL (e.g. about >70-77 mg/dL), especially in high-risk groups such as those with CVDs plus diabetes, smoking, poorly controlled hypertension, metabolic syndrome, or previous myocardial infarction. In the context of present invention, statin resistance relates to resistance concerning the treatment with any statin, such as with any of the statins as listed herein.

The term "statin intolerance" as used herein refers to the inability to tolerate at least two different statins at the lowest approved dose and dose regimen (i.e. for example being intolerant to treatment with atorvastatin and being intolerant to treatment with rosuvastatin and possibly also being intolerant to treatment with one or more other statins).

The terms "Liver function" or "hepatic impairment", as used herein and especially if used in the context of mildly or moderately impaired liver function or mild or moderate hepatic impairment: As used herein, the liver function or hepatic impairment is preferably determined according to the Child-Pugh score. The Child-Pugh score (sometimes the Child-Turcotte-Pugh score) is used to assess the prognosis of chronic liver disease, mainly cirrhosis. Although it was originally

used to predict mortality during surgery, it is now frequently used to determine the prognosis, as well as the required strength of treatment and the necessity of liver transplantation. Scoring: The score employs five clinical measures of liver disease. Each measure is scored 1-3, with 3 indicating most severe derangement.

| Measure | 1 point | 2 points | 3 points |
|---|---|---|---|
| Total bilirubin, μmol/l (mg/dl) | <34 (</=2) | 34-50 (2-3) | >50 (>3) |
| Serum albumin, g/l | >35 | 28-35 | <28 |
| PT INR | <1.7 | 1.71-2.30 | > 2.30 |
| Ascites | None | Mild | Moderate to Severe |
| Hepatic encephalopathy | None | Grade I-II (or suppressed with medication) | Grade III-IV (or refractory) |

Different textbooks and publications use different measures. Some older reference works substitute PT prolongation for INR. In primary sclerosing cholangitis (PSC) and primary biliary cirrhosis (PBC), the bilirubin references are changed to reflect the fact that these diseases feature high conjugated bilirubin levels. The upper limit for 1 point is 68 μmol/l (4 mg/dl) and the upper limit for 2 points is 170 μmol/l (10 mg/dl). Interpretation: Chronic liver disease is classified into Child-Pugh class A to C, employing the added score from above.

| Points | Class | One year survival | Two year survival |
|---|---|---|---|
| 5-6 | A | 100% | 85% |
| 7-9 | B | 81% | 57% |
| 10-15 | C | 45% | 35% |

In the context of present invention, the term mild hepatic impairment preferably refers to Child-Pugh Class A, the term moderate hepatic impairment preferably refers to Child-Pugh Class B and the term severe hepatic impairment preferably refers to Child-Pugh Class C. A liver status of 0-4 points in the Child-Pugh Scoring system corresponds to a healthy hepatic liver function or a healthy liver status. There are also other classes of scoring system for determining the severity of impairment of liver function. The skilled artisan knows to which classes of another scoring system a certain Child-Pugh Class corresponds.

In the context of present invention, the term "lipid modifying agent" refers to any agent that is capable of modifying the lipid level in the body, particularly in the plasma and/or liver, lipid modifying agents encompass lipid lowering agents.

The term "lipid lowering agent" or "lipid modifying agent" particularly refers to any agent that is capable of lowering the total cholesterol and/or LDL-C level in the body (particularly in the plasma and/or liver) and/or of increasing HDL-C levels in the body (particularly in the plasma and/or liver) and/or of lowering the triglyceride levels in the body (particularly in the plasma and/or liver).

A lipid lowering or modifying agent can be any synthetic, proteinaceous, nucleic acid or other agent, composition or isolated natural product. The term "lipid lowering agent" is used synonymously with the term "hypolipidemic agent", or "antihyperlipidemic agent" or "lipid-lowering drug (LLD)" and refers to a diverse group of agents or compositions that can be used in the treatment of one or more hyperlipidemia.

Several classes of LLDs are known in the art. The known LLDs may differ in both their impact on the cholesterol profile and adverse effects, while some may have a stronger LDL-lowering effect, others may more strongly increase HDL-C levels. The individual choice of an agent to be prescribed will depend on the patient's cholesterol profile, cardiovascular risk, and the liver and kidney functions of the patient, evaluated against the balancing of risks and benefits of the medications, that lies within the skill of the physician.

The term lipid lowering agent or LLD includes, but is not limited to any agent that

(1) induces a cellular depletion of cholesterol synthesis by inhibiting 3-hydroxy-3-methylglutaryl (HMG)-coenzyme A (CoA) reductase, such as cerivastatin, atorvastatin, simvastatin, pitavastatin, rosuvastatin, fluvastatin, lovastatin, pravastatin;
(2) inhibits cholesterol uptake in the intestine and or bile acid re-absorption, e.g. ezetimibe or phytosterols;
(3) increases lipoprotein catabolism (such as niacin and activators of the LXR transcription factor that plays a role in cholesterol elimination such as 22-hydroxycholesterol)

(4) other lipid lowering agents such as omega-3-fatty acid ethyl esters (for example, omacor, Orlistat), fibrates or activators of peroxisome proliferator activated receptor alpha (PPARalpha), such as fenofibrate), or

(5) combinations of any of 1-4 such as ezetimibe plus a HMG-CoA inhibitor, ezetimibe plus a statin, ezetimibe plus a PPARalpha activator such as fenofibrate, ezetimibe plus a fibrate such as fenofibrate.

Particular lipid lowering agents are lipid lowering agents decreasing cholesterol uptake or absorption into the intestine, such as azetidines or more particularly ezetimibe. Ezetimibe is an inhibitor of intestinal cholesterol (and related phytosterol) absorption and belongs to the class azetidine compounds. It is indicated as an adjunct to diet mainly to reduce LDL-C in various clinical situations. After oral administration, ezetimibe is absorbed and extensively conjugated to a pharmacologically active phenolic glucuronide (ezetimibe-glucuronide). Both ezetimibe and ezetimibe-glucuronide are eliminated from plasma with a half-life of approximately 22 hours. As used herein, the term ezetimibe comprises unmodified ezetimibe as well as derivatives of ezetimibe such as conjugated ezetimibe, e.g. ezetimibe-glucoronide.

Particular other lipid lowering agents are PPARalpha activators or fibrates such as bezafibrate, ciprofibrate, clofibrate, gemfibrozil or fenofibrate and more particularly fenofibrate. Fenofibrate is a peroxisome proliferator receptor alpha (PPAR$\alpha$) activator and belongs to the class of fibrates. It is indicated as an adjunct to diet mainly to reduce LDL-C and triglycerides in various clinical situations. Fenofibrate is a pro-drug of the active moiety fenofibric acid. Fenofibric acid is eliminated with a half-life of 20 hours. As used herein, the term "fenofibrate" comprises the prodrug fenofibrate as well as the active moiety fenofibric acid.

The term "cardiovascular disease" as used herein refers to any disease that affects the cardiovascular system, principally cardiac disease, heart disease, vascular diseases of the brain and kidney, and peripheral arterial disease and also comprises coronary heart disease. As used herein, the term particularly includes: coronary heart disease (a disease of the blood vessels supplying the heart muscle), cerebrovascular disease (a disease of the blood vessels supplying the brain), peripheral arterial disease (a disease of blood vessels supplying the arms and legs), rheumatic heart disease (a damage to the heart muscle and heart valves from rheumatic fever, caused by streptococcal bacteria), congenital heart disease (malformations of heart structure existing at birth), deep vein thrombosis (blood clots in the leg veins, which can dislodge and move to the heart and lungs), pulmonary embolism (disloged blood clogs move to the heart lungs and obstruct vessels), heart attack and stroke (both mainly caused by a blockage that prevents blood from flowing to the heart or brain. The most common reason is a buildup of fatty deposits (atherosclerotic plaques) on the inner walls of the blood vessels). Strokes can also be caused by bleeding from a blood vessel in the brain or by blood clots.

The terms "cardiovascular disease" and "cardiovascular diseases" are used interchangeably herein.

The term "coronary heart disease" (CHD) as used herein is known in the art and particularly refers to the narrowing or blockage of the coronary arteries, usually caused by atherosclerosis, and can result in a heart attack. Atherosclerosis occurs when cholesterol and other fatty substances accumulate on the inner wall of the arteries. They attract fibrous tissue, blood components, and calcium, and harden into cholesterol-containing artery-clogging plaques. Atherosclerotic plaques often form blood clots that also can block the coronary arteries (coronary thrombosis). Congenital defects and muscle spasms can also block blood flow. Recent research indicates that infection from organisms such as chlamydia bacteria may be responsible for some cases of coronary artery disease. CHD particularly refers to but is not limited to coronary artery disease (CAD), particularly atherosclerotic coronary artery disease.

In the context of present invention, the term "high cardiovascular risk" refers to a high risk of suffering from or developing a cardiovascular disease. Thus, a patient or subject having a high cardiovascular risk or being classified as subject or patient with high cardiovascular risk has a high (e.g. statistically significantly increased risk when compared with other patients or subjects) of suffering from or developing a cardiovascular disease.

A number of major contributing factors increase the risk of developing cardiovascular disease and particularly coronary heart and more particularly coronary artery disease. Subjects having more of these risk factors are more likely to develop cardiovascular disease, particularly CHD and more particularly coronary artery disease (CAD). Major CHD/CAD risk factors are: Heredity, Gender (men have a higher risk up to 60 years of age, then the risk is equal), age (men of 45 years or older, women of 55 years or older have a higher risk), smoking, elevated cholesterol levels (i.e. levels of equal to or more than 240mg/dL total Cholesterol or equal or more than 130-159 mg/dL LDL-Cholesterol, the risk of developing coronary artery disease increases steadily as blood cholesterol levels increase above 160 mg/dL. When a person has other risk factors, the risk multiplies), high blood pressure (It increases the risk of heart attack, stroke, kidney failure, and congestive heart failure. A blood pressure of 140 over 90 or above is considered high. As the numbers rise, high blood pressure goes from Stage 1 (mild) to Stage 4 (very severe). In combination with obesity, smoking, high cholesterol, or diabetes, high blood pressure raises the risk of heart attack or stroke several times.), lack of physical exercise, Type II diabetes (The risk of developing coronary artery disease is seriously increased for diabetics. More than 80% of diabetics die of some type of heart or blood vessel disease.), hormone replacement therapy, obesity, excessive stress.

The term "CHD-risk equivalent" (also known as CHD equivalent) as used herein is known in the art. Subjects who do not have established CHD, but do have a risk for developing major coronary events (myocardial infarction (MI)

and coronary death) equal to someone with established CHD (>20% per 10 years according to Framingham risk scoring) or equal to someone who already suffered from a major coronary event, are said to have a CHD risk equivalent. These subjects belong in the highest risk category for primary prevention and have the lowest LDL-C goal (<100 mg/dL). Thus, as used herein, the term "CHD-risk equivalent" refers to a risk of developing a major coronary event that is equal to a subject having established CHD or that is equal to a subject having suffered from a major coronary event and particularly refers to a risk of developing a major coronary event that is >20% per 10 years according to Framingham risk scoring.

CHD risk equivalents include

- clinical forms of noncoronary atherosclerosis such as peripheral arterial disease (PAD), carotid artery disease (transient ischemic attack or stroke of carotid origin, or >50% stenosis on angiography or ultrasound), and abdominal aortic aneurysm (AAA)

- type 1 and type 2 diabetes; however, the differences between the two necessitate different considerations and approaches to treatment

    - In persons with type 1 diabetes, the intensity of LDL-C-lowering therapy depends on clinical judgment. Recent-onset type 1 diabetes need not be considered a CHD risk equivalent; therefore, reduction of LDL-C to <130 mg/dL is sufficient. With increasing duration of type 1 diabetes, however, a lower goal of <100 mg/dL should be considered. Nonetheless, ATP III favours starting drug therapy along with lifestyle therapies when LDL-C levels are >130 mg/dL.
    - For persons with type 2 diabetes, treatment for LDL-C lowering should follow ATP III recommendations for those with established CHD. Clinical judgment should be used as to the intensity of therapy for younger persons with type 2 diabetes, who otherwise are at lower risk; however, consideration should be given to using LDL-C-lowering drugs when LDL-C levels are >130 mg/dL.

- presence of one or more risk factors according to ATP III Framingham risk scoring (high CHD risk occurs at presence of multiple risk factors according to ATP III Framingham risk scoring)

- abdominal aortic aneurysm

- peripheral vascular disease

- carotid artery disease

- a 10-year risk of having heart disease greater than 20% according to the Framingham Cardiac Risk Calculator

- elevated cholesterol levels

- any condition, factor or event leading to a 10-year risk of having heart disease greater than 20% according to the Framingham Cardiac Risk Calculator or ATPIII Framingham risk scoring.

ASPECTS OF THE INVENTION

The present invention will now be further described. In the following passages different aspects of the invention are defined in more detail and preferred embodiments of the invention will be described. Each aspect and embodiment so defined may be combined with any other aspect or aspects or embodiment or embodiments unless clearly indicated to the contrary. In particular, any feature indicated as being preferred or advantageous may be combined with any other feature or features indicated as being preferred or advantageous, unless clearly indicated to the contrary.

All methods of treatment as herein described, especially the methods according to the first, second and third, aspect should also be understood to relate to the PCSK9 antibody of the present invention according to the fourth aspect (or a formulation, pharmaceutical composition or unit dosage form comprising the antibody), , i.e. for use in a method according to the first, the second and the third aspect and for use in any combination of said methods. In addition, all methods of treatment as herein described, especially the methods according to the first, second and third aspect should also be understood to relate to the use of the PCSK9 antibody of the present invention or a formulation, pharmaceutical composition or unit dosage form comprising the antibody in the manufacture of a medicament for treating a disorder

according to one of the aspects 1, 2 and 3, i.e. for treating a disorder according to the first, the second or the third aspect and any combination of said methods.

In a first aspect, present invention concerns a method for treating a disease or condition in which PCSK9 expression or activity causes an impact comprising administering a therapeutic amount of an antibody or antigen-binding fragment thereof which specifically binds hPCSK9 (human proprotein convertase subtilisin/kexin type 9) to a subject in need thereof, wherein the method comprises administering of the antibody or antigen-binding fragment thereof together with

a) a therapeutic amount of a lipid lowering agent acting by decreasing cholesterol adsorption in the intestine, or

b) a therapeutic amount of a fibrate, or

c) a therapeutic amount of the lipid lowering agent of a) and a therapeutic amount of a fibrate.

In a second aspect, present invention concerns a method of lowering cholesterol levels in a subject in need thereof, comprising:

(a) selecting a subject with a blood low density lipoprotein cholesterol (LDL-C) level > 100 mg/dL; and
(b) administering to said subject a composition comprising an antibody or antigen-binding fragment thereof or antigen binding fragment thereof that specifically binds to human proprotein convertase subtilisin/kexin type 9 (hPCSK9); thereby lowering cholesterol levels in the subject in need thereof, wherein the method comprises administering of the antibody or antigen- binding fragment thereof or fragment thereof together with

a) a therapeutic amount of a lipid lowering agent acting by decreasing cholesterol adsorption in the intestine, or

b) a therapeutic amount of a fibrate, or

c) a therapeutic amount of the lipid lowering agent of a) and a therapeutic amount of a fibrate.

In a third aspect, present invention concerns a method of preventing effects of a (persistently) increased LDL-C level in the blood comprising administering a therapeutic amount of an antibody or antigen-binding fragment thereof which specifically binds hPCSK9 (human proprotein convertase subtilisin/kexin type 9) to a subject having an increased LDL-C level in the blood, and

a. administering a therapeutic amount of a lipid lowering agent acting by decreasing cholesterol adsorption in the intestine, or

b. administering a therapeutic amount of a fibrate, or

c. administering a therapeutic amount of the lipid lowering agent of a) and a therapeutic amount of a fibrate.

According to one embodiment of the first, second and third aspect, the antibody or antigen-binding fragment thereof is administered together with the lipid lowering agent.

According to another embodiment of the first, second and third aspect, the antibody or antigen-binding fragment thereof is administered together with the fibrate.

According to another embodiment of the first, second and third aspect, the antibody or antigen-binding fragment thereof is administered together with the lipid lowering agent and the fibrate.

The fibrate to be used in the context of all aspects (i.e. aspects 1-10) of present invention can be any fibrate having cholesterol lowering effect. Examples comprise fenofibrate, bezafibrate, ciprofibrate, clofibrate and gemfibrozil. According to one embodiment of the different aspects of present invention, the fibrate is an activator of PPARalpha. According to one particular embodiment of the different aspects of present invention, the fibrate is fenofibrate.

The lipid lowering agent acting by decreasing cholesterol adsorption in the intestine can be any lipid lowering agent acting by decreasing cholesterol adsorption or uptake in the intestine (possibly in addition to other lipid lowering effects) and preferably is a azetidine such as ezetimibe. According to one embodiment of the different aspects of present invention, the lipid lowering agent is ezetimibe.

Antibodies specifically binding human PCSK9 are known in the art, see e.g. WO 2008/057457, WO 2008/057458, WO 2008/057459, WO 2008/063382, WO 2008/125623, and US 2008/0008697 and particularly US 2010/0166768 A1. The skilled artisan knows how to express and purify such antibodies. Thus, in the context of the

different aspects (1,2,3,4,5,6,7,8,9,10) of present invention, any antibody specifically binding human PCSK9 can be used as long as it has the desired effect for practicing present invention. Selecting the right antibody for practicing the different aspects of present invention among the known and readily available ones lies within the skill of the artisan.

Particularly suitable antibodies and antigen-binding fragments for practicing all aspects of present invention can be gained from the section "preferred antibodies for practicing present invention" herein.

According to one embodiment of the first, second, third aspect (and all other aspects) of present invention, the antibody or antigen-binding fragment thereof is a recombinant human antibody.

According to another embodiment of the first, second, third aspect (and all other aspects) of present invention, the antibody or antigen-binding fragment thereof or the antigen-binding fragment thereof is characterized by one or more of the following:

(i) being capable of reducing serum total cholesterol at least about 25 to about 35% and sustaining the reduction over at least a 24 day period relative to a predose level;

(ii) being capable of reducing serum LDL cholesterol at least about 65-80% and sustaining the reduction over at least a 24 day period relative to a predose level;

(iii) being capable of reducing serum triglyceride at least about 25-40% relative to predose level;

(iv) achieving one or more of (i)-(iii) without reducing serum HDL cholesterol or reducing serum HDL cholesterol no more than 5% relative to predose level;

(v) achieving one or more of (i)-(iii) with little or no measurable effect on liver function, as determined by ALT (Alanine Aminotransferase) and AST (Aspartate Aminotransferase) measurements.

According to another embodiment of the first, second, third aspect (and all other aspects) of present invention, the antibody or antigen-binding fragment thereof or the antigen-binding fragment thereof comprises

a) a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; or

b) a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736, or

c) the heavy chain of a) and the light chain of b).

According to another embodiment of the first, second, third aspect (and all other aspects) of present invention, the antibody or antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

According to another embodiment of the first, second, third aspect (and all other aspects) of present invention, the antibody or antigen-binding fragment thereof binds to the same epitope on hPCSK9 as an antibody or antigen-binding fragment thereof comprising heavy and light chain CDR amino acid sequences as shown in SEQ ID NOs: 76, 78, 80, 84, 86, and 88.

According to another embodiment of the first, second, third aspect (and all other aspects) of present invention, the antibody or antigen-binding fragment thereof competes for binding to hPCSK9 with an antibody or antigen-binding fragment thereof comprising heavy and light chain CDR amino acid sequences as shown in SEQ ID NOs: 76, 78, 80, 84, 86, and 88.

According to other embodiments of the first, the second, the third and all other aspects of present invention, the antibody or antigen-binding fragment thereof is antibody 316P or an antigen-binding fragment thereof or is antibody 300N or an antigen-binding fragment thereof.

The administered lipid lowering agent can e.g. decrease or increase PCSK9 expression or can have no significant effect on PCSK9 expression.

The administered fibrate can e.g. decrease or increase PCSK9 expression or can have no significant effect on

PCSK9 expression.

According to another embodiment of the first, second and third aspect, the administration of the antibody or antigen-binding fragment thereof together with the lipid lowering agent and/or the fibrate is an adjunctive therapy to diet and preferably to lipid-lowering or cholesterol-lowering diet.

The combination treatment according to the different aspects of present invention comprises the administration of effective dosages of the antibody together with the fibrate or the lipid lowering drug or both. The effective dosage for a given drug and a given patient depends on various factors and the skilled person can readily determine the individually best dosage for each component of the combination treatment as herein described.

According to one embodiment of the first, second, third aspect, the method comprises administration of the antibody or antigen-binding fragment thereof in a dosage amount ranging from 5 mg to 500 mg and preferably ranging from 50 to 300 mg and more preferably of about 150 mg.

According to one embodiment, the fibrate and/or the lipid lowering drug acting by decreasing cholesterol uptake in the intestine are administered once a day.

The combination treatment comprising administration of the antibody or fragment thereof together with the fibrate (particularly a fenofibrate) and/or together with a lipid lowering drug acting by decreasing the cholesterol uptake in the intestine (particularly ezetimibe) can be performed according to any suitable administration and dosing regime (particularly as described herein). According to some embodiments of the first, the second or the third aspect, all three components are administered as separate unit dosage forms (e.g. the antibody by injection of a solution, the fibrate by oral administration of a suitable unit dosage form (e.g. a tablet) and the lipid lowering drug also by oral administration of a separate suitable unit dosage form (e.g. a tablet). According to other embodiments of the first, the second or the third aspect, all three components are administered together as one unit dosage form (e.g. all as solution for injection). According to other embodiments of the first, the second or the third aspect, the antibody or fragment thereof is administered as a unit dosage form and the other two components are administered together as one unit dosage form comprising both other components (e.g. a unit dosage form suitable for oral administration comprising the fibrate such as fenofibrate and the lipid lowering drug such as ezetimibe, particularly a tablet or capsule comprising a fenofibrate and ezetimibe).

Effective dosages (e.g. daily, weekly, biweekly, four-weekly or monthly dosages) depend on various factors and the skilled person can readiliy determine the best dosage.

Conceivable daily dosages of the lipid lowering agent in the context of the first, second or third aspect of present invention e.g. about1, 3, 5, 7, 10, 11, 12, 15, 20, 25, 30, 40 or 50 mg and preferably of 5 to 20 mg and preferably of an azetidone and more preferably ezetimibe. According to one particular embodiment of the first, the second or the third aspect, the method comprises administering a daily dose of about 10 mg ezetimibe.

A conceivable daily dose of fibrate in the context of the first, second or third aspect of present invention is e.g. about 50, 75, 100, 150, 160, 170, 180, 190 or 200 mg and preferably of 75 to 180 mg of the fibrate and preferably of fenofibrate. According to one particular embodiment of the first, the second or the third aspect, the method comprises administering a daily dose of about 160mg fenofibrate.

According to another embodiment of the first, second or third aspect, the method comprises administration of the antibody or antigen-binding fragment thereof in a dosage amount ranging from 50 mg to 300 mg and preferably of about 150 mg and comprising administration of the lipid lowering agent and preferably ezetimibe in a daily dose of 5 to 20 and preferably of about 10 mg.

According to another embodiment of the first, second or third aspect, the method comprises administration of the antibody or antigen-binding fragment thereof in a dosage amount ranging from 50 mg to 300 and preferably of about 150 mg and comprises administration of the fibrate and preferably of fenofibrate in a daily dose of 75 to 180 mg and preferably of about 160 mg.

According to another embodiment of the first, second or third aspect, the method comprises administration of the antibody or antigen-binding fragment thereof in a dosage amount ranging from 50 mg to 300 mg and preferably of about 150 mg and comprises administration of fenofibrate in a daily dose of about between 75 to 180 mg and preferably of about 160 mg and comprises administration of ezetimibe in a daily dose of between 5 to 20 mg and preferably of about 10 mg.

According to another embodiment of the first, second, third aspect, the lipid lowering agent, the fibrate or both are administered three times per day, twice per day, or once per day and preferably once per day. One embodiment of the first, second or third aspect comprises administering the fibrate once per day. One embodiment of the first, second or third aspect comprises administering the lipid lowering agent acting by decreasing cholesterol uptake once per day.

According to another embodiment of the first, second or third aspect, the lipid lowering agent, the fibrate or both are administered every day, every other day, every third day, every fourth day, every fifth day, or every sixth day and preferably every day. One embodiment of the first, second or third aspect comprises administering the fibrate once per day every day. One embodiment of the first, second or third aspect comprises administering the lipid lowering agent acting by decreasing cholesterol uptake once per day every day.

The lipid lowering agent and/or the fibrate can be administered at some particular point during the day- or night

time or at varying time points during day- or night time. According to one embodiment of the first, second, third aspect, the lipid lowering agent, the fibrate or both are administered at about the same time point during the day- or night-time, e.g. in the morning, at noon or in the evening and preferably in the evening.

The lipid lowering agent and/or the fibrate can be administered in connection with meal-time or irrespective to meals. According to one embodiment of the first, second or third aspect, the lipid lowering agent, the fibrate or both are administered before, during or after a meal (e.g. about 30 min before beginning of the meal (at maximum) or during the meal or until about 30 min (at maximum) after end of the meal).

According to someembodiments of the first, second or third aspect, the antibody or antigen-binding fragment thereof is administered every week, every other week, every third week, every month or every fourth week and preferably every fourth week (i.e. once per four weeks or 28 days) or every month (i.e. once per calendar-month that can comprise e.g. 28, 29, 30 or 31 days).

Administration every fourth week or administration once a month (i.e. once per calendar month, e.g. every first, second etc. day of the month or every first, second or third Monday, Tuesday etc. every month, in contrast to administration every fourth week) is preferred in view of patient compliance. Administration every other week is preferred in view of a very low variation of blood cholesterol levels. Other suitable time schedules for administration of the antibody or antigen-binding fragment thereof include without limitation an administration once per day, every other day, every third day, every fourth day, every fifth day, every sixth day, every week, every third week, every fifth week, every sixth week, every eighth week, every tenth week, and every twelfth week.

According to one preferred embodiment, the antibody is administered every fourth week or every month.

The combination treatment of present invention comprising e.g. the co-administration of an anti-PCSK9 antibody with a fibrate such as fenofibrate or with a lipid lowering drug such as ezetimibe advantageously needs relatively low amounts of PCSK9 antibody, so that already an administration of relatively low dosage amounts of antibody of e.g. about 150mg or 200 mg per 4-weeks or per month suffice for achieving significant effects.

Thus, according to some other embodiments of the first, second or third aspect, the antibody or antigen-binding fragment thereof has one or more of the following characteristics:

(i) reduces low-density lipoprotein (LDL-C) levels of at least about -20% relative to a predose level with a sustained reduction over at least a 14 day-period, wherein the sustained reduction is preferably at least -25% and more preferably at least -30% relative to a predose level, wherein the antibody or antigen-binding fragment thereof is preferably administered in a dose of about 100 to about 200 mg E4W and particularly about 150mg E4W;

(ii) reduces low-density lipoprotein (LDL-C) of at least about -35% to relative to a predose level with a sustained reduction over at least a 14 day-period, wherein the sustained reduction is preferably at least -40% and more preferably at least -45% relative to a predose level, wherein the antibody or antigen-binding fragment thereof is preferably administered in a dose of about 100 to about 200 mg E4W and particularly about 150mg E4W;

(iii) reduces low-density lipoprotein (LDL-C) of at least about -55 % relative to a predose level with a sustained reduction over at least a 14 day-period, wherein the sustained reduction is preferably at least -55% and more preferably at least -60% relative to a predose level, wherein the antibody or antigen-binding fragment thereof is preferably administered in a dose of about 100 to about 200 mg E4W and particularly about 150mg E4W;

(iv) reduces low-density lipoprotein (LDL-C) of at least about 30% with a sustained reduction over at least a 28 day period, wherein the sustained reduction is preferably at least -35% and more preferably at least -40% relative to a predose level, wherein the antibody or antigen-binding fragment thereof is preferably administered in a dose of about 100 to about 200 mg E4W and particularly about 150mg E4W;

(v) reduces low-density lipoprotein (LDL- C) of at least about -35 % relative to a predose level with a sustained reduction over at least a 28 day-period, wherein the sustained reduction is preferably at least -40% and more preferably at least -45% or - 50% relative to a predose level, wherein the antibody or antigen-binding fragment thereof is preferably administered in a dose of about 100 to about 200 mg E4W and particularly about 150mg E4W;

(vi) increases of serum HDL cholesterol levels of at least 2%, at least 2.5%, at least, 3%, at least 3.5%, at least 4%, at least 4.5%, at least 5% or at least 5.5% relative to a predose level, wherein the antibody or antigen-binding fragment thereof is preferably administered in a dose of about 100 to about 200 mg E4W and particularly about 150mg E4W;

(vii) reduces serum total cholesterol at least about 20% and preferably about at least 25 or 30% relative to a predose level with sustained reduction over at least a 24 day period;

(viii) reduces serum total cholesterol at least about 60% and preferably at least about 65, 70, 75 or 80% relative to a predose level with sustained reduction over at least a 24 day period, wherein the antibody or antigen-binding fragment thereof is preferably administered in a dose of about 100 to about 200 mg E4W and particularly about 150mg E4W;

(ix) reduces serum triglyceride levels at least about 20%, preferably about 30, 35 or about 40% relative to a predose level, wherein the antibody or antigen-binding fragment thereof is preferably administered in a dose of about 100 to about 200 mg E4W and particularly about 150mg E4W

(x) has little or no measurable effect on liver function, as determined by ALT and AST measurements, or on troponin levels;

(xi) increases one or more of: Total-Cholesterol levels, ApoB levels, non HDL-C levels, Apo-B/ApoA-1 ratio, wherein the antibody or antigen-binding fragment thereof is preferably administered in a dose of about 100 to about 200 mg E4W and particularly about 150mg E4W.

According to some embodiments of the first, second or third aspect of present invention, the antibody is administered every fourth week or every month and administering the fibrate each day once a day.

Another embodiment of the first, second or third aspect of present invention comprises administering the antibody every fourth week or every month and administering the lipid lowering agent acting by decreasing cholesterol uptake in the intestine each day once a day.

Another embodiment of the first, second or third aspect of present invention comprises administering the antibody every fourth week or every month and administering the lipid lowering agent acting by decreasing cholesterol uptake in the intestine each day once a day and administering the fibrate each day once a day.

Suitable formulations for the antibody, the lipid lowering drug acting by cholesterol uptake into the intestine and the fibrate can e.g. be taken from the general definitions section. The skilled artisan knows about suitable formulations for a given administration regime.According to one embodiment of the first, second or third aspect, the antibody or antigen-binding fragment thereof or fragment thereof is administered per injection and preferably subcutaneous injection and more preferably per subcutaneous injection into the abdomen. Thus, according to another embodiment formulated as injection solution and particularly injection solution suitable for subcutaneous injection

According to another embodiment of the first, second or third aspect, the lipid lowering drug or the fibrate or both are administered orally. Thus, according to yet another embodiment of the first, second or third aspect, the lipid lowering drug acting by decreasing cholesterol uptake into the intestine or the fibrate or both are formulated for oral administration.According to oner particular embodiment of the first, second or third aspect, the lipid lowering drug or the fibrate of both are formulated as tablet, capsule or (over-) encapsulated tablet.

According to another embodiment of the first, second or third aspect, the subject in need thereof falls into one or more of the following groups of subjects:

(i) Subjects having a serum LDL cholesterol (LDL-C) level of at least 100 mg/dL, *[at least 130 mg/dL, at least 160 mg/dL / at least 200 mg/dL]* (e.g. a level of at least 130mg/dL if said LDL-C level is determined absence of any lipid lowering treatment, or a level of at least 100 mg/dL if said LDL-C level is determined under lipid lowering treatment such as treatment with ezetimibe or fenofibrate).

(ii) Subjects having a serum HDL-C level of less than 40 mg/dL; (particularly if said LDL-C level is determined absence of any lipid lowering treatment or if said LDL-C level is determined under treatment with ezetimibe or fenofibrate).

(iii) Subjects having a serum cholesterol level of at least 200 mg/dL *[240 mgldL];* (particularly if said LDL-C level is determined absence of any lipid lowering treatment or if said LDL-C level is determined under lipid lowering treatment, e.g.with ezetimibe or fenofibrate).

(iv) Subjects having a serum triacylglycerol level of at least 150 mg/dL *[at least 200 mgldL; at least 500 mgldL],* wherein said triacylglycerol level is determined after fasting for at least 8 hours; (particularly if said LDL-C level is determined absence of any lipid lowering treatment or if said LDL-C level is determined under lipid lowering treatment, e.g.with ezetimibe or fenofibrate).

(v) Subjects being at least 18 years old *[at least 18 /20 /25 /30/ 35 /40/ 45 / 50 / 55 / 60 /65 / 70 / 75 years old].*

(vi) Subjects being at maximum 75 years old *[75 /70 /65 / 60 / 55 / 50 / 45 / 40/35/ 30/ 25/ 20 years].*

(vii) Subjects having a *BMI of 25 [26127128129130131 1321331341351361371 38 / 39]* or more.

(viii) Male subjects.

(ix) Female subjects.

(x) Subjects in which the administration of said antibody or antigen-binding fragment thereof leads to a reduction in the serum LDL-C level by at least 30 mg/dL *[40 mgldL; 50 mgldL; 60 mgldL; 70 mgldL]* relative to predose level.

(xi) Subjects in which the administration of said antibody or antigen-binding fragment thereof leads to a reduction in the serum LDL-C level by at least 20% *[30%; 40%; 50%; 60%]* relative to predose level.

(xii) Subjects having mild and/or moderate hepatic impairment. It has surprisingly been found, that administration of the antibody of present invention is well tolerable for patients and that no increases of ALT or AST levels have been observed. Thus, the present combination treatment - especially in the context of administration of present PCSK9 antibody or fragment thereof - is a suitable treatment for patients having mild or moderate hepatic impairment, particularly as measured with the child-Pugh score.

(xiii) Subjects with normal hepatic liver function.

(ix) Subjects with high cardiovascular risk.

(x) Subjects with coronary heart disease.

(xi) Subjects with cardiovascular disease.

(xii) Subjects undergoing lipid-lowering diet.

According to another embodiment of the first, second or third aspect, the subject in need thereof does not fall into one or more of the following groups of subjects:

(i) smokers;

(ii) persons being 76 years old or older;

(iii) persons being 17 years or younger;

(iv) persons suffering from hypertension;

(v) women who are pregnant;

(vi) women who are trying to become pregnant;

(vii) women who are breast-feeding;

(viii) persons who have or ever had severe hepatic impairment;

(ix) persons who had any unexplained abnormal results from blood tests for liver function;

(x) persons who drink excessive amounts of alcohol;

(xi) persons having kidney problems;

(xii) persons suffering from hypothyroidism;

(xiii) persons suffering from muscle disorders;

(xiv) persons having encountered previous muscular problems during treatment with lipid-lowering medicine;

(xv) persons having serious problems with their breathing;

(xvi) persons taking one or more of the following medicines: medicines altering the way the immune systems works (e.g. cyclosporine or antihistamines), antibiotics or antifungal medicines (e.g. erythromycin, clarithromycin, ketoconazole, itraconazole, rifampicin, fusidic acid), medicines regulating lipid levels (e.g. gemfibrozil, colestipol), calcium channel blockers (e.g. verapamil, diltiazem), medicines regulating the heart rhythm (digoxin, amiodarone), protease inhibitors used in the treatment of HIV (e.g. nelfinavir), warfarin, oral contraceptives, antacids or St. John's Wort; or

(xvii) persons drinking more than 0.1 L of grapefruit juice per day; or

(xviii) persons having a body mass index (BMI) of more than 40; or

(xix) persons having a body mass index (BMI) of less than 18; or

(xx) persons suffering from type 1 diabetes or type 2 diabetes; or

(xxi) persons positive for hepatitis B or hepatitis C; or

(xxii) persons having a known sensitivity to monoclonal antibody or antigen-binding fragment thereof therapeutics; or

(xxiii) persons having acute hepatitis; or

(xxiv) persons having hepatic encephalopathy grade 2, 3, or 4; or

(xxv)persons having uncontrolled clinically relevant cardiovascular, pulmonary, gastrointestinal, metabolic, hematological, neurological, psychiatric, systemic, ocular, gynecologic (if female), or infectious disease, or signs of acute illness; or

(xxvi) persons having history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness; or

(xxvii) persons having experienced an adverse event after earlier administration of a fibrate and preferably fenofibrate; or

(xxviii)persons having experienced an adverse event after earlier administration of ezetimibe; or

(xxix) persons having experienced an adverse event after earlier administration of antibodies against human PCSK9; or

(xxx)persons having one or more of: neutropenia, thrombocytopenia, an increase in ALT, acute renal failure and rhabdomyolysis

and wherein the subject preferably falls into one or more of the subject groups according to the previous embodiment.

According to another embodiment of the first, second or third aspect, the subject in need is a subject indicated for LDL apheresis, a subject with PCSK9-activating mutations, a subject with heterozygous Familial Hypercholesterolemia, a subject with primary hypercholesterolemia who is statin uncontrolled, a subject at risk for developing hypercholesterolemia, a subject with hypercholesterolemia, hyperlipidemia, dyslipidemia such as mixed dyslipidemia, atherosclerosis, a high risk of developing or suffering from cardiovascular disease, a subject with a cardiovascular disease such as coronary heart disease or a coronary heart disease risk-equivalent.

In further preferred embodiments of the first, second or third and any aspect of present invention, the subject in need thereof is a subject with a cardiovascular disease, a subject with high cardiovascular risk, a subject with (established) coronary heart disease (CHD) or a subject with a CHD-risk equivalent. The subject can also be a subject with one or more of high cardiovascular risk, cardiovascular disease, (established) coronary heart disease (CHD), and CHD-risk equivalent AND falling into one or more of the other patient populations as listed above, e.g. having hypercholesterolemia, particularly forms of familial hypercholesterolemia such as heterozygous familial hypercholesterolemia. Some

preferred subjects are thus subjects with hypercholesterolemia, particularly heterozygous familial hypercholesterolemia and having a cardiovascular disease. Other preferred subjects are thus subjects with hypercholesterolemia, particularly heterozygous familial hypercholesterolemia and having a high cardiovascular risk. Other preferred subjects are thus subjects with hypercholesterolemia, particularly heterozygous familial hypercholesterolemia and having a CHD-risk equivalent.

According to one embodiment of the first, second or third (as well any other) aspect of present invention, a disease or condition in which PCSK9 expression or activity causes an impact is thus a cardiovascular disease.

According to another embodiment of the first (as well as the second, third, fourth, fifth, sixth, seventh, eighth or ninth) aspect of present invention, a disease or condition in which PCSK9 expression or activity causes an impact is thus high cardiovascular risk.

According to another embodiment of the first (as well as the second, third, fourth, fifth, sixth, seventh, eighth or ninth) aspect of present invention, a disease or condition in which PCSK9 expression or activity causes an impact is thus (established) coronary heart disease (CHD).

According to another embodiment of the first (as well as the second, third, fourth, fifth, sixth, seventh, eighth or ninth) aspect of present invention, a disease or condition in which PCSK9 expression or activity causes an impact is thus a CHD-risk equivalent.

According to another embodiment of the first, second or third aspect, the disease or condition in which PCSK9 expression or activity causes an impact is ameliorated, improved, inhibited or prevented with a PCSK9 antagonist.

Exemplary diseases or conditions that can be ameliorated, improved, inhibited or prevented by administration of an PCSK9 antibody in the context of present invention include without limitation: hypercholesterolemia, statin-uncontrolled hypercholesterolemia, non-familial hypercholesterolemia, heterozygous familiar hypercholesterolemia, hyperlipidemia, dyslipidemia such as mixed dyslipidemia, mixed dyslipidemia, atherosclerosis, cardiovascular disease, coronary heart disease and a coronary heart disease-risk equivalent.

According to one embodiment of the first, the second or the third aspect of present invention, the disease or disorder is hypercholesterolemia, wherein the hypercholesterolemia can be any hypercholesterolemia, e.g. familial or non-familial hypercholesterolemia, statin-controlled or statin-uncontrolled hypercholesterolemia.

According to another embodiment of the first, the second or the third aspect of present invention, the disease or disorder is hyperlipidemia.

According to another embodiment of the first, the second or the third aspect of present invention, the disease or disorder is dyslipidemia (such as mixed dyslipidemia).

According to another embodiment of the first, the second, the third, the fourth, the fifth, the sixth, the seventh, the eighth and the ninth aspect of present invention, the disease or disorder is atherosclerosis.

According to another embodiment of the first, the second or the third aspect of present invention,, the disease or disorder is cardiovascular disease.

According to another embodiment of the first, second or third aspect, the treatment with the antibody or antigen-binding fragment thereof together with the lipid lowering agent or the fibrate or together with the lipid lowering agent and the fibrate does not induce does not induce any of: neutropenia, acute renal failure, rhabdomyolysis, increase in ALT or AST, thrombocytopenia, neutropenia.

According to another embodiment of the first, second or third aspect, the treatment with the antibody or antigen-binding fragment thereof together with the lipid lowering agent or the fibrate or together with the lipid lowering agent and the fibrate improves lipid parameters critical for the development, persistence or worsening of atherosclerosis and/or cardiovascular disease such as coronary heart disease or coronary-heart disease risk equivalents.

According to another embodiment of the first, second or third aspect, the treatment positively affects one or more of the following serum levels: LDL-C, total-C, triglycerides, HDL-C, non-HDL-C, apolipoprotein B, apolipoprotein A1, lipoprotein a).

Additional embodiments of the first, second or third aspect can e.g. be taken from the following aspects of present invention of the general definitions section. Further suitable formulations, unit dosage forms for the combination treatments can for example be found in the general definitions section or in the description of the $5^{th}$ $6^{th}$ and $7^{th}$ aspect.

In a fourth aspect, present invention concerns an antibody or antigen-binding fragment thereof which specifically binds hPCSK9 (human proprotein convertase subtilisin/kexin type 9) for use in one or more of the following methods (1), (2) and (3):

(1) for use in a method according to the first aspect, i.e. a method for treating a disease or condition in which PCSK9 expression or activity causes an impact comprising administering a therapeutic amount of an antibody or antigen-binding fragment thereof which specifically binds hPCSK9 (human proprotein convertase subtilisin/kexin type 9) to a subject in need thereof, wherein the method comprises administering of the antibody or antigen-binding fragment thereof together with

a) a therapeutic amount of a lipid lowering agent acting by decreasing cholesterol adsorption in the intestine, or

b) a therapeutic amount of a fibrate, or

c) a therapeutic amount of the lipid lowering agent of a) and a therapeutic amount of a fibrate;

(2) for use in a method of lowering cholesterol levels in a subject in need thereof, comprising:

(a) selecting a subject with a blood low density lipoprotein cholesterol (LDL-C) level > 100 mg/dL; and
(b) administering to said subject a composition comprising an antibody or antigen-binding fragment thereof or antigen binding fragment thereof that specifically binds to human proprotein convertase subtilisin/kexin type 9 (hPCSK9); thereby lowering cholesterol levels in the subject in need thereof, wherein the method comprises administering of the antibody or antigen- binding fragment thereof or fragment thereof together with

(i) a therapeutic amount of a lipid lowering agent acting by decreasing cholesterol adsorption in the intestine, or
(ii) a therapeutic amount of a fibrate, or
(iii) a therapeutic amount of the lipid lowering agent of a) and a therapeutic amount of a fibrate; or

(3) for use in a method of preventing effects of a (persistently) increased LDL-C level in the blood comprising administering a therapeutic amount of an antibody or antigen-binding fragment thereof which specifically binds hPCSK9 (human proprotein convertase subtilisin/kexin type 9) to a subject having an increased LDL-C level in the blood, and

a. administering a therapeutic amount of a lipid lowering agent acting by decreasing cholesterol adsorption in the intestine, or

b. administering a therapeutic amount of a fibrate, or

c. administering a therapeutic amount of the lipid lowering agent of a) and a therapeutic amount of a fibrate.

Different embodiments concerning the fourth aspect of present invention can e.g. be taken from the description of the first, second and third aspect (particularly as regards the individual features of the methods of treatment for which the antibody is to be used such as the antibodies, fibrate, the lipid lowering agent, indications, subjects in need, administration and dosage regimens etc.) as well as from the general definitions section and the section "preferred antibodies for practicing present invention" or any other section, herein.

Antibodies specifically binding human PCSK9 are known in the art, see e.g. WO 2008/057457, WO 2008/057458, WO 2008/057459, WO 2008/063382, WO 2008/125623, and US 2008/0008697 and particularly US 2010/0166768 A1. The skilled artisan knows how to express and purify such antibodies. Thus, in the context of the fourth aspect of present invention, any antibody specifically binding human PCSK9 can be used as long as it has the desired effect for practicing present invention. Selecting the right antibody for practicing the different aspects of present invention among the known and readily available ones lies within the skill of the artisan.

Particularly suitable antibodies for practicing the fourth aspect of present invention can be gained from the section "Preferred antibodies for practicing present invention" herein.

According to one embodiment of the fourth aspect of present invention, the antibody or antigen-binding fragment thereof is a recombinant human antibody.

According to another embodiment of the fourth aspect of present invention, the antibody or antigen-binding fragment thereof is characterized by one or more of the following:

(i) being capable of reducing serum total cholesterol at least about 25 to about 35% and sustaining the reduction over at least a 24 day period relative to a predose level;

(ii) being capable of reducing serum LDL cholesterol at least about 65-80% and sustaining the reduction over at least a 24 day period relative to a predose level;

(iii) being capable of reducing serum triglyceride at least about 25-40% relative to predose level;

(iv) achieving one or more of (i)-(iii) without reducing serum HDL cholesterol or reducing serum HDL cholesterol no

more than 5% relative to predose level;

(v) achieving one or more of (i)-(iii) with little or no measurable effect on liver function, as determined by ALT (Alanine Aminotransferase) and AST (Aspartate Aminotransferase) measurements.

According to another preferred embodiment of the fourth aspect, the antibody or antigen-binding fragment thereof is administered to the subject every other week (E2W), every fourth week (E4W) or once a month.

According to another preferred embodiment of the fourth aspect, the antibody or antigen-binding fragment thereof has one or more of the following characteristics:

(i) is for use in the reduction of low-density lipoprotein (LDL-C) levels of at least about - 20% relative to a predose level with a sustained reduction over at least a 14 day-period, wherein the sustained reduction is preferably at least -25% and more preferably at least -30% relative to a predose level, wherein the antibody or antigen-binding fragment thereof is preferably administered in a dose of about 100 to about 200 mg E4W and particularly about 150mg E4W;

(ii) is for use in the reduction of low-density lipoprotein (LDL-C) of at least about -35% to relative to a predose level with a sustained reduction over at least a 14 day-period, wherein the sustained reduction is preferably at least -40% and more preferably at least -45% relative to a predose level, wherein the antibody or antigen-binding fragment thereof is preferably administered in a dose of about 100 to about 200 mg E4W and particularly about 150mg E4W;

(iii) is for use in the reduction of low-density lipoprotein (LDL-C) of at least about -55 % relative to a predose level with a sustained reduction over at least a 14 day-period, wherein the sustained reduction is preferably at least -55% and more preferably at least -60% relative to a predose level, wherein the antibody or antigen-binding fragment thereof is preferably administered in a dose of about 100 to about 200 mg E4W and particularly about 150mg E4W;

(iv) is for use in the reduction of low-density lipoprotein (LDL-C) of at least about 30% with a sustained reduction over at least a 28 day period, wherein the sustained reduction is preferably at least -35% and more preferably at least -40% relative to a predose level, wherein the antibody or antigen-binding fragment thereof is preferably administered in a dose of about 100 to about 200 mg E4W and particularly about 150mg E4W;

(v) is for use in the reduction of low-density lipoprotein (LDL-C) of at least about -35 % relative to a predose level with a sustained reduction over at least a 28 day-period, wherein the sustained reduction is preferably at least -40% and more preferably at least -45% or -50% relative to a predose level, wherein the antibody or antigen-binding fragment thereof is preferably administered in a dose of about 100 to about 200 mg E4W and particularly about 150mg E4W;

(vi) is for use in the increase of serum HDL cholesterol levels of at least 2%, at least 2.5%, at least, 3%, at least 3.5%, at least 4%, at least 4.5%, at least 5% or at least 5.5% relative to a predose level, wherein the antibody or antigen-binding fragment thereof is preferably administered in a dose of about 100 to about 200 mg E4W and particularly about 150mg E4W;

(vii) is for use in the reduction of serum total cholesterol at least about 20% and preferably about at least 25 or 30% relative to a predose level with sustained reduction over at least a 24 day period;

(viii) is for use in the reduction of serum total cholesterol at least about 60% and preferably at least about 65, 70, 75 or 80% relative to a predose level with sustained reduction over at least a 24 day period, wherein the antibody or antigen-binding fragment thereof is preferably administered in a dose of about 100 to about 200 mg E4W and particularly about 150mg E4W;

(ix) is for use in the reduction of serum triglyceride levels at least about 20%, preferably about 30, 35 or about 40% relative to a predose level, wherein the antibody or antigen-binding fragment thereof is preferably administered in a dose of about 100 to about 200 mg E4W and particularly about 150mg E4W

(x) has little or no measurable effect on liver function, as determined by ALT and AST measurements, or on troponin levels;

(xi) is for use in the increase of one or more of: Total-Cholesterol levels, ApoB levels, non HDL-C levels, Apo-B/ApoA-1 ratio, wherein the antibody or antigen-binding fragment thereof is preferably administered in a dose of about

100 to about 200 mg E4W and particularly about 150mg E4W.

According to another embodiment of the fourth aspect of present invention, the antibody or antigen-binding fragment thereof or the antigen-binding fragment thereof comprises

a) a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; or

b) a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736, or

c) the heavy chain of a) and the light chain of b).

According to yet another embodiment of the fourth aspect of present invention, the antibody or antigen-binding fragment thereof or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

According to another embodiment of the fourth aspect of present invention, the antibody or antigen-binding fragment thereof binds to the same epitope on hPCSK9 as an antibody or antigen-binding fragment thereof comprising heavy and light chain CDR amino acid sequences as shown in SEQ ID NOs: 76, 78, 80, 84, 86, and 88.

According to another embodiment of the fourth aspect of present invention, the antibody or antigen-binding fragment thereof competes for binding to hPCSK9 with an antibody or antigen-binding fragment thereof comprising heavy and light chain CDR amino acid sequences as shown in SEQ ID NOs: 76, 78, 80, 84, 86, and 88.

According to another embodiment of the fourth aspect, the antibody or antigen-binding fragment thereof is administered in a dosage amount ranging from 5 mg to 500 mg and preferably ranging from 50 to 300 mg and more preferably of about 150 mg.

Particular embodiments of the fourth aspect comprise the medical use of one of the antibodies 316P or 300N.

The fibrate to be used in the context of the fourth aspect of present invention can be any fibrate having cholesterol lowering effect. According to one embodiment, the fibrate is an activator of PPARalpha. Examples comprise fenofibrate, bezafibrate, ciprofibrate, clofibrate and gemfibrozil. According to one embodiment of the different aspects of present invention, the fibrate is fenofibrate. According to one particular embodiment, the fibrate is fenofibrate. Suitable dosages, administration regimes and formulations etc. can be taken from the description of the first, second or third, fifth or sixth aspect and from the general descriptions section.

The lipid lowering agent acting by decreasing cholesterol adsorption in the intestine can be any lipid lowering agent acting by decreasing cholesterol adsorption or uptake in the intestine (possibly in addition to other kind(s) of lipid lowering activity) and preferably is an azetidine such as ezetimibe. According to one embodiment of the different aspects of present invention, the lipid lowering agent is ezetimibe. Suitable dosages, administration regimes and formulations etc. can be taken from the description of the first, second or third, fifth, sixth or seventh aspect and from the general descriptions section.

According to one embodiment of the fourth aspect, the action of the antibody or antigen-binding fragment thereof comprises - inter alia - counteracting the increase of PCSK9 expression by the lipid-lowering agent and particularly of ezetimibe.

According to another embodiment of the fourth aspect, the action of the antibody or antigen-binding fragment thereof comprises - inter alia - counteracting the increase of PCSK9 expression by the fibrate and particularly of fenofibrate.

In a fifth aspect, present invention concerns a pharmaceutical composition comprising a pharmaceutically effective amount of antibody or antigen-binding fragment thereof of an antibody or antigen-binding fragment thereof which specifically binds hPCSK9 and preferably of an antibody as described in the first, second and third aspect of present invention and a pharmaceutically acceptable excipient together with a pharmaceutically effective amount of a fibrate and preferably of fenofibrate.

In a sixth aspect, present invention concerns a pharmaceutical composition comprising a pharmaceutically effective amount of antibody or antigen-binding fragment thereof of an antibody or antigen-binding fragment thereof which specifically binds hPCSK9 and preferably of an antibody as described in the first, second,third or fourth aspect of present invention, in a method according to one of the aspects 1 to 3.

Different embodiments concerning the fifth or sixth aspect of present invention can e.g. be taken from the

description of the first, second and third aspect (particularly as regards the individual features of the methods of treatment for which the pharmaceutical composition of the sixth aspect is to be used, as well as the antibodies, the fibrate, the lipid lowering agent, indications, subjects in need, administration and dosage regimens etc of the fifth and sixth aspect.) as well as from the general definitions section and the section "preferred antibodies for practicing present invention" or any other section, herein. All of these embodiments can readiliy be combined with any of the embodiments of the fifth and sixth aspect described in more detail herein below, if not indicated to the contrary.

Antibodies specifically binding human PCSK9 are known in the art, see e.g. WO 2008/057457, WO 2008/057458, WO 2008/057459, WO 2008/063382, WO 2008/125623, and US 2008/0008697 and particularly US 2010/0166768 A1. The skilled artisan knows how to express and purify such antibodies. Thus, in the context of the fifth and sixth aspect of present invention, any antibody specifically binding human PCSK9 can be used as long as it has the desired effect for practicing present invention. Selecting the right antibody for practicing the fifth and sixth aspects of present invention among the known and readily available ones lies within the skill of the artisan. Particularly suitable antibodies for practicing the fifth or sixth aspect of present invention can be gained from the section "preferred antibodies for practicing present invention" herein, as well as from the description of the first, second, third or fourth aspect of present invention

According to one embodiment of the sixth aspect, the pharmaceutical composition further comprises a pharmaceutically effective amount of a fibrate.

The fibrate to be used in the context of the fifth or sixth aspect of present invention can be any fibrate having cholesterol lowering effect. According to one embodiment, the fibrate is an activator of PPARalpha. Examples comprise fenofibrate, bezafibrate, ciprofibrate, clofibrate and gemfibrozil. According to one embodiment of the different aspects of present invention, the fibrate is fenofibrate. According to one particular embodiment, the fibrate is fenofibrate. Suitable dosages, administration regimes and formulations etc. can be taken from the description of the first, second or third aspect and from the general descriptions section.

According to one embodiment, the pharmaceutical composition of the fifth or sixth aspect comprises fenofibrate, e.g. about 160 mg.

According to another embodiment of the sixth aspect, the pharmaceutical composition further comprises a pharmaceutically effective amount of a lipid lowering drug acting by decreasing cholesterol uptake in the intestine.

The lipid lowering agent acting by decreasing cholesterol adsorption in the intestine can be any lipid lowering agent acting by decreasing cholesterol adsorption or uptake in the intestine (possibly in addition to one or more other lipid lowering actions) and preferably is an azetidine such as ezetimibe. According to one embodiment of the different aspects of present invention, the lipid lowering agent is ezetimibe.

According to one embodiment, the pharmaceutical composition of the fifth or sixth aspect comprises ezetimibe, e.g. about 10 mg.

According to another embodiment of the sixth aspect, the pharmaceutical composition further comprises a pharmaceutically effective amount of a fibrate and preferably of fenofibrate, e.g. about 160mg and a pharmaceutically effective amount of a lipid lowering drug acting by decreasing cholesterol uptake in the intestine and preferably of ezetimibe, e.g. about 10 mg.

The pharmaceutical composition can be formulated according to any pharmaceutically applicable formulation as known in the art, and specifically as herein described, such as dry formulation for dissolution or liquid formulation. Suitable formulations of antibodies are known in the art and comprise dry formulations (e.g. freeze-dried, spray-dried or lyophilized, water-free concentrate) as well as liquid formulations (e.g. solutions).

The term "carrier" in the context of present invention refers to a diluent, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Examples of suitable pharmaceutical carriers are described in "Remington's Pharmaceutical Sciences" by E. W. Martin. Such compositions will contain a prophylactically or therapeutically effective amount of the antibody, preferably in purified form, together with a suitable amount of carrier so as to provide the form for proper administration to the patient. The composition may further contain one or more other active ingredients such as an HMG-CoA reductase inhibitor. The formulation should suit the mode of administration.

According to one embodiment, the pharmaceutical composition comprises the antibody or antigen-binding fragment thereof as dry formulation for dissolution such as a lyophilized powder, freeze-dried or spray-dried powder or water free concentrate.

According to another embodiment, the pharmaceutical composition comprises the antibody or antigen-binding fragment thereof as liquid formulation, e.g. injection or infusion solution and particularly as solution for subcutaneous injection.

According to another preferred embodiment, the pharmaceutical composition comprises the fibrate and/or the lipid lowering agent acting by reducing cholesterol uptake in the intestine as oral or peroral formulation, e.g. capsule or tabled, or as liquid formulation, e.g. suspension, dispersion or solution, e.g. for peroral administration, injection or infusion.

According to another preferred embodiment the pharmaceutical composition is for use in the treatment of a disease or disorder for use in the treatment of a disease or condition in which PCSK9 expression or activity causes an impact or for lowering elevated total cholesterol or elevated LDL-C levels. Further preferred uses, dosage regimens, administration regimens of the antibody or fragment thereof or of the HMG-CoA reductase inhibitor, or populations to be treated with the pharmaceutical composition described in present application, for example at the other aspects of present invention such as the first or second aspect.

The pharmaceutical composition of the antibody as used in the different aspects of present invention, e.g. the sixth or seventh aspect, can e.g. be a liquid formulation as herein described comprising the antibody or antigen-binding fragment thereof of present invention, and preferably comprising about 40 mg to about 200 mg or about 50 to about 200 mg, e.g. about 40 mg, about 50 mg, about 75 mg, at about 100 mg, about 150 mg or about 200 mg of the antibody or antigen-binding fragment thereof per 1 ml volume.

The pharmaceutical composition of the antibody as used in the different aspects of present invention, e.g. the sixth or seventh aspect, can e.g. be a dry formulation as herein described comprising the antibody or antigen-binding fragment thereof of present invention, and preferably comprising about 40 mg to about 500 mg, 50 to about 500 mg, about 50 to about 400, about 50 to about 300 e.g. about 40 mg, about 50 mg, about 75 mg, at about 100 mg, about 150 mg, about 200 mg, about 250 mg, about 300 mg, about 350 mg, about 400 mg, about 450 mg or about 500mg and more preferably about 50 mg, about 100 mg, about 150 mg, about 200 mg, about 250 mg, about 300 mg and even more preferably about 150 mg, about 200 mg or about 300 mg of the antibody or antigen-binding fragment thereof per dose.

The formulations of present invention can comprise further active ingredients such as an HMG-CoA reductase inhibitor as herein described.

Suitable formulations of antibodies in general are known in the art and comprise dry formulations (e.g. freeze-dried, spray-dried or lyophilized, water-free concentrate) as well as liquid formulations (e.g. solutions). Suitable formulations of statins are as well known in the art and comprise dry formulations as well as liquid formulations, e.g suspensions, dispersions and solutions (for a reference, see e.g. "Statins therapy: a review on conventional and novel formulation approaches" R. Tiwari and K. Pathak, Journal of Pharmacy and Pharmacology, 2011, that is hereby incorporated in entirety).

According to a further preferred embodiment of the seventh aspect of present invention, the pharmaceutical composition comprising the antibody or fragment thereof is comprised in a unit dosage form as herein described (e.g. as). In this particular embodiment, the present invention relates to a unit dosage form comprising the antibody or fragment thereof for use in a method according to the 1st, 2nd, 3rd, 4th or 5th aspect of present invention. In a particularly preferred embodiment, the unit dosage form is for use in a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function. Further embodiments of the unit dosage form can be as e.g. described in the Definitions section or in the eighth aspect of present invention. Further embodiments of the pharmaceutical use of the unit dosage form are e.g. as described in the Definitions section or in the 1st, 2nd, 3rd, 4th or 5th aspect of present invention. Further embodiments of the antibody comprised in the unit dosage form are e.g. described in the section "*Preferred antibodies for practicing present invention"* or in the sixth aspect.

In a seventh aspect, present invention concerns an article of manufacture comprising

(a) a packaging material or container;

(b) an antibody or antigen-binding fragment thereof which specifically binds hPCSK9; and

(c) a fibrate and/or a lipid lowering drug acting by decreasing cholesterol adsorption in the intestine; and

(d) a label or packaging insert.

Antibodies specifically binding human PCSK9 are known in the art, see e.g. WO 2008/057457, WO 2008/057458, WO 2008/057459, WO 2008/063382, WO 2008/125623, and US 2008/0008697 and particularly US 2010/0166768 A1. The skilled artisan knows how to express and purify such antibodies. Thus, in the context of the

seventh as well as all other aspects of present invention, any antibody specifically binding human PCSK9 can be used as long as it has the desired effect for practicing present invention. Selecting the right antibody for practicing the different aspects of present invention among the known and readily available ones lies within the skill of the artisan.

Particularly suitable antibodies for practicing all aspects such as the seventh aspect of present invention can be gained from the section "preferred antibodies for practicing present invention" herein.

The fibrate to be used in the context of the seventh and the other aspects of present invention can be any fibrate having cholesterol lowering effect. Examples comprise fenofibrate, bezafibrate, ciprofibrate, clofibrate and gemfibrozil. According to one embodiment of the different aspects of present invention, the fibrate is fenofibrate.

The lipid lowering agent acting by decreasing cholesterol adsorption in the intestine for use in the context of the seventh and the other aspects of present invention can be any lipid lowering agent acting by decreasing cholesterol adsorption or uptake in the intestine and preferably is an azetidine such as ezetimibe. According to one embodiment of the different aspects of present invention, the lipid lowering agent is ezetimibe.

According to one embodiment of the seventh aspect, the label or packaging insert contains reference to any one of the methods according to any of aspects 1 to 3.

According to another embodiment of the seventh aspect, the article of manufacture comprises a fibrate, wherein the fibrate is preferably fenofibrate.

According to another embodiment of the seventh aspect, the article of manufacture comprises the lipid lowering drug, wherein the lipid lowering drug is preferably an azetidone and more preferably ezetimibe.

According to another embodiment of the seventh aspect, the article of manufacture comprises the lipid lowering drug, wherein the lipid lowering drug is preferably an azetidone and more preferably ezetimibe and the article of manufacture further comprises a fibrate, wherein the fibrate is preferably fenofibrate.

According to another embodiment of the seventh aspect, the antibody or antigen-binding fragment thereof is comprised in a pharmaceutical formulation together with a pharmaceutically acceptable excipient. Suitable pharmaceutical compositions for practicing the seventh aspect and the other aspects of present invention are for example described in the definition section of present application or in the context of the fifth or sixth aspect of present invention.

Different embodiments of the pharmaceutical composition comprising the antibody or fragment thereof for use in the eighth aspect of present invention are listed below and can also be taken from the general Definition section or from the description of the fifth or sixth aspect of present invention.

The pharmaceutical composition can be formulated according to any pharmaceutically applicable formulation as known in the art, and specifically as herein described, such as dry formulation for dissolution or liquid formulation. Suitable formulations of antibodies are known in the art and comprise dry formulations (e.g. freeze-dried, spray-dried or lyophilized, water-free concentrate) as well as liquid formulations (e.g. solutions).

Suitable formulations of fibrates or azetidines, e.g. of fenofibrate or ezetimibe are also well known in the art and comprise dry formulations as well as liquid formulations. However, as fenofibrate and ezetimibe are usually formulated for oral administration, dry formulations for oral administration may often be suitable.

The term "carrier" in the context of present invention refers to a diluent, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a prophylactically or therapeutically effective amount of the antibody, preferably in purified form, together with a suitable amount of carrier so as to provide the form for proper administration to the patient. The composition may further contain one or more other active ingredients such as a fibrate and/or a lipid lowering agent acting by decreasing cholesterol uptake in the intestine and/or an HMG-CoA reductase inhibitor. The formulation should suit the mode of administration.

According to one embodiment, the fibrate and the lipid lowering agent acting by decreasing cholesterol uptake in the intestine can be formulated together, e.g. as one unit dosage form, e.g. a unit dosage form for oral administration such as a tablet or capsule.

According to another embodiment of the seventh aspect, the antibody or antigen-binding fragment thereof or antigen-binding fragment is an antibody or antigen-binding fragment thereof or antigen-binding fragment as specified in any of aspects 1 to 3.

According to another embodiment of the seventh aspect, the label or packaging insert contains reference to any of the methods according to aspect 1 and/or 2 and/or 3.

According to another embodiment of the seventh aspect, the label or packaging insert has one or more of the following features:

(a) the label or packaging insert indicates that patients receiving treatment with said antibody or antigen-binding fragment thereof or antigen-binding fragment can be treated for a disease or condition selected from the group consisting of: hypercholesterolemia, statin-uncontrolled hypercholesterolemia, non-familial hypercholesterolemia, heterozygous familiar hypercholesterolemia, hyperlipidemia, dyslipidemia such as mixed dyslipidemia, mixed dyslipidemia, atherosclerosis, cardiovascular disease, coronary heart disease and a coronary heart disease-risk equivalent;

(b) the label or packaging insert indicates the treatment of patients in a method according to one of the aspects 1 to 3 is contra-indicated in subjects falling into one or more of the following groups of subjects:

smokers; persons being 76 years old or older; persons being 17 years or younger; persons suffering from hypertension; women who are pregnant; women who are trying to become pregnant; women who are breast-feeding; persons who have or ever had severe hepatic impairment; persons who had any unexplained abnormal results from blood tests for liver function; persons who drink excessive amounts of alcohol; persons having kidney problems; persons suffering from hypothyroidism; persons suffering from muscle disorders; persons having encountered previous muscular problems during treatment with lipid-lowering medicine; persons having serious problems with their breathing; persons taking one or more of the following medicines: medicines altering the way the immune systems works (e.g. cyclosporine or antihistamines), antibiotics or antifungal medicines (e.g. erythromycin, clarithromycin, ketoconazole, itraconazole, rifampicin, fusidic acid), medicines regulating lipid levels (e.g. gemfibrozil, colestipol), calcium channel blockers (e.g. verapamil, diltiazem), medicines regulating the heart rhythm (digoxin, amiodarone), protease inhibitors used in the treatment of HIV (e.g. nelfinavir), warfarin, oral contraceptives, antacids or St. John's Wort; or persons drinking more than 0.1 L of grapefruit juice per day; or persons having a body mass index (BMI) of more than 40; or persons having a body mass index (BMI) of less than 18; or persons suffering from type 1 diabetes or type 2 diabetes; or persons positive for hepatitis B or hepatitis C; or persons having a known sensitivity to monoclonal antibody or antigen-binding fragment thereof therapeutics; or persons having acute hepatitis; or persons having hepatic encephalopathy grade 2, 3, or 4; or persons having uncontrolled clinically relevant cardiovascular, pulmonary, gastrointestinal, metabolic, hematological, neurological, psychiatric, systemic, ocular, gynecologic (if female), or infectious disease, or signs of acute illness; or persons having history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness; or persons having experienced an adverse event after earlier administration of a fibrate and preferably fenofibrate; or persons having experienced an adverse event after earlier administration of ezetimibe; or persons having experienced an adverse event after earlier administration of antibodies against human PCSK9; or persons having one or more of: neutropenia, thrombocytopenia, an increase in ALT, acute renal failure and rhabdomyolysis;

(c) the label or packaging insert indicates the treatment of patients in a method according to one of the aspects 1 to 3 is possible the subjects falling into one or more of the following groups of subjects:

subjects having a serum LDL cholesterol (LDL-C) level of at least 100 mg/dL, *[at least 130 mg/dL, at least 160 mg/dL / at least 200 mg/dL]* (particularly if said LDL-C level is determined absence of any lipid lowering treatment or if said LDL-C level is determined under treatment with ezetimibe or fenofibrate); subjects having a serum HDL-C level of less than 40 mg/dL (particularly if said LDL-C level is determined absence of any lipid lowering treatment or if said LDL-C level is determined under treatment with ezetimibe or fenofibrate); subjects having a serum cholesterol level of at least 200 mg/dL *[240 mg/dL]*; (particularly if said LDL-C level is determined absence of any lipid lowering treatment or if said LDL-C level is determined under treatment with ezetimibe or fenofibrate); subjects having a serum triacylglycerol level of at least 150 mg/dL *[at least 200 mg/dL; at least 500 mg/dL],* wherein said triacylglycerol level is determined after fasting for at least 8 hours (particularly if said LDL-C level is determined absence of any lipid lowering treatment or if said LDL-C level is determined under treatment with ezetimibe or fenofibrate); subjects being at least 18 years old *[at least 18 /20 /25 /30/ 35 /40/ 45 / 50 / 55 / 60 / 65 / 70 / 75 years old];* subjects being at maximum 75 years old *[75 /70 /65 / 60 / 55 / 50 / 45 / 40/35/ 30/ 25/ 20 years]*; subjects having a BMI of 25 *[26/ 27 / 28 / 29 / 30 / 31 / 32 / 33 / 34 / 35 / 36 / 37 / 38 / 39]* or more;

male subjects; female subjects; subjects in which the administration of said antibody or antigen-binding fragment thereof leads to a reduction in the serum LDL-C level by at least 30 mg/dL *[40 mg/dL; 50 mg/dL; 60 mg/dL; 70 mg/dL]* relative to predose level; or subjects in which the administration of said antibody or antigen-binding fragment thereof leads to a reduction in the serum LDL-C level by at least 20% *[30%; 40%; 50%; 60%]* relative to predose level; subjects having mild and/or moderate hepatic impairment; subjects with normal hepatic liver function; subjects with high cardiovascular risk; subjects with coronary heart disease; subjects with cardiovascular disease; subjects undergoing lipid-lowering diet.

According to one embodiment of the seventh aspect, the label or packaging insert indicates that the antibody or pharmaceutical composition comprising the antibody is administered in a combination treatment according to the first, second or third aspect of present invention..

According to yet another embodiment of the seventh aspect of present invention, the label or packaging insert has one or more of the following features:

(a) the label or packaging insert indicates that patients receiving treatment with said antibody or antigen-binding fragment can be treated for a disease or condition selected from the group consisting of hypercholesterolemia, hyperlipidemia, dyslipidemia such as mixed dyslipidemia, atherosclerosis and cardiovascular diseases such as CHD or a CHD risk equivalent;

(b) the label or packaging insert indicates the treatment of patients with said antibody or antigen-binding fragment thereof together with the administration of a fibrate such as fenofibrate;

(c) the label or packaging insert indicates the treatment of patients with said antibody or antigen-binding fragment thereof together with the application of a lipid lowering agent acting by decreasing cholesterol uptake in the intestine such as ezetimibe;

(d) the label or packaging insert indicates the treatment of patients with said antibody or antigen-binding fragment thereof together with the application of a fibrate such as fenofibrate and together with the application of a lipid lowering agent such as ezetimibe, particularly a combination treatment of the antibody or antigen-binding fragment thereof together with fenofibrate and ezetimibe;

(e) the label or packaging insert indicates that the treatment of patients according to (b), (c) or (d) above, is possible for patients belonging to one or more of the subject groups as described as patient groups for which the combination treatment according to present invention is possible in the first, second or third aspect;

(f) the label or packaging insert indicates that the treatment of patients with said antibody or antigen-binding fragment thereof according to (b), (c) or (d), is contraindicated for patients belonging to one or more of the subject groups as described as subject groups for which the combination treatment according to present invention is contraindicated in the description of the first, second or third aspect.

The label or package insert of the article of manufacture preferably states for which period or duration of treatment the article of manufacture is intended (e.g. one-time treatment, treatment for two weeks or fourteen days, treatment for four weeks, treatment for one month, treatment for one year etc) and more preferably also indicates the administration regime for the antibody or fragment thereof and the administration regime of the fibrate and/or the lipid lowering agent acting by decreasing cholesterol uptake in the intestine.

The label or packaging insert as applicable for the different aspects and embodiments of the invention, particularly in respect to the different articles of manufacture of the invention, can be any kind of data carrier suitable to be arranged within the package(s) or container(s) or on the outside of the package(s) or container(s) comprised within the article of manufacture. Preferably, the data carrier (i.e. label or, chip, bar code or leaflet or label comprising a bar code etc.) comprises information such as

(i) composition, formulation, concentration and total amount, identity of active ingredient (s) contained in the article of manufacture, i.e. of the antibody or antigen-fragments, HMG-CoA reductase inhibitor, pharmaceutical composition, unit dosage form or formulation of present invention

(ii) number and composition of unit dosage form contained in the article of manufacture

(iii) indications, contra-indications of the antibody or antigen-fragments, pharmaceutical composition, unit dosage

form or formulation of present invention

(iv) subjects/patients or subject/patient populations indicated or contra-indicated for treatment with the antibody or antigen-fragments, pharmaceutical composition, unit dosage form or formulation of present invention,

(v) instructions for use, dosage regimens and/or administration regimes,

(vi) quality information such as information about the lot/batch number of the of the antibody or antigen-fragments, pharmaceutical composition, unit dosage form or formulation of present invention, the manufacturing or assembly site or the expiry or sell-by date

(vii) information concerning the correct storage or handling of the article of manufacture, of the device for application, or of the antibody or antigen-fragments, pharmaceutical composition, unit dosage form or formulation of present invention,

(viii) information concerning the composition of the buffer(s), diluent(s), reagent(s), excipients, carriers, formulations of of the antibody or antigen-fragments, pharmaceutical composition, unit dosage form or formulation of present invention,

(ix) a warning concerning possible consequences when applying unsuitable dosage or administration regimens and/or use in contraindicated indications and/or for unsuitable patient populations.

In preferred embodiments the label or packaging insert contains reference to a method of treatment or medical use according to the $1^{st}$, $2^{nd}$ or $3^{rd}$ aspect and the embodiments of the $1^{st}$, $2^{nd}$, $3^{rd}$, $4^{th}$, $5^{th}$ or $6^{th}$ aspect as described herein.

The article of manufacture of present invention can also be a package comprising the different features of the ninth aspect and/or of the different embodiments thereof as herein described.

According to one embodiment, the article of manufacture comprises one or more effective dosages of the lipid lowering agent and preferably of ezetimibe for lowering LDL-C levels by administration once per day.

According to another embodiment, the article of manufacture comprises an effective dose of ezetimibe for lowering LDL-C levels by administration once per day.

According to one embodiment of the seventh aspect, the effective dosage comprises about 10 mg ezetimibe.

According to another embodiment, the article of manufacture comprises ezetimibe as one or more unit dosage forms for oral administration.

According to another embodiment, the article of manufacture comprises one or more effective dosages of the fibrate and preferably of fenofibrate for lowering LDL-C levels by administration once per day.

According to another embodiment, the article of manufacture comprises an effective dose of fenofibrate for lowering LDL-C levels by administration once per day.

According to another embodiment, the effective dosage comprises about 100 mg of fenofibrate.

According to another embodiment, the article of manufacture comprises fenofibrate as one or more unit dosage forms for oral administration.

According to another embodiment, the article of manufacture comprises the antibody or antigen-binding fragment thereof as injection solution and preferably comprising about 40 mg to about 200 mg or about 40 to about 200 mg, e.g. about 40 mg, about 50 mg, about 75 mg, about 100 mg, about 150 mg or about 200 mg of the antibody or antigen-binding fragment thereof per 0,5 ml or per 1 ml volume injection solution and preferably about 150mg per 1ml volume injection solution.

According to another embodiment, the article of manufacture comprises the antibody or antigen-binding fragment thereof as dry formulation, preferably comprising about 40 mg to about 500 mg, 50 to about 500 mg, about 50 to about 400, about 50 to about 300 e.g. about 40 mg, about 50 mg, about 75 mg, at about 100 mg, about 150 mg, about 200 mg, about 250 mg, about 300 mg, about 350 mg, about 400 mg, about 450 mg or about 500mg and preferably about 50 mg, about 100 mg, about 150 mg, about 200 mg, about 250 mg, about 300 mg and even more preferably about 150 mg, about 200 mg or about 300 mg and particularly about 150mg of the antibody or antigen-binding fragment thereof per dose.

According to another embodiment, the article of manufacture comprises the antibody or antigen-binding fragment thereof in one or more unit dosage forms and preferably comprising the antibody or antigen-binding fragment thereof antibody or antigen-binding fragment thereof as liquid formulation in a hermetically sealed container such as a vial, a sachette, a pre-filled syringe, a pre-filled a

As used in the different aspects and embodiments of present invention and in particularly of the eighth seventh, $9^{th}$ and $10^{th}$ aspect, the term "unit dosage form" refers to physically discrete units suitable as unitary dosages for human

and animal subjects, each unit containing a predetermined quantity of active material (e.g., about 40mg to about 500mg and preferably about 150mg of PCSK5 antibody and/or of e.g. 140 to 180 and preferably of about 160mg fenofibrate and/or about 8 to 12 mg and preferably about 10 mg of ezetimibe) calculated to produce the desired therapeutic effect in association with the required pharmaceutical diluent, carrier or vehicle. The specifications for the unit dosage forms to be used in the context of present invention are dictated by and are directly dependent on (a) the unique characteristics of the active material and the particular therapeutic effect to be achieved, and (b) the limitation inherent in the art of compounding such an active material for therapeutic use in animals or humans, as disclosed in this specification, these being features of the present invention. The term "active material" refers to any material with therapeutic activity, such as one or more active ingredients. The active ingredients to be employed as therapeutic agents can be easily prepared in such unit dosage form with the employment of pharmaceutical materials which themselves are available in the art and can be prepared by established procedures. Preferred active ingredients of present invention are the antibody or fragment thereof, the fibrate and/or the lipid lowering agent as described e.g. in the first, second, third, fourth, fifth, sixth and seventh aspect herein.

Examples of suitable unit dosage forms in accord with the different aspects of this invention and particularly in the context of the eighth aspect of present invention are vials, tablets, capsules, troches, suppositories, powder packets, wafers, cachets, ampules, segregated multiples of any of the foregoing, and other forms as herein described or generally known in the art. One or more such unit dosage forms of the antibody or fragment thereof can be comprised in an article of manufacture of present invention, optionally further comprising one or more unit dosage forms of a fibrate such as fenofibrate (e.g. a tablets comprising as blister of active ingredient fibrate) and optionally further comprising one or more unit dosage forms of the lipid lowering agent acting by decreasing cholesterol uptake such as ezetimibe (e.g. a blister of tablets or capsules comprising as active ingredient the lipid lowering drug such as ezetimibe).

According to one embodiment, the article of manufacture of present invention comprises one or more separate unit dosage forms of the antibody or fragment thereof, the pharmaceutical composition comprising the antibody or fragment thereof, the dry formulation of the antibody or fragment thereof or the injection solution of the antibody or fragment thereof and one or more separate unit dosage forms of the fibrate such as fenofibrate. According to another embodiment, the article of manufacture additionally comprises one or more separate unit dosage forms of the lipid lowering agent acting by decreasing cholesterol uptake in the intestine such as ezetimibe.

In a preferred embodiment of the seventh aspect, the unit dosage form comprises 40 - about 500 mg and particularly about 150mg of the antibody or an antigen-binding fragment of present invention. According to another preferred embodiment, the unit dosage form comprises about 40 mg, about 50 mg, about 75 mg, about 100 mg, about 150 mg, about 200 mg, about 250 mg, about 300 mg, about 350 mg, about 400 mg, about 450 mg, or about 500 mg and more preferably about 150 mg, about 200 mg or about 300 mg and even more preferably of about 150 mg of the antibody or antigen-binding fragment thereof. Further preferred dosages, and dosage regimens are as described elsewhere in the application, such as in the Definition section or in the first, second or third aspect of present invention.

According to another embodiment, the article of manufacture comprises one or more separate unit dosage forms each of the antibody or antigen-binding fragment thereof and fenofibrate and/or ezetimibe.

In one embodiment, each unit dosage form of fibrate comprises the fibrate, e.g. the fenofibrate in an effective dose for administration once per day.

In another embodiment, each unit dosage form of lipid lowering drug acting by decreasing cholesterol uptake in the intestine comprises the lipid lowering drug, e.g. ezetimibe in an effective dose for administration once per day.

In another embodiment, each unit dosage form of antibody or antigen-binding fragment thereof comprises the antibody or fragment in an effective dose for administration E2W, E4W or once a month.

According to a preferred embodiment of the eight and other aspects of present invention, each unit dosage form of fenofibrate comprises about 140 mg to about 180 mg fenofibrate and more preferably about 160mg fenofibrate.

According to a preferred embodiment of the eight and other aspects of present invention, each unit dosage form of ezetimibe comprises about 5 mg, about 8 mg, about 9 mg, about 10 mg, about 11 mg, about 15 mg and particularly about 10 mg of ezetimibe.

According to another preferred embodiment, the unit dosage form comprising the fibrate is suitable for oral administration, e.g. a pill, capsule, troche or tablet.

According to another preferred embodiment, the unit dosage form comprising the lipid lowering agent acting by decreasing cholesterol uptake into the intestine is suitable for oral administration, e.g. a pill, capsule, troche or tablet.

According to another embodiment, the article of manufacture comprises one or more unit dosage forms comprising the antibody or antigen-binding fragment thereof as dry formulation for dissolution in a hermetically sealed container such as a vial, an ampoule or sachette

According to another preferred embodiment, the unit dosage form comprising the antibody is a sachette, a disposable or reusable pre-filled syringe, a disposable or reusable pre-filled autoinjector or a cartridge for a disposable or reusable syringe or applicator, herein the unit dosage form especially comprises injection solution for injection of 0,5ml, 1ml or 2ml and preferably 1ml injection solution per shot.According to another embodiment, the article of manufacture comprises the following components a) to d):

a) one or more unit dosage forms comprising the antibody;

b) one or more unit dosage forms comprising ezetimibe and/or one or more unit dosage forms of fenofibrate;

c) the label or package insert;

d) a device for application of the antibody or antigen-binding fragment thereof such as a syringe and instructions for use of the device.

According to another embodiment, the article of manufacture comprises one or more unit dosage forms of ezetimibe and/or fenofibrate as tablet or capsule or other formulation for oral administration in a hermetically sealed container or blister.

According to another embodiment of the seventh aspect, the quantity of active ingredient is indicated on the hermetically-sealed container or blister.

According to some embodiments of the seventh aspect, the article of manufacture comprises sufficient unit dosage forms of antibody or antigen-binding fragment thereof for a bi-weekly administration regime or a four-weekly administration regime or a monthly administration regime.

According to some other embodiments of the seventh aspect, the article of manufacture comprises sufficient unit dosage forms of and ezetimibe and/or fenofibrate for a daily administration regime.

According to another embodiment of the seventh aspect, the article of manufacture comprises sufficient unit dosage forms of the antibody or antigen-binding fragment thereof and ezetimibe and/or fenofibrate, for one single administration of antibody or antigen-binding fragment thereof and ezetimibe and/or fenofibrate, for a two-week (i.e. 14-day) treatment with antibody or antigen-binding fragment thereof and ezetimibe and/or fenofibrate, for a four week (i.e, 28-day) treatment with antibody or antigen-binding fragment thereof and ezetimibe and/or fenofibrate or for a one-month treatment with antibody or antigen-binding fragment thereof and ezetimibe and/or fenofibrate.

Article of manufacture for E4W or once-a-month administration scheme antibody and daily administration scheme fibrate or lipid lowering agent acting by decreasing cholesterol absorption:

a) According to one suitable embodiment, the article of manufacture comprises sufficient unit dosage forms of the antibody or antigen-binding fragment thereof and ezetimibe, for one single administration of antibody or antigen-binding fragment thereof and for a four week (i.e, 28-day) treatment with ezetimibe (i.e. 1 unit dosage form of antibody such as 1 ml injection solution comprising 1 dosage of about 150mg and about 28 dosage forms of ezetimibe, e.g. 10 mg each). In a one-month administration scheme, the article comprises about 31 or 32 unit dosage forms of ezetimibe and 1 unit dosage form of antibody.

b) According to another suitable embodiment, the article of manufacture comprises sufficient unit dosage forms of the antibody or antigen-binding fragment thereof and fenofibrate, for one single administration of antibody or antigen-binding fragment thereof and for a four week (i.e, 28-day) treatment with fenofibrate (i.e. 1 unit dosage form of antibody such as 1 ml injection solution comprising 1 dosage of about 150mg and about 28 dosage forms of fenofibrate, e.g. 160 mg each). In a one-month administration scheme, the article comprises about 31 or 32 unit dosage forms of fenofibrate and 1 unit dosage form of antibody.

c) According to another suitable embodiment, the article of manufacture comprises sufficient unit dosage forms of the antibody or antigen-binding fragment thereof and fenofibrate and ezetimibe, for one single administration of antibody or antigen-binding fragment thereof and for a four week (i.e, 28-day) treatment with fenofibrate and ezetimibe (i.e. 1 unit dosage form of antibody such as 1 ml injection solution comprising 1 dosage of about 150mg and about 28 dosage forms of fenofibrate, e.g. 160 mg each and about 28 dosage forms of ezitimibe, e.g. about 10 mg each). In a one-month administration scheme, the article comprises about 31 or 32 unit dosage forms of fenofibrate and about 31 or 32 unit dosage forms of ezetimibe and 1 unit dosage form of antibody.

Suitable would also be articles of manufacture taking into account a weekly or biweekly administration of the antibody, wherein these articles of manufacture could readily be assembled by the skilled artisan to include sufficient unit dosage forms of the antibody, the antibody and ezetimibe, the antibody and fenofibrate, or the antibody and ezetimibe and fenofibrate for a treatment period of e.g one week, two weeks, four weeks or one month or several months.

Additional embodiments concerning seventh aspect of present invention can e.g. be taken from the description of the first, second, third, fourth, fifth and sixth aspect (particularly as regards the individual features of the methods of

treatment, the pharmaceutical compositions, the antibodies, the fibrate, the lipid lowering agent, indications, subjects in need, administration and dosage regimens etc) as well as from the general definitions section and the section "preferred antibodies for practicing present invention" or any other section, herein. All of these embodiments can readiliy be combined with any of the embodiments of the seventh aspect described in more detail hereinabove, if not explicitly stated to the contrary.

In an eighth aspect, present invention concerns a method for preparing an article of manufacture according to the seventh aspect comprising packaging an antibody or antigen-binding fragment thereof which specifically binds hPCSK9 and packaging the lipid lowering agent and/or the fibrate in a container and assembling them with a label or packaging insert and optionally together with one or more of the following: a label or package insert, instructions for use, an application device.

Antibodies specifically binding human PCSK9 are known in the art, see e.g. WO 2008/057457, WO 2008/057458, WO 2008/057459, WO 2008/063382, WO 2008/125623, and US 2008/0008697 and particularly US 2010/0166768 A1. The skilled artisan knows how to express and purify such antibodies. Thus, in the context of the eighth aspect of present invention, any antibody specifically binding human PCSK9 can be used as long as it has the desired effect for practicing present invention. Selecting the right antibody for practicing the different aspects of present invention among the known and readily available ones lies within the skill of the artisan.

Particularly suitable antibodies for practicing all aspects of present invention can be gained from the section "preferred antibodies for practicing present invention" herein.

The fibrate to be used in the context of the eighth aspect of present invention can be any fibrate having cholesterol lowering effect. Examples comprise fenofibrate, bezafibrate, ciprofibrate, clofibrate and gemfibrozil. According to one embodiment of the different aspects of present invention, the fibrate is fenofibrate.

The lipid lowering agent acting by decreasing cholesterol adsorption in the intestine can be any lipid lowering agent acting by decreasing cholesterol adsorption or uptake in the intestine and preferably is an azetidine such as ezetimibe. According to one embodiment of the eighth aspect of present invention, the lipid lowering agent is ezetimibe.

Suitable dosages of antibody, ezetimibe and fenofibrate etc. and other embodiments of the eighth aspect can be gained from the other aspects of present invention, e.g. the $1^{st}$ to the seventh aspect.

According to one embodiment of the eighth aspect, the antibody or antigen-binding fragment thereof is comprised in a pharmaceutical composition in the form of a liquid or dry formulation and preferably assembled as unit dosage form.

According to another embodiment of the eighth aspect, the lipid lowering agent and/or the fibrate are comprised in pharmaceutical compositions formulated for oral administration and preferably assembled as unit dosage forms for oral administration (e.g. tablet or capsule or over-encapsulated tablet).

Additional embodiments concerning eight aspect of present invention can e.g. be taken from the description of the first, second, third, fourth, fifth and sixth seventh aspect (particularly as regards the individual features of the methods of treatment, the pharmaceutical compositions, the antibodies, the fibrate, the lipid lowering agent, indications, subjects in need, administration and dosage regimens etc) as well as from the general definitions section and the section "preferred antibodies for practicing present invention" or any other section, herein. In particular, further details and embodiments concerning the article of manufacture to be assembled according to the eighth aspect can be found in the description of the seventh aspect hereinabove. All of these embodiments can readiliy be combined with any of the embodiments of the eight aspect described in more detail hereinabove, if not explicitly stated to the contrary.

In a ninth aspect, present invention concerns a unit dosage form comprising an antibody or antigen-binding fragment thereof which specifically binds hPCSK9 (human proprotein convertase subtilisin/kexin type 9) together with a fibrate.

In a tenth aspect, present invention concerns a unit dosage form comprising an antibody or antigen-binding fragment thereof which specifically binds hPCSK9 (human proprotein convertase subtilisin/kexin type 9) for use in a method according to one of the aspects 1 to 3.

Antibodies specifically binding human PCSK9 are known in the art, see e.g. WO 2008/057457, WO 2008/057458, WO 2008/057459, WO 2008/063382, WO 2008/125623, and US 2008/0008697 and particularly US 2010/0166768 A1. The skilled artisan knows how to express and purify such antibodies. Thus, in the context of the ninth and tenth aspect of present invention, any antibody specifically binding human PCSK9 can be used as long as it has the desired effect for practicing present invention. Selecting the right antibody for practicing the different aspects of present invention among the known and readily available ones lies within the skill of the artisan.

Particularly suitable antibodies for practicing all aspects of present invention can be gained from the section "preferred antibodies for practicing present invention" herein.

The fibrate to be used in the context of the eighth aspect of present invention can be any fibrate having cholesterol lowering effect. Examples comprise fenofibrate, bezafibrate, ciprofibrate, clofibrate and gemfibrozil. According to one embodiment of the ninth and tenth aspect of present invention, the fibrate is fenofibrate.

The lipid lowering agent acting by decreasing cholesterol adsorption in the intestine can be any lipid lowering agent acting by decreasing cholesterol adsorption or uptake in the intestine and preferably is an azetidine such as

ezetimibe. According to one embodiment of the ninth and tenth aspect of present invention, the lipid lowering agent is ezetimibe.

Additional embodiments concerning the ninth and tenth aspect of present invention can e.g. be taken from the description of the first, second, third, fourth, fifth, sixth, seventh and eight aspect (the first to third aspect particularly as regards the individual features of the methods of treatment, the pharmaceutical compositions, the antibodies, the fibrate, the lipid lowering agent, indications, subjects in need, administration and dosage regimens etc; the fifth to seventh aspect particularly as regards the unit dosage forms, formulations and pharmaceutical compositions) as well as from the general definitions section and the section "preferred antibodies for practicing present invention" or any other section, herein. All of these embodiments can readiliy be combined with any of the embodiments of the seventh aspect described in more detail hereinabove, if not explicitly stated to the contrary.

Preferred embodiments of the ninth and tenth aspect (e.g. as concerns the container, the antibody, the fibrate, the lipid lowering agent, the label or package insert, the package, the instructions for use or the application device, the unit dosage forms etc.) can be taken from the description of the $1^{st}$, $2^{nd}$, $3^{rd}$, $4^{th}$, $5^{th}$, $6^{th}$ or$7^{th}$ or eighth aspect and from the section "preferred antibodies for practicing present invention" herein below.

Several aspects of the invention can be combined with each other. For example, the method for treating a disease or condition according to the first aspect and the method for treating a disease or condition according to the $2^{nd}$ and $3^{rd}$ aspect can be combined. The skilled artisan will recognize other preferred embodiments resulting of suitable combinations of different aspects and embodiments of present invention.

PREFERRED ANTIBODIES FOR PRACTICING THE PRESENT INVENTION

The following section describes functional and structural features of antibodies and antigen-binding fragments thereof that can be used for practicing all aspects of the present invention. Thus, expressions such as "in preferred embodiments", "in some embodiments", "in another preferred embodiment" and similar expressions should be understood as referring to embodiments of the first aspect of the present invention, the second aspect of the present invention, the third aspect of the present invention, the fourth aspect of the present invention, the fifth aspect of the present invention, the sixth aspect of the present invention, the seventh aspect of the present invention, the eighth aspect of the present invention, the ninth aspect of the present invention or to any of the preferred aspects 1-51 as described in this application.

All antibodies or antigen-binding fragments thereof suitable for practicing the present invention specifically bind hPCSK9. In preferred embodiments of any aspect of the present invention, the antibody or antigen-binding fragment thereof is a recombinant human antibody or fragment thereof. In more specific embodiments, the antibody or antigen-binding fragment thereof is a fully human monoclonal antibody or antigen-binding fragment thereof that specifically binds hPCSK9 and neutralizes PCSK9 activity.

The mAbs usable in the present invention can be full-length (e.g., an IgG1 or IgG4 antibody) or may comprise only an antigen-binding portion (e.g., a Fab, $F(ab')_2$ or scFv fragment), and may be modified to affect functionality, e.g., to eliminate residual effector functions (Reddy et al. (2000) J. Immunol. 164:1925-1933).

In preferred embodiments, the antibodies of present invention are characterized by one or more of the following features upon administration to a subject, preferably a human or non-human mammal and more preferably a human:

- reduction of low density lipoprotein-C (LDL-C) levels of at least about -25% to about -40% relative to a predose level with a sustained reduction over at least a 14 day-period, wherein the sustained reduction is preferably at least -25% and more preferably at least -30% relative to a predose level, particularly if administered in a dose of about 40 to about 60 mg, preferably about 45 to about 55 mg and more preferably about 50 mg in a biweekly administration regime (every other week, E2W),

- reduction of low density lipoprotein- C (LDL-C) of at least about -50% to about -65% relative to a predose level with a sustained reduction over at least a 14 day-period, wherein the sustained reduction is preferably at least -40% and more preferably at least -45% relative to a predose level, particularly if administered in a dose of about 100 mg E2W,

- reduction of low-density lipoprotein-C (LDL-C) of at least about -60 % to at least about -75% [e.g. at least about -60 %, at least about -65%, at least about -70 or at least about -75%] relative to a predose level with a sustained reduction over at least a 14 day-period, wherein the sustained reduction is preferably at least -55% and more preferably at least -60% relative to a predose level, particularly when administered in a dose of about 150 mg E2W,

- reduction of low density lipoprotein-C (LDL-C) of at least about 40% to about 75% relative to a predose level with a sustained reduction over at least a 28 day period, wherein the sustained reduction is preferably at least -35% and more preferably at least -40% relative to a predose level, particularly when administered in a dose of about 200 mg E4W,

- reduction of low density lipoprotein-C (LDL-C) of at least about -50 % to about -75% relative to a predose level with a sustained reduction over at least a 28 day-period, wherein the sustained reduction is preferably at least -40% and more preferably at least -45% relative to a predose level, particularly when administered in a dose of about 300 mg E4W,

- increase of serum HDL cholesterol levels of at least 2%, at least 2.5%, at least, 3%, at least 3.5%, at least 4%, at least 4.5%, at least 5% or at least 5.5% relative to a predose level, particularly when administered in a dose of about 150 mg E2W,

- little or no detectable effect on troponin levels,

- Increase of one or more of: Total-Cholesterol levels, ApoB levels, non HDL-C levels, Apo-B/ApoA-1 ratio,

The antibodies according to present invention exhibit the above properties preferably if administered in combination with an HMG-CoA reductase inhibitor treatment. Preferred embodiments of HMG-CoA reductase inhibitors to be used in conjunction with the antibody of the invention and dosage and administration regimes thereof can be found throughout the specification, particularly as described in the aspects related to medical uses and methods of treatment.

According to another preferred embodiment of the antibodies and antigen-binding fragments thereof of present invention, the antibody or antigen binding fragment thereof has one or more of the following characteristics:

- The antibody or the antigen-binding fragment comprises the heavy and light chain CDRs of a HCVR/LCVR amino acid sequence pair as shown in SEQ ID NOs: 90/92.

- The antibody or antigen-binding fragment thereof comprises a HCVR/LCVR amino acid sequence pair as shown in SEQ ID NOs: 90/92.

- The antibody or antigen-binding fragment thereof competes for binding to hPCSK9 with an antibody or antigen-binding fragment comprising a HCVR/LCVR amino acid sequence pair as shown in SEQ ID NOs: 90/92.

According to another preferred embodiment of the antibodies and antigen-binding fragments thereof of present invention, the antibody or antigen binding fragment thereof has one or more of the following characteristics:

- overcomes statin resistance in mammals, especially in rodents such as hamster

- increase in LDLR expression in mammals, particularly in rodents such as hamster

- decreases serum LDL-C in rodents such as hamster

- synergistic decrease of LDL-C in conjunction with HMG-CoA reductase inhibitor administration, particularly in rodents such as hamster, wherein the HMG-CoA reductase inhibitor is preferably Atorvastatin.

In preferred embodiments, the antibody or the antigen-binding fragment thereof is characterized by one or more of the following:

(i) capable of reducing serum total cholesterol at least about 25 to about 35% and sustaining the reduction over at least a 24 day period relative to a predose level, preferably the reduction in serum total cholesterol is at least about 30-40%;

(ii) capable of reducing serum LDL cholesterol at least about 65-80% and sustaining the reduction over at least a 24 day period relative to a predose level;

(iii) capable of reducing serum triglyceride at least about 25-40% relative to predose level;

(iv) achieves one or more of (i)-(iii) without reducing serum HDL cholesterol or reducing serum HDL cholesterol no more than 5% relative to predose level;

(v) achieves one or more of (i)-(iii) with little or no measurable effect on liver function, as determined by ALT and AST measurements.

In preferred embodiments, the antibody or the antigen-binding fragment thereof is characterized by one or more of the following:

(i) capable of reducing serum LDL cholesterol at least about 40-70% and sustaining the reduction over at least a 60 or 90 day period relative to a predose level;

(ii) capable of reducing serum triglyceride at least about 25-40% relative to predose level;

(iii) does not reduce serum HDL cholesterol or reduces serum HDL cholesterol no more than 5% relative to predose level.

In one embodiment, the antibody or the antigen-binding fragment thereof is characterized as binding an epitope comprising amino acid residue 238 of hPCSK9 (SEQ ID NO: 755). In a more specific embodiment, the antibody or antigen-binding fragment binds an epitope comprising one or more of amino acid residues at positions 238, 153, 159 and 343 of hPCSK9 (SEQ ID NO: 755). In a more specific embodiment, the antibody or fragment thereof is characterized as binding an epitope which does not comprise an amino acid residue at positions 192, 194, 197 and/or 237 of SEQ ID NO: 755.

In one embodiment, the antibody or the antigen-binding fragment thereof is characterized as binding an epitope comprising amino acid residue 366 of hPCSK9 (SEQ ID NO: 755). In a more specific embodiment, the antibody or antigen-binding fragment binds an epitope comprising one or more of amino acid residues at positions 147, 366 and 380 of hPCSK9 (SEQ ID NO: 755). In a more specific embodiment, the antibody or antigen-binding fragment of an antibody is characterized as binding an epitope which does not comprise an amino acid residue at position 215 or 238 of SEQ ID NO: 755.

In one embodiment, the antibody or the antigen-binding fragment thereof comprises a heavy chain variable region (HCVR) selected from the group consisting of SEQ ID NO:2, 18, 22, 26, 42, 46, 50, 66, 70, 74, 90, 94, 98, 114, 118, 122, 138, 142, 146, 162, 166, 170, 186, 190, 194, 210, 214, 218, 234, 238, 242, 258, 262, 266, 282, 286, 290, 306, 310, 314, 330, 334, 338, 354, 358, 362, 378, 382, 386, 402, 406, 410, 426, 430, 434, 450, 454, 458, 474, 478, 482, 498, 502, 506, 522, 526, 530, 546, 550, 554, 570, 574, 578, 594, 598, 602, 618, 622, 626, 642, 646, 650, 666, 670, 674, 690, 694, 698, 714, 718, 722, 738 and 742, or a substantially similar sequence thereof having at least 90%, at least 95%, at least 98% or at least 99% sequence identity. In one embodiment, the HCVR comprises an amino acid sequence selected from the group consisting of SEQ ID NO: 50, 66, 70, 74, 90, 94, 122, 138, 142, 218, 234, 238, 242, 258, 262, 314, 330 and 334. In a more specific embodiment, the HCVR comprises SEQ ID NO: 90 or 218.

In one embodiment, the antibody or the antigen-binding fragment thereof further comprises a light chain variable region (LCVR) selected from the group consisting of SEQ ID NO: 10, 20, 24, 34, 44, 48, 58, 68, 72, 82, 92, 96, 106, 116, 120, 130, 140, 144, 154, 164, 168, 178, 188, 192, 202, 212, 216, 226, 236, 240, 250, 260, 264, 274, 284, 288, 298, 308, 312, 322, 332, 336, 346, 356, 360, 370, 380, 384, 394, 404, 408, 418, 428, 432, 442, 452, 456, 466, 476, 480, 490, 500, 504, 514, 524, 528, 538, 548, 552, 562, 572, 576, 586, 596, 600, 610, 620, 624, 634, 644, 648, 658, 668, 672, 682, 692, 696, 706, 716, 720, 730, 740 and 744, or a substantially similar sequence thereof having at least 90%, at least 95%, at least 98% or at least 99% sequence identity. In one embodiment, the LCVR comprises an amino acid sequence selected from the group consisting of SEQ ID NO: 58, 68, 72, 82, 92, 96, 130, 140, 144, 226, 236, 240, 250, 260, 264, 322, 332 and 336. In a more specific embodiment, the LCVR comprises SEQ ID NO: 92 or 226.

In specific embodiments, the antibody or the antigen-binding fragment thereof comprises a HCVR and LCVR (HCVR/LCVR) sequence pair selected from the group consisting of SEQ ID NO: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744. In one embodiment, the HCVR and LCVR sequence pair comprises one of SEQ ID NO: 50/58, 66/68, 70/72, 74/82, 90/92, 94/96, 122/130, 138/140, 142/144, 218/226, 234/236, 238/240, 242/250, 258/260, 262/264, 314/322, 330/332 and 334/336. In preferred embodiments, the antibody or antigen-binding fragment thereof comprises an HCVR amino acid sequence as shown in SEQ ID NO: 90 and an LCVR amino acid sequence as shown in SEQ ID NO: 92. In another preferred embodiment, the antibody or antigen-binding fragment thereof comprises an HCVR amino acid sequence as shown in SEQ ID NO: 218 and an LCVR amino acid sequence as shown in SEQ ID NO: 226.

In preferred embodiments, the antibody or the antigen-binding fragment thereof comprises a heavy chain CDR3 (HCDR3) domain selected from the group consisting of SEQ ID NO: 8, 32, 56, 80, 104, 128, 152, 176, 200, 224, 248,

272, 296, 320, 344, 368, 392, 416, 440, 464, 488, 512, 536, 560, 584, 608, 632, 656, 680, 704 and 728, or a substantially similar sequence thereof having at least 90%, at least 95%, at least 98% or at least 99% sequence identity; and a light chain CDR3 (LCDR3) domain selected from the group consisting of SEQ ID NO: 16, 40, 64, 88, 112, 136, 160, 184, 208, 232, 256, 280, 304, 328, 352, 376, 400, 424, 448, 472, 496, 520, 544, 568, 592, 616, 640, 664, 688, 712 and 736, or substantially similar sequences thereof having at least 90%, at least 95%, at least 98% or at least 99% sequence identity. In one embodiment, the HCDR3/LCDR3 sequence pair is selected from the group consisting of SEQ ID NO: 56/64, 80/88, 128/136, 224/232, 248/256 and 320/328. In more preferred embodiments, the antibody or the antigen-binding fragment thereof comprises a HCDR3 domain as shown in SEQ ID NO: 80 and a LCDR3 domain as shown in SEQ ID NO: 88. In another preferred embodiment, the antibody or the antigen-binding fragment thereof comprises a HCDR3 domain as shown in SEQ ID NO: 224 and a LCDR3 domain as shown in SEQ ID NO: 232.

In a further embodiment, the antibody or the antigen-binding fragment thereof further comprises a heavy chain CDR1 (HCDR1) domain selected from the group consisting of SEQ ID NO: 4, 28, 52, 76, 100, 124, 148, 172, 196, 220, 244, 268, 292, 316, 340, 364, 388, 412, 436, 460, 484, 508, 532, 556, 580, 604, 628, 652, 676, 700 and 724, or a substantially similar sequence thereof having at least 90%, at least 95%, at least 98% or at least 99% sequence identity; a heavy chain CDR2 (HCDR2) domain selected from the group consisting of SEQ ID NO: 6, 30, 54, 78, 102, 126, 150, 174, 198, 222, 246, 270, 294, 318, 342, 366, 390, 414, 438, 462, 486, 510, 534, 558, 582, 606, 630, 654, 678, 702 and 726, or a substantially similar sequence thereof having at least 90%, at least 95%, at least 98% or at least 99% sequence identity; a light chain CDR1 (LCDR1) domain selected from the group consisting of SEQ ID NO: 12, 36, 60, 84, 108, 132, 156, 180, 204, 228, 252, 276, 300, 324, 348, 372, 396, 420, 444, 468, 492, 516, 540, 564, 588, 612, 636, 660, 684, 708 and 732, or a substantially similar sequence thereof having at least 90%, at least 95%, at least 98% or at least 99% sequence identity; and a light chain CDR2 (LCDR2) domain selected from the group consisting of SEQ ID NO: 14, 38, 62, 86, 110, 134, 158, 182, 206, 230, 254, 278, 302, 326, 350, 374, 398, 422, 446, 470, 494, 518, 542, 566, 590, 614, 638, 662, 686, 710 and 734, or a substantially similar sequence thereof having at least 90%, at least 95%, at least 98% or at least 99% sequence identity. In one embodiment, the heavy and light chain CDR sequences comprise a sequence selected from the group consisting of SEQ ID NO: 52, 54, 56, 60, 62, 64; 76, 78, 80, 84, 86, 88; 124, 126, 128, 132, 134, 136; 220, 222, 224, 228, 230, 232; 244, 246, 248, 252, 254, 256; and 316, 318, 320, 324, 326, 328. In more specific embodiments, the CDR sequences comprise SEQ ID NO: 76, 78, 80, 84, 86, 88; or 220, 222, 224, 228, 230, 232. In preferred embodiments, the antibody or antigen-binding fragment thereof comprises heavy and light chain CDR amino acid sequences as shown in SEQ ID NOs: 76, 78, 80, 84, 86 and 88. In another preferred embodiment, the antibody or antigen-binding fragment thereof comprises heavy and light chain CDR amino acid sequences as shown in SEQ ID NOs: 220, 222, 224, 228, 230 and 232.

In a related embodiment, the antibody or antigen-binding fragment thereof comprises heavy and light chain CDR domains contained within heavy and light chain sequence pairs selected from the group consisting of SEQ ID NO: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744. In one embodiment, the CDR sequences are contained within HCVR and LCVR selected from the amino acid sequence pairs of SEQ ID NO: 50/58, 66/68, 70/72, 74/82, 90/92, 94/96, 122/130, 138/140, 142/144, 218/226, 234/236, 238/240, 242/250, 258/260, 262/264, 314/322, 330/332 and 334/336. In more specific embodiments, the CDR sequences are comprised within HCVR/LCVR sequences selected from SEQ ID NO: 90/92 or 218/226. In preferred embodiments, the antibody or the antigen-binding fragment thereof comprises the heavy and light chain CDRs of an HCVR/LCVR amino acid sequence pair as shown in SEQ ID NOs: 90/92. In another preferred embodiment, the antibody or the antigen-binding fragment thereof comprises the heavy and light chain CDRs of an HCVR/LCVR amino acid sequence pair as shown in SEQ ID NOs: 218/226.

In one specific embodiment, the antibody or the antigen-binding fragment thereof comprises the heavy chain variable region (HCVR), of SEQ ID NO: 90 or a substantially similar sequence thereof having at least 90%, at least 95%, at least 98% or at least 99% sequence identity..

In one specific embodiment, the antibody or the antigen-binding fragment thereof further comprises the light chain variable region (LCVR) of SEQ Id NO: 92 or a substantially similar sequence thereof having at least 90%, at least 95%, at least 98% or at least 99% sequence identity.

In specific embodiments, the antibody or the antigen-binding fragment thereof comprises HCVR amino acid sequence as shown in SEQ ID NO: 90 and an LCVR amino acid sequence as shown in SEQ ID NO: 92.

In specific embodiments, the antibody or the antigen-binding fragment thereof comprises a heavy chain CDR3 (HCDR3) domain of SEQ ID NO: 80 or a substantially similar sequence thereof having at least 90%, at least 95%, at

least 98% or at least 99% sequence identity; and/or a light chain CDR3 (LCDR3) domain of SEQ ID NO: 88, or substantially similar sequences thereof having at least 90%, at least 95%, at least 98% or at least 99% sequence identity. In one embodiment, the HCDR3/LCDR3 sequence pair is SEQ ID NO: 80/88 . In more preferred embodiments, the antibody or the antigen-binding fragment thereof comprises a HCDR3 domain as shown in SEQ ID NO: 80 and a LCDR3 domain as shown in SEQ ID NO: 88.

In a further specific embodiment, the antibody or the antigen-binding fragment thereof further comprises the heavy chain CDR1 (HCDR1) domain of SEQ ID NO: 76, or a substantially similar sequence thereof having at least 90%, at least 95%, at least 98% or at least 99% sequence identity; and/or the heavy chain CDR2 (HCDR2) domain of SEQ ID NO: 78 or a substantially similar sequence thereof having at least 90%, at least 95%, at least 98% or at least 99% sequence identity; and/or a light chain CDR1 (LCDR1) domain of SEQ ID NO: 84or a substantially similar sequence thereof having at least 90%, at least 95%, at least 98% or at least 99% sequence identity; and/or a light chain CDR2 (LCDR2) domain of SEQ ID NO: 86, or a substantially similar sequence thereof having at least 90%, at least 95%, at least 98% or at least 99% sequence identity. In one embodiment, the heavy and light chain CDR sequences comprise a sequence selected from the group consisting of SEQ ID NO: 76, 78, 80, 84, 86, 88. In preferred embodiments, the antibody or antigen-binding fragment thereof comprises heavy and light chain CDR amino acid sequences as shown in SEQ ID NOs: 76, 78, 80, 84, 86 and 88.

In another specific embodiment, the antibody or antigen-binding fragment thereof comprises heavy and light chain CDR domains contained within the heavy and light chain sequence pair of SEQ ID NO: 90/92.

A particularly preferred embodiment concerns an antibody comprising HCVR/LCVR sequences SEQ ID Nos: 90/92 and/or CDR sequences SEQ ID Nos: 76, 78, 80 and/or CDR sequences SEQ ID NOs: 84, 86, 88. Another particularly preferred embodiment concerns an antibody comprising the HCVR/LCVR sequences SEQ ID Nos: 90/92 and the CDR sequences SEQ ID Nos: 76, 78, 80 and the CDR sequences SEQ ID Nos: 84, 86, 88 ("316P").

In one embodiment, the antibody or antigen-binding fragment thereof comprises an HCVR encoded by a nucleic acid sequence selected from the group consisting of SEQ ID NO: 1, 17, 21, 25, 41, 45, 49, 65, 69, 73, 89, 93, 97, 113, 117, 121, 137, 141, 145, 161, 165, 169, 185, 189, 193, 209, 213, 217, 233, 237, 241, 257, 261, 265, 281, 285, 289, 305, 309, 313, 329, 333, 337, 353, 357, 361, 377, 381, 385, 401, 405, 409, 425, 429, 433, 449, 453, 457, 473, 477, 481, 497, 501, 505, 521, 525, 529, 545, 549, 553, 569, 573, 577, 593, 597, 601, 617, 621, 625, 641, 645, 649, 665, 669, 673, 689, 693, 697, 713, 717, 721, 737 and 741, or a substantially identical sequence having at least 90%, at least 95%, at least 98%, or at least 99% sequence identity thereof. In one embodiment, the HCVR is encoded by a nucleic acid sequence selected from the group consisting of SEQ ID NO: 49, 65, 69, 73, 89, 93, 121, 137, 141, 217, 233, 237, 241, 257, 261, 313, 329 and 333. In more specific embodiments, the HCVR is encoded by a nucleic acid sequence selected from the group consisting of SEQ ID NO: 89 and 217.

In one embodiment, the antibody or fragment thereof further comprises an LCVR encoded by a nucleic acid sequence selected from the group consisting of SEQ ID NO: 9, 19, 23, 33, 43, 47, 57, 67, 71, 81, 91, 95, 105, 115, 119, 129, 139, 143, 153, 163, 167, 177, 187, 191, 201, 211, 215, 225, 235, 239, 249, 259, 263, 273, 283, 287, 297, 307, 311, 321, 331, 335, 345, 355, 359, 369, 379, 383, 393, 403, 407, 417, 427, 431, 441, 451, 455, 465, 475, 479, 489, 499, 503, 513, 523, 527, 537, 547, 551, 561, 571, 575, 585, 595, 599, 609, 619, 623, 633, 643, 647, 657, 667, 671, 681, 691, 695, 705, 715, 719, 729, 739 and 743, or a substantially identical sequence having at least 90%, at least 95%, at least 98%, or at least 99% sequence identity thereof. In one embodiment, the LCVR is encoded by a nucleic acid sequence selected from the group consisting of SEQ ID NO: 57, 67, 71, 81, 91, 95, 129, 139, 143, 225, 235, 239, 249, 259, 263, 321, 331 and 335. In more specific embodiments, the LCVR is encoded by a nucleic acid sequence selected from the group consisting of SEQ ID NO: 91 and 225.

In one embodiment, the antibody or antigen-binding fragment thereof comprises an HCDR3 domain encoded by a nucleotide sequence selected from the group consisting of SEQ ID NO: 7, 31, 55, 79, 103, 127, 151, 175, 199, 223, 247, 271, 295, 319, 343, 367, 391, 415, 439, 463, 487, 511, 535, 559, 583, 607, 631, 655, 679, 703 and 727, or a substantially identical sequence having at least 90%, at least 95%, at least 98%, or at least 99% sequence identity thereof; and a LCDR3 domain encoded by a nucleotide sequence selected from the group consisting of SEQ ID NO: 15, 39, 63, 87, 111, 135, 159, 183, 207, 231, 255, 279, 303, 327, 351, 375, 399, 423, 447, 471, 495, 519, 543, 567, 591, 615, 639, 663, 687, 711 and 735, or a substantially identical sequence having at least 90%, at least 95%, at least 98%, or at least 99% sequence identity thereof. In one embodiment, the HCDR3 and LCDR3 comprise a sequence pair encoded by the nucleic acid sequence of SEQ ID NO: 55/63, 79/87, 127/135, 223/231, 247/255 and 319/327, respectively. In more specific embodiments, the HCDR3 and LCDR3 comprise a sequence pair encoded by the nucleic acid sequence of SEQ ID NO: 79/87 and 223/231.

In a further embodiment, the antibody or antigen-binding fragment thereof further comprises: an HCDR1 domain encoded by a nucleotide sequence selected from the group consisting of SEQ ID NO: 3, 27, 51, 75, 99, 123, 147, 171, 195, 219, 243, 267, 291, 315, 339, 363, 387, 411, 435, 459, 483, 507, 531, 555, 579, 603, 627, 651, 675, 699 and 723, or a substantially identical sequence having at least 90%, at least 95%, at least 98%, or at least 99% sequence identity thereof; an HCDR2 domain encoded by a nucleotide sequence selected from the group consisting of SEQ ID NO:5, 29,

53, 77, 101, 125, 149, 173, 197, 221, 245, 269, 293, 317, 341, 365, 389, 413, 437, 461, 485, 509, 533, 557, 581, 605, 629, 653, 677, 701 and 725, or a substantially identical sequence having at least 90%, at least 95%, at least 98%, or at least 99% sequence identity thereof; an LCDR1 domain encoded by a nucleotide sequence selected from the group consisting of SEQ ID NO: 11, 35, 59, 83, 107, 131, 155, 179, 203, 227, 251, 275, 299, 323, 347, 371, 395, 419, 443, 467, 491, 515, 539, 563, 587, 611, 635, 659, 683, 707 and 731, or a substantially identical sequence having at least 90%, at least 95%, at least 98%, or at least 99% sequence identity thereof; and an LCDR2 domain encoded by a nucleotide sequence selected from the group consisting of SEQ ID NO: 13, 37, 61, 85, 109, 133, 157, 181, 205, 229, 253, 277, 301, 325, 349, 373, 397, 421, 445, 469, 493, 517, 541, 565, 589, 613, 637, 661, 685, 709 and 733, or a substantially identical sequence having at least 90%, at least 95%, at least 98%, or at least 99% sequence identity thereof. In one embodiment, the heavy and light chain CDR sequences are encoded by the nucleic acid sequences of SEQ ID NO: 51, 53, 55, 59, 61, 63; 75, 77, 79, 83, 85, 87; 123, 125, 127, 131, 133, 135; 219, 221, 223, 227, 229, 231; 243, 245, 247, 251, 253, 255; and 315, 317, 319, 323, 325, 327. In more specific embodiments, the heavy and light chain CDR sequences are encoded by the nucleic acid sequences of SEQ ID NO: 75, 77, 79, 83, 85, 87; and 219, 221, 223, 227, 229, 231.

In a further embodiment, the antibody or antigen-binding fragment thereof comprises an HCDR3 and an LCDR3, wherein HCDR3 comprises an amino acid sequence of the formula $X^1$ - $X^2$ - $X^3$ - $X^4$ - $X^5$ - $X^6$- $X^7$ - $X^8$ - $X^9$ - $X^{10}$ - $X^{11}$ - $X^{12}$ - $X^{13}$ - $X^{14}$ - $X^{15}$ - $X^{16}$ - $X^{17}$ - $X^{18}$ - $X^{19}$ - $X^{20}$ (SEQ ID NO: 747), wherein $X^1$ is Ala, $X^2$ is Arg or Lys, $X^3$ is Asp, $X^4$ is Ser or Ile, $X^5$ is Asn or Val, $X^6$ is Leu or Trp, $X^7$ is Gly or Met, $X^8$ is Asn or Val, $X^9$ is Phe or Tyr, $X^{10}$ is Asp, $X^{11}$ is Leu or Met, $X^{12}$ is Asp or absent, $X^{13}$ is Tyr or absent, $X^{14}$ is Tyr or absent, $X^{15}$ is Tyr or absent, $X^{16}$ is Tyr or absent, $X^{17}$ is Gly or absent, $X^{18}$ is Met or absent, $X^{19}$ is Asp or absent, and $X^{20}$ is Val or absent; and LCDR3 comprises an amino acid sequence of the formula $X^1$ - $X^2$ - $X^3$ - $X^4$ - $X^5$ - $X^6$ - $X^7$ - $X^8$ - $X^9$ (SEQ ID NO:750), wherein $X^1$ is Gln or Met, $X^2$ is Gln, $X^3$ is Tyr or Thr, $X^4$ is Tyr or Leu, $X^5$ is Thr or Gln, $X^6$ is Thr, $X^7$ is Pro, $X^8$ is Tyr or Leu, and $X^9$ is Thr.

In a further embodiment, the antibody or antigen-binding fragment thereof further comprises an HCDR1 sequence of the formula $X^1$ - $X^2$ - $X^3$ - $X^4$ - $X^5$ - $X^6$ - $X^7$ - $X^8$ (SEQ ID NO: 745), wherein $X^1$ is Gly, $X^2$ is Phe, $X^3$ is Thr, $X^4$ is Phe, $X^5$ is Ser or Asn, $X^6$ is Ser or Asn, $X^7$ is Tyr or His, and $X^8$ is Ala or Trp; a HCDR2 sequence of the formula $X^1$ - $X^2$ - $X^3$ - $X^4$ - $X^5$ - $X^6$ - $X^7$ - $X^8$ (SEQ ID NO: 746), wherein $X^1$ is Ile, $X^2$ is Ser or Asn, $X^3$ is Gly or Gln, $X^4$ is Asp or Ser, $X^5$ is Gly, $X^6$ is Ser or Gly, $X^7$ is Thr or Glu, and $X^8$ is Thr or Lys; a LCDR1 sequence of the formula $X^1$ - $X^2$ - $X^3$ - $X^4$ - $X^5$ - $X^6$ - $X^7$ - $X^8$ - $X^9$ - $X^{10}$ - $X^{11}$ - $X^{12}$ (SEQ ID NO: 748) wherein $X^1$ is Gln, $X^2$ is Ser, $X^3$ is Val or Leu, $X^4$ is Leu, $X^5$ is His or Tyr, $X^6$ is Arg or Ser, $X^7$ is Ser or Asn, $X^8$ is Asn or Gly, $X^9$ is Asn, $X^{10}$ is Arg or Asn, $X^{11}$ is Asn or Tyr, and $X^{12}$ is Phe or absent; an LCDR2 sequence of the formula $X^1$ - $X^2$ - $X^3$ (SEQ ID NO: 749) wherein $X^1$ is Trp or Leu, $X^2$ is Ala or Gly, and $X^3$ is Ser.

In a further embodiment, the antibody or antigen-binding fragment thereof is a human anti-PCSK9 antibody or antigen-binding fragment thereof comprising a heavy chain variable region (HCVR) encoded by nucleotide sequence segments derived from $V_H$, $D_H$ and $J_H$ germline sequences, and a light chain variable region (LCVR) encoded by nucleotide sequence segments derived from $V_K$ and $J_K$ germline sequences, wherein the germline sequences are (a) $V_H$ gene segment 3-23, $D_H$ gene segment 7-27, $J_H$ gene segment 2, $V_K$ gene segment 4-1 and $J_K$ gene segment 2; or (b) $V_H$ gene segment 3-7, $D_H$ gene segment 2-8, $J_H$ gene segment 6, $V_K$ gene segment 2-28 and $J_K$ gene segment 4.

In preferred embodiments, the antibody or antigen-binding fragment thereof binds to the same epitope on hPCSK9 as an antibody comprising heavy and light chain CDR amino acid sequences as shown in SEQ ID NOs: 76, 78, 80, 84, 86, and 88 or as shown in SEQ ID NOs: 220, 222, 224, 228, 230 and 232.

In preferred embodiments, the antibody or antigen-binding fragment thereof competes for binding to hPCSK9 with an antibody comprising heavy and light chain CDR amino acid sequences as shown in SEQ ID NOs: 76, 78, 80, 84, 86, and 88 or as shown in SEQ ID NOs: 220, 222, 224, 228, 230 and 232.

Two exemplary anti-PCSK9 antibodies and the antigen-binding fragments derived therefrom that are suitable for all the different aspects and embodiments of present invention are antibodies 316P and 300N.

Antibody 316P:

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

Antibody 300N:

Antibody 300N is a fully human antibody comprising a HCVR as shown in SEQ ID NO: 218 and LCVR as shown in SEQ ID NO: 226 of the sequence listing. The CDR sequences are shown in SEQ ID NOs: 220, 222, and 224 (CDR1, CDR2, CDR3 of the heavy chain) as well as in SEQ ID NOs: 228, 230, and 232 (CDR1, CDR2, CDR3 of the light chain).

The invention encompasses anti-PCSK9 antibodies having a modified glycosylation pattern. In some applications, modification to remove undesirable glycosylation sites may be useful, or e.g., removal of a fucose moiety to increase antibody dependent cellular cytotoxicity (ADCC) function (see Shield et al. (2002) JBC 277:26733). In other applications, modification of galactosylation can be made in order to modify complement dependent cytotoxicity (CDC).

Some preferred sequences related to preferred antibodies for practicing present invention:

SEQ ID NO: 76: Gly Phe Thr Phe Asn Asn Tyr Ala

SEQ ID NO: 78: Ile Ser Gly Ser Gly Gly Thr Thr

SEQ ID NO: 80: Ala Lys Asp Ser Asn Trp Gly Asn Phe Asp Leu

SEQ ID NO: 84: Gln Ser Val Leu Tyr Arg Ser Asn Asn Arg Asn Phe

SEQ ID NO: 86: Trp Ala Ser

SEQ ID NO: 88: Gln Gln Tyr Tyr Thr Thr Pro Tyr Thr

SEQ ID NO: 90:

```
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Phe Asn Asn Tyr
            20                  25                  30
Ala Met Asn Trp Val Arg Gln Ala Pro Gly Lys Gly Leu Asp Trp Val
        35                  40                  45
Ser Thr Ile Ser Gly Ser Gly Gly Thr Thr Asn Tyr Ala Asp Ser Val
    50                  55                  60
Lys Gly Arg Phe Ile Ile Ser Arg Asp Ser Ser Lys His Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Ser Asn Trp Gly Asn Phe Asp Leu Trp Gly Arg Gly Thr
            100                 105                 110
Leu Val Thr Val Ser Ser
    115
```

SEQ ID NO:92:

```
Asp Ile Val Met Thr Gln Ser Pro Asp Ser Leu Ala Val Ser Leu Gly
 1               5                  10                  15
Glu Arg Ala Thr Ile Asn Cys Lys Ser Ser Gln Ser Val Leu Tyr Arg
            20                  25                  30
Ser Asn Asn Arg Asn Phe Leu Gly Trp Tyr Gln Gln Lys Pro Gly Gln
        35                  40                  45
Pro Pro Asn Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Thr Thr Pro Tyr Thr Phe Gly Gln Gly Thr Lys Leu Glu Ile
            100                 105                 110
Lys
```

SEQ ID NO: 755 (hPCSK9):

```
Met Gly Thr Val Ser Ser Arg Arg Ser Trp Trp Pro Leu Pro Leu Leu
 1               5                  10                  15
Leu Leu Leu Leu Leu Leu Leu Gly Pro Ala Gly Ala Arg Ala Gln Glu
            20                  25                  30
Asp Glu Asp Gly Asp Tyr Glu Glu Leu Val Leu Ala Leu Arg Ser Glu
        35                  40                  45
Glu Asp Gly Leu Ala Glu Ala Pro Glu His Gly Thr Thr Ala Thr Phe
```

50 55 60

His Arg Cys Ala Lys Asp Pro Trp Arg Leu Pro Gly Thr Tyr Val Val
65 70 75 80

Val Leu Lys Glu Glu Thr His Leu Ser Gln Ser Glu Arg Thr Ala Arg
85 90 95

Arg Leu Gln Ala Gln Ala Ala Arg Arg Gly Tyr Leu Thr Lys Ile Leu
100 105 110

His Val Phe His Gly Leu Leu Pro Gly Phe Leu Val Lys Met Ser Gly
115 120 125

Asp Leu Leu Glu Leu Ala Leu Lys Leu Pro His Val Asp Tyr Ile Glu
130 135 140

Glu Asp Ser Ser Val Phe Ala Gln Ser Ile Pro Trp Asn Leu Glu Arg
145 150 155 160

Ile Thr Pro Pro Arg Tyr Arg Ala Asp Glu Tyr Gln Pro Pro Asp Gly
165 170 175

Gly Ser Leu Val Glu Val Tyr Leu Leu Asp Thr Ser Ile Gln Ser Asp
180 185 190

His Arg Glu Ile Glu Gly Arg Val Met Val Thr Asp Phe Glu Asn Val
195 200 205

Pro Glu Glu Asp Gly Thr Arg Phe His Arg Gln Ala Ser Lys Cys Asp
210 215 220

Ser His Gly Thr His Leu Ala Gly Val Val Ser Gly Arg Asp Ala Gly
225 230 235 240

Val Ala Lys Gly Ala Ser Met Arg Ser Leu Arg Val Leu Asn Cys Gln
245 250 255

Gly Lys Gly Thr Val Ser Gly Thr Leu Ile Gly Leu Glu Phe Ile Arg
260 265 270

Lys Ser Gln Leu Val Gln Pro Val Gly Pro Leu Val Val Leu Leu Pro
275 280 285

Leu Ala Gly Gly Tyr Ser Arg Val Leu Asn Ala Ala Cys Gln Arg Leu
290 295 300

Ala Arg Ala Gly Val Val Leu Val Thr Ala Ala Gly Asn Phe Arg Asp
305 310 315 320

Asp Ala Cys Leu Tyr Ser Pro Ala Ser Ala Pro Glu Val Ile Thr Val

```
                325                 330                 335
Gly Ala Thr Asn Ala Gln Asp Gln Pro Val Thr Leu Gly Thr Leu Gly
            340                 345                 350
Thr Asn Phe Gly Arg Cys Val Asp Leu Phe Ala Pro Gly Glu Asp Ile
        355                 360                 365
Ile Gly Ala Ser Ser Asp Cys Ser Thr Cys Phe Val Ser Gln Ser Gly
    370                 375                 380
Thr Ser Gln Ala Ala Ala His Val Ala Gly Ile Ala Ala Met Met Leu
385                 390                 395                 400
Ser Ala Glu Pro Glu Leu Thr Leu Ala Glu Leu Arg Gln Arg Leu Ile
                405                 410                 415
His Phe Ser Ala Lys Asp Val Ile Asn Glu Ala Trp Phe Pro Glu Asp
            420                 425                 430
Gln Arg Val Leu Thr Pro Asn Leu Val Ala Ala Leu Pro Pro Ser Thr
        435                 440                 445
His Gly Ala Gly Trp Gln Leu Phe Cys Arg Thr Val Trp Ser Ala His
    450                 455                 460
Ser Gly Pro Thr Arg Met Ala Thr Ala Val Ala Arg Cys Ala Pro Asp
465                 470                 475                 480
Glu Glu Leu Leu Ser Cys Ser Ser Phe Ser Arg Ser Gly Lys Arg Arg
                485                 490                 495
Gly Glu Arg Met Glu Ala Gln Gly Gly Lys Leu Val Cys Arg Ala His
            500                 505                 510
Asn Ala Phe Gly Gly Glu Gly Val Tyr Ala Ile Ala Arg Cys Cys Leu
        515                 520                 525
Leu Pro Gln Ala Asn Cys Ser Val His Thr Ala Pro Pro Ala Glu Ala
    530                 535                 540
Ser Met Gly Thr Arg Val His Cys His Gln Gln Gly His Val Leu Thr
545                 550                 555                 560
Gly Cys Ser Ser His Trp Glu Val Glu Asp Leu Gly Thr His Lys Pro
                565                 570                 575
Pro Val Leu Arg Pro Arg Gly Gln Pro Asn Gln Cys Val Gly His Arg
            580                 585                 590
Glu Ala Ser Ile His Ala Ser Cys Cys His Ala Pro Gly Leu Glu Cys
```

```
        595                 600 605
Lys Val Lys Glu His Gly Ile Pro Ala Pro Gln Glu Gln Val Thr Val
    610                 615                 620
Ala Cys Glu Glu Gly Trp Thr Leu Thr Gly Cys Ser Ala Leu Pro Gly
625                 630                 635                 640
Thr Ser His Val Leu Gly Ala Tyr Ala Val Asp Asn Thr Cys Val Val
                645                 650                 655
Arg Ser Arg Asp Val Ser Thr Thr Gly Ser Thr Ser Glu Gly Ala Val
            660                 665                 670
Thr Ala Val Ala Ile Cys Cys Arg Ser Arg His Leu Ala Gln Ala Ser
        675                 680                 685
Gln Glu Leu Gln
    690
```

Preparation of Human Antibodies

Methods for generating human antibodies in transgenic mice are known (see for example, US 6,596,541, Regeneron Pharmaceuticals, VELOCIMMUNE™). The VELOCIMMUNE™ technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody. In specific embodiment, the cell is a CHO cell.

Antibodies may be therapeutically useful in blocking a ligand-receptor interaction or inhibiting receptor component interaction, rather than by killing cells through fixation of complement and participation in complement-dependent cytotoxicity (CDC), or killing cells through antibody-dependent cell-mediated cytotoxicity (ADCC). The constant region of an antibody is thus important in the ability of an antibody to fix complement and mediate cell-dependent cytotoxicity. Thus, the isotype of an antibody may be selected on the basis of whether it is desirable for the antibody to mediate cytotoxicity. Human antibodies can exist in two forms that are associated with hinge heterogeneity. In one form, an antibody molecule comprises a stable four-chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, CH2 or CH3 region which may be desirable, for example, in production, to improve the yield of the desired antibody form.

Generally, a VELOCIMMUNE™ mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant

region. As described below, the antibodies are characterized and selected for desirable characteristics, including affinity, selectivity, epitope, etc. The mouse constant regions are replaced with a desired human constant region to generate the fully human antibody of the invention, for example wild-type or modified IgG1 or IgG4 (for example, SEQ ID NO: 751, 752, 753). While the constant region selected may vary according to specific use, high affinity antigen-binding and target specificity characteristics reside in the variable region.

Epitope Mapping and Related Technologies

To screen for antibodies that bind to a particular epitope (e.g., those which block binding of IgE to its high affinity receptor), a routine cross-blocking assay such as that described Antibodies, Harlow and Lane (Cold Spring Harbor Press, Cold Spring Harb., NY) can be performed. Other methods include alanine scanning mutants, peptide blots (Reineke (2004) Methods Mol Biol 248:443-63; herein specifically incorporated by reference in its entirety), or peptide cleavage analysis. In addition, methods such as epitope excision, epitope extraction and chemical modification of antigens can be employed (Tomer (2000) Protein Science 9: 487-496; herein specifically incorporated by reference in its entirety).

The term "epitope" refers to a site on an antigen to which B and/or T cells respond. B-cell epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, and more usually, at least 5 or 8-10 amino acids in a unique spatial conformation.

Modification-Assisted Profiling (MAP), also known as Antigen Structure-based Antibody Profiling (ASAP) is a method that categorizes large numbers of monoclonal antibodies (mAbs) directed against the same antigen according to the similarities of the binding profile of each antibody to chemically or enzymatically modified antigen surfaces (US 2004/0101920, herein specifically incorporated by reference in its entirety). Each category may reflect a unique epitope either distinctly different from or partially overlapping with epitope represented by another category. This technology allows rapid filtering of genetically identical mAbs, such that characterization can be focused on genetically distinct mAbs. When applied to hybridoma screening, MAP may facilitate identification of rare hybridoma clones that produce mAbs having the desired characteristics. MAP may be used to sort the anti-PCSK9 mAbs of the invention into groups of mAbs binding different epitopes.

In various embodiments, the anti-hPCSK9 antibody or antigen-binding fragment of an antibody binds an epitope within the catalytic domain, which is about 153 to 425 of SEQ ID NO:755); more specifically, an epitope from about 153 to about 250 or from about 250 to about 425; more specifically, the antibody or antibody fragment of the invention binds an epitope within the fragment from about 153 to about 208, from about 200 to about 260, from about 250 to about 300, from about 275 to about 325, from about 300 to about 360, from about 350 to about 400, and/or from about 375 to about 425.

In various embodiments, the anti-hPCSK9 antibody or antigen-binding fragment of an antibody binds an epitope within the propeptide domain (residues 31 to 152 of SEQ ID NO:755); more specifically, an epitope from about residue 31 to about residue 90 or from about residue 90 to about residue 152; more specifically, the antibody or antibody fragment of the invention binds an epitope within the fragment from about residue 31 to about residue 60, from about residue 60 to about residue 90, from about residue 85 to about residue 110, from about residue 100 to about residue 130, from about residue 125 to about residue 150, from about residue 135 to about residue 152, and/or from about residue 140 to about residue 152.

In some embodiments, the anti-hPCSK9 antibody or antigen-binding fragment of an antibody binds an epitope within the C-terminal domain, (residues 426 to 692 of SEQ ID NO:755); more specifically, an epitope from about residue 426 to about residue 570 or from about residue 570 to about residue 692; more specifically, the antibody or antibody fragment of the invention binds an epitope within the fragment from about residue 450 to about residue 500, from about residue 500 to about residue 550, from about residue 550 to about residue 600, and/or from about residue 600 to about residue 692.

In some embodiments, the antibody or antibody fragment binds an epitope which includes more than one of the enumerated epitopes within the catalytic, propeptide or C-terminal domain, and/or within two or three different domains (for example, epitopes within the catalytic and C-terminal domains, or within the propeptide and catalytic domains, or within the propeptide, catalytic and C-terminal domains.

In some embodiments, the antibody or antigen-binding fragment binds an epitope on hPCSK9 comprising amino acid residue 238 of hPCSK9 (SEQ ID NO:755). Experimental results (see US 2010/0166768) showed that when D238 was mutated, the $K_D$ of mAb 316P exhibited >400-fold reduction in binding affinity (~1 $x10^{-9}$ M to ~410 $x10^{-9}$ M) and $T_{1/2}$ decreased >30-fold (from ~37 to ~1 min). In a specific embodiment, the mutation was D238R. In specific embodiments, the antibody or antigen-binding fragment of the invention binds an epitope of hPCSK9 comprising two or more of amino acid residues at positions 153, 159, 238 and 343.

As shown before (see US 2010/0166768), a mutation in amino acid residue 153, 159 or 343 resulted in about a 5- to 10-fold decrease in affinity or similar shortening in $T_{1/2}$. In specific embodiments, the mutation was S153R, E159R

and/or D343R.

In some embodiments, the antibody or antigen-binding fragment binds an epitope on hPCSK9 comprising amino acid residue 366 of hPCSK9 (SEQ ID NO:755). Experimental results (see US 2010/0166768) showed that when E366 was mutated, the affinity of mAb 300N exhibited about 50-fold decrease (~0.7 x$10^{-9}$ M to ~36 x$10^{-9}$ M) and a similar shortening in $T_{1/2}$ (from ~120 to ~2 min). In a specific embodiment, the mutation is E366K.

The present invention includes anti-PCSK9 antibodies that bind to the same epitope as any of the specific exemplary antibodies described herein. Likewise, the present invention also includes anti-PCSK9 antibodies that compete for binding to PCSK9 or a PCSK9 fragment with any of the specific exemplary antibodies described herein.

One can easily determine whether an antibody binds to the same epitope as, or competes for binding with, a reference anti-PCSK9 antibody by using routine methods known in the art. For example, to determine if a test antibody binds to the same epitope as a reference anti-PCSK9 antibody of the invention, the reference antibody is allowed to bind to a PCSK9 protein or peptide under saturating conditions. Next, the ability of a test antibody to bind to the PCSK9 molecule is assessed. If the test antibody is able to bind to PCSK9 following saturation binding with the reference anti-PCSK9 antibody, it can be concluded that the test antibody binds to a different epitope than the reference anti-PCSK9 antibody. On the other hand, if the test antibody is not able to bind to the PCSK9 molecule following saturation binding with the reference anti-PCSK9 antibody, then the test antibody may bind to the same epitope as the epitope bound by the reference anti-PCSK9 antibody of the invention.

To determine if an antibody competes for binding with a reference anti-PCSK9 antibody, the above-described binding methodology is performed in two orientations: In a first orientation, the reference antibody is allowed to bind to a PCSK9 molecule under saturating conditions followed by assessment of binding of the test antibody to the PCSK9 molecule. In a second orientation, the test antibody is allowed to bind to a PCSK9 molecule under saturating conditions followed by assessment of binding of the reference antibody to the PCSK9 molecule. If, in both orientations, only the first (saturating) antibody is capable of binding to the PCSK9 molecule, then it is concluded that the test antibody and the reference antibody compete for binding to PCSK9. As will be appreciated by a person of ordinary skill in the art, an antibody that competes for binding with a reference antibody may not necessarily bind to the identical epitope as the reference antibody, but may sterically block binding of the reference antibody by binding an overlapping or adjacent epitope.

Two antibodies bind to the same or overlapping epitope if each competitively inhibits (blocks) binding of the other to the antigen. That is, a 1-, 5-, 10-, 20- or 100-fold excess of one antibody inhibits binding of the other by at least 50% but preferably 75%, 90% or even 99% as measured in a competitive binding assay (see, e.g., Junghans et al., Cancer Res. 1990 50:1495-1502). Alternatively, two antibodies have the same epitope if essentially all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Additional routine experimentation (e.g., peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

In a specific embodiment, the invention comprises an anti-PCSK9 antibody or antigen binding fragment of an antibody that binds an PCSK9 protein of SEQ ID NO:755, wherein the binding between the antibody or fragment thereof to PCSK9 and a variant PCSK9 protein is less than 50% of the binding between the antibody or fragment and the PCSK9 protein of SEQ ID NO:755. In one specific embodiment, the variant PCSK9 protein comprises at least one mutation of a residue at a position selected from the group consisting of 153, 159, 238 and 343. In a more specific embodiment, the at least one mutation is S153R, E159R, D238R, and/or D343R. In another specific embodiment, the variant PCSK9 protein comprises at least one mutation of a residue at a position selected from the group consisting of 366. In one specific embodiment, the variant PCSK9 protein comprises at least one mutation of a residue at a position selected from the group consisting of 147, 366 and 380. In a more specific embodiment, the mutation is S147F, E366K and V380M.

## Immunoconjugates

The invention encompasses a human anti-PCSK9 monoclonal antibody conjugated to a therapeutic moiety ("immunoconjugate"), such as a cytotoxin, a chemotherapeutic drug, an immunosuppressant or a radioisotope. Cytotoxin agents include any agent that is detrimental to cells. Examples of suitable cytotoxin agents and chemotherapeutic agents for forming immunoconjugates are known in the art, see for example, WO 05/103081.

Bispecifics

The antibodies of the present invention may be monospecific, bispecific, or multispecific. Multispecific mAbs may be specific for different epitopes of one target polypeptide or may contain antigen-binding domains specific for more than one target polypeptide. See, e.g., Tutt et al. (1991) J. Immunol. 147:60-69. The human anti-PCSK9 mAbs can be linked to or co-expressed with another functional molecule, e.g., another peptide or protein. For example, an antibody or fragment thereof can be functionally linked (e.g., by chemical coupling, genetic fusion, noncovalent association or otherwise) to one or more other molecular entities, such as another antibody or antibody fragment, to produce a bispecific or a multispecific antibody with a second binding specificity.

An exemplary bi-specific antibody format that can be used in the context of the present invention involves the use of a first immunoglobulin (Ig) CH3 domain and a second Ig CH3 domain, wherein the first and second Ig CH3 domains differ from one another by at least one amino acid, and wherein at least one amino acid difference reduces binding of the bispecific antibody to Protein A as compared to a bi-specific antibody lacking the amino acid difference. In one embodiment, the first Ig CH3 domain binds Protein A and the second Ig CH3 domain contains a mutation that reduces or abolishes Protein A binding such as an H95R modification (by IMGT exon numbering; H435R by EU numbering). The second CH3 may further comprise a Y96F modification (by IMGT; Y436F by EU). Further modifications that may be found within the second CH3 include: D16E, L18M, N44S, K52N, V57M, and V821 (by IMGT; D356E, L358M, N384S, K392N, V397M, and V4221 by EU) in the case of IgG1 antibodies; N44S, K52N, and V821 (IMGT; N384S, K392N, and V4221 by EU) in the case of IgG2 antibodies; and Q15R, N44S, K52N, V57M, R69K, E79Q, and V821 (by IMGT; Q355R, N384S, K392N, V397M, R409K, E419Q, and V4221 by EU) in the case of IgG4 antibodies. Variations on the bi-specific antibody format described above are contemplated within the scope of the present invention.

Bioequivalents

The anti-PCSK9 antibodies and antibody fragments of the present invention encompass proteins having amino acid sequences that vary from those of the described mAbs, but that retain the ability to bind human PCSK9. Such variant mAbs and antibody fragments comprise one or more additions, deletions, or substitutions of amino acids when compared to parent sequence, but exhibit biological activity that is essentially equivalent to that of the described mAbs. Likewise, the anti-PCSK9 antibody-encoding DNA sequences of the present invention encompass sequences that comprise one or more additions, deletions, or substitutions of nucleotides when compared to the disclosed sequence, but that encode an anti-PCSK9 antibody or antibody fragment that is essentially bioequivalent to an anti-PCSK9 antibody or antibody fragment of the invention. Examples of such variant amino acid and DNA sequences are discussed above.

Two antigen-binding proteins, or antibodies, are considered bioequivalent if, for example, they are pharmaceutical equivalents or pharmaceutical alternatives whose rate and extent of absorption do not show a significant difference when administered at the same molar dose under similar experimental conditions, either single does or multiple dose. Some antibodies will be considered equivalents or pharmaceutical alternatives if they are equivalent in the extent of their absorption but not in their rate of absorption and yet may be considered bioequivalent because such differences in the rate of absorption are intentional and are reflected in the labeling, are not essential to the attainment of effective body drug concentrations on, e.g., chronic use, and are considered medically insignificant for the particular drug product studied. In one embodiment, two antigen-binding proteins are bioequivalent if there are no clinically meaningful differences in their safety, purity, and potency.

In one embodiment, two antigen-binding proteins are bioequivalent if a patient can be switched one or more times between the reference product and the biological product without an expected increase in the risk of adverse effects, including a clinically significant change in immunogenicity, or diminished effectiveness, as compared to continued therapy without such switching.

In one embodiment, two antigen-binding proteins are bioequivalent if they both act by a common mechanism or mechanisms of action for the condition or conditions of use, to the extent that such mechanisms are known.

Bioequivalence may be demonstrated by *in vivo* and *in vitro* methods. Bioequivalence measures include, e.g., (a) an *in vivo* test in humans or other mammals, in which the concentration of the antibody or its metabolites is measured in blood, plasma, serum, or other biological fluid as a function of time; (b) an *in vitro* test that has been correlated with and is reasonably predictive of human *in vivo* bioavailability data; (c) an *in vivo* test in humans or other mammals in which the appropriate acute pharmacological effect of the antibody (or its target) is measured as a function of time; and (d) in a well-controlled clinical trial that establishes safety, efficacy, or bioavailability or bioequivalence of an antibody.

Bioequivalent variants of anti-PCSK9 antibodies of the invention may be constructed by, for example, making various substitutions of residues or sequences or deleting terminal or internal residues or sequences not needed for biological activity. For example, cysteine residues not essential for biological activity can be deleted or replaced with other amino acids to prevent formation of unnecessary or incorrect intramolecular disulfide bridges upon renaturation.

Treatment Population

The invention provides therapeutic methods for treating a human patient in need of a composition of the invention. While modifications in lifestyle and conventional drug treatment are often successful in reducing cholesterol levels, not all patients are able to achieve the recommended target cholesterol levels with such approaches. Various conditions, such as familial hypercholesterolemia (FH), appear to be resistant to lowering of LDL-C levels in spite of aggressive use of conventional therapy. Homozygous and heterozygous familial hypercholesterolemia (hoFH, heFH) is a condition associated with premature atherosclerotic vascular disease. However, patients diagnosed with hoFH are largely unresponsive to conventional drug therapy and have limited treatment options. Specifically, treatment with statins, which reduce LDL-C by inhibiting cholesterol synthesis and upregulating the hepatic LDL receptor, may have little effect in patients whose LDL receptors are non-existent or defective. A mean LDL-C reduction of only less than about 20% has been recently reported in patients with genotype-confirmed hoFH treated with the maximal dose of statins. The addition of ezetimibe 10 mg/day to this regimen resulted in a total reduction of LDL-C levels of 27%, which is still far from optimal. Likewise, many patients are statin non-responsive, poorly controlled with statin therapy, or cannot tolerate statin therapy; in general, these patients are unable to achieve cholesterol control with alternative treatments. There is a large unmet medical need for new treatments that can address the short-comings of current treatment options.

Specific populations treatable by the therapeutic methods of the invention include subjects indicated for LDL apheresis, subjects with PCSK9-activating mutations (gain of function mutations, "GOF"), subjects with heterozygous Familial Hypercholesterolemia (heFH); subjects with primary hypercholesterolemia who are statin intolerant or statin uncontrolled; and subjects at risk for developing hypercholesterolemia who may be preventably treated. Other indications include hyperlipidemia and dyslipidemia (such as mixed dyslipidemia) associated with secondary causes such as Type 2 diabetes mellitus, cholestatic liver diseases (primary biliary cirrhosis), nephrotic syndrome, hypothyroidism, obesity; and the prevention and treatment of atherosclerosis and cardiovascular diseases. However, depending on the severity of the afore-mentioned diseases and conditions, the treatment of subjects with the antibodies and antigen-binding fragments of the invention may be contraindicated for certain diseases and conditions.

Therapeutic Administration and Formulations

The invention provides therapeutic compositions comprising the anti-PCSK9 antibodies or antigen-binding fragments thereof of the present invention. The administration of therapeutic compositions in accordance with the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

The dose may vary depending upon the age and the size of a subject to be administered, target disease, conditions, route of administration, and the like. When the antibody of the present invention is used for treating various conditions and diseases associated with PCSK9, including hypercholesterolemia, disorders associated with LDL and apolipoprotein B, and lipid metabolism disorders, and the like, in an adult patient, it is advantageous to intravenously administer the antibody of the present invention normally at a single dose of about 0.01 to about 20 mg/kg body weight, more preferably about 0.02 to about 7, about 0.03 to about 5, or about 0.05 to about 3 mg/kg body weight. Depending on the severity of the condition, the frequency and the duration of the treatment can be adjusted.

Various delivery systems are known and can be used to administer the pharmaceutical compositions of the invention, e.g., encapsulation in liposomes, microparticles, microcapsules, recombinant cells capable of expressing the mutant viruses, receptor mediated endocytosis (see, e.g., Wu et al. (1987) J. Biol. Chem. 262:4429-4432). Methods of introduction include, but are not limited to, intradermal, intramuscular, intraperitoneal, intravenous, subcutaneous, intranasal, epidural, and oral or peroral routes. If the antibody of present invention is administered per injection, subcutaneous injection is preferred. Oral or peroral administration is preferred for the HMG-CoA reductase inhibitor, e.g. the statin.

The composition may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. The pharmaceutical composition can be also delivered in a vesicle, in particular a liposome (see Langer (1990) Science 249:1527-1533; Treat et al. (1989) in Liposomes in the Therapy of Infectious Disease and Cancer, Lopez Berestein and Fidler (eds.), Liss, New York, pp. 353-365; Lopez-Berestein, ibid., pp. 317-327; see generally ibid.).

In certain situations, the pharmaceutical composition can be delivered in a controlled release system. In one

embodiment, a pump may be used (see Langer, supra; Sefton (1987) CRC Crit. Ref. Biomed. Eng. 14:201). In another embodiment, polymeric materials can be used; see, Medical Applications of Controlled Release, Langer and Wise (eds.), CRC Pres., Boca Raton, Florida (1974). In yet another embodiment, a controlled release system can be placed in proximity of the composition's target, thus requiring only a fraction of the systemic dose (see, e.g., Goodson, in Medical Applications of Controlled Release, supra, vol. 2, pp. 115-138, 1984).

The injectable preparations may include dosage forms for intravenous, subcutaneous, intracutaneous and intramuscular injections, drip infusions, etc. These injectable preparations may be prepared by methods publicly known. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but certainly are not limited to AUTOPEN™ (Owen Mumford, Inc., Woodstock, UK), DISETRONIC™ pen (Disetronic Medical Systems, Burghdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™ , and OPTICLIK™ (sanofi-aventis, Frankfurt, Germany), to name only a few. Examples of disposable pen delivery devices having applications in subcutaneous delivery of a pharmaceutical composition of the present invention include, but certainly are not limited to the SOLOSTAR™ pen (sanofi-aventis), the FLEXPEN™ (Novo Nordisk), and the KWIKPEN™ (Eli Lilly).

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc. The amount of the aforesaid antibody contained is generally about 4 to about 500 mg or from about, from about 5 to about 500 mg per dosage form in a unit dose; especially in the form of injection, it is preferred that the aforesaid antibody is contained in about 5 to about 400 mg (such as from about 50 to about 200 mg per 1 ml injection solution), particularly about 150mg and in about 10 to about 250 mg or to about 500 mg for the other dosage forms.

The invention provides therapeutic methods in which the antibody or antibody fragment of the invention is useful to treat hypercholesterolemia associated with a variety of conditions involving hPCSK9. The anti-PCSK9 antibodies or antibody fragments of the invention are particularly useful for the treatment of hypercholesterolemia and the like, particularly in the types of hypercholesterolemia as herein described.

In some embodiments, the antibodies or antibody fragments of the invention and as herein described are for use in combination therapies for treatment or prevention of the herein described indications. Combination therapies of present invention may include the antibody or fragment described together with a fibrate and/or the lipid lowering agent acting by decreasing cholesterol uptake into the intestine and optionally one or more additional lipid-lowering agents.

The above combination therapies are particularly suitable in the context of the method of the first, second, third, aspect and for the use according to the fourth aspect of present invention. The present invention also relates to a pharmaceutical composition for use in the above combination therapy and a pharmaceutical composition comprising (in addition to the antibody or fragment of present invention) one or more of the above-indicated additional lipid lowering agents. The present invention also relates to an article of manufacture of present invention, e.g. according to the eighth aspect of present invention that is adapted for the combination therapy as herein described, e.g. by comprising in addition to the antibody or fragment thereof of present invention one or more of the herein described additional lipid lowering agents and/or by comprising instructions for a use adapted to a particular combination treatment and/or comprising instructions for a dosage or administration regime of the additional lipid lowering agent.

PREFERRED ASPECTS OF PRESENT INVENTION

In the following, some preferred aspects of present invention will be listed:

1. A method for treating a disease or condition in which PCSK9 expression or activity causes an impact comprising administering a therapeutic amount of an antibody or antigen-binding fragment thereof which specifically binds hPCSK9 (human proprotein convertase subtilisin/kexin type 9) to a subject in need thereof, wherein the method comprises administering of the antibody or antigen-binding fragment thereof together with

(a) a therapeutic amount of a lipid lowering agent acting by decreasing cholesterol adsorption in the intestine, or

(b) a therapeutic amount of a fibrate, or

(c) a therapeutic amount of the lipid lowering agent of a) and a therapeutic amount of a fibrate.

2. A method of lowering cholesterol levels in a subject in need thereof, comprising:

(a) selecting a subject with a blood low density lipoprotein cholesterol (LDL-C) level > 100 mg/dL; and
(b) administering to said subject a composition comprising an antibody or antigen-binding fragment thereof or antigen binding fragment thereof that specifically binds to human proprotein convertase subtilisin/kexin type 9 (hPCSK9); thereby lowering cholesterol levels in the subject in need thereof, wherein the method comprises administering of the antibody or antigen-binding fragment thereof or fragment thereof together with

(i) a therapeutic amount of a lipid lowering agent acting by decreasing cholesterol adsorption in the intestine, or

(ii) a therapeutic amount of a fibrate, or

(iii) a therapeutic amount of the lipid lowering agent of a) and a therapeutic amount of a fibrate.

3. A method of preventing effects of a (persistently) increased LDL-C level in the blood comprising administering a therapeutic amount of an antibody or antigen-binding fragment thereof which specifically binds hPCSK9 (human proprotein convertase subtilisin/kexin type 9) to a subject having an increased LDL-C level in the blood, and

(a) administering a therapeutic amount of a lipid lowering agent acting by decreasing cholesterol adsorption in the intestine, or

(b) administering a therapeutic amount of a fibrate, or

(c) administering a therapeutic amount of the lipid lowering agent of a) and a therapeutic amount of a fibrate.

4. The method according to any one of the aspects 1 to 3 comprising administering the antibody or antigen-binding fragment thereof together with the lipid lowering agent.

5. The method according to any one of the aspects 1 to 4 comprising administering the antibody or antigen-binding fragment thereof together with the fibrate.

6. The method according to aspect 5 comprising the administration of the antibody or antigen-binding fragment thereof together with the lipid lowering agent and the fibrate.

7. The method according to one of the aspects 4 or 6, wherein the lipid lowering agent decreases or increases PCSK9 expression or has no significant effect on PCSK9 expression.

8. The method according to one of the aspects 5 to 7, wherein the fibrate decreases or increases PCSK9 expression or has no significant effect on PCSK9 expression.

9. The method according to any one of the aspects 1 to 8, wherein the administration of the antibody or antigen-binding fragment thereof together with the lipid lowering agent and/or the fibrate is an adjunctive therapy to diet and

preferably to lipid-lowering or cholesterol-lowering diet.

10. The method according to any one of the aspects 1 to 9, wherein the lipid lowering agent is an azetidone and preferably ezetimibe.

11. The method according to any one of the aspects 1 to 10, wherein the fibrate is bezafibrate, ciprofibrate, clofibrate, gemfibrozil or fenofibrate and preferably fenofibrate.

12. The method of any one of aspects 1 to 11, wherein the antibody or antigen-binding fragment thereof is a recombinant human antibody.

13. The method of any one of aspects 1 to 12, wherein the antibody or antigen-binding fragment thereof or the antigen-binding fragment thereof is characterized by one or more of the following:

(i) being capable of reducing serum total cholesterol at least about 25 to about 35% and sustaining the reduction over at least a 24 day period relative to a predose level;

(ii) being capable of reducing serum LDL cholesterol at least about 65-80% and sustaining the reduction over at least a 24 day period relative to a predose level;

(iii) being capable of reducing serum triglyceride at least about 25-40% relative to predose level;

(iv) achieving one or more of (i)-(iii) without reducing serum HDL cholesterol or reducing serum HDL cholesterol no more than 5% relative to predose level;

(v) achieving one or more of (i)-(iii) with little or no measurable effect on liver function, as determined by ALT (Alanine Aminotransferase) and AST (Aspartate Aminotransferase) measurements.

14. The method of any one of aspects 1 to 13, wherein the antibody or antigen-binding fragment thereof or the antigen-binding fragment thereof comprises

(a) a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; or

(b) a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736, or

(c) the heavy chain of a) and the light chain of b).

15. The method of any one of aspects 1 to 14, wherein the antibody or antigen-binding fragment thereof or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

16. The method of any one of aspects 1 to 15, wherein the antibody or antigen-binding fragment thereof binds to the same epitope on hPCSK9 as an antibody or antigen-binding fragment thereof comprising heavy and light chain CDR amino acid sequences as shown in SEQ ID NOs: 76, 78, 80, 84, 86, and 88.

17. The method of any one of aspects 1 to 16, wherein the antibody or antigen-binding fragment thereof competes for binding to hPCSK9 with an antibody or antigen-binding fragment thereof comprising heavy and light chain CDR amino acid sequences as shown in SEQ ID NOs: 76, 78, 80, 84, 86, and 88.

18. The method according to one of the aspects 1 to 17 comprising administration of the antibody or antigen-binding fragment thereof in a dosage amount ranging from 5 mg to 500 mg and preferably ranging from 50 to 300 mg and

more preferably of about 150 mg.

19. The method according to any one of the aspects 1 to 18, comprising administering a daily dose of 1, 3, 5, 7, 10, 15, 20, 25, 30, 40 or 50 mg and preferably of 5 to 20 mg of the lipid lowering agent and preferably of an azetidone and more preferably ezetimibe.

20. The method according to any one of the aspects 1 to 19, comprising administering a daily dose of 50, 75, 100, 150, 160, 170, 180, 190 or 200 mg and preferably of 75 to 180 mg of the fibrate and preferably of fenofibrate.

21. The method according to aspect 18 or 19 comprising administration of the antibody or antigen-binding fragment thereof in a dosage amount ranging from 50 mg to 300 mg and preferably of about 150 mg and comprising administration of the lipid lowering agent and preferably ezetimibe in a daily dose of 5 to 20 and preferably of about 10 mg.

22. The method according to one of the aspects 18 to 21 comprising administration of the antibody or antigen-binding fragment thereof in a dosage amount ranging from 50 mg to 300 and preferably of about 150 mg and comprising administration of the fibrate and preferably of fenofibrate in a daily dose of 75 to 180 mg and preferably of about 160 mg.

23. The method according to aspect 22 comprising administration of the antibody or antigen-binding fragment thereof in a dosage amount ranging from 50 mg to 300 mg and preferably of about 150 mg and comprising administration of fenofibrate in a daily dose of about between 75 to 180 mg and preferably of about 160 mg and comprising administration of ezetimibe in a daily dose of between 5 to 20 mg and preferably of about 10 mg.

24. The method of any one of aspects 1 to 23, wherein the lipid lowering agent, the fibrate or both are administered three times per day, twice per day, or once per day and preferably once per day.

25. The method of any one of aspects 1 to 24, wherein the lipid lowering agent, the fibrate or both are administered every day, every other day, every third day, every fourth day, every fifth day, or every sixth day and preferably every day.

26. The method or the antibody or antigen-binding fragment thereof of any one of aspects 1 to 25, wherein the lipid lowering agent, the fibrate or both are administered in the morning, at noon or in the evening and preferably in the evening.

27. The method or the antibody or antigen-binding fragment thereof of any one of aspects 1 to 26, wherein the lipid lowering agent, the fibrate or both are administered before, during or after a meal.

28. The method of aspects 1 to 27, wherein the antibody or antigen-binding fragment thereof is administered every week, every other week, every third week, every month or every fourth week and preferably every fourth week or every month.

29. The method according to any one of aspects 1 to 28, wherein the antibody or antigen-binding fragment thereof is formulated as injection solution.

30. The method according to any one of aspects 1 to 29, wherein the antibody or antigen-binding fragment thereof or fragment thereof is administered per injection and preferably subcutaneous injection and more preferably per subcutaneous injection into the abdomen.

31. The method according to any one of the aspects 1 to 30, wherein the lipid lowering drug or the fibrate or both are formulated for oral administration.

32. The method according to any one of the aspects 1 to 31, wherein the lipid lowering drug or the fibrate of both are formulated as tablet, capsule or (over-) encapsulated tablet.

33. The method according to any one of the aspects 1 to 32, wherein the lipid lowering drug or the fibrate or both are administered orally.

34. The method according to any one of the aspects 1 to 33 wherein the subject in need thereof falls into one or

more of the following groups of subjects:

(i) subjects having a serum LDL cholesterol (LDL-C) level of at least 100 mg/dL, *[at least 130 mgldL, at least 160 mgldL / at least 200 mgldL],* (particularly if said LDL-C level is determined absence of any lipid lowering treatment or if said LDL-C level is determined under treatment with ezetimibe or fenofibrate)

(ii) subjects having a serum HDL-C level of less than 40 mg/dL; (particularly if said LDL-C level is determined absence of any lipid lowering treatment or if said LDL-C level is determined under treatment with ezetimibe or fenofibrate)

(iii) subjects having a serum cholesterol level of at least 200 mg/dL *[240 mg/dL];* (particularly if said LDL-C level is determined absence of any lipid lowering treatment or if said LDL-C level is determined under treatment with ezetimibe or fenofibrate)

(iv) subjects having a serum triacylglycerol level of at least 150 mg/dL *[at least 200 mg/dL; at least 500 mg/dL],* wherein said triacylglycerol level is determined after fasting for at least 8 hours; (particularly if said LDL-C level is determined absence of any lipid lowering treatment or if said LDL-C level is determined under treatment with ezetimibe or fenofibrate)

(v) subjects being at least 18 years old *[at least 18 /20 /25 /30/ 35 /40/ 45 / 50 / 55 / 60 /65 / 70 / 75 years old];*

(vi) subjects being at maximum 75 years old *[75 /70 /65 / 60 / 55 / 50 / 45 / 40/ 35/ 30/ 25/ 20 years]l*

(vii) subjects having a BMI of *25 [26 / 271 28 / 29 / 30 / 31 /32 / 33 / 34 / 35 / 36 / 37 I 38 / 39]* or more;

(viii) male subjects;

(ix) female subjects;

(x) subjects in which the administration of said antibody or antigen-binding fragment thereof leads to a reduction in the serum LDL-C level by at least 30 mg/dL *[40 mg/dL; 50 mg/dL; 60 mg/dL; 70 mg/dL]* relative to predose level; or

(xi) subjects in which the administration of said antibody or antigen-binding fragment thereof leads to a reduction in the serum LDL-C level by at least 20% *[30%; 40%; 50%; 60%]* relative to predose level

(xii) subjects having mild and/or moderate hepatic impairment

(xiii) subjects with normal hepatic liver function

(xiv) subjects with high cardiovascular risk

(xv) subjects with coronary heart disease

(xvi) subjects with cardiovascular disease

(xvii) subjects undergoing lipid-lowering diet.

35. The method according to any one of the aspects 1 to 34, wherein the subject in need thereof does not fall into one or more of the following groups of subjects:

(i) smokers;

(ii) persons being 76 years old or older;

(iii) persons being 17 years or younger;

(iv) persons suffering from hypertension;

(v) women who are pregnant;

(vi) women who are trying to become pregnant;

(vii) women who are breast-feeding;

(viii) persons who have or ever had severe hepatic impairment;

(ix) persons who had any unexplained abnormal results from blood tests for liver function;

(x) persons who drink excessive amounts of alcohol;

(xi) persons having kidney problems;

(xii) persons suffering from hypothyroidism;

(xiii) persons suffering from muscle disorders;

(xiv) persons having encountered previous muscular problems during treatment with lipid-lowering medicine;

(xv) persons having serious problems with their breathing;

(xvi) persons taking one or more of the following medicines: medicines altering the way the immune systems works (e.g. cyclosporine or antihistamines), antibiotics or antifungal medicines (e.g. erythromycin, clarithromycin, ketoconazole, itraconazole, rifampicin, fusidic acid), medicines regulating lipid levels (e.g. gemfibrozil, colestipol), calcium channel blockers (e.g. verapamil, diltiazem), medicines regulating the heart rhythm (digoxin, amiodarone), protease inhibitors used in the treatment of HIV (e.g. nelfinavir), warfarin, oral contraceptives, antacids or St. John's Wort; or

(xvii) persons drinking more than 0.1 L of grapefruit juice per day; or

(xviii) persons having a body mass index (BMI) of more than 40; or

(xix) persons having a body mass index (BMI) of less than 18; or

(xx) persons suffering from type 1 diabetes or type 2 diabetes; or

(xxi) persons positive for hepatitis B or hepatitis C; or

(xxii) persons having a known sensitivity to monoclonal antibody or antigen-binding fragment thereof therapeutics; or

(xxiii) persons having acute hepatitis; or

(xxiv) persons having hepatic encephalopathy grade 2, 3, or 4; or

(xv) persons having uncontrolled clinically relevant cardiovascular, pulmonary, gastrointestinal, metabolic, hematological, neurological, psychiatric, systemic, ocular, gynecologic (if female), or infectious disease, or signs of acute illness; or

(xvi) persons having history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness; or

(xvii) persons having experienced an adverse event after earlier administration of a fibrate and preferably fenofibrate; or

(xviii) persons having experienced an adverse event after earlier administration of ezetimibe; or

(xix) persons having experienced an adverse event after earlier administration of antibodies against human PCSK9; or

(xx) persons having one or more of: neutropenia, thrombocytopenia, an increase in ALT, acute renal failure and rhabdomyolysis

and wherein the subject preferably falls into one or more of the subject groups according to aspect 34.

36. The method of any one of aspects 1 to 35, wherein the subject in need is a subject indicated for LDL apheresis, a subject with PCSK9-activating mutations, a subject with heterozygous Familial Hypercholesterolemia, a subject with primary hypercholesterolemia who is statin uncontrolled, a subject at risk for developing hypercholesterolemia, a subject with hypercholesterolemia, hyperlipidemia, dyslipidemia such as mixed dyslipidemia, atherosclerosis, a high risk of developing or suffering from cardiovascular disease, a subject with a cardiovascular disease such as coronary heart disease or a coronary heart disease risk-equivalent.

37. The method according to one of the aspects 1 to 36, wherein the disease or condition in which PCSK9 expression or activity causes an impact is ameliorated, improved, inhibited or prevented with a PCSK9 antagonist.

38. The method according to any one of the aspects 1 to 37, wherein the disease or condition in which PCSK9 expression or activity causes an impact is selected from the group consisting of: hypercholesterolemia, statin-uncontrolled hypercholesterolemia, non-familial hypercholesterolemia, heterozygous familiar hypercholesterolemia, hyperlipidemia, dyslipidemia such as mixed dyslipidemia, mixed dyslipidemia, atherosclerosis, cardiovascular disease, coronary heart disease and a coronary heart disease-risk equivalent.

39. The method of any one of aspects 1 to 38, wherein the treatment with the antibody or antigen-binding fragment thereof together with the lipid lowering agent or the fibrate or together with the lipid lowering agent and the fibrate does not induce does not induce any of: neutropenia, acute renal failure, rhabdomyolysis, increase in ALT or AST, thrombocytopenia, neutropenia.

40. The method of any one of aspects 1 to 39, wherein the treatment with the antibody or antigen-binding fragment thereof together with the lipid lowering agent or the fibrate or together with the lipid lowering agent and the fibrate improves lipid parameters critical for the development, persistence or worsening of atherosclerosis and/or cardiovascular disease such as coronary heart disease or coronary-heart disease risk equivalents.

41. The method of aspect 40, wherein the treatment positively affects one or more of the following serum levels: LDL-C, total-C, triglycerides, HDL-C, non-HDL-C, apolipoprotein B, apolipoprotein A1, lipoprotein a).

42. An antibody or antigen-binding fragment thereof which specifically binds hPCSK9 (human proprotein convertase subtilisin/kexin type 9) for use in a method according to any one of aspects 1 to 41.

43. The antibody or antigen-binding fragment thereof or antigen binding fragment according to aspect 42, wherein the action of the antibody or antigen-binding fragment thereof comprises counteracting the increase of PCSK9 expression by the lipid-lowering agent and particularly of ezetimibe.

44. The antibody or antigen-binding fragment thereof or antigen binding fragment thereof according to aspect 42 or 43, wherein the action of the antibody or antigen-binding fragment thereof comprises counteracting the increase of PCSK9 expression by the fibrate and particularly of fenofibrate.

45. Pharmaceutical composition comprising a pharmaceutically effective amount of antibody or antigen-binding fragment thereof of an antibody or antigen-binding fragment thereof which specifically binds hPCSK9 and preferably of an antibody according to one of the aspects 12 to 17 or 43 to 44 and a pharmaceutically acceptable excipient together with a pharmaceutically effective amount of a fibrate and preferably of fenofibrate.

46. Pharmaceutical composition comprising a pharmaceutically effective amount of antibody or antigen-binding fragment thereof of an antibody or antigen-binding fragment thereof which specifically binds hPCSK9 and preferably of an antibody according to one of the aspects 12 to 17 or 43 to 44, in a method according to one of the aspects 1 to 41.

47. The pharmaceutical composition according to claim 46 further comprising a pharmaceutically effective amount

of a fibrate and preferably fenofibrate.

48. The pharmaceutical composition according to one of the claims 45 to 47 further comprising a pharmaceutically effective amount of a lipid lowering drug acting by decreasing cholesterol uptake in the intestine and preferably ezetimibe.

49. Article of manufacture comprising

(a) a packaging material or container;

(b) an antibody or antigen-binding fragment thereof which specifically binds hPCSK9; and

(c) a fibrate and/or a lipid lowering drug acting by decreasing cholesterol adsorption in the intestine; and

(d) a label or packaging insert.

50. The article of manufacture according to claim 49, wherein the label or packaging insert contains reference to any one of the methods according to any of aspects 1 to 41.

51. The article of manufacture according to one of the aspects 49 or 50, comprising a fibrate, wherein the fibrate is preferably fenofibrate.

52. The article of manufacture according to one of the aspects 49 to 51, comprising the lipid lowering drug, wherein the lipid lowering drug is preferably an azetidone and more preferably ezetimibe.

53. The article of manufacture according to one of the aspects 49-52, wherein the antibody or antigen-binding fragment thereof is comprised in a pharmaceutical formulation together with a pharmaceutically acceptable excipient.

54. The article of manufacture according to one of the aspects 49 to 53, wherein the antibody or antigen-binding fragment thereof or antigen-binding fragment is an antibody or antigen-binding fragment thereof or antigen-binding fragment as specified in any of aspects 12 to 17.

55. The article of manufacture according to any of aspects 49 to 54, wherein the label or packaging insert has one or more of the following features:

(a) the label or packaging insert indicates that patients receiving treatment with said antibody or antigen-binding fragment thereof or antigen-binding fragment can be treated for a disease or condition selected from the group as defined in aspect 38

(b) the label or packaging insert indicates the treatment of patients in a method according to one of the aspects 1 to 41 is contra-indicated in the subjects according to aspect 35;

(c) the label or packaging insert indicates the treatment of patients in a method according to one of the aspects 1 to 41 is possible for the subjects according to aspects 34 and 36.

56. The article of manufacture according to one of the aspects 49 to 55 comprising one or more effective dosages of the lipid lowering agent and preferably of ezetimibe for lowering LDL-C levels by administration once per day.

57. The article of manufacture according to aspect 56, comprising an effective dose of ezetimibe for lowering LDL-C levels by administration once per day.

58. The article of manufacture according to aspect 57, wherein the effective dosage comprises about 10 mg ezetimibe.

59. The article of manufacture according to one of aspects 56 to 58 comprising ezetimibe as one or more unit dosage forms for oral administration.

60. The article of manufacture according to one of the aspects 49 to 59 comprising one or more effective dosages of the fibrate and preferably of fenofibrate for lowering LDL-C levels by administration once per day.

61. The article of manufacture according to aspect 60, comprising an effective dose of fenofibrate for lowering LDL-C levels by administration once per day.

62. The article of manufacture according to aspect 61, wherein the effective dosage comprises about 100 mg of fenofibrate.

63. The article of manufacture according to one of aspects 60 to 62 comprising fenofibrate as one or more unit dosage forms for oral administration.

64. The article of manufacture according to one of aspects 49 to 63, comprising the antibody or antigen-binding fragment thereof as injection solution and preferably comprising about 40 mg to about 200 mg or about 40 to about 200 mg, e.g. about 40 mg, about 50 mg, about 75 mg, about 100 mg, about 150 mg or about 200 mg of the antibody or antigen-binding fragment thereof per 0,5 ml or per 1 ml volume injection solution and preferably about 150mg per 1ml volume injection solution.

65. The article of manufacture according to aspect 64 comprising the antibody or antigen-binding fragment thereof in one or more unit dosage forms and preferably comprising the antibody or antigen-binding fragment thereof antibody or antigen-binding fragment thereof as liquid formulation in a hermetically sealed container such as a vial, a sachette, a pre-filled syringe, a pre-filled autoinjector, a cartridge for a reusable syringe, pen, autoinjector or applicator.

66. The article of manufacture according to one of aspects 49 to 63, comprising the antibody or antigen-binding fragment thereof as dry formulation, preferably comprising about 40 mg to about 500 mg, 50 to about 500 mg, about 50 to about 400, about 50 to about 300 e.g. about 40 mg, about 50 mg, about 75 mg, at about 100 mg, about 150 mg, about 200 mg, about 250 mg, about 300 mg, about 350 mg, about 400 mg, about 450 mg or about 500mg and preferably about 50 mg, about 100 mg, about 150 mg, about 200 mg, about 250 mg, about 300 mg and even more preferably about 150 mg, about 200 mg or about 300 mg and particularly about 150mg of the antibody or antigen-binding fragment thereof per dose.

67. The article of manufacture according to aspect 66, comprising one or more unit dosage forms comprising the antibody or antigen-binding fragment thereof as dry formulation for dissolution in a hermetically sealed container such as a vial, an ampoule or sachette

68. The article of manufacture according to one of the aspects 49 to 67 comprising one or more separate unit dosage forms each of the antibody or antigen-binding fragment thereof and fenofibrate and/or ezetimibe.

69. The article of manufacture according to one of the aspects 46 to 68 comprising the following components a) to d):

(a) one or more unit dosage forms comprising the antibody;

(b) one or more unit dosage forms comprising ezetimibe and/or one or more unit dosage forms of fenofibrate;

(c) the label or package insert;

(d) a device for application of the antibody or antigen-binding fragment thereof such as a syringe and instructions for use of the device.

70. Article of manufacture according to one of the aspects 49 to 69 comprising one or more unit dosage forms of ezetimibe and/or fenofibrate as tablet or capsule or other formulation for oral administration in a hermetically sealed container or blister

71. Article of manufacture according to aspect 70, wherein the quantity of active ingredient is indicated on the hermetically-sealed container or blister.

72. Article of manufacture according to one of the aspects 49 to 71 comprising sufficient unit dosage forms of the antibody or antigen-binding fragment thereof and ezetimibe and/or fenofibrate, for one single administration of antibody or antigen-binding fragment thereof and ezetimibe and/or fenofibrate, for a two-week (i.e. 14-day) treatment with antibody or antigen-binding fragment thereof and ezetimibe and/or fenofibrate, for a four week (i.e, 28-day) treatment with antibody or antigen-binding fragment thereof and ezetimibe and/or fenofibrate or for a one-month

treatment with antibody or antigen-binding fragment thereof and ezetimibe and/or fenofibrate.

73. Article of manufacture according to one of the aspects 49 to 72, comprising sufficient unit dosage forms of antibody or antigen-binding fragment thereof for a bi-weekly administration regime or a four-weekly administration regime or a monthly administration regime.

74. Article of manufacture according to one of the aspects 49 to 73 comprising sufficient unit dosage forms of and ezetimibe and/or fenofibrate for a daily administration regime.

75. A method for preparing an article of manufacture according to one of the aspects 49 to 74 comprising packaging an antibody or antigen-binding fragment thereof which specifically binds hPCSK9 and packaging the lipid lowering agent and/or the fibrate in a container and assembling them with a label or packaging insert.

76. The method according to aspect 75, wherein the antibody or antigen-binding fragment thereof is comprised in a pharmaceutical composition in the form of a liquid or dry formulation and preferably assembled as unit dosage form.

77. The method according to aspect 75 or 76, wherein the lipid lowering agent and/or the fibrate are comprised in pharmaceutical compositions formulated for oral administration and preferably assembled as unit dosage forms for oral administration (e.g. tablet or capsule or over-encapsulated tablet).

78. Unit dosage form comprising an antibody or antigen-binding fragment thereof which specifically binds hPCSK9 (human proprotein convertase subtilisin/kexin type 9) together with a fibrate.

79. Unit dosage form comprising an antibody or antigen-binding fragment thereof which specifically binds hPCSK9 (human proprotein convertase subtilisin/kexin type 9) for use in a method according to one of the aspects 1 to 41, wherein the antibody or antigen-binding fragment thereof preferably is an antibody or antigen-binding fragment thereof according to one of the aspects 12 to 17.

In the following, the invention will be described in more detail by means of figures and examples that serve to illustrate but do not intend to limit present invention.

DESCRIPTION OF THE FIGURES:

Figure 1: Graphical study design of study1 of present examples comprising an administration regime of the antibody 316P in 150 mg per 1ml injection volume (subcutaneous, abdominal) administered every fourth week together with daily administration of either 160 mg of fenofibrate of 10 mg ezetimibe or ezetimibe placebo.
Gp A: 316P + ezetimibe placebo (ezetimibe placebo from D-28 to D120)
Gp B: 316P + ezetimibe (ezetimibe from D-28 to D120)
Gp C: 316P + fenofibrate (fenofibrate from D-28 to D120)

EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used but some experimental errors and deviations should be accounted for. Unless indicated otherwise, molecular weight is average molecular weight, temperature is in degrees Centigrade, and pressure is at or near atmospheric.

Study 1

Title: A randomized, partial blind, 3 parallel groups study of the pharmacodynamic profile of antibody 316P administered as multiple 150 mg subcutaneous doses, either alone or on top of ezetimibe or fenofibrate administered as multiple oral doses in healthy subjects.

1.1 STUDY OBJECTIVES:

Primary objective: To assess the pharmacodynamic profile of antibody 316P administered either alone or on top of ezetimibe or fenofibrate, based on LDL-C Secondary objective(s):

To assess the pharmacodynamic profile of antibody 316P administered either alone or on top of ezetimibe or fenofibrate, based on other lipid parameters
To assess the pharmacokinetic profile of antibody 316P administered either alone or on top of ezetimibe or fenofibrate.
To document exposure to ezetimibe and fenofibrate.
To assess the effect of either ezetimibe or fenofibrate on PCSK9 levels.
To assess the safety of concomitant administration of antibody 316P and either ezetimibe or fenofibrate.

1.2 STUDY DESIGN

DESCRIPTION OF THE PROTOCOL

This is a phase I multi-centre study.

It is a randomized, partial blind, controlled study conducted in 3 parallel groups, with a 4-week run-in period with either ezetimibe or fenofibrate or placebo ezetimibe (i.e.: group A: antibody 316P + ezetimibe placebo, group B: antibody 316P + ezetimibe, group C: antibody 316P + fenofibrate).

Subjects will receive repeated doses of ezetimibe or fenofibrate or placebo ezetimibe since the beginning of the run-in period, and repeated doses of antibody 316P on top of continuing ezetimibe or fenofibrate or placebo ezetimibe.

Antibody 316P is a fully human monoclonal antibody that binds proprotein convertase subtilisin kexin type 9 (PCSK9).

Proprotein convertase subtilisin kexin type 9 belongs to the subtilisin family of serine proteases and is highly expressed in the liver. PCSK9 is involved in regulating the levels of the low-density lipoprotein receptor (LDLR) protein. In animals and humans, antibody 316P reduces LDL-C levels in circulation. Antibody 316P may be an effective treatment to lower LDL-C and to reduce the risk for cardiovascular disease.

Seven Phase 1 and 3 Phase 2 studies have been conducted with antibody 316P.

In Phase 1 studies conducted in healthy subjects and in patients with familial and non familial hypercholesterolemia (FH), administration of antibody 316P induced rapid, substantial, and sustained reductions from baseline in LDL-C of up to 60%. The magnitude and duration of these reductions were positively related to the dose administered.

After a single SC administration of 50 to 250 mg, maximum serum concentrations ($C_{max}$) of antibody 316P were observed at Days 5 to 7. Exposure (area under the curve, AUC) increased in a slightly greater than dose-proportional manner with a 6.7-fold increase in AUC for a 5-fold increase in dose. Antibody 316P was eliminated with a terminal half-life ranging between 5.5 and 7.6 days.

In 2 Phase 2 dose/dose regimen-finding studies (exploring different doses and every 2 weeks [Q2W]/every 4 weeks [Q4W] dosing regimens), results showed statistically significant decreases from baseline in LDL-C at 12 weeks in all antibody 316P groups compared to the placebo group.

In the third Phase 2 study, which evaluated the dose of 150 mg Q2W, the same magnitude of effect was shown for this dose with a statistically significant decrease in LDL-C at 8 weeks in the antibody 316P 150 mg + atorvastatin 80 mg group compared with the placebo + atorvastatin 80 mg group.

In completed Phase 1 studies, administration of all doses of antibody 316P to healthy subjects and patients, either by intravenous (IV) or subcutaneous (SC) route, was generally well tolerated with a favourable safety profile regardless of the concomitant administration of atorvastatin (10 mg to 40 mg). No dose relationship for treatment emergent adverse events (TEAEs) was observed. Injection site reactions were the most frequently reported TEAEs regardless of the dose. No clinically significant abnormalities in liver transaminases, troponin I, hematology or chemistry parameters were observed. Occasional non-clinically significant QTc changes were observed; these changes were transient and did not demonstrate a clear dose- or time-dependent pattern.

In the 3 Phase 2 studies, antibody 316P was well tolerated during the whole treatment period in all treatment groups. No particular signal was noted for TEAEs related to musculoskeletal or connective tissue disorders and there were no elevations in liver enzymes. Given the limited published data on the safety of LDL-C level <25 mg/dL, a prespecified statistical analysis was conducted in patients reaching LDL-C value < 25mg/dL in all the Phase 2 studies with no specific safety signal identified during the course of the studies. In the two dose finding studies, the proportion of patients reaching an LDL-C < 25 mg/dL in the 150 mg Q2W group was approximately 30% to 45%. In the study with atorvastatin 80 mg the proportion in the 150 mg Q2W group was approximately 50%.

Ezetimibe is an inhibitor of intestinal cholesterol (and related phytosterol) absorption indicated as an adjunct to diet mainly to reduce LDL-C in various clinical situations. After oral administration, ezetimibe is absorbed and extensively conjugated to a pharmacologically active phenolic glucuronide (ezetimibe-glucuronide). Both ezetimibe and ezetimibe-glucuronide are eliminated from plasma with a half-life of approximately 22 hours.

Frequent adverse events reported with ezetimibe include abdominal pain, diarrhea, flatulence, fatigue, increase ALT/AST, headache and myalgia. Less frequently reported adverse events include increase GGT, increase CPK, cough, dyspepsia, gastro-oesophageal reflux, nausea, arthralgia, muscle cramps, neck pain, loss of appetite, hot flush, hypertension, chest pain, pain, parethesia, dryness of mouth, gastritis, pruritus, rash, urticaria, lumbar pain, muscle weakness, weakness of extremities, asthenia, peripheral edema.

Fenofibrate is a peroxisome proliferator receptor alpha (PPAR$\alpha$) activator indicated as an adjunct to diet mainly to reduce LDL-C and triglycerides in various clinical situations. Fenofibrate is a pro-drug of the active moiety fenofibric acid. Fenofibric acid is eliminated with a half-life of 20 hours.

Frequent adverse events reported with fenofibrate include abdominal pain, nausea, vomiting, diarrhea, flatulence, increase ALT/AST. Less frequently reported adverse events include pancreatitis, thromboembolism (pulmonary embolism, deep venous thrombosis), biliary lithiasis, rash, pruritus, urticaria, photosensitivity reaction, increase creatinine and increase urea.

1.3 RATIONALE

Study rationale

It is known that PCSK9 (target of antibody 316P) expression is upregulated upon administration of statins and ezetimibe, however there is limited or no information on the effect of ezetimibe alone or fenofibrate on the expression of PCSK9 in human. As antibody 316P undergoes target mediated drug disposition (TMDD), the pharmacodynamics and pharmacokinetics of antibody 316P could be altered with concomitant ezetimibe or fenofibrate administration, should any of these drug alter the expression of PCSK9. Such situation would occur in hyperlipidemic patients who cannot be treated by statins and have to receive ezetimibe or fenofibrate.

Design rationale and risk assessment

Given the primary objective and end-point of this study (effect on LDL-C), the design chosen is a partial blind, parallel groups multiple dose study. The run-in period with either ezetimibe or fenofibrate allows to reach steady- state of both compounds and achieve full pharmacodynamic (lipid lowering) effect, and to assess their effect on the expression of PCSK9.

The duration of treatment with antibody 316P is sufficient to allow an accurate assessment of the effect on lipid parameters, and to assess the extent and duration of effect of all 3 treatments on lipid parameters during coadministration.

The duration of observation after the last dose of antibody 316P is sufficient to cover 5 elimination half-lives of antibody 316P, and the duration of observation after the last dose of fenofibrate or ezetimibe covers also 5 elimination half-lives of fenofibric acid, ezetimibe, and ezetimibe-glucuronide.

The selected dose of antibody 316P (150 mg) is one of the doses used in phase 3. The dosing regimen for antibody 316P (E4W) is based on the longer duration of action observed in patients not receiving concomitant administration of a statin. The dosing regimen and doses of ezetimibe and fenofibrate correspond to the recommendation from package insert of both compounds for hypercholesterolemia.

No effect of antibody 316P on the exposure to ezetimibe or fenofibrate (or their metabolites) is expected and no effect of ezetimibe or fenofibrate on risk related to increased exposure to antibody 316P is expected.

Healthy male and female subjects, 18-65 years old, with serum LDL-C levels >130 mg/dL but not treated with statin or other lipid lowering therapy, and with LDL-C levels still $\geq$ 100 mg/dL at Day - 1 assessment (done prior to dosing with antibody 316P) are enrolled in this study to allow robust pharmacodynamic evaluation. A decrease in LDL-C of about 15% with either fenofibrate or ezetimibe is expected in this population.

Specific parameters rationale

Due to the mechanism of action of antibody 316P, LDL-C is expected to be reduced by antibody 316P at the dose chosen for this study. Therefore, LDL-C serves as a PD marker. Other lipid markers (TC, HDL-C, non HDL-C, ApoB, ApoA1, Lp(a) and PCSK9 will be explored.

1.4 DURATION OF STUDY PARTICIPATION

Duration of study participation for each subject

- Screening: 2 to 21 days
- Run-in period (with ezetimibe, ezetimibe placebo, or fenofibrate): 4 weeks
- Treatment period (with reference to antibody 316P) including treatment and post-treatment phase: 17 weeks
- End of study: day 126 $\pm$ 3 (with a minimum of 4 days after the last dosing with ezetimibe, placebo or fenofibrate)
- Total duration from screening per subject: 22 to 25 weeks at maximum

Determination of end of clinical trial (all subjects):

The end of the clinical trial is defined as the day the last subject completed his/her last visit planned in the protocol (EOS visit).

1.5 STUDY POPULATION

Main selection criteria: Healthy male or female subjects, aged 18 to 65 years old, with LDL-C > 130 mg/dL not receiving lipid lowering therapy, and LDL-C $\geq$ 100 mg/dL at Day-1 baseline, before initiating antibody 316P (REGN727)

1.5.1 SELECTION OF SUBJECTS

1.5.1.1 INCLUSION CRITERIA

Demography

I 01. Male or female subjects, not receiving lipid lowering therapy and between 18 and 65 years of age, inclusive
I 02. Body weight between 50.0 and 95.0 kg, inclusive, if male, and between 40.0 and 85.0 kg, inclusive, if female, body mass index between 18.0 and 30.0 kg/m$^2$, inclusive.

Health status

I 03. Certified as healthy by a comprehensive clinical assessment (detailed medical history and complete physical examination).
I 04. Normal vital signs after 10 minutes resting in supine position:

- 95 mmHg < systolic blood pressure (SBP) <140 mmHg
- 45 mmHg < diastolic blood pressure (DBP) <90 mmHg
- 40 bpm < heart rate (HR) <100 bpm

I 05. Normal standard 12-lead electrocardiogram (ECG) after 10 minutes resting in supine position; 120 ms<PR<220 ms, QRS<120 ms, QTc$\leq$450 ms if male, $\leq$470 ms if female.
I 06. Laboratory parameters within the normal range (or defined screening threshold for the Investigator site), unless the Investigator considers an abnormality to be clinically irrelevant for healthy subjects; however serum creatinine, alkaline phosphatase, hepatic enzymes (aspartate aminotransferase, alanine aminotransferase), and total bilirubin (unless the subject has documented Gilbert syndrome) and any parameter for which there is an explicit stopping rule should not exceed the upper laboratory norm.
I 07. If female, subject must use a double contraception method, except if she has undergone sterilization at least 3 months earlier or is postmenopausal. The accepted double contraception methods include use of a highly effective method of birth control (intrauterine device or hormonal contraception) in addition to one of the following contraceptive options: (1) condom; (2) diaphragm or cervical/vault cap; (3) spermicide. Menopause is defined as being over 60 years of age, being amenorrheic for at least 2 years, or between 45 and 60 years of age and being amenorrheic for

at least 2 years with plasma FSH level > 30 IU/L.

Regulatory

- I 08 to I 10: Standard for clinical phase 1 studies

Specific to the study

I 11. Serum LDL-C levels >130 mg/dL (initial or repeat measurement): Subjects whose screening LDL-C level is >120 mg/dL may return for a repeat serum LDL-C draw within6 days after the initial measurement and will be eligible if the repeat measurement of LDL- C is >130 mg/dL.

1.5.1.2 EXCLUSION CRITERIA

Medical history and clinical status

E 01. Any history or presence of clinically relevant cardiovascular, pulmonary, gastrointestinal, hepatic, renal, metabolic, hematological, neurological, osteomuscular, articular, psychiatric, systemic, ocular, gynaecologic (if female), or infectious disease, or signs of acute illness.
E 02. Frequent headaches and/or migraine, recurrent nausea and/or vomiting (more than twice a month).
E 03. Blood donation, any volume, within 1 month before inclusion.
E 04. Symptomatic postural hypotension, irrespective of the decrease in blood pressure, or asymptomatic postural hypotension defined as a decrease in systolic blood pressure≥20 mmHg within 3 minutes when changing from supine to standing position.
E 05. Presence or history of drug hypersensitivity, or allergic disease diagnosed and
E 06. History or presence of drug or alcohol abuse (alcohol consumption more than 40 g per day).
E 07. Smoking more than 10 cigarettes or equivalent per day.
E 08. Excessive consumption of beverages containing xanthine bases (more than 4 cups or glasses per day).
E 09. If female, pregnancy (defined as positive β-HCG blood test), breast-feeding.

Interfering substance

E 10. Any medication (including St John's Wort) within 14 days before inclusion or within 5 times the elimination half-life or pharmacodynamic half-life of the medication, with the exception of hormonal contraception or menopausal hormone replacement therapy; any vaccination within the last 28 days.

General conditions

E 11 to E14 standard for phase I clinical studies

Biological status

E 15. Positive result on any of the following tests: hepatitis B surface (HBs Ag) antigen, anti- hepatitis B core antibodies (anti-HBc Ab), anti-hepatitis C virus (anti-HCV) antibodies, anti-human immunodeficiency virus 1 and 2 antibodies (anti-HIV1 and anti HIV2 Ab).
E 16. Positive result on urine drug screen (amphetamines/methamphetamines, barbiturates, benzodiazepines, cannabinoids, cocaine, opiates).
E 17. Positive alcohol test.

Specific to the study

E 18. Subjects indicated for the use of statins, ezetimibe or fenofibrate according to investigator opinion.
E 19. Initiation of a new diet or major change to a previous diet within 4 weeks prior to Screening (Day -50 to -29). Subjects must be willing to maintain a consistent diet for the duration of the study.
E 20. Use of a medication or nutraceutical in order to alter serum lipids within 4 weeks prior to screening (Day -50 to -29), including but not limited to statins, ezetimibe, fibrates, niacin, bile acid resins, or red yeast rice.
E 21. Fasting serum triglycerides >200 mg/dL measured after at least a 10 hour fast.
E 22. Contraindication to treatment by either ezetimibe of fenofibrate
E 23. Serum LDL-C levels < 100 mg/dL at the baseline control on Day -1 (i.e.: before initiation of antibody 316P)

1.6 STUDY TREATMENTS:

Investigational medicinal products/formulation/route of administration/dose regimen

INVESTIGATIONAL MEDICINAL PRODUCTS

antibody 316P: The liquid formulation for antibody 316P is supplied ready-to-use at concentrations of 150 mg/mL for SC injection, antibody 316P drug product in liquid formulation is supplied as a drug-device combination product consisting of the liquid drug product in a prefilled syringe that will be part of a disposable auto-injector (DAI).

- Dose of drug per administration: 150 mg
- Formulation: Ready-to-use liquid formulation for SC injection at concentrations of 150 mg/mL
- Administration: Subcutaneous injection in the abdomen; starting on Day 1, after the run-in period. Administration can be done without regard to meal time.
- Dose regimen: 150 mg, 1 injection every 4 weeks, for a total of 3 injections

Ezetimibe: 10 mg over-encapsulated tablet for oral administration

- Matching placebo ezetimibe capsule
- Administration: oral, without regard to meal; however subjects are instructed to maintain a consistent dosing time and conditions throughout the study.
- Dose regimen: 10mg (or placebo), once daily administration for a total duration of 21 weeks (4 weeks of run-in followed by 17 weeks after initial administration of antibody 316P.

Fenofibrate: 160 mg coated tablets (Lipanthyl® 160 mg) for oral administration

- Administration: oral during a meal; subjects are instructed to maintain a consistent dosing time and conditions throughout the study.
- Dose regimen: 160 mg, once daily administration for a total duration of 21 weeks (4 weeks of run-in followed by 17 weeks after initial administration of antibody 316P.

1.7 PACKAGING AND LABELING

- antibody 316P 150 mg/mL pre- filled pen areprovided as a single unit prepared into an open label carton.
- Ezetimibe 10 mg over-encapsulated tablets areprovided as 36 count open labelled wallets.
- Placebo capsule is provided as 36 count open labelled wallet.
- Commercial packs of fenofibrate (Lipanthyl® 160 mg) are provided by the investigator sites.

The content of the labelling is in accordance with the local regulatory specifications and requirements.

STORAGE CONDITIONS AND SHELF LIFE

The antibody 316P 150 mg/mL cartons are stored in a refrigerator between +2°C and +8°C.
The ezetimibe 10 mg over-encapsulated tablet and placebo capsule wallets are stored between +2°C and +30°C by the site.
Fenofibrate tablets should be stored according to manufacturer's instructions (i.e. not above 25°C).

1.8 PRIMARY ENDPOINT(S) AND MAIN SECONDARY ENDPOINT(S)

Pharmacodynamics:

- Primary endpoint: serum low density lipoprotein cholesterol (LDL-C) parameter analyzed in percent change from baseline.

- Secondary endpoints: serum LDL-C analyzed in absolute change from baseline, other lipid parameters (HDL-C, non-HDL-C, total cholesterol, triglycerides, apolipoprotein B, apolipoprotein A1, lipoprotein a) analyzed in percent and absolute changes from baseline.

Safety: Clinical, laboratory, vital signs, ECG parameters (automatic on-site reading), adverse events (including local tolerability).

Pharmacokinetics:

Total serum antibody 316P concentrations, as well as total and free PCSK9 concentrations are analyzed. Plasma concentrations of total and unconjugated ezetimibe as well as fenofibric acid concentrations are analyzed.
Serum PK samples are analyzed using a validated enzyme-linked immunosorbent assay (ELISA) for the determination of total concentrations of antibody 316P (i.e., free antibody 316P and antibody 316P present in PCSK9: antibody 316P complexes) in human serum.
Antibody 316P PK samples are analyzed for the determination of the total and free PCSK9 levels using validated enzyme-linked immunosorbent assays (ELISA). The lower limit of quantification is 0.156 μg/mL for the total PCSK9 assay and 0.0312 μg/mL for the free PCSK9 assay.
Plasma PK samples are analyzed using validated high performance liquid chromatographic methods for the determination of concentrations of total and unconjugated ezetimibe and fenofibric acid in human plasma.
Target levels (free and total PCSK9) are measured using PK serum samples.

1.9 ASSESSMENT OF INVESTIGATIONAL MEDICINAL PRODUCT

1.9.1 ACTIVITY OR PHARMACODYNAMICS

Total cholesterol (TC), HDL-C, Triglycerides (TG), apolipoprotein B (ApoB), apolipoprotein A1 (ApoA1) and Lipoprotein a (Lp(a)) are directly measured. LDL-C levels are calculated using the Friedewald formula (LDL-C = TC - HDL-C - TG/5). Non-HDL-C is calculated in subtracting HDL cholesterol from the TC.

Assessment methods

The blood sampling for lipid parameters [i.e., TC, LDL-C, HDL-C, TG, ApoB, ApoA1, Lp(a)] should be performed in the morning, in fasting condition (at least 10-hour fast and refrain from smoking), before any drug intake. Alcohol consumption within 24 hours and intense physical exercise within 48 hours preceding the blood sampling are discouraged. If the subject is not fasting at any visits, he/she is asked to come the day after for the blood sampling.

Pharmacodynamic parameters

*Primary parameter(s)*

Percent change in calculated serum LDL-C from baseline.

*Secondary parameter(s)*

Serum LDL-C determined in absolute change from baseline, TC, HDL-C, TG, non-HDL-C, ApoB, ApoA1, and Lp(a) analyzed in percent and absolute change from baseline.

Pharmacodynamics

The percentage and absolute changes from baseline of LDL-C are summarized by descriptive statistics for each treatment and at each timepoint.
Statistical analyses compare antibody 316P co- administered with ezetimibe or antibody 316P co- administered with fenofibrate versus antibody 316P alone at each timepoint throughout the study.

Pharmacokinetics

PK parameters are summarized by treatment using descriptive statistics.
Statistical analyses will compare antibody 316P co- administered with ezetimibe or antibody 316P co- administered with fenofibrate versus antibody 316P alone.
The targets levels of PCSK9 (total and free) are summarized by treatment group. Concentrations of ezetimibe and fenofibrate in plasma are summarized.
Results of anti-antibody 316P antibodies are summarized by treatment using descriptive statistics.

Primary pharmacodynamic variable(s)

The primary PD variables will be determined as following:

- percent change from baseline in calculated serum LDL-C defined as:

$$\frac{100 \times (\text{calculated LDL - C value at Day X} - \text{calculated LDL - C value at baseline})}{\text{calculated LDL - C value at baseline}},$$

- absolute change from baseline in calculated serum LDL-C defined as:

  calculated LDL-C value at day X - calculated LDL-C value at baseline.

Secondary pharmacodynamic variable(s)

The percent change from baseline and the absolute change from baseline will also be derived for TC, HDL-C, non-HDL-C, TG, apoB, apoA1 and Lp(a) parameters.

Pharmacodynamic analyses

Statistical analyses compare percent changes LDL-C under antibody 316P co-administered with ezetimibe or antibody 316P co-administered with fenofibrate versus antibody 316P alone at each day throughout the study.

1.10 DURATION OF STUDY PERIOD:

Duration of each part of the study for one subject:

- Screening: 3 weeks (D-50 to D-29)
- Treatment period:
  - Run-in period (ezetimibe, ezetimibe placebo or fenofibrate): D-28 to D-1
  - Administration of antibody 316P on D1, D29, D57

● Continuing administration of ezetimibe, ezetimibe placebo or fenofibrate: from D1 to D120 (± 3 days)

● Follow-up visits from D64 up to day D126

- EOS; D126 (± 3 days) : with a minimum of 4 days after the last dosing with ezetimibe, placebo or fenofibrate

Duration of the study for one subject (excluding screening: 22 weeks (D-28 to D126).

1.11 DEFINITIONS OF ADVERSE EVENTS

Adverse event

An adverse event (AE) is any untoward medical occurrence in a subject administered a pharmaceutical product and which does not necessarily have to have a causal relationship with this treatment.

● Mild = no modification of daily activities and does not require mandatory corrective/symptomatic treatment.

● Moderate = hinders normal daily activities and/or requires mandatory corrective/symptomatic treatment.

● Severe = prevents daily activities and requires mandatory corrective/symptomatic treatment.

Serious adverse event

A serious adverse event (SAE) is any untoward medical occurrence that at any dose:

● Results in death, or
● Is life-threatening, or

Note: The term "life-threatening" in the definition of "serious" refers to an event in which the subject was at risk of death at the time of the event; it does not refer to an event which hypothetically might have caused death if it were more severe.

● Requires inpatient hospitalization or prolongation of existing hospitalization, or
● Results in persistent or significant disability/incapacity, or
● Is a congenital anomaly/birth defect, or
● Is a medically important event:

- Medical and scientific judgment should be exercised in deciding whether expedited reporting is appropriate in other situations, such as important medical events that may not be immediately life-threatening or result in death or hospitalization but may jeopardize the subject or may require intervention to prevent one of the other outcomes listed in the definition above.

Note: Examples of such events are intensive treatment in an emergency room or at home for allergic bronchospasm, blood dyscrasias, convulsions, ALT > 3 x ULN + total bilirubin > 2 x ULN or asymptomatic ALT increase > 10 x ULN, or development of drug dependency or drug abuse.

1.12. ACTIVE COMPOUNDS USED IN STUDY 1:

Active compounds:

Antibody 316P

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

Ezetimibe.

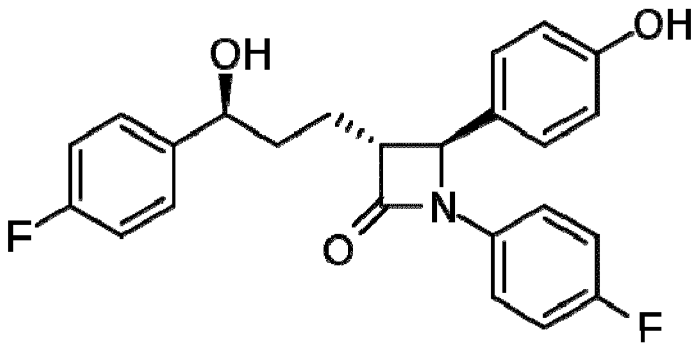

Formula of ezetimibe

Fenofibrate

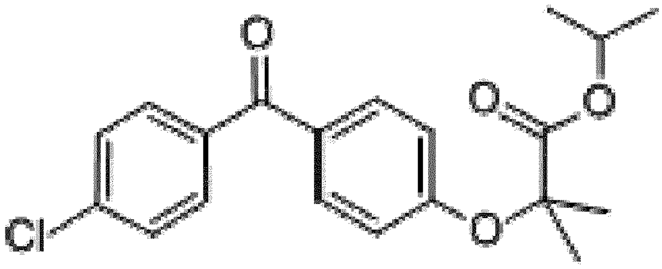

Formula of fenofibrate

Study 2:

Efficacy and Safety of antibody 316P versus placebo on top of lipid-modifying therapy in patients with high cardiovascular risk and hypercholesterolemia

This is a randomized, double-blind, placebo-controlled, parallel group study to evaluate the efficacy and safety of antibody 316P in ihgh cardiovascular risk patients with hypercholesterolemia not adequately controlled with their lipid-modifying therapy.

Therapeutic area: Nutritional and metabolic diseases, particularly hypercholesterolaemia

Primary subject is to demonstrate the reduction of low-density lipoprotein cholesterol (LDL-C) by antibody 316P as add-on therapy to stable maximally tolerated daily statin therapy with or without other lipid-modifying therapy (LMT) in comparison with placebo after 24 weeks of treatment in high cardiovascular (CV) risk patients with hypercholesterolemia.

Secondary subjects are:

To evaluate the effect of antibody 316P in comparison with placebo on LDL-C after 12 weeks of treatment
to evaluate the effect of antibody 316P on other lipid parameter
to evaluate the safety and tolerability of antibody 316P

An optional pharmacogenetic sub-study will be conducted to identify genetic associations with clinical or biomarker response to PCSK9 inhibition, hyperlipidemia, or cardiovascular disease.

Study design is a randomized, parallel, double blind phase 3 study.

Administration is antibody 316P versus placebo via subcutanous injection.

Study population are patients of both gender with minimum age of 18 years and no upper age-limit.

- Inclusion criteria:

  Patients with hypercholesterolemia and established coronary heart disease (CHD) or CHD risk equivalents who are not adequately controlled with a maximally tolerated daily dose of statin with or without other LMT, both at stable dose for at least 4 weeks to 6 weeks prior to screening (Week -2)

- Exclusion criteria:

Age < 18 or legal age of adulthood, whichever is greater

Patients without established coronary heart disease (CHD) or CHD risk equivalent

LDL-C <70 mg/dL (<1.81 mmol/L) at the screening visit and patients with an history of documented cardiovascular disease

LDL-C <100 mg/dL (<2.59 mmol/L) at the screening visit and patients without history of documented cardiovascular disease

Not on a stable dose of LMT (including statin) for at least 4 Weeks and/or fenofibrate for at least 6 weeks, as applicable, prior to the screening visit (Week -2) and from screening to randomization.

Fasting serum triglycerides >400 mg/dL (>4.52 mmol/L) during the screening period.

Specific vulnerable population: Women of child-bearing potential using contraception

Investigational medicinal product is antibody 316P formulated as solution for injection. Injection is subcutaneous and control is a matching placebo.

Active compounds: Antibody 316P

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

Primary endpoint is percent change of LDL-C from baseline to week 24. Secondary endpoint is the percent change in LDL-C from baseline to weeks 12 and 52. Further secondary endpoint is the percent change in other lipid parameters from baseline to weeks 12, 24 and 52.
Duration of the study per subject/patient is 2 weeks, screening period: 2 weeks, double-blind treatment period: 52 weeks, follow-up period: 8 weeks
Study 3 - Efficacy and Safety of antibody 316P versus Ezetimibe on top of statin in high cardiovascular risk patients with hypercholesterolemia.
This is a randomized, double-blind, parallel group study to evaluate the efficacy and safety of antibody 316P versus ezetimibe in high cardiovascular risk patients with hypercholesterolemia not adequately controlled with their statin therapy

Therapeutic area: Nutritional and metabolic diseases, preferably: Hypercholesterolemia

Primary Study objective is to demonstrate the reduction of low-density lipoprotein cholesterol (LDL-C) by antibody 316P as add-on therapy to stable maximally tolerated daily statin therapy in comparison with ezetimibe 10 mg daily after 24 weeks of treatment in patients with hypercholesterolemia at high cardiovascular (CV) risk.
Secondary objectives are:

To evaluate the effect of antibody 316P 75 mg in comparison with ezetimibe on LDL-C after 12 weeks of treatment

To evaluate the effect of antibody 316P on other lipid parameters.

To evaluate the safety and tolerability of antibody 316P

An optional pharmacogenetic sub-study is conducted to identify genetic associations with clinical or biomarker response to PCSK9 inhibition, hyperlipidemia, or cardiovascular disease.
Study design: This is a randomized, parallel, double blind phase 3 study. The study has two arms with one arm receiving antibody 316P as injection through subcutaneous administration and receiving an ezetimibe placebo administered orally (316P plus ezetimibe-placebo). The second arm receives a 316P placebo as injection through subcutaneous administration and receives ezetimibe administered orally (316P-placebo plus ezetimibe).
Study population are patients of both gender with a minimum age of 18 years and no upper age limit (~330

adults of 18-64 years of age, ~330 elderly with 65 years of age or older).

Inclusion criteria: Patients with hypercholesterolemia and established coronary heart disease (CHD) or CHD risk equivalents who are not adequately controlled with a maximally tolerated daily dose of statin at stable dose for at least 4 weeks prior to the screening visit (Week -2).

Exclusion criteria:

Age < 18 years or under legal age of adulthood whatever is higher

Patients without established CHD or CHD risk equivalents

LDL-C <70 mg/dL (<1.81 mmol/L) at the screening visit (Week-2) and patients with an history of documented CV disease as described in section 7.1)

LDL-C <100 mg/dL (<2.59 mmol/L) at the screening visit (Week-2) and patients without history of documented CV disease as described in section 7.1)

Fasting serum triglycerides >400 mg/dL (>4.52 mmol/L) during the screening period.

Specific vulnerable population: Women of child-bearing potential using contraception.

Investigational medicinal products are antibody 316P as solution for injection with subcutaneous route of administration and ezetimibe as encapsulated tablets for oral administration. The placebo for 316P is in a matching formulation as injection solution for subcutaneous administration, the placebo for ezetimibe is in a matching formulation as capsules for oral administration.

Active compounds:

Antibody 316P:

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

Ezetimibe:

Formula of ezetimibe:

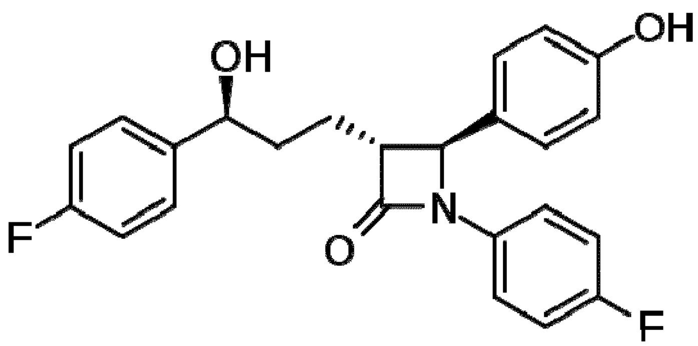

Primary endpoint is the percent change in LDL-C from baseline to week 24.

Secondary endpoints are the percent change in LDL-C from baseline to week 12 and the percent change in other lipid parameters at weeks 24 and 12.

Study duration per subject/patient is 114 weeks with a screening period of 2 weeks, a double-blind treatement period of 104 weeks and a follow-up period: 8 weeks.

Study 4 - Efficacy and Safety of antibody 316P versus placebo on top of lipid-modifying therapy in patients with heterozygous familial hypercholesterolemia

This is a randomized, double-blind, placebo-controlled, parallel group study to evaluate the efficacy and safety of antibody 316P in patients with heterozygous familial hypercholesterolemia and LDL-C higher or equal to 160mg/dL with their lipid-modifying therapy

Therapeutic area: Nutritional and metabolic diseases, preferably: Hypercholesterolemia

Primary objective: To evaluate the effect of antibody 316P on low-density lipoprotein cholesterol (LDL-C) levels after 24 weeks of treatment in comparison with placebo.
Secondary objectives:

To evaluate the effect of antibody 316P in comparison with placebo on LDL-C at other time points

To evaluate the effects of antibody 316P on other lipid parameters

To evaluate the safety and tolerability of antibody 316P

Study design: This is a randomized, parallel, double blind phase 3 study.
Study arms:

First arm (experimental): Injection of antibody 316P through subcutaneous (SC) administration. Background statin therapy or other lipid lowering therapy is administered according to site investigator discretion as background therapy.

Second arm (Placebo Comparator): injection of placebo through subcutaneous (SC) administration. Background statin therapy or other lipid lowering therapy will be administered according to site investigator discretion as background therapy.

Study population: Minimum age of 18 years without upper age limit.

Inclusion criteria: Patients with heterozygous familial hypercholesterolemia who are not adequately controlled with their lipid-modifying therapy.
Exclusion criteria:

Age < 18 years

LDL-C < 160 mg/dL (< 4.14 mmol/L) at the screening visit (Week-2).

Fasting serum triglycerides > 400 mg/dL (> 4.52 mmol/L) during the screening period.

Known history of homozygous familial hypercholesterolemia.

Specific vulnerable population: Women of child-bearing potential using contraception.
Number of subjects/patients: ~200 (~ 180 adults with age of 18-64 years and ~ 20 elderly with age of 65 years or more).
Investigational medicinal product is antibody 316P as solution for injection with subcutaneous administration, the placebo is equally formulated as for injection with subcutaneous administration.

Active compounds: Antibody 316P

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).
Primary endpoint: percent change in LDL-C from baseline to week 24.
Secondary endpoints: Percent change in LDL-C from baseline up to week 78 and percent change in other lipid parameters from baseline up to week 78.
Duration of study per subject/patient: The maximum study duration will be 88 weeks per patient, including a

78-week randomized treatment period.

## Claims

1. A method for treating a disease or condition in which PCSK9 expression or activity causes an impact comprising administering a therapeutic amount of an antibody or antigen-binding fragment thereof which specifically binds hPCSK9 (human proprotein convertase subtilisin/kexin type 9) to a subject in need thereof, wherein the method comprises administering of the antibody or antigen-binding fragment thereof together with

   (a) a therapeutic amount of a lipid lowering agent acting by decreasing cholesterol adsorption in the intestine, or
   (b) a therapeutic amount of a fibrate, or
   (c) a therapeutic amount of the lipid lowering agent of a) and a therapeutic amount of a fibrate.

2. A method of lowering cholesterol levels in a subject in need thereof, comprising:

   (a) selecting a subject with a blood low density lipoprotein cholesterol (LDL-C) level > 100 mg/dL; and
   (b) administering to said subject a composition comprising an antibody or antigen-binding fragment thereof or antigen binding fragment thereof that specifically binds to human proprotein convertase subtilisin/kexin type 9 (hPCSK9); thereby lowering cholesterol levels in the subject in need thereof, wherein the method comprises administering of the antibody or antigen-binding fragment thereof or fragment thereof together with

      (i) a therapeutic amount of a lipid lowering agent acting by decreasing cholesterol adsorption in the intestine, or
      (ii) a therapeutic amount of a fibrate, or
      (iii) a therapeutic amount of the lipid lowering agent of a) and a therapeutic amount of a fibrate.

3. A method of preventing effects of a (persistently) increased LDL-C level in the blood comprising administering a therapeutic amount of an antibody or antigen-binding fragment thereof which specifically binds hPCSK9 (human proprotein convertase subtilisin/kexin type 9) to a subject having an increased LDL-C level in the blood, and

   (a) administering a therapeutic amount of a lipid lowering agent acting by decreasing cholesterol adsorption in the intestine, or
   (b) administering a therapeutic amount of a fibrate, or
   (c) administering a therapeutic amount of the lipid lowering agent of a) and a therapeutic amount of a fibrate.

4. The method according to any one of the claims 1 to 3, wherein the lipid lowering agent is ezetimibe.

5. The method according to any one of the claims 1 to 4, wherein the fibrate is fenofibrate.

6. The method of any one of claims 1 to 5, wherein the antibody or antigen-binding fragment thereof is a recombinant human antibody.

7. The method of any one of claims 1 to 6, wherein the antibody or antigen-binding fragment thereof or the antigen-binding fragment thereof comprises

   (a) a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; or
   (b) a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736, or
   (c) the heavy chain of (a) and the light chain of (b).

8. The method of any one of claims 1 to 7, wherein the antibody or antigen-binding fragment thereof or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

9. The method of any one of claims 1 to 8, wherein the antibody or antigen-binding fragment thereof binds to the same epitope on hPCSK9 as an antibody or antigen-binding fragment thereof comprising heavy and light chain CDR amino acid sequences as shown in SEQ ID NOs: 76, 78, 80, 84, 86, and 88.

10. The method of any one of claims 1 to 9, wherein the antibody or antigen-binding fragment thereof competes for binding to hPCSK9 with an antibody or antigen-binding fragment thereof comprising heavy and light chain CDR amino acid sequences as shown in SEQ ID NOs: 76, 78, 80, 84, 86, and 88.

11. The method according to one of the claims 1 to 10 comprising administration of the antibody or antigen-binding fragment thereof in a dosage amount ranging from 5 mg to 500 mg and preferably ranging from 50 to 300 mg and more preferably of about 150 mg.

12. The method according to any one of the claims 1 to 11, comprising administering a daily dose of 1, 3, 5, 7, 10, 15, 20, 25, 30, 40 or 50 mg and preferably of 5 to 20 mg and more preferably of about 10 mg of the lipid lowering agent and preferably of an azetidone and more preferably of ezetimibe.

13. The method according to any one of the claims 1 to 12, comprising administering a daily dose of 50, 75, 100, 150, 160, 170, 180, 190 or 200 mg and preferably of 75 to 180 mg and more preferably of about 160 mg of the fibrate and preferably of fenofibrate.

14. The method according to any one of the claims 1 to 13, wherein the administration of the antibody or antigen-binding fragment thereof together with the lipid lowering agent and/or the fibrate is an adjunctive therapy to diet and preferably to lipid-lowering or cholesterol-lowering diet.

**15.** The method of claims 1 to 14, wherein the antibody or antigen-binding fragment thereof is administered every week, every other week, every third week, every month or every fourth week.

**16.** The method according to any one of the claims 1 to 15, wherein the disease or condition in which PCSK9 expression or activity causes an impact is selected from the group consisting of: hypercholesterolemia, statin-uncontrolled hypercholesterolemia, non-familial hypercholesterolemia, heterozygous familiar hypercholesterolemia, hyperlipidemia, dyslipidemia such as mixed dyslipidemia, mixed dyslipidemia, atherosclerosis, cardiovascular disease, coronary heart disease and a coronary heart disease-risk equivalent.

**17.** The method of any one of claims 1 to 16, wherein the treatment with the antibody or antigen-binding fragment thereof together with the lipid lowering agent or the fibrate or together with the lipid lowering agent and the fibrate improves lipid parameters critical for the development, persistence or worsening of atherosclerosis and/or cardiovascular disease such as coronary heart disease or coronary-heart disease risk equivalents.

**18.** A pharmaceutical composition comprising a therapeutic amount of an antibody or antigen-binding fragment thereof which specifically binds hPCSK9 (human proprotein convertase subtilisin/kexin type 9) together with a therapeutic amount of a fibrate and a pharmaceutically acceptable excipient.

**19.** Pharmaceutical composition comprising a therapeutic amount of an antibody or antigen-binding fragment thereof which specifically binds hPCSK9 (human proprotein convertase subtilisin/kexin type 9) and a pharmaceutically acceptable excipient for use in a method according to one of the claims 1 to 17.

**20.** Article of manufacture comprising

(a) a packaging material or container;
(b) an antibody or antigen-binding fragment thereof which specifically binds hPCSK9; and
(c) a fibrate and/or a lipid lowering drug acting by decreasing cholesterol adsorption in the intestine; and
(d) a label or packaging insert.

**21.** Article of manufacture according to claim 20, wherein the lipid lowering drug is ezetimibe.

**22.** Article of manufacture according to one of the claims 20 or 21, wherein the fibrate is fenofibrate.

**23.** A method for preparing an article of manufacture according to one of the claims 20 to 22 comprising packaging an antibody or antigen-binding fragment thereof which specifically binds hPCSK9 and packaging the lipid lowering agent and/or the fibrate in a container and assembling them with a label or packaging insert.

**24.** Unit dosage form comprising an antibody or antigen-binding fragment thereof which specifically binds hPCSK9 (human proprotein convertase subtilisin/kexin type 9) together with a fibrate.

**25.** Unit dosage form comprising an antibody or antigen-binding fragment thereof which specifically binds hPCSK9 (human proprotein convertase subtilisin/kexin type 9) for use in a method according to one of the claims 1 to 17, wherein the antibody or antigen-binding fragment thereof preferably is an antibody or antigen-binding fragment thereof according to one of the claims 6 to 10.

Figure 1

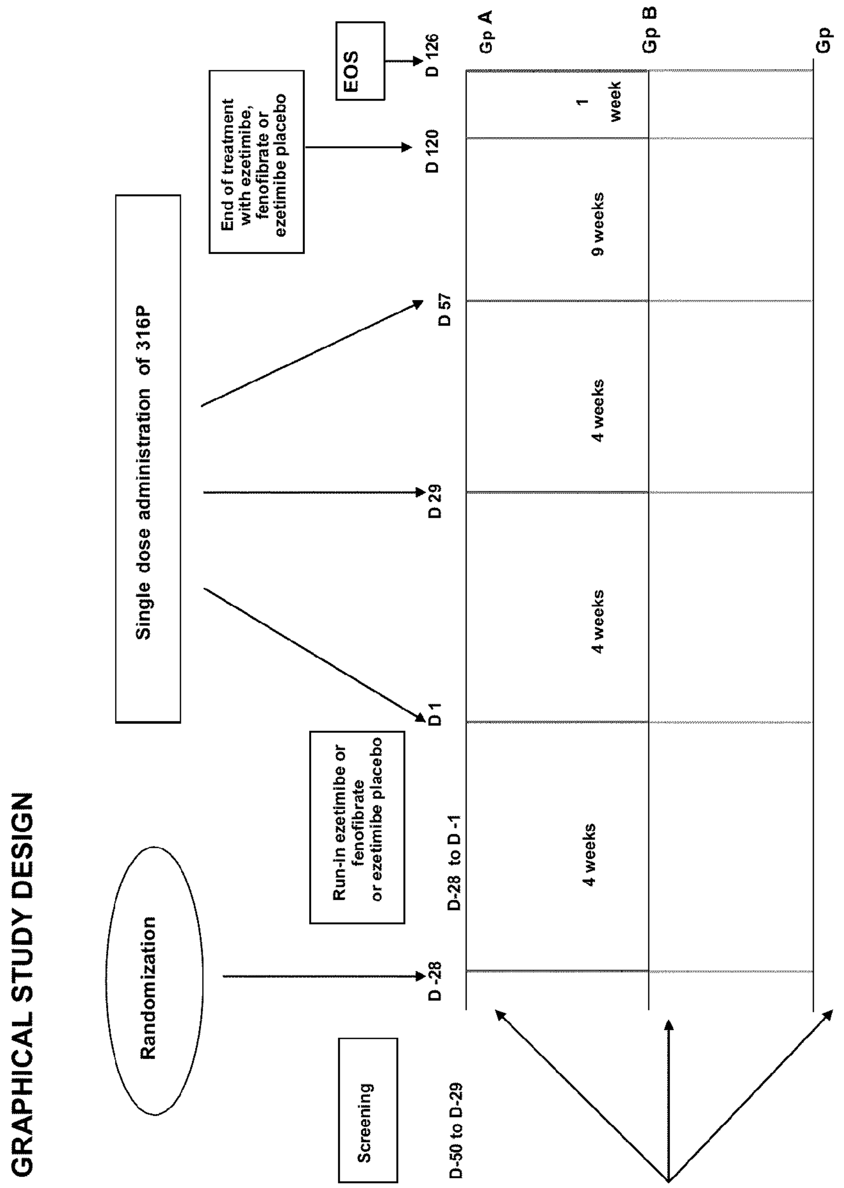
GRAPHICAL STUDY DESIGN
Screening
D-50 to D-29
Randomization
D -28
Run-in ezetimibe or fenofibrate or ezetimibe placebo
D-28 to D -1
4 weeks
D 1
Single dose administration of 316P
4 weeks
D 29
4 weeks
D 57
9 weeks
End of treatment with ezetimibe, fenofibrate or ezetimibe placebo
D 120
1 week
EOS
D 126
Gp A
Gp B
Gp

Europäisches Patentamt
European Patent Office
Office européen des brevets

# EUROPEAN SEARCH REPORT

Application Number
EP 12 30 6068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EVAN A STEIN ET AL: "Effect of a monoclonal antibody to PCSK9, REGN727/SAR236553, to reduce low-density lipoprotein cholesterol in patients with heterozygous familial hypercholesterolaemia on stable statin dose with or without ezetimibe therapy: a phase 2 randomised controlled trial", THE LANCET, vol. 380, no. 9836, 1 July 2012 (2012-07-01), pages 29-36, XP055041201, ISSN: 0140-6736, DOI: 10.1016/S0140-6736(12)60771-5 | 1-4,6, 11, 14-17, 19-21, 23,25 | INV. C07K16/40 A61K39/395 |
| Y | * abstract * * page 36, left-hand column, last paragraph * ----- | 5,13,18, 22,24 | |
| X | EVAN A. STEIN ET AL: "Effect of a Monoclonal Antibody to PCSK9 on LDL Cholesterol", NEW ENGLAND JOURNAL OF MEDICINE, vol. 366, no. 12, 22 March 2012 (2012-03-22), pages 1108-1118, XP055049842, ISSN: 0028-4793, DOI: 10.1056/NEJMoa1105803 * abstract * * page 1113, left-hand column, paragraph 2 * ----- | 1-3,6, 11,15-17 | TECHNICAL FIELDS SEARCHED (IPC): C07K A61K |

-/--

The present search report has been drawn up for all claims

2

| Place of search | Date of completion of the search | Examiner |
|---|---|---|
| Munich | 16 January 2013 | Malamoussi, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 6068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/101251 A1 (SANOFI SA [FR]; HANOTIN CORINNE [FR]; BESSAC LAURENCE [FR]; CHAUDHARI) 2 August 2012 (2012-08-02)<br>* sequences 90,92 *<br>* page 178, lines 12-22 *<br>* page 182, line 22 - page 184, line 15 *<br>* page 187, line 2 - line 12 *<br>* page 202, line 1 - line 8 *<br>* page 202, line 16 - line 18 *<br>* page 213, line 4 - line 11 *<br>----- | 1-4, 6-12, 14-17, 19-21, 23,25 | |
| X | JAMES M. MCKENNEY ET AL: "Safety and Efficacy of a Monoclonal Antibody to Proprotein Convertase Subtilisin/Kexin Type 9 Serine Protease, SAR236553/REGN727, in Patients With Primary Hypercholesterolemia Receiving Ongoing Stable Atorvastatin Therapy",<br>JOURNAL OF THE AMERICAN COLLEGE OF CARDIOLOGY,<br>vol. 59, no. 25, 1 June 2012 (2012-06-01), pages 2344-2353, XP055049859,<br>ISSN: 0735-1097, DOI: 10.1016/j.jacc.2012.03.007<br>* abstract *<br>----- | 1-3,6, 11,16, 17,19, 20,23,25 | |
| | -/-- | | TECHNICAL FIELDS SEARCHED (IPC) |

The present search report has been drawn up for all claims

| Place of search | Date of completion of the search | Examiner |
|---|---|---|
| Munich | 16 January 2013 | Malamoussi, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 30 6068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Sanofi and Regeneron Announce Publication of Positive Phase 2 Results for Lipid-Lowering PCSK9 Antibody in The Lancet", , 26 May 2012 (2012-05-26), pages 1-4, XP002690376, Retrieved from the Internet: URL:http://files.shareholder.com/downloads/REGN/2265016371x0x572527/35552a52-cb45-4a7a-85c2-2cd8ce685038/REGN_News_2012_5_26_General_Releases.pdf [retrieved on 2013-01-11] * the whole document * ----- | 1-4,6, 11,12, 14-17, 20,21, 23,25 | |
| X | PAYAL KOHLI ET AL: "Design and Rationale of the LAPLACE-TIMI 57 Trial: A Phase II, Double-Blind, Placebo-Controlled Study of the Efficacy and Tolerability of a Monoclonal Antibody Inhibitor of PCSK9 in Subjects With Hypercholesterolemia on Background Statin Therapy", CLINICAL CARDIOLOGY, vol. 35, no. 7, 1 July 2012 (2012-07-01), pages 385-391, XP055041199, ISSN: 0160-9289, DOI: 10.1002/clc.22014 * abstract * ----- -/-- | 1-4,6, 11,12, 14-17, 20,21, 23,25 | TECHNICAL FIELDS SEARCHED (IPC) |

The present search report has been drawn up for all claims

2

| Place of search | Date of completion of the search | Examiner |
|---|---|---|
| Munich | 16 January 2013 | Malamoussi, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 6068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2011/142849 A1 (RUE SARAH [US] ET AL) 16 June 2011 (2011-06-16)<br>* page 1, paragraph 0008 - paragraph 0009 *<br>* page 20, paragraph 261 *<br>* page 16, paragraph 224 *<br>* page 3, paragraph 0093 - paragraph 0100 *<br>* page 17, paragraph 0236 - paragraph 0238 *<br>* claims 33, 46 *<br>----- | 5,13,18, 22,24 | |
| Y | KONRAD ROBERT J ET AL: "Effects of currently prescribed LDL-C-lowering drugs on PCSK9 and implications for the next generation of LDL-C-lowering agents", LIPIDS IN HEALTH AND DISEASE, BIOMED CENTRAL, LONDON, GB, vol. 10, no. 1, 28 February 2011 (2011-02-28), page 38, XP021088517, ISSN: 1476-511X, DOI: 10.1186/1476-511X-10-38<br>* abstract *<br>* page 43, right-hand column, paragraph 1 *<br>* page 44, right-hand column, paragraph 4 *<br>* page 45, left-hand column, paragraph 1 *<br>-----<br>-/-- | 5,13,18, 22,24 | TECHNICAL FIELDS SEARCHED (IPC) |

The present search report has been drawn up for all claims

| Place of search | Date of completion of the search | Examiner |
|---|---|---|
| Munich | 16 January 2013 | Malamoussi, A |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 6068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LI HAI ET AL: "Recent patents on PCSK9: a new target for treating hypercholesterolemia", RECENT PATENTS ON DNA & GENE SEQUENCES,, vol. 3, no. 3, 1 November 2009 (2009-11-01), pages 201-212, XP002619049, ISSN: 1872-2156, DOI: 10.2174/187221509789318388 * the whole document * ----- | 1-25 | |
| A | COSTET P: "PCSK9 inhibitors as LDL cholesterol-lowering agents: Rationale, concerns and preliminary outcomes", DRUGS OF THE FUTURE, PROUS SCIENCE, ES, vol. 37, no. 5, 1 May 2012 (2012-05-01), pages 331-341, XP009163740, ISSN: 0377-8282, DOI: 10.1358/DOF.2012.37.5.1790302 * the whole document * ----- | 1-25 | |
| | | | TECHNICAL FIELDS SEARCHED (IPC) |
| A | BERTRAND CARIOU ET AL: "Clinical aspects of PCSK9", ATHEROSCLEROSIS, ELSEVIER IRELAND LTD, IE, vol. 216, no. 2, 12 April 2011 (2011-04-12), pages 258-265, XP028226555, ISSN: 0021-9150, DOI: 10.1016/J.ATHEROSCLEROSIS.2011.04.018 [retrieved on 2011-04-22] * the whole document * ----- -/-- | 1-25 | |

The present search report has been drawn up for all claims

| Place of search | Date of completion of the search | Examiner |
|---|---|---|
| Munich | 16 January 2013 | Malamoussi, A |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 6068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHAN D C ET AL: "Fenofibrate concomitantly decreases serum proprotein convertase subtilisin/kexin type 9 and very-low-density lipoprotein particle concentrations in statin-treated type 2 diabetic patients", DIABETES, OBESITY AND METABOLISM, BLACKWELL SCIENCE, vol. 12, no. 9, 1 September 2010 (2010-09-01), pages 752-756, XP002685484, ISSN: 1462-8902 [retrieved on 2010-03-22] * abstract * ----- | 1-25 | |
| A | LAMBERT GILLES ET AL: "Plasma PCSK9 concentrations correlate with LDL and total cholesterol in diabetic patients and are decreased by fenofibrate treatment", CLINICAL CHEMISTRY, AMERICAN ASSOCIATION FOR CLINICAL CHEMISTRY, WASHINGTON, DC, vol. 54, no. 6, 1 June 2008 (2008-06-01), pages 1038-1045, XP009104835, ISSN: 0009-9147, DOI: 10.1373/CLINCHEM.2007.099747 * abstract * ----- | 1-25 | TECHNICAL FIELDS SEARCHED (IPC) |
| A | MAYNE JANICE ET AL: "Plasma PCSK9 levels are significantly modified by statins and fibrates in humans", LIPIDS IN HEALTH AND DISEASE, BIOMED CENTRAL, LONDON, GB, vol. 7, no. 1, 11 June 2008 (2008-06-11), page 22, XP021037016, ISSN: 1476-511X * abstract * * page 24, right-hand column, paragraph 1 * ----- | 1-25 | |

The present search report has been drawn up for all claims

| Place of search | Date of completion of the search | Examiner |
|---|---|---|
| Munich | 16 January 2013 | Malamoussi, A |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 30 6068

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2013

| Patent document cited in search report | Publication date | Patent family member(s) | Publication date |
|---|---|---|---|
| WO 2012101251 A1 | 02-08-2012 | WO 2012101251 A1 | 02-08-2012 |
| | | WO 2012101252 A2 | 02-08-2012 |
| | | WO 2012101253 A1 | 02-08-2012 |
| US 2011142849 A1 | 16-06-2011 | AR 079336 A1 | 18-01-2012 |
| | | AU 2010327924 A1 | 05-07-2012 |
| | | CA 2781050 A1 | 16-06-2011 |
| | | CN 102652139 A | 29-08-2012 |
| | | CR 20120371 A | 27-08-2012 |
| | | EP 2510013 A1 | 17-10-2012 |
| | | KR 20120098873 A | 05-09-2012 |
| | | TW 201136605 A | 01-11-2011 |
| | | US 2011142849 A1 | 16-06-2011 |
| | | WO 2011072263 A1 | 16-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008057457 A
- WO 2008057458 A
- WO 2008057459 A
- WO 2008063382 A
- WO 2008125623 A
- US 20080008697 A
- US 20100166768 A1
- US 20030118592 A
- US 20030133939 A
- US 5939598 A, Kucherlapati & Jakobovits
- US 6596541 B
- US 20040101920 A
- US 20100166768 A
- WO 05103081 A


### Non-patent literature cited in the description


- A multilingual glossary of biotechnological terms: (IUPAC Recommendations. Helvetica Chimica Acta, 1995
- **PADLAN et al.** *FASEB J.,* 1995, vol. 9, 133-139
- **VAJDOS et al.** *J Mol Biol,* 2002, vol. 320, 415-428
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HEAP CJ et al.** *J. Gen. Virol.,* 2005, vol. 86, 1791-1800
- **QIU X-Q et al.** *Nature biotechnology,* 2007, vol. 25 (8), 921-929
- **ALMAGRO JC ; FRANSSON J.** *Frontiers in Bioscience,* 2008, vol. 13, 1619-1633
- **HOLLIGER P. et al.** *Proc. Natl. Acad. Sci. U.S.A.,* 1993, vol. 90 (14), 6444-6448
- **PEARSON.** *Methods Mol. Biol.,* 1994, vol. 24, 307-331
- **GONNET et al.** *Science,* 1992, vol. 256, 1443-45
- **ALTSCHUL et al.** *J. Mol. Biol.,* 1990, vol. 215, 403-410
- *ucleic Acids Res.,* 1997, vol. 25, 3389-402
- United States Pharmacopeia-33/National Formulary-28 Reissue. United States Pharmacopeia Convention, Inc, April 2010
- **C. ROWE ; P.J. SESKEY ; S.C. OWEN.** Handbook of Pharmaceutical Excipients. Pharmaceutical Press
- **R. TIWARI ; K. PATHAK.** Statins therapy: a review on conventional and novel formulation approaches. *Journal of Pharmacy and Pharmacology,* 2011
- **REDDY et al.** *J. Immunol.,* 2000, vol. 164, 1925-1933
- **ANGAL et al.** *Molecular Immunology,* 1993, vol. 30, 105
- **HARLOW ; LANE.** Antibodies. Cold Spring Harbor Press
- **REINEKE.** *Methods Mol Biol,* 2004, vol. 248, 443-63
- **TOMER.** *Protein Science,* 2000, vol. 9, 487-496
- **JUNGHANS et al.** *Cancer Res.,* 1990, vol. 50, 1495-1502
- **TUTT et al.** *J. Immunol.,* 1991, vol. 147, 60-69
- Remington's Pharmaceutical Sciences. Mack Publishing Company
- **POWELL et al.** Compendium of excipients for parenteral formulations. *J Pharm Sci Technol,* 1998, vol. 52, 238-311
- **WU et al.** *J. Biol. Chem.,* 1987, vol. 262, 4429-4432
- **LANGER.** *Science,* 1990, vol. 249, 1527-1533
- **TREAT et al.** Liposomes in the Therapy of Infectious Disease and Cancer. Liss, 1989, 353-365
- **LOPEZ-BERESTEIN.** LIPOSOMES IN THE THERAPY OF INFECTIOUS DISEASE AND CANCER. 317-327
- **SEFTON.** *CRC Crit. Ref. Biomed. Eng.,* 1987, vol. 14, 201
- **GOODSON.** *Medical Applications of Controlled Release,* 1984, vol. 2, 115-138